# EUROPEAN PATENT SPECIFICATION

(11) **EP 3 016 684 B1**
(45) Date of publication and mention of the grant of the patent: **23.02.2022**
(21) Application number: 14820173.4
(22) Date of filing: 03.07.2014
(51) Int. Cl.: A61K 39/395, A61P 3/08, C07K 16/26

(54) **REGULATION OF GLUCOSE METABOLISM USING ANTI-CGRP ANTIBODIES**
REGULIERUNG DES GLUCOSESTOFFWECHSELS MIT ANTI-CGRP-ANTIKÖRPERN
RÉGULATION DE MÉTABOLISME DE GLUCOSE À L'AIDE D'ANTICORPS ANTI-CGRP

(30) Priority: 03.07.2013 US 201361842745 P; 22.04.2014 US 201461982611 P
(43) Date of publication of application: 11.05.2016
(73) Proprietor: H. Lundbeck A/S, 2500 Valby (DK)
(72) Inventor: LATHAM, John A., Seattle Washington 98119 (US); SMITH, Jeffrey T.L., Bellevue Washington 98004 (US); BAKER, Brian, Kirkland Washington 98034 (US)
(74) Representative: H. Lundbeck A/S
(86) International application number: PCT/US2014/045389
(87) International publication number: WO 2015/003122

(56) References cited:
- US-A- 5 266 561
- US-A- 5 364 841
- US-A- 5 364 841
- US-A- 5 942 227
- US-A1- 2012 294 797
- US-A1- 2012 294 797
- Giuseppe Derosa ET AL: "Optimizing combination treatment in the management of type 2 diabetes Introduction Type 2 diabetes: from lifestyle and diet to pharmacological treatment", Vascular Health and Risk Management, 1 January 2007 (2007-01-01), pages 665-671, XP055337147, Retrieved from the Internet: URL:https://www.ncbi.nlm.nih.gov/pmc/artic les/PMC2291311/pdf/VHRM_0305-665.pdf
- HALIMI S ET AL: "Combination treatment in the management of type 2 diabetes: Focus on vildagliptin and metformin as a single tablet", VASCULAR HEALTH AND RISK MANAGEMENT, DOVE MEDICAL PRESS LTD, NZ, vol. 4, no. 3, 1 January 2008 (2008-01-01) , pages 481-492, XP009105436, ISSN: 1176-6344
- ALISON E. TEDSTONE ET AL: "The effect of islet amyloid polypeptide (amylin) and calcitonin gene-related peptide on glucose removal in the anaesthetized rat and on insulin secretion from rat pancreatic isletsin vitro", BIOSCIENCE REPORTS, vol. 162, no. 4, 1 August 1990 (1990-08-01), pages 876-345, XP055337076, US ISSN: 0144-8463, DOI: 10.1007/BF01117233
- A M GÓMEZ-FOIX ET AL: "Anti-insulin effects of amylin and calcitonin-gene-related peptide on hepatic glycogen metabolism", BIOCHEMICAL JOURNAL, vol. 276, no. 3, 15 June 1991 (1991-06-15) , pages 607-610, XP055336562, GB ISSN: 0264-6021, DOI: 10.1042/bj2760607
- CHRISTOPHER S. WALKER ET AL: "Mice Lacking the Neuropeptide [alpha]-Calcitonin Gene-Related Peptide Are Protected Against Diet-Induced Obesity", ENDOCRINOLOGY, vol. 151, no. 9, 1 September 2010 (2010-09-01), pages 4257-4269, XP055336922, US ISSN: 0013-7227, DOI: 10.1210/en.2010-0284
- JOSEPH M MOLINA ET AL: "Rapid Publications Induction of Insulin Resistance In Vivo by Amylin and Calcitonin Gene-Related Peptide", DIABETES, vol. 39, 1 February 1990 (1990-02-01), pages 260-265, XP055336927,
- BRENDAN LEIGHTON ET AL: "Pancreatic amylin and calcitonin gene-related peptide cause resistance to insulin in skeletal muscle in vitro", NATURE, vol. 335, no. 6191, 13 October 1988 (1988-10-13), pages 632-635, XP055336929, United Kingdom ISSN: 0028-0836, DOI: 10.1038/335632a0

### SEQUENCE LISTING

<110> ALDER BIOPHARMACEUTICALS, INC.
<120> REGULATION OF GLUCOSE METABOLISM USING ANTI-CGRP ANTIBODIES
<130> 43257.3013
<141> 2014-07-03
<150> 61/982,611 <151> 2014-04-22
<150> 61/842,745 <151> 2013-07-03
<160> 284
<170> PatentIn version 3.5
<210> 1
   <211> 113
   <212> PRT
   <213> Artificial Sequence
<220>
   <223> Description of Artificial Sequence: Synthetic polypeptide
<400> 1
   Gln Val Leu Thr Gln Thr Ala Ser Pro Val Ser Ala Ala Val Gly Ser
   1 5 10 15
   Thr Val Thr Ile Asn Cys Gln Ala Ser Gln Ser Val Tyr Asp Asn Asn
   20 25 30
   Tyr Leu Ala Trp Tyr Gln Gln Lys Pro Gly Gln Pro Pro Lys Gln Leu
   35 40 45
   Ile Tyr Ser Thr Ser Thr Leu Ala Ser Gly Val Ser Ser Arg Phe Lys
   50 55 60
   Gly Ser Gly Ser Gly Thr Gln Phe Thr Leu Thr Ile Ser Asp Leu Glu
   65 70 75 80
   Cys Ala Asp Ala Ala Thr Tyr Tyr Cys Leu Gly Ser Tyr Asp Cys Ser
   85 90 95
   Ser Gly Asp Cys Phe Val Phe Gly Gly Gly Thr Glu Val Val Val Lys
   100 105 110
   Arg
<210> 2
   <211> 219
   <212> PRT
   <213> Artificial Sequence
<220>
   <223> Description of Artificial Sequence: Synthetic polypeptide
<400> 2
   Gln Val Leu Thr Gln Thr Ala Ser Pro Val Ser Ala Ala Val Gly Ser
   1               5                   10                  15
   Thr Val Thr Ile Asn Cys Gln Ala Ser Gln Ser Val Tyr Asp Asn Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Lys
       50                  55                  60
   Gly Ser Gly Ser Gly Thr Gln Phe Thr Leu Thr Ile Ser Asp Leu Glu
   65                  70                  75                  80
   Cys Ala Asp Ala Ala Thr Tyr Tyr Cys Leu Gly Ser Tyr Asp Cys Ser
                   85                  90                  95
   Ser Gly Asp Cys Phe Val Phe Gly Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
                       210                 215
<210> 3
   <211> 109
   <212> PRT
   <213> Artificial Sequence
<220>
   <223> Description of Artificial Sequence: Synthetic polypeptide
<400> 3
   Gln Ser Leu Glu Glu Ser Gly Gly Arg Leu Val Thr Pro Gly Thr Pro
   1               5                   10                  15
   Leu Thr Leu Thr Cys Thr Val Ser Gly Leu Asp Leu Ser Ser Tyr Tyr
               20                  25                  30
   Met Gln Trp Val Arg Gln Ala Pro Gly Lys Gly Leu Glu Trp Ile Gly
           35                  40                  45
   Val Ile Gly Ile Asn Asp Asn Thr Tyr Tyr Ala Ser Trp Ala Lys Gly
       50                  55                  60
   Arg Phe Thr Ile Ser Arg Ala Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser
               100                 105
<210> 4
   <211> 439
   <212> PRT
   <213> Artificial Sequence
<220>
   <223> Description of Artificial Sequence: Synthetic polypeptide
<400> 4
   Gln Ser Leu Glu Glu Ser Gly Gly Arg Leu Val Thr Pro Gly Thr Pro
   1               5                   10                  15
   Leu Thr Leu Thr Cys Thr Val Ser Gly Leu Asp Leu Ser Ser Tyr Tyr
               20                  25                  30
   Met Gln Trp Val Arg Gln Ala Pro Gly Lys Gly Leu Glu Trp Ile Gly
           35                  40                  45
   Val Ile Gly Ile Asn Asp Asn Thr Tyr Tyr Ala Ser Trp Ala Lys Gly
       50                  55                  60
   Arg Phe Thr Ile Ser Arg Ala Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Arg Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Ala Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Tyr Thr Leu Pro Pro Ser Arg Glu Glu Met Thr Lys
               340                 345                 350
   Asn Gln Val Ser Leu Thr Cys Leu Val Lys Gly Phe Tyr Pro Ser Asp
           355                 360                 365
   Ile Ala Val Glu Trp Glu Ser Asn Gly Gln Pro Glu Asn Asn Tyr Lys
       370                 375                 380
   Thr Thr Pro Pro Val Leu Asp Ser Asp Gly Ser Phe Phe Leu Tyr Ser
   385                 390                 395                 400
   Lys Leu Thr Val Asp Lys Ser Arg Trp Gln Gln Gly Asn Val Phe Ser
                   405                 410                 415
   Cys Ser Val Met His Glu Ala Leu His Asn His Tyr Thr Gln Lys Ser
               420                 425                 430
   Leu Ser Leu Ser Pro Gly Lys
           435
<210> 5
   <211> 13
   <212> PRT
   <213> Oryctolagus cuniculus
<400> 5
   Gln Ala Ser Gln Ser Val Tyr Asp Asn Asn Tyr Leu Ala
   1               5                   10
<210> 6
   <211> 7
   <212> PRT
   <213> Oryctolagus cuniculus
<400> 6
   Ser Thr Ser Thr Leu Ala Ser
   1               5
<210> 7
   <211> 13
   <212> PRT
   <213> Oryctolagus cuniculus
<400> 7
   Leu Gly Ser Tyr Asp Cys Ser Ser Gly Asp Cys Phe Val
   1               5                   10
<210> 8
   <211> 5
   <212> PRT
   <213> Oryctolagus cuniculus
<400> 8
   Ser Tyr Tyr Met Gln
   1               5
<210> 9
   <211> 16
   <212> PRT
   <213> Oryctolagus cuniculus
<400> 9
   Val Ile Gly Ile Asn Asp Asn Thr Tyr Tyr Ala Ser Trp Ala Lys Gly
   1               5                   10                  15
<210> 10
   <211> 3
   <212> PRT
   <213> Oryctolagus cuniculus
<400> 10
   Gly Asp Ile
   1
<210> 11
   <211> 113
   <212> PRT
   <213> Artificial Sequence
<220>
   <223> Description of Artificial Sequence: Synthetic polypeptide
<400> 11
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg
<210> 12
   <211> 219
   <212> PRT
   <213> Artificial Sequence
<220>
   <223> Description of Artificial Sequence: Synthetic polypeptide
<400> 12
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 13
   <211> 111
   <212> PRT
   <213> Artificial Sequence
<220>
   <223> Description of Artificial Sequence: Synthetic polypeptide
<400> 13
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Leu Asp Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ile Asn Asp Asn Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                      80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 14
   <211> 441
   <212> PRT
   <213> Artificial Sequence
<220>
   <223> Description of Artificial Sequence: Synthetic polypeptide
<400> 14
   Glu Val Gln Leu Val Glu Ser Gly Gly Gly Leu Val Gln Pro Gly Gly
   1 5 10 15
   Ser Leu Arg Leu Ser Cys Ala Val Ser Gly Leu Asp Leu Ser Ser Tyr
   20 25 30
   Tyr Met Gln Trp Val Arg Gln Ala Pro Gly Lys Gly Leu Glu Trp Val
   35 40 45
   Gly Val Ile Gly Ile Asn Asp Asn Thr Tyr Tyr Ala Ser Trp Ala Lys
   50 55 60
   Gly Arg Phe Thr Ile Ser Arg Asp Asn Ser Lys Thr Thr Val Tyr Leu
   65 70 75 80
   Gln Met Asn Ser Leu Arg Ala Glu Asp Thr Ala Val Tyr Phe Cys Ala
   85 90 95
   Arg Gly Asp Ile Trp Gly Gln Gly Thr Leu Val Thr Val Ser Ser Ala
   100 105 110
   Ser Thr Lys Gly Pro Ser Val Phe Pro Leu Ala Pro Ser Ser Lys Ser
   115 120 125
   Thr Ser Gly Gly Thr Ala Ala Leu Gly Cys Leu Val Lys Asp Tyr Phe
   130 135 140
   Pro Glu Pro Val Thr Val Ser Trp Asn Ser Gly Ala Leu Thr Ser Gly
   145 150 155 160
   Val His Thr Phe Pro Ala Val Leu Gln Ser Ser Gly Leu Tyr Ser Leu
   165 170 175
   Ser Ser Val Val Thr Val Pro Ser Ser Ser Leu Gly Thr Gln Thr Tyr
   180 185 190
   Ile Cys Asn Val Asn His Lys Pro Ser Asn Thr Lys Val Asp Lys Arg
   195 200 205
   Val Glu Pro Lys Ser Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 15
   <211> 13
   <212> PRT
   <213> Oryctolagus cuniculus
<400> 15
   Gln Ala Ser Gln Ser Val Tyr Asp Asn Asn Tyr Leu Ala
   1               5                   10
<210> 16
   <211> 7
   <212> PRT
   <213> Oryctolagus cuniculus
<400> 16
   Ser Thr Ser Thr Leu Ala Ser
   1 5
<210> 17
   <211> 13
   <212> PRT
   <213> Oryctolagus cuniculus
<400> 17
   Leu Gly Ser Tyr Asp Cys Ser Ser Gly Asp Cys Phe Val
   1 5 10
<210> 18
   <211> 5
   <212> PRT
   <213> Oryctolagus cuniculus
<400> 18
   Ser Tyr Tyr Met Gln
   1 5
<210> 19
   <211> 16
   <212> PRT
   <213> Oryctolagus cuniculus
<400> 19
   Val Ile Gly Ile Asn Asp Asn Thr Tyr Tyr Ala Ser Trp Ala Lys Gly
   1 5 10 15
<210> 20
   <211> 3
   <212> PRT
   <213> Oryctolagus cuniculus
<400> 20
   Gly Asp Ile
   1
<210> 21
   <211> 113
   <212> PRT
   <213> Artificial Sequence
<220>
   <223> Description of Artificial Sequence: Synthetic polypeptide
<400> 21
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg
<210> 22
   <211> 219
   <212> PRT
   <213> Artificial Sequence
<220>
   <223> Description of Artificial Sequence: Synthetic polypeptide
<400> 22
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asp Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Ser Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 23
   <211> 111
   <212> PRT
   <213> Artificial Sequence
<220>
   <223> Description of Artificial Sequence: Synthetic polypeptide
<400> 23
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Leu Asp Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ile Asn Asp Asn Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 24
   <211> 441
   <212> PRT
   <213> Artificial Sequence
<220>
   <223> Description of Artificial Sequence: Synthetic polypeptide
<400> 24
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Leu Asp Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ile Asn Asp Asn Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Ala Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 25
   <211> 13
   <212> PRT
   <213> Oryctolagus cuniculus
<400> 25
   Gln Ala Ser Gln Ser Val Tyr Asp Asn Asn Tyr Leu Ala
   1               5                   10
<210> 26
   <211> 7
   <212> PRT
   <213> Oryctolagus cuniculus
<400> 26
   Ser Thr Ser Thr Leu Ala Ser
   1               5
<210> 27
   <211> 13
   <212> PRT
   <213> Oryctolagus cuniculus
<400> 27
   Leu Gly Ser Tyr Asp Cys Ser Ser Gly Asp Cys Phe Val
   1               5                   10
<210> 28
   <211> 5
   <212> PRT
   <213> Oryctolagus cuniculus
<400> 28
   Ser Tyr Tyr Met Gln
   1               5
<210> 29
   <211> 16
   <212> PRT
   <213> Oryctolagus cuniculus
<400> 29
   Val Ile Gly Ile Asn Asp Asn Thr Tyr Tyr Ala Ser Trp Ala Lys Gly
   1               5                   10                  15
<210> 30
   <211> 3
   <212> PRT
   <213> Oryctolagus cuniculus
<400> 30
   Gly Asp Ile
   1
<210> 31
   <211> 113
   <212> PRT
   <213> Artificial Sequence
<220>
   <223> Description of Artificial Sequence: Synthetic polypeptide
<400> 31
   Gln Val Leu Thr Gln Thr Pro Ser Pro Val Ser Ala Ala Val Gly Ser
   1               5                   10                  15
   Thr Val Thr Ile Asn Cys Gln Ala Ser Gln Ser Val Tyr His Asn Thr
               20                  25                  30
   Tyr Leu Ala Trp Tyr Gln Gln Lys Pro Gly Gln Pro Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Gln Phe Thr Leu Thr Ile Ser Gly Val Gln
   65                  70                  75                  80
   Cys Asn Asp Ala Ala Ala Tyr Tyr Cys Leu Gly Ser Tyr Asp Cys Thr
                   85                  90                  95
   Asn Gly Asp Cys Phe Val Phe Gly Gly Gly Thr Glu Val Val Val Lys
               100                 105                 110
   Arg
<210> 32
   <211> 219
   <212> PRT
   <213> Artificial Sequence
<220>
   <223> Description of Artificial Sequence: Synthetic polypeptide
<400> 32
   Gln Val Leu Thr Gln Thr Pro Ser Pro Val Ser Ala Ala Val Gly Ser
   1               5                   10                  15
   Thr Val Thr Ile Asn Cys Gln Ala Ser Gln Ser Val Tyr His Asn Thr
               20                  25                  30
   Tyr Leu Ala Trp Tyr Gln Gln Lys Pro Gly Gln Pro Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Gln Phe Thr Leu Thr Ile Ser Gly Val Gln
   65                  70                  75                  80
   Cys Asn Asp Ala Ala Ala Tyr Tyr Cys Leu Gly Ser Tyr Asp Cys Thr
                   85                  90                  95
   Asn Gly Asp Cys Phe Val Phe Gly Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 33
   <211> 109
   <212> PRT
   <213> Artificial Sequence
<220>
   <223> Description of Artificial Sequence: Synthetic polypeptide
<400> 33
   Gln Ser Leu Glu Glu Ser Gly Gly Arg Leu Val Thr Pro Gly Thr Pro
   1               5                   10                  15
   Leu Thr Leu Thr Cys Ser Val Ser Gly Ile Asp Leu Ser Gly Tyr Tyr
               20                  25                  30
   Met Asn Trp Val Arg Gln Ala Pro Gly Lys Gly Leu Glu Trp Ile Gly
           35                  40                  45
   Val Ile Gly Ile Asn Gly Ala Thr Tyr Tyr Ala Ser Trp Ala Lys Gly
       50                  55                  60
   Arg Phe Thr Ile Ser Lys Thr Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser
               100                 105
<210> 34
   <211> 439
   <212> PRT
   <213> Artificial Sequence
<220>
   <223> Description of Artificial Sequence: Synthetic polypeptide
<400> 34
   Gln Ser Leu Glu Glu Ser Gly Gly Arg Leu Val Thr Pro Gly Thr Pro
   1               5                   10                  15
   Leu Thr Leu Thr Cys Ser Val Ser Gly Ile Asp Leu Ser Gly Tyr Tyr
               20                  25                  30
   Met Asn Trp Val Arg Gln Ala Pro Gly Lys Gly Leu Glu Trp Ile Gly
           35                  40                  45
   Val Ile Gly Ile Asn Gly Ala Thr Tyr Tyr Ala Ser Trp Ala Lys Gly
       50                  55                  60
   Arg Phe Thr Ile Ser Lys Thr Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Arg Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Ala Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Tyr Thr Leu Pro Pro Ser Arg Glu Glu Met Thr Lys
               340                 345                 350
   Asn Gln Val Ser Leu Thr Cys Leu Val Lys Gly Phe Tyr Pro Ser Asp
           355                 360                 365
   Ile Ala Val Glu Trp Glu Ser Asn Gly Gln Pro Glu Asn Asn Tyr Lys
       370                 375                 380
   Thr Thr Pro Pro Val Leu Asp Ser Asp Gly Ser Phe Phe Leu Tyr Ser
   385                 390                 395                 400
   Lys Leu Thr Val Asp Lys Ser Arg Trp Gln Gln Gly Asn Val Phe Ser
                   405                 410                 415
   Cys Ser Val Met His Glu Ala Leu His Asn His Tyr Thr Gln Lys Ser
               420                 425                 430
   Leu Ser Leu Ser Pro Gly Lys
           435
<210> 35
   <211> 13
   <212> PRT
   <213> Oryctolagus cuniculus
<400> 35
   Gln Ala Ser Gln Ser Val Tyr His Asn Thr Tyr Leu Ala
   1               5                   10
<210> 36
   <211> 7
   <212> PRT
   <213> Oryctolagus cuniculus
<400> 36
   Asp Ala Ser Thr Leu Ala Ser
   1               5
<210> 37
   <211> 13
   <212> PRT
   <213> Oryctolagus cuniculus
<400> 37
   Leu Gly Ser Tyr Asp Cys Thr Asn Gly Asp Cys Phe Val
   1               5                   10
<210> 38
   <211> 5
   <212> PRT
   <213> Oryctolagus cuniculus
<400> 38
   Gly Tyr Tyr Met Asn
   1               5
<210> 39
   <211> 16
   <212> PRT
   <213> Oryctolagus cuniculus
<400> 39
   Val Ile Gly Ile Asn Gly Ala Thr Tyr Tyr Ala Ser Trp Ala Lys Gly
   1               5                   10                  15
<210> 40
   <211> 3
   <212> PRT
   <213> Oryctolagus cuniculus
<400> 40
   Gly Asp Ile
   1
<210> 41
   <211> 113
   <212> PRT
   <213> Artificial Sequence
<220>
   <223> Description of Artificial Sequence: Synthetic polypeptide
<400> 41
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr His Asn Thr
               20                  25                  30
   Tyr Leu Ala Trp Tyr Gln Gln Lys Pro Gly Lys Val Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Thr
                   85                  90                  95
   Asn Gly Asp Cys Phe Val Phe Gly Gly Gly Thr Lys Val Glu Ile Lys
               100                 105                 110
   Arg
<210> 42
   <211> 219
   <212> PRT
   <213> Artificial Sequence
<220>
   <223> Description of Artificial Sequence: Synthetic polypeptide
<400> 42
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr His Asn Thr
               20                  25                  30
   Tyr Leu Ala Trp Tyr Gln Gln Lys Pro Gly Lys Val Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Thr
                   85                  90                  95
   Asn Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 43
   <211> 111
   <212> PRT
   <213> Artificial Sequence
<220>
   <223> Description of Artificial Sequence: Synthetic polypeptide
<400> 43
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 44
   <211> 441
   <212> PRT
   <213> Artificial Sequence
<220>
   <223> Description of Artificial Sequence: Synthetic polypeptide
<400> 44
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
   100 105 110
   Ser Thr Lys Gly Pro Ser Val Phe Pro Leu Ala Pro Ser Ser Lys Ser
   115 120 125
   Thr Ser Gly Gly Thr Ala Ala Leu Gly Cys Leu Val Lys Asp Tyr Phe
   130 135 140
   Pro Glu Pro Val Thr Val Ser Trp Asn Ser Gly Ala Leu Thr Ser Gly
   145 150 155 160
   Val His Thr Phe Pro Ala Val Leu Gln Ser Ser Gly Leu Tyr Ser Leu
   165 170 175
   Ser Ser Val Val Thr Val Pro Ser Ser Ser Leu Gly Thr Gln Thr Tyr
   180 185 190
   Ile Cys Asn Val Asn His Lys Pro Ser Asn Thr Lys Val Asp Lys Arg
   195 200 205
   Val Glu Pro Lys Ser Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Ala Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Tyr Thr Leu Pro Pro Ser Arg Glu Glu Met
   340 345 350
   Thr Lys Asn Gln Val Ser Leu Thr Cys Leu Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro Gly Lys
   435 440
<210> 45
   <211> 13
   <212> PRT
   <213> Oryctolagus cuniculus
<400> 45
   Gln Ala Ser Gln Ser Val Tyr His Asn Thr Tyr Leu Ala
   1 5 10
<210> 46
   <211> 7
   <212> PRT
   <213> Oryctolagus cuniculus
<400> 46
   Asp Ala Ser Thr Leu Ala Ser
   1 5
<210> 47
   <211> 13
   <212> PRT
   <213> Oryctolagus cuniculus
<400> 47
   Leu Gly Ser Tyr Asp Cys Thr Asn Gly Asp Cys Phe Val
   1 5 10
<210> 48
   <211> 5
   <212> PRT
   <213> Oryctolagus cuniculus
<400> 48
   Gly Tyr Tyr Met Asn
   1 5
<210> 49
   <211> 15
   <212> PRT
   <213> Oryctolagus cuniculus
<400> 49
   Ile Gly Ile Asn Gly Ala Thr Tyr Tyr Ala Ser Trp Ala Lys Gly
   1 5 10 15
<210> 50
   <211> 3
   <212> PRT
   <213> Oryctolagus cuniculus
<400> 50
   Gly Asp Ile
   1
<210> 51
   <211> 113
   <212> PRT
   <213> Artificial Sequence
<220>
   <223> Description of Artificial Sequence: Synthetic polypeptide
<400> 51
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr His Asn Thr
               20                  25                  30
   Tyr Leu Ala Trp Tyr Gln Gln Lys Pro Gly Lys Val Pro Lys Gln Leu
           35                  40                  45
   Ile Tyr Asp Ala Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Thr
                   85                  90                  95
   Asn Gly Asp Cys Phe Val Phe Gly Gly Gly Thr Lys Val Glu Ile Lys
               100                 105                 110
   Arg
<210> 52
   <211> 219
   <212> PRT
   <213> Artificial Sequence
<220>
   <223> Description of Artificial Sequence: Synthetic polypeptide
<400> 52
   Gln Val Leu Thr Gln Ser Pro Ser Ser Leu Ser Ala Ser Val Gly Asp
   1 5 10 15
   Arg Val Thr Ile Asn Cys Gln Ala Ser Gln Ser Val Tyr His Asn Thr
   20 25 30
   Tyr Leu Ala Trp Tyr Gln Gln Lys Pro Gly Lys Val Pro Lys Gln Leu
   35 40 45
   Ile Tyr Asp Ala Ser Thr Leu Ala Ser Gly Val Pro Ser Arg Phe Ser
   50 55 60
   Gly Ser Gly Ser Gly Thr Asp Phe Thr Leu Thr Ile Ser Ser Leu Gln
   65 70 75 80
   Pro Glu Asp Val Ala Thr Tyr Tyr Cys Leu Gly Ser Tyr Asp Cys Thr
   85 90 95
   Asn Gly Asp Cys Phe Val Phe Gly Gly Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 53
   <211> 111
   <212> PRT
   <213> Artificial Sequence
<220>
   <223> Description of Artificial Sequence: Synthetic polypeptide
<400> 53
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 54
   <211> 441
   <212> PRT
   <213> Artificial Sequence
<220>
   <223> Description of Artificial Sequence: Synthetic polypeptide
<400> 54
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Gly Tyr
               20                  25                  30
   Tyr Met Asn Trp Val Arg Gln Ala Pro Gly Lys Gly Leu Glu Trp Val
           35                  40                  45
   Gly Val Ile Gly Ile Asn Gly Ala Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Ala Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Tyr Thr Leu Pro Pro Ser Arg Glu Glu Met
   340 345 350
   Thr Lys Asn Gln Val Ser Leu Thr Cys Leu Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro Gly Lys
   435 440
<210> 55
   <211> 13
   <212> PRT
   <213> Oryctolagus cuniculus
<400> 55
   Gln Ala Ser Gln Ser Val Tyr His Asn Thr Tyr Leu Ala
   1 5 10
<210> 56
   <211> 7
   <212> PRT
   <213> Oryctolagus cuniculus
<400> 56
   Asp Ala Ser Thr Leu Ala Ser
   1 5
<210> 57
   <211> 13
   <212> PRT
   <213> Oryctolagus cuniculus
<400> 57
   Leu Gly Ser Tyr Asp Cys Thr Asn Gly Asp Cys Phe Val
   1 5 10
<210> 58
   <211> 5
   <212> PRT
   <213> Oryctolagus cuniculus
<400> 58
   Gly Tyr Tyr Met Asn
   1 5
<210> 59
   <211> 15
   <212> PRT
   <213> Oryctolagus cuniculus
<400> 59
   Ile Gly Ile Asn Gly Ala Thr Tyr Tyr Ala Ser Trp Ala Lys Gly
   1 5 10 15
<210> 60
   <211> 3
   <212> PRT
   <213> Oryctolagus cuniculus
<400> 60
   Gly Asp Ile
   1
<210> 61
   <211> 113
   <212> PRT
   <213> Artificial Sequence
<220>
   <223> Description of Artificial Sequence: Synthetic polypeptide
<400> 61
   Gln Val Leu Thr Gln Thr Ala Ser Pro Val Ser Ala Ala Val Gly Ser
   1               5                   10                  15
   Thr Val Thr Ile Asn Cys Gln Ala Ser Gln Ser Val Tyr Asn Tyr Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Lys
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Thr Gly Asp Cys Phe Val Phe Gly Gly Gly Thr Glu Val Val Val Lys
               100                 105                 110
   Arg
<210> 62
   <211> 219
   <212> PRT
   <213> Artificial Sequence
<220>
   <223> Description of Artificial Sequence: Synthetic polypeptide
<400> 62
   Gln Val Leu Thr Gln Thr Ala Ser Pro Val Ser Ala Ala Val Gly Ser
   1               5                   10                  15
   Thr Val Thr Ile Asn Cys Gln Ala Ser Gln Ser Val Tyr Asn Tyr Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Lys
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Thr Gly Asp Cys Phe Val Phe Gly Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 63
   <211> 110
   <212> PRT
   <213> Artificial Sequence
<220>
   <223> Description of Artificial Sequence: Synthetic polypeptide
<400> 63
   Gln Glu Gln Leu Lys Glu Ser Gly Gly Arg Leu Val Thr Pro Gly Thr
   1               5                   10                  15
   Ser Leu Thr Leu Thr Cys Thr Val Ser Gly Ile Asp Leu Ser Asn His
               20                  25                  30
   Tyr Met Gln Trp Val Arg Gln Ala Pro Gly Lys Gly Leu Glu Trp Ile
           35                  40                  45
   Gly Val Val Gly Ile Asn Gly Arg Thr Tyr Tyr Ala Ser Trp Ala Lys
       50                  55                  60
   Gly Arg Phe Thr Ile Ser Arg Thr Ser Ser Thr Thr Val Asp Leu Lys
   65                  70                  75                  80
   Met Thr Arg Leu Thr Thr Glu Asp Thr Ala Thr Tyr Phe Cys Ala Arg
                   85                  90                  95
   Gly Asp Ile Trp Gly Pro Gly Thr Leu Val Thr Val Ser Ser
               100                 105                 110
<210> 64
   <211> 440
   <212> PRT
   <213> Artificial Sequence
<220>
   <223> Description of Artificial Sequence: Synthetic polypeptide
<400> 64
   Gln Glu Gln Leu Lys Glu Ser Gly Gly Arg Leu Val Thr Pro Gly Thr
   1               5                   10                  15
   Ser Leu Thr Leu Thr Cys Thr Val Ser Gly Ile Asp Leu Ser Asn His
               20                  25                  30
   Tyr Met Gln Trp Val Arg Gln Ala Pro Gly Lys Gly Leu Glu Trp Ile
           35                  40                  45
   Gly Val Val Gly Ile Asn Gly Arg Thr Tyr Tyr Ala Ser Trp Ala Lys
       50                  55                  60
   Gly Arg Phe Thr Ile Ser Arg Thr Ser Ser Thr Thr Val Asp Leu Lys
   65                  70                  75                  80
   Met Thr Arg Leu Thr Thr Glu Asp Thr Ala Thr Tyr Phe Cys Ala Arg
                   85                  90                  95
   Gly Asp Ile Trp Gly Pro Gly Thr Leu Val Thr Val Ser Ser Ala Ser
               100                 105                 110
   Thr Lys Gly Pro Ser Val Phe Pro Leu Ala Pro Ser Ser Lys Ser Thr
           115                 120                 125
   Ser Gly Gly Thr Ala Ala Leu Gly Cys Leu Val Lys Asp Tyr Phe Pro
       130                 135                 140
   Glu Pro Val Thr Val Ser Trp Asn Ser Gly Ala Leu Thr Ser Gly Val
   145                 150                 155                 160
   His Thr Phe Pro Ala Val Leu Gln Ser Ser Gly Leu Tyr Ser Leu Ser
                   165                 170                 175
   Ser Val Val Thr Val Pro Ser Ser Ser Leu Gly Thr Gln Thr Tyr Ile
               180                 185                 190
   Cys Asn Val Asn His Lys Pro Ser Asn Thr Lys Val Asp Lys Arg Val
           195                 200                 205
   Glu Pro Lys Ser Cys Asp Lys Thr His Thr Cys Pro Pro Cys Pro Ala
       210                 215                 220
   Pro Glu Leu Leu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser His Glu Asp Pro Glu Val Lys Phe Asn Trp Tyr Val
               260                 265                 270
   Asp Gly Val Glu Val His Asn Ala Lys Thr Lys Pro Arg Glu Glu Gln
           275                 280                 285
   Tyr Ala Ser Thr Tyr Arg Val Val Ser Val Leu Thr Val Leu His Gln
       290                 295                 300
   Asp Trp Leu Asn Gly Lys Glu Tyr Lys Cys Lys Val Ser Asn Lys Ala
   305                 310                 315                 320
   Leu Pro Ala Pro Ile Glu Lys Thr Ile Ser Lys Ala Lys Gly Gln Pro
                   325                 330                 335
   Arg Glu Pro Gln Val Tyr Thr Leu Pro Pro Ser Arg Glu Glu Met Thr
               340                 345                 350
   Lys Asn Gln Val Ser Leu Thr Cys Leu Val Lys Gly Phe Tyr Pro Ser
           355                 360                 365
   Asp Ile Ala Val Glu Trp Glu Ser Asn Gly Gln Pro Glu Asn Asn Tyr
       370                 375                 380
   Lys Thr Thr Pro Pro Val Leu Asp Ser Asp Gly Ser Phe Phe Leu Tyr
   385                 390                 395                 400
   Ser Lys Leu Thr Val Asp Lys Ser Arg Trp Gln Gln Gly Asn Val Phe
                   405                 410                 415
   Ser Cys Ser Val Met His Glu Ala Leu His Asn His Tyr Thr Gln Lys
               420                 425                 430
   Ser Leu Ser Leu Ser Pro Gly Lys
           435                 440
<210> 65
   <211> 13
   <212> PRT
   <213> Oryctolagus cuniculus
<400> 65
   Gln Ala Ser Gln Ser Val Tyr Asn Tyr Asn Tyr Leu Ala
   1               5                   10
<210> 66
   <211> 7
   <212> PRT
   <213> Oryctolagus cuniculus
<400> 66
   Ser Thr Ser Thr Leu Ala Ser
   1               5
<210> 67
   <211> 13
   <212> PRT
   <213> Oryctolagus cuniculus
<400> 67
   Leu Gly Ser Tyr Asp Cys Ser Thr Gly Asp Cys Phe Val
   1               5                   10
<210> 68
   <211> 5
   <212> PRT
   <213> Oryctolagus cuniculus
<400> 68
   Asn His Tyr Met Gln
   1               5
<210> 69
   <211> 16
   <212> PRT
   <213> Oryctolagus cuniculus
<400> 69
   Val Val Gly Ile Asn Gly Arg Thr Tyr Tyr Ala Ser Trp Ala Lys Gly
   1               5                   10                  15
<210> 70
   <211> 3
   <212> PRT
   <213> Oryctolagus cuniculus
<400> 70
   Gly Asp Ile
   1
<210> 71
   <211> 113
   <212> PRT
   <213> Artificial Sequence
<220>
   <223> Description of Artificial Sequence: Synthetic polypeptide
<400> 71
   Gln Val Leu Thr Gln Ser Pro Ser Ser Leu Ser Ala Ser Val Gly Asp
   1 5 10 15
   Arg Val Thr Ile Asn Cys Gln Ala Ser Gln Ser Val Tyr Asn Tyr Asn
   20 25 30
   Tyr Leu Ala Trp Tyr Gln Gln Lys Pro Gly Lys Val Pro Lys Gln Leu
   35 40 45
   Ile Tyr Ser Thr Ser Thr Leu Ala Ser Gly Val Pro Ser Arg Phe Ser
   50 55 60
   Gly Ser Gly Ser Gly Thr Asp Phe Thr Leu Thr Ile Ser Ser Leu Gln
   65 70 75 80
   Pro Glu Asp Val Ala Thr Tyr Tyr Cys Leu Gly Ser Tyr Asp Cys Ser
   85 90 95
   Thr Gly Asp Cys Phe Val Phe Gly Gly Gly Thr Lys Val Glu Ile Lys
   100 105 110
   Arg
<210> 72
   <211> 219
   <212> PRT
   <213> Artificial Sequence
<220>
   <223> Description of Artificial Sequence: Synthetic polypeptide
<400> 72
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Ser Val Tyr Asn Tyr Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Thr Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 73
   <211> 111
   <212> PRT
   <213> Artificial Sequence
<220>
   <223> Description of Artificial Sequence: Synthetic polypeptide
<400> 73
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Asn His
               20                  25                  30
   Tyr Met Gln Trp Val Arg Gln Ala Pro Gly Lys Gly Leu Glu Trp Val
           35                  40                  45
   Gly Val Val Gly Ile Asn Gly Arg Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 74
   <211> 441
   <212> PRT
   <213> Artificial Sequence
<220>
   <223> Description of Artificial Sequence: Synthetic polypeptide
<400> 74
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Leu Ser Asn His
               20                  25                  30
   Tyr Met Gln Trp Val Arg Gln Ala Pro Gly Lys Gly Leu Glu Trp Val
           35                  40                  45
   Gly Val Val Gly Ile Asn Gly Arg Thr Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 75
   <211> 13
   <212> PRT
   <213> Oryctolagus cuniculus
<400> 75
   Gln Ala Ser Gln Ser Val Tyr Asn Tyr Asn Tyr Leu Ala
   1 5 10
<210> 76
   <211> 7
   <212> PRT
   <213> Oryctolagus cuniculus
<400> 76
   Ser Thr Ser Thr Leu Ala Ser
   1 5
<210> 77
   <211> 13
   <212> PRT
   <213> Oryctolagus cuniculus
<400> 77
   Leu Gly Ser Tyr Asp Cys Ser Thr Gly Asp Cys Phe Val
   1 5 10
<210> 78
   <211> 5
   <212> PRT
   <213> Oryctolagus cuniculus
<400> 78
   Asn His Tyr Met Gln
   1 5
<210> 79
   <211> 16
   <212> PRT
   <213> Oryctolagus cuniculus
<400> 79
   Val Val Gly Ile Asn Gly Arg Thr Tyr Tyr Ala Ser Trp Ala Lys Gly
   1 5 10 15
<210> 80
   <211> 3
   <212> PRT
   <213> Oryctolagus cuniculus
<400> 80
   Gly Asp Ile
   1
<210> 81
   <211> 113
   <212> PRT
   <213> Artificial Sequence
<220>
   <223> Description of Artificial Sequence: Synthetic polypeptide
<400> 81
   Gln Val Leu Thr Gln Thr Pro Ser Pro Val Ser Ala Ala Val Gly Ser
   1               5                   10                  15
   Thr Val Thr Ile Asn Cys Gln Ala Ser Gln Asn Val Tyr Asn Asn Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Arg
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Arg Gly Asp Cys Phe Val Phe Gly Gly Gly Thr Glu Val Val Val Lys
               100                 105                 110
   Arg
<210> 82
   <211> 219
   <212> PRT
   <213> Artificial Sequence
<220>
   <223> Description of Artificial Sequence: Synthetic polypeptide
<400> 82
   Gln Val Leu Thr Gln Thr Pro Ser Pro Val Ser Ala Ala Val Gly Ser
   1               5                   10                  15
   Thr Val Thr Ile Asn Cys Gln Ala Ser Gln Asn Val Tyr Asn Asn Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Arg
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Arg Gly Asp Cys Phe Val Phe Gly Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 83
   <211> 109
   <212> PRT
   <213> Artificial Sequence
<220>
   <223> Description of Artificial Sequence: Synthetic polypeptide
<400> 83
   Gln Ser Leu Glu Glu Ser Gly Gly Arg Leu Val Thr Pro Gly Thr Pro
   1               5                   10                  15
   Leu Thr Leu Thr Cys Thr Val Ser Gly Ile Gly Leu Ser Ser Tyr Tyr
               20                  25                  30
   Met Gln Trp Val Arg Gln Ser Pro Gly Arg Gly Leu Glu Trp Ile Gly
           35                  40                  45
   Val Ile Gly Ser Asp Gly Lys Thr Tyr Tyr Ala Thr Trp Ala Lys Gly
       50                  55                  60
   Arg Phe Thr Ile Ser Lys Thr Ser Ser Thr Thr Val Asp Leu Arg Met
   65                  70                  75                  80
   Ala Ser Leu Thr Thr Glu Asp Thr Ala Thr Tyr Phe Cys Thr Arg Gly
                   85                  90                  95
   Asp Ile Trp Gly Pro Gly Thr Leu Val Thr Val Ser Ser
               100                 105
<210> 84
   <211> 439
   <212> PRT
   <213> Artificial Sequence
<220>
   <223> Description of Artificial Sequence: Synthetic polypeptide
<400> 84
   Gln Ser Leu Glu Glu Ser Gly Gly Arg Leu Val Thr Pro Gly Thr Pro
   1               5                   10                  15
   Leu Thr Leu Thr Cys Thr Val Ser Gly Ile Gly Leu Ser Ser Tyr Tyr
               20                  25                  30
   Met Gln Trp Val Arg Gln Ser Pro Gly Arg Gly Leu Glu Trp Ile Gly
           35                  40                  45
   Val Ile Gly Ser Asp Gly Lys Thr Tyr Tyr Ala Thr Trp Ala Lys Gly
       50                  55                  60
   Arg Phe Thr Ile Ser Lys Thr Ser Ser Thr Thr Val Asp Leu Arg Met
   65                  70                  75                  80
   Ala Ser Leu Thr Thr Glu Asp Thr Ala Thr Tyr Phe Cys Thr Arg Gly
                   85                  90                  95
   Asp Ile Trp Gly Pro Gly Thr Leu Val Thr Val Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Arg Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Ala Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Tyr Thr Leu Pro Pro Ser Arg Glu Glu Met Thr Lys
               340                 345                 350
   Asn Gln Val Ser Leu Thr Cys Leu Val Lys Gly Phe Tyr Pro Ser Asp
           355                 360                 365
   Ile Ala Val Glu Trp Glu Ser Asn Gly Gln Pro Glu Asn Asn Tyr Lys
       370                 375                 380
   Thr Thr Pro Pro Val Leu Asp Ser Asp Gly Ser Phe Phe Leu Tyr Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro Gly Lys
   435
<210> 85
   <211> 13
   <212> PRT
   <213> Oryctolagus cuniculus
<400> 85
   Gln Ala Ser Gln Asn Val Tyr Asn Asn Asn Tyr Leu Ala
   1 5 10
<210> 86
   <211> 7
   <212> PRT
   <213> Oryctolagus cuniculus
<400> 86
   Ser Thr Ser Thr Leu Ala Ser
   1 5
<210> 87
   <211> 13
   <212> PRT
   <213> Oryctolagus cuniculus
<400> 87
   Leu Gly Ser Tyr Asp Cys Ser Arg Gly Asp Cys Phe Val
   1 5 10
<210> 88
   <211> 5
   <212> PRT
   <213> Oryctolagus cuniculus
<400> 88
   Ser Tyr Tyr Met Gln
   1 5
<210> 89
   <211> 16
   <212> PRT
   <213> Oryctolagus cuniculus
<400> 89
   Val Ile Gly Ser Asp Gly Lys Thr Tyr Tyr Ala Thr Trp Ala Lys Gly
   1               5                   10                  15
<210> 90
   <211> 3
   <212> PRT
   <213> Oryctolagus cuniculus
<400> 90
   Gly Asp Ile
   1
<210> 91
   <211> 113
   <212> PRT
   <213> Artificial Sequence
<220>
   <223> Description of Artificial Sequence: Synthetic polypeptide
<400> 91
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Asn Val Tyr Asn Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Arg Gly Asp Cys Phe Val Phe Gly Gly Gly Thr Lys Val Glu Ile Lys
               100                 105                 110
   Arg
<210> 92
   <211> 219
   <212> PRT
   <213> Artificial Sequence
<220>
   <223> Description of Artificial Sequence: Synthetic polypeptide
<400> 92
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Asn Val Tyr Asn Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Arg Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 93
   <211> 111
   <212> PRT
   <213> Artificial Sequence
<220>
   <223> Description of Artificial Sequence: Synthetic polypeptide
<400> 93
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Gly Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ser Asp Gly Lys Thr Tyr Tyr Ala Thr Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Thr
                   85                  90                  95
   Arg Gly Asp Ile Trp Gly Gln Gly Thr Leu Val Thr Val Ser Ser
               100                 105                 110
<210> 94
   <211> 441
   <212> PRT
   <213> Artificial Sequence
<220>
   <223> Description of Artificial Sequence: Synthetic polypeptide
<400> 94
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Gly Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ser Asp Gly Lys Thr Tyr Tyr Ala Thr Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Thr
   85 90 95
   Arg Gly Asp Ile Trp Gly Gln Gly Thr Leu Val Thr Val Ser Ser Ala
   100 105 110
   Ser Thr Lys Gly Pro Ser Val Phe Pro Leu Ala Pro Ser Ser Lys Ser
   115 120 125
   Thr Ser Gly Gly Thr Ala Ala Leu Gly Cys Leu Val Lys Asp Tyr Phe
   130 135 140
   Pro Glu Pro Val Thr Val Ser Trp Asn Ser Gly Ala Leu Thr Ser Gly
   145 150 155 160
   Val His Thr Phe Pro Ala Val Leu Gln Ser Ser Gly Leu Tyr Ser Leu
   165 170 175
   Ser Ser Val Val Thr Val Pro Ser Ser Ser Leu Gly Thr Gln Thr Tyr
   180 185 190
   Ile Cys Asn Val Asn His Lys Pro Ser Asn Thr Lys Val Asp Lys Arg
   195 200 205
   Val Glu Pro Lys Ser Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Ala Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 95
   <211> 13
   <212> PRT
   <213> Oryctolagus cuniculus
<400> 95
   Gln Ala Ser Gln Asn Val Tyr Asn Asn Asn Tyr Leu Ala
   1               5                   10
<210> 96
   <211> 7
   <212> PRT
   <213> Oryctolagus cuniculus
<400> 96
   Ser Thr Ser Thr Leu Ala Ser
   1               5
<210> 97
   <211> 13
   <212> PRT
   <213> Oryctolagus cuniculus
<400> 97
   Leu Gly Ser Tyr Asp Cys Ser Arg Gly Asp Cys Phe Val
   1               5                   10
<210> 98
   <211> 5
   <212> PRT
   <213> Oryctolagus cuniculus
<400> 98
   Ser Tyr Tyr Met Gln
   1               5
<210> 99
   <211> 16
   <212> PRT
   <213> Oryctolagus cuniculus
<400> 99
   Val Ile Gly Ser Asp Gly Lys Thr Tyr Tyr Ala Thr Trp Ala Lys Gly
   1               5                   10                  15
<210> 100
   <211> 3
   <212> PRT
   <213> Oryctolagus cuniculus
<400> 100
   Gly Asp Ile
   1
<210> 101
   <211> 113
   <212> PRT
   <213> Artificial Sequence
<220>
   <223> Description of Artificial Sequence: Synthetic polypeptide
<400> 101
   Gln Val Leu Thr Gln Thr Ala Ser Pro Val Ser Pro Ala Val Gly Ser
   1               5                   10                  15
   Thr Val Thr Ile Asn Cys Arg Ala Ser Gln Ser Val Tyr Tyr Asn Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Lys
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Asn Gly Asp Cys Phe Val Phe Gly Gly Gly Thr Glu Val Val Val Lys
               100                 105                 110
   Arg
<210> 102
   <211> 219
   <212> PRT
   <213> Artificial Sequence
<220>
   <223> Description of Artificial Sequence: Synthetic polypeptide
<400> 102
   Gln Val Leu Thr Gln Thr Ala Ser Pro Val Ser Pro Ala Val Gly Ser
   1               5                   10                  15
   Thr Val Thr Ile Asn Cys Arg Ala Ser Gln Ser Val Tyr Tyr Asn Asn
               20                  25                  30
   Tyr Leu Ala Trp Tyr Gln Gln Lys Pro Gly Gln Pro Pro Lys Gln Leu
           35                  40                  45
   Ile Tyr Ser Thr Ser Thr Leu Ala Ser Gly Val Ser Ser Arg Phe Lys
       50                  55                  60
   Gly Ser Gly Ser Gly Thr Gln Phe Thr Leu Thr Ile Ser Asp Val Gln
   65                  70                  75                  80
   Cys Asp Asp Ala Ala Thr Tyr Tyr Cys Leu Gly Ser Tyr Asp Cys Ser
                   85                  90                  95
   Asn Gly Asp Cys Phe Val Phe Gly Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
               195                 200                 205
   Pro Val Thr Lys Ser Phe Asn Arg Gly Glu Cys
       210                 215
<210> 103
   <211> 109
   <212> PRT
   <213> Artificial Sequence
<220>
   <223> Description of Artificial Sequence: Synthetic polypeptide
<400> 103
   Gln Ser Leu Glu Glu Ser Gly Gly Arg Leu Val Thr Pro Gly Gly Ser
   1               5                   10                  15
   Leu Thr Leu Thr Cys Thr Val Ser Gly Ile Asp Val Thr Asn Tyr Tyr
               20                  25                  30
   Met Gln Trp Val Arg Gln Ala Pro Gly Lys Gly Leu Glu Trp Ile Gly
           35                  40                  45
   Val Ile Gly Val Asn Gly Lys Arg Tyr Tyr Ala Ser Trp Ala Lys Gly
       50                  55                  60
   Arg Phe Thr Ile Ser Lys Thr Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser
               100                 105
<210> 104
   <211> 439
   <212> PRT
   <213> Artificial Sequence
<220>
   <223> Description of Artificial Sequence: Synthetic polypeptide
<400> 104
   Gln Ser Leu Glu Glu Ser Gly Gly Arg Leu Val Thr Pro Gly Gly Ser
   1               5                   10                  15
   Leu Thr Leu Thr Cys Thr Val Ser Gly Ile Asp Val Thr Asn Tyr Tyr
               20                  25                  30
   Met Gln Trp Val Arg Gln Ala Pro Gly Lys Gly Leu Glu Trp Ile Gly
           35                  40                  45
   Val Ile Gly Val Asn Gly Lys Arg Tyr Tyr Ala Ser Trp Ala Lys Gly
       50                  55                  60
   Arg Phe Thr Ile Ser Lys Thr Ser Ser Thr Thr Val Asp Leu Lys Met
   65                  70                  75                  80
   Thr Ser Leu Thr Thr Glu Asp Thr Ala Thr Tyr Phe Cys Ala Arg Gly
                   85                  90                  95
   Asp Ile Trp Gly Pro Gly Thr Leu Val Thr Val Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Arg Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Ala Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Tyr Thr Leu Pro Pro Ser Arg Glu Glu Met Thr Lys
               340                 345                 350
   Asn Gln Val Ser Leu Thr Cys Leu Val Lys Gly Phe Tyr Pro Ser Asp
           355                 360                 365
   Ile Ala Val Glu Trp Glu Ser Asn Gly Gln Pro Glu Asn Asn Tyr Lys
       370                 375                 380
   Thr Thr Pro Pro Val Leu Asp Ser Asp Gly Ser Phe Phe Leu Tyr Ser
   385                 390                 395                 400
   Lys Leu Thr Val Asp Lys Ser Arg Trp Gln Gln Gly Asn Val Phe Ser
                   405                 410                 415
   Cys Ser Val Met His Glu Ala Leu His Asn His Tyr Thr Gln Lys Ser
               420                 425                 430
   Leu Ser Leu Ser Pro Gly Lys
           435
<210> 105
   <211> 13
   <212> PRT
   <213> Oryctolagus cuniculus
<400> 105
   Arg Ala Ser Gln Ser Val Tyr Tyr Asn Asn Tyr Leu Ala
   1               5                   10
<210> 106
   <211> 7
   <212> PRT
   <213> Oryctolagus cuniculus
<400> 106
   Ser Thr Ser Thr Leu Ala Ser
   1               5
<210> 107
   <211> 13
   <212> PRT
   <213> Oryctolagus cuniculus
<400> 107
   Leu Gly Ser Tyr Asp Cys Ser Asn Gly Asp Cys Phe Val
   1 5 10
<210> 108
   <211> 5
   <212> PRT
   <213> Oryctolagus cuniculus
<400> 108
   Asn Tyr Tyr Met Gln
   1 5
<210> 109
   <211> 16
   <212> PRT
   <213> Oryctolagus cuniculus
<400> 109
   Val Ile Gly Val Asn Gly Lys Arg Tyr Tyr Ala Ser Trp Ala Lys Gly
   1 5 10 15
<210> 110
   <211> 3
   <212> PRT
   <213> Oryctolagus cuniculus
<400> 110
   Gly Asp Ile
   1
<210> 111
   <211> 113
   <212> PRT
   <213> Artificial Sequence
<220>
   <223> Description of Artificial Sequence: Synthetic polypeptide
<400> 111
   Gln Val Leu Thr Gln Ser Pro Ser Ser Leu Ser Ala Ser Val Gly Asp
   1 5 10 15
   Arg Val Thr Ile Asn Cys Arg Ala Ser Gln Ser Val Tyr Tyr Asn Asn
   20 25 30
   Tyr Leu Ala Trp Tyr Gln Gln Lys Pro Gly Lys Val Pro Lys Gln Leu
   35 40 45
   Ile Tyr Ser Thr Ser Thr Leu Ala Ser Gly Val Pro Ser Arg Phe Ser
   50 55 60
   Gly Ser Gly Ser Gly Thr Asp Phe Thr Leu Thr Ile Ser Ser Leu Gln
   65 70 75 80
   Pro Glu Asp Val Ala Thr Tyr Tyr Cys Leu Gly Ser Tyr Asp Cys Ser
   85 90 95
   Asn Gly Asp Cys Phe Val Phe Gly Gly Gly Thr Lys Val Glu Ile Lys
   100 105 110
   Arg
<210> 112
   <211> 219
   <212> PRT
   <213> Artificial Sequence
<220>
   <223> Description of Artificial Sequence: Synthetic polypeptide
<400> 112
   Gln Val Leu Thr Gln Ser Pro Ser Ser Leu Ser Ala Ser Val Gly Asp
   1               5                   10                  15
   Arg Val Thr Ile Asn Cys Arg Ala Ser Gln Ser Val Tyr Tyr Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Asn Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 113
   <211> 111
   <212> PRT
   <213> Artificial Sequence
<220>
   <223> Description of Artificial Sequence: Synthetic polypeptide
<400> 113
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Val Thr Asn Tyr
               20                  25                  30
   Tyr Met Gln Trp Val Arg Gln Ala Pro Gly Lys Gly Leu Glu Trp Val
           35                  40                  45
   Gly Val Ile Gly Val Asn Gly Lys Arg Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser
               100                 105                 110
<210> 114
   <211> 441
   <212> PRT
   <213> Artificial Sequence
<220>
   <223> Description of Artificial Sequence: Synthetic polypeptide
<400> 114
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Asp Val Thr Asn Tyr
               20                  25                  30
   Tyr Met Gln Trp Val Arg Gln Ala Pro Gly Lys Gly Leu Glu Trp Val
           35                  40                  45
   Gly Val Ile Gly Val Asn Gly Lys Arg Tyr Tyr Ala Ser Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Ala
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
           435                 440
<210> 115
   <211> 13
   <212> PRT
   <213> Oryctolagus cuniculus
<400> 115
   Arg Ala Ser Gln Ser Val Tyr Tyr Asn Asn Tyr Leu Ala
   1               5                   10
<210> 116
   <211> 7
   <212> PRT
   <213> Oryctolagus cuniculus
<400> 116
   Ser Thr Ser Thr Leu Ala Ser
   1 5
<210> 117
   <211> 13
   <212> PRT
   <213> Oryctolagus cuniculus
<400> 117
   Leu Gly Ser Tyr Asp Cys Ser Asn Gly Asp Cys Phe Val
   1 5 10
<210> 118
   <211> 5
   <212> PRT
   <213> Oryctolagus cuniculus
<400> 118
   Asn Tyr Tyr Met Gln
   1 5
<210> 119
   <211> 16
   <212> PRT
   <213> Oryctolagus cuniculus
<400> 119
   Val Ile Gly Val Asn Gly Lys Arg Tyr Tyr Ala Ser Trp Ala Lys Gly
   1 5 10 15
<210> 120
   <211> 3
   <212> PRT
   <213> Oryctolagus cuniculus
<400> 120
   Gly Asp Ile
   1
<210> 121
   <211> 113
   <212> PRT
   <213> Artificial Sequence
<220>
   <223> Description of Artificial Sequence: Synthetic polypeptide
<400> 121
   Ala Ile Val Met Thr Gln Thr Pro Ser Ser Lys Ser Val Pro Val Gly
   1               5                   10                  15
   Asp Thr Val Thr Ile Asn Cys Gln Ala Ser Glu Ser Leu Tyr Asn Asn
               20                  25                  30
   Asn Ala Leu Ala Trp Phe Gln Gln Lys Pro Gly Gln Pro Pro Lys Arg
           35                  40                  45
   Leu Ile Tyr Asp Ala Ser Lys Leu Ala Ser Gly Val Pro Ser Arg Phe
       50                  55                  60
   Ser Gly Gly Gly Ser Gly Thr Gln Phe Thr Leu Thr Ile Ser Gly Val
   65                  70                  75                  80
   Gln Cys Asp Asp Ala Ala Thr Tyr Tyr Cys Gly Gly Tyr Arg Ser Asp
                   85                  90                  95
   Ser Val Asp Gly Val Ala Phe Ala Gly Gly Thr Glu Val Val Val Lys
               100                 105                 110
   Arg
<210> 122
   <211> 219
   <212> PRT
   <213> Artificial Sequence
<220>
   <223> Description of Artificial Sequence: Synthetic polypeptide
<400> 122
   Ala Ile Val Met Thr Gln Thr Pro Ser Ser Lys Ser Val Pro Val Gly
   1               5                   10                  15
   Asp Thr Val Thr Ile Asn Cys Gln Ala Ser Glu Ser Leu Tyr Asn Asn
               20                  25                  30
   Asn Ala Leu Ala Trp Phe Gln Gln Lys Pro Gly Gln Pro Pro Lys Arg
           35                  40                  45
   Leu Ile Tyr Asp Ala Ser Lys Leu Ala Ser Gly Val Pro Ser Arg Phe
       50                  55                  60
   Ser Gly Gly Gly Ser Gly Thr Gln Phe Thr Leu Thr Ile Ser Gly Val
   65                  70                  75                  80
   Gln Cys Asp Asp Ala Ala Thr Tyr Tyr Cys Gly Gly Tyr Arg Ser Asp
                   85                  90                  95
   Ser Val Asp Gly Val Ala Phe Ala Gly Gly Thr Glu Val Val Val Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 123
   <211> 111
   <212> PRT
   <213> Artificial Sequence
<220>
   <223> Description of Artificial Sequence: Synthetic polypeptide
<400> 123
   Gln Ser Val Glu Glu Ser Gly Gly Gly Leu Val Gln Pro Glu Gly Ser
   1               5                   10                  15
   Leu Thr Leu Thr Cys Thr Ala Ser Gly Phe Asp Phe Ser Ser Asn Ala
               20                  25                  30
   Met Trp Trp Val Arg Gln Ala Pro Gly Lys Gly Leu Glu Trp Ile Gly
           35                  40                  45
   Cys Ile Tyr Asn Gly Asp Gly Ser Thr Tyr Tyr Ala Ser Trp Val Asn
       50                  55                  60
   Gly Arg Phe Ser Ile Ser Lys Thr Ser Ser Thr Thr Val Thr Leu Gln
   65                  70                  75                  80
   Leu Asn Ser Leu Thr Val Ala Asp Thr Ala Thr Tyr Tyr Cys Ala Arg
                   85                  90                  95
   Asp Leu Asp Leu Trp Gly Pro Gly Thr Leu Val Thr Val Ser Ser
               100                 105                 110
<210> 124
   <211> 441
   <212> PRT
   <213> Artificial Sequence
<220>
   <223> Description of Artificial Sequence: Synthetic polypeptide
<400> 124
   Gln Ser Val Glu Glu Ser Gly Gly Gly Leu Val Gln Pro Glu Gly Ser
   1               5                   10                  15
   Leu Thr Leu Thr Cys Thr Ala Ser Gly Phe Asp Phe Ser Ser Asn Ala
               20                  25                  30
   Met Trp Trp Val Arg Gln Ala Pro Gly Lys Gly Leu Glu Trp Ile Gly
           35                  40                  45
   Cys Ile Tyr Asn Gly Asp Gly Ser Thr Tyr Tyr Ala Ser Trp Val Asn
       50                  55                  60
   Gly Arg Phe Ser Ile Ser Lys Thr Ser Ser Thr Thr Val Thr Leu Gln
   65                  70                  75                  80
   Leu Asn Ser Leu Thr Val Ala Asp Thr Ala Thr Tyr Tyr Cys Ala Arg
                   85                  90                  95
   Asp Leu Asp Leu Trp Gly Pro Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385                 390                 395                 400
   Tyr Ser Lys Leu Thr Val Asp Lys Ser Arg Trp Gln Gln Gly Asn Val
                   405                 410                 415
   Phe Ser Cys Ser Val Met His Glu Ala Leu His Asn His Tyr Thr Gln
               420                 425                 430
   Lys Ser Leu Ser Leu Ser Pro Gly Lys
   435 440
<210> 125
   <211> 13
   <212> PRT
   <213> Oryctolagus cuniculus
<400> 125
   Gln Ala Ser Glu Ser Leu Tyr Asn Asn Asn Ala Leu Ala
   1 5 10
<210> 126
   <211> 7
   <212> PRT
   <213> Oryctolagus cuniculus
<400> 126
   Asp Ala Ser Lys Leu Ala Ser
   1 5
<210> 127
   <211> 12
   <212> **PRT**
   <213> Oryctolagus cuniculus
<400> 127
   Gly Gly Tyr Arg Ser Asp Ser Val Asp Gly Val Ala
   1 5 10
<210> 128
   <211> 5
   <212> PRT
   <213> Oryctolagus cuniculus
<400> 128
   Ser Asn Ala Met Trp
   1 5
<210> 129
   <211> 17
   <212> PRT
   <213> Oryctolagus cuniculus
<400> 129
   Cys Ile Tyr Asn Gly Asp Gly Ser Thr Tyr Tyr Ala Ser Trp Val Asn
   1 5 10 15
   Gly
<210> 130
   <211> 4
   <212> PRT
   <213> Oryctolagus cuniculus
<400> 130
   Asp Leu Asp Leu
   1
<210> 131
   <211> 113
   <212> PRT
   <213> Artificial Sequence
<220>
   <223> Description of Artificial Sequence: Synthetic polypeptide
<400> 131
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Asn Val Tyr Asn Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Arg Gly Asp Cys Phe Val Phe Gly Gly Gly Thr Lys Val Glu Ile Lys
               100                 105                 110
   Arg
<210> 132
   <211> 219
   <212> PRT
   <213> Artificial Sequence
<220>
   <223> Description of Artificial Sequence: Synthetic polypeptide
<400> 132
   Gln Val Leu Thr Gln Ser Pro Ser Ser Leu Ser Ala Ser Val Gly Asp
   1               5                   10                  15
   Arg Val Thr Ile Asn Cys Gln Ala Ser Gln Asn Val Tyr Asn Asn Asn
               20                  25                  30
   Tyr Leu Ala Trp Tyr Gln Gln Lys Pro Gly Lys Val Pro Lys Gln Leu
           35                  40                  45
   Ile Tyr Ser Thr Ser Thr Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Val Ala Thr Tyr Tyr Cys Leu Gly Ser Tyr Asp Cys Ser
                   85                  90                  95
   Arg Gly Asp Cys Phe Val Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 133
   <211> 111
   <212> PRT
   <213> Artificial Sequence
<220>
   <223> Description of Artificial Sequence: Synthetic polypeptide
<400> 133
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Gly Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ser Asp Gly Lys Thr Tyr Tyr Ala Thr Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Thr
                   85                  90                  95
   Arg Gly Asp Ile Trp Gly Gln Gly Thr Leu Val Thr Val Ser Ser
               100                 105                 110
<210> 134
   <211> 441
   <212> PRT
   <213> Artificial Sequence
<220>
   <223> Description of Artificial Sequence: Synthetic polypeptide
<400> 134
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Gly Leu Ser Ser Tyr
               20                  25                  30
   Tyr Met Gln Trp Val Arg Gln Ala Pro Gly Lys Gly Leu Glu Trp Val
           35                  40                  45
   Gly Val Ile Gly Ser Asp Gly Lys Thr Tyr Tyr Ala Thr Trp Ala Lys
       50                  55                  60
   Gly Arg Phe Thr Ile Ser Arg Asp Asn Ser Lys Thr Thr Val Tyr Leu
   65                  70                  75                  80
   Gln Met Asn Ser Leu Arg Ala Glu Asp Thr Ala Val Tyr Phe Cys Thr
                   85                  90                  95
   Arg Gly Asp Ile Trp Gly Gln Gly Thr Leu Val Thr Val Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Ala Arg
           195                 200                 205
   Val Glu Pro Lys Ser Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Ala Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Glu Glu Met
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro Gly Lys
   435 440
<210> 135
   <211> 13
   <212> PRT
   <213> Oryctolagus cuniculus
<400> 135
   Gln Ala Ser Gln Asn Val Tyr Asn Asn Asn Tyr Leu Ala
   1 5 10
<210> 136
   <211> 7
   <212> PRT
   <213> Oryctolagus cuniculus
<400> 136
   Ser Thr Ser Thr Leu Ala Ser
   1 5
<210> 137
   <211> 13
   <212> PRT
   <213> Oryctolagus cuniculus
<400> 137
   Leu Gly Ser Tyr Asp Cys Ser Arg Gly Asp Cys Phe Val
   1 5 10
<210> 138
   <211> 5
   <212> PRT
   <213> Oryctolagus cuniculus
<400> 138
   Ser Tyr Tyr Met Gln
   1 5
<210> 139
   <211> 16
   <212> PRT
   <213> Oryctolagus cuniculus
<400> 139
   Val Ile Gly Ser Asp Gly Lys Thr Tyr Tyr Ala Thr Trp Ala Lys Gly
   1               5                   10                  15
<210> 140
   <211> 3
   <212> PRT
   <213> Oryctolagus cuniculus
<400> 140
   Gly Asp Ile
   1
<210> 141
   <211> 339
   <212> DNA
   <213> Artificial Sequence
<220>
   <223> Description of Artificial Sequence: Synthetic polynucleotide
<400> 141
   caagtgctga cccagactgc atcccccgtg tctgcagctg tgggaagcac agtcaccatc 60
   aattgccagg ccagtcagag tgtttatgat aacaactacc tagcctggta tcagcagaaa 120
   ccagggcagc ctcccaagca actgatctat tctacatcca ctctggcatc tggggtctca 180
   tcgcggttca aaggcagtgg atctgggaca cagttcactc tcaccatcag cgacctggag 240
   tgtgccgatg ctgccactta ctactgtcta ggcagttatg attgtagtag tggtgattgt 300
   tttgttttcg gcggagggac cgaggtggtg gtcaaacgt 339
<210> 142
   <211> 660
   <212> DNA
   <213> Artificial Sequence
<220>
   <223> Description of Artificial Sequence: Synthetic polynucleotide
<400> 142
   caagtgctga cccagactgc atcccccgtg tctgcagctg tgggaagcac agtcaccatc 60
   aattgccagg ccagtcagag tgtttatgat aacaactacc tagcctggta tcagcagaaa 120
   ccagggcagc ctcccaagca actgatctat tctacatcca ctctggcatc tggggtctca 180
   tcgcggttca aaggcagtgg atctgggaca cagttcactc tcaccatcag cgacctggag 240
   tgtgccgatg ctgccactta ctactgtcta ggcagttatg attgtagtag tggtgattgt 300
   tttgttttcg gcggagggac cgaggtggtg gtcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 143
   <211> 327
   <212> DNA
   <213> Artificial Sequence
<220>
   <223> Description of Artificial Sequence: Synthetic polynucleotide
<400> 143
   cagtcgctgg aggagtccgg gggtcgcctg gtcacgcctg ggacacccct gacactcacc 60
   tgcacagtct ctggactcga cctcagtagc tactacatgc aatgggtccg ccaggctcca 120
   gggaaggggc tggaatggat cggagtcatt ggtattaatg ataacacata ctacgcgagc 180
   tgggcgaaag gccgattcac catctccaga gcctcgtcga ccacggtgga tctgaaaatg 240
   accagtctga caaccgagga cacggccacc tatttctgtg ccagagggga catctggggc 300
   ccaggcaccc tcgtcaccgt ctcgagc 327
<210> 144
   <211> 1320
   <212> DNA
   <213> Artificial Sequence
<220>
   <223> Description of Artificial Sequence: Synthetic polynucleotide
<400> 144
   cagtcgctgg aggagtccgg gggtcgcctg gtcacgcctg ggacacccct gacactcacc        60
   tgcacagtct ctggactcga cctcagtagc tactacatgc aatgggtccg ccaggctcca       120
   gggaaggggc tggaatggat cggagtcatt ggtattaatg ataacacata ctacgcgagc       180
   tgggcgaaag gccgattcac catctccaga gcctcgtcga ccacggtgga tctgaaaatg       240
   accagtctga caaccgagga cacggccacc tatttctgtg ccagagggga catctggggc       300
   ccaggcaccc tcgtcaccgt ctcgagcgcc tccaccaagg gcccatcggt cttccccctg       360
   gcaccctcct ccaagagcac ctctgggggc acagcggccc tgggctgcct ggtcaaggac       420
   tacttccccg aaccggtgac ggtgtcgtgg aactcaggcg ccctgaccag cggcgtgcac       480
   accttcccgg ctgtcctaca gtcctcagga ctctactccc tcagcagcgt ggtgaccgtg       540
   ccctccagca gcttgggcac ccagacctac atctgcaacg tgaatcacaa gcccagcaac       600
   accaaggtgg acaagagagt tgagcccaaa tcttgtgaca aaactcacac atgcccaccg       660
   tgcccagcac ctgaactcct ggggggaccg tcagtcttcc tcttcccccc aaaacccaag       720
   gacaccctca tgatctcccg gacccctgag gtcacatgcg tggtggtgga cgtgagccac       780
   gaagaccctg aggtcaagtt caactggtac gtggacggcg tggaggtgca taatgccaag       840
   acaaagccgc gggaggagca gtacgccagc acgtaccgtg tggtcagcgt cctcaccgtc       900
   ctgcaccagg actggctgaa tggcaaggag tacaagtgca aggtctccaa caaagccctc       960
   ccagccccca tcgagaaaac catctccaaa gccaaagggc agccccgaga accacaggtg      1020
   tacaccctgc ccccatcccg ggaggagatg accaagaacc aggtcagcct gacctgcctg      1080
   gtcaaaggct tctatcccag cgacatcgcc gtggagtggg agagcaatgg gcagccggag      1140
   aacaactaca agaccacgcc tcccgtgctg gactccgacg gctccttctt cctctacagc      1200
   aagctcaccg tggacaagag caggtggcag caggggaacg tcttctcatg ctccgtgatg      1260
   catgaggctc tgcacaacca ctacacgcag aagagcctct ccctgtctcc gggtaaatga      1320
<210> 145
   <211> 39
   <212> DNA
   <213> Oryctolagus cuniculus
<400> 145
   caggccagtc agagtgttta tgataacaac tacctagcc 39
<210> 146
   <211> 21
   <212> DNA
   <213> Oryctolagus cuniculus
<400> 146
   tctacatcca ctctggcatc t 21
<210> 147
   <211> 39
   <212> DNA
   <213> Oryctolagus cuniculus
<400> 147
   ctaggcagtt atgattgtag tagtggtgat tgttttgtt 39
<210> 148
   <211> 15
   <212> DNA
   <213> Oryctolagus cuniculus
<400> 148
   agctactaca tgcaa 15
<210> 149
   <211> 48
   <212> DNA
   <213> Oryctolagus cuniculus
<400> 149
   gtcattggta ttaatgataa cacatactac gcgagctggg cgaaaggc 48
<210> 150
   <211> 9
   <212> DNA
   <213> Oryctolagus cuniculus
<400> 150
   ggggacatc 9
<210> 151
   <211> 339
   <212> DNA
   <213> Artificial Sequence
<220>
   <223> Description of Artificial Sequence: Synthetic polynucleotide
<400> 151
   caagtgctga cccagtctcc atcctccctg tctgcatctg taggagacag agtcaccatc 60
   aattgccagg ccagtcagag tgtttatgat aacaactacc tagcctggta tcagcagaaa 120
   ccagggaaag ttcctaagca actgatctat tctacatcca ctctggcatc tggggtccca 180
   tctcgtttca gtggcagtgg atctgggaca gatttcactc tcaccatcag cagcctgcag 240
   cctgaagatg ttgcaactta ttactgtcta ggcagttatg attgtagtag tggtgattgt 300
   tttgttttcg gcggaggaac caaggtggaa atcaaacgt 339
<210> 152
   <211> 660
   <212> DNA
   <213> Artificial Sequence
<220>
   <223> Description of Artificial Sequence: Synthetic polynucleotide
<400> 152
   caagtgctga cccagtctcc atcctccctg tctgcatctg taggagacag agtcaccatc        60
   aattgccagg ccagtcagag tgtttatgat aacaactacc tagcctggta tcagcagaaa       120
   ccagggaaag ttcctaagca actgatctat tctacatcca ctctggcatc tggggtccca       180
   tctcgtttca gtggcagtgg atctgggaca gatttcactc tcaccatcag cagcctgcag       240
   cctgaagatg ttgcaactta ttactgtcta ggcagttatg attgtagtag tggtgattgt       300
   tttgttttcg gcggaggaac caaggtggaa atcaaacgta cggtggctgc accatctgtc       360
   ttcatcttcc cgccatctga tgagcagttg aaatctggaa ctgcctctgt tgtgtgcctg       420
   ctgaataact tctatcccag agaggccaaa gtacagtgga aggtggataa cgccctccaa       480
   tcgggtaact cccaggagag tgtcacagag caggacagca aggacagcac ctacagcctc       540
   agcagcaccc tgacgctgag caaagcagac tacgagaaac acaaagtcta cgcctgcgaa       600
   gtcacccatc agggcctgag ctcgcccgtc acaaagagct tcaacagggg agagtgttag       660
<210> 153
   <211> 333
   <212> DNA
   <213> Artificial Sequence
<220>
   <223> Description of Artificial Sequence: Synthetic polynucleotide
<400> 153
   gaggtgcagc ttgtggagtc tgggggaggc ttggtccagc ctggggggtc cctgagactc        60
   tcctgtgcag tctctggact cgacctcagt agctactaca tgcaatgggt ccgtcaggct       120
   ccagggaagg ggctggagtg ggtcggagtc attggtatca atgataacac atactacgcg       180
   agctgggcga aaggccgatt caccatctcc agagacaatt ccaagaccac ggtgtatctt       240
   caaatgaaca gcctgagagc tgaggacact gctgtgtatt tctgtgctag aggggacatc       300
   tggggccaag ggaccctcgt caccgtctcg agc                                    333
<210> 154
   <211> 1326
   <212> DNA
   <213> Artificial Sequence
<220>
   <223> Description of Artificial Sequence: Synthetic polynucleotide
<400> 154
   gaggtgcagc ttgtggagtc tgggggaggc ttggtccagc ctggggggtc cctgagactc      60
   tcctgtgcag tctctggact cgacctcagt agctactaca tgcaatgggt ccgtcaggct     120
   ccagggaagg ggctggagtg ggtcggagtc attggtatca atgataacac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacaa gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                               1326
<210> 155
   <211> 39
   <212> DNA
   <213> Oryctolagus cuniculus
<400> 155
   caggccagtc agagtgttta tgataacaac tacctagcc 39
<210> 156
   <211> 21
   <212> DNA
   <213> Oryctolagus cuniculus
<400> 156
   tctacatcca ctctggcatc t 21
<210> 157
   <211> 39
   <212> DNA
   <213> Oryctolagus cuniculus
<400> 157
   ctaggcagtt atgattgtag tagtggtgat tgttttgtt 39
<210> 158
   <211> 15
   <212> DNA
   <213> Oryctolagus cuniculus
<400> 158
   agctactaca tgcaa 15
<210> 159
   <211> 48
   <212> DNA
   <213> Oryctolagus cuniculus
<400> 159
   gtcattggta tcaatgataa cacatactac gcgagctggg cgaaaggc 48
<210> 160
   <211> 9
   <212> DNA
   <213> Oryctolagus cuniculus
<400> 160
   ggggacatc 9
<210> 161
   <211> 339
   <212> DNA
   <213> Artificial Sequence
<220>
   <223> Description of Artificial Sequence: Synthetic polynucleotide
<400> 161
   caagtgctga cccagtctcc atcctccctg tctgcatctg taggagacag agtcaccatc    60
   aattgccagg ccagtcagag tgtttatgat aacaactacc tagcctggta tcagcagaaa   120
   ccagggaaag ttcctaagca actgatctat tctacatcca ctctggcatc tggggtccca   180
   tctcgtttca gtggcagtgg atctgggaca gatttcactc tcaccatcag cagcctgcag   240
   cctgaagatg ttgcaactta ttactgtcta ggcagttatg attgtagtag tggtgattgt   300
   tttgttttcg gcggaggaac caaggtggaa atcaaacgt                          339
<210> 162
   <211> 660
   <212> DNA
   <213> Artificial Sequence
<220>
   <223> Description of Artificial Sequence: Synthetic polynucleotide
<400> 162
   caagtgctga cccagtctcc atcctccctg tctgcatctg taggagacag agtcaccatc      60
   aattgccagg ccagtcagag tgtttatgat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtcta ggcagttatg attgtagtag tggtgattgt     300
   tttgttttcg gcggaggaac caaggtggaa atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 163
   <211> 333
   <212> DNA
   <213> Artificial Sequence
<220>
   <223> Description of Artificial Sequence: Synthetic polynucleotide
<400> 163
   gaggtgcagc ttgtggagtc tgggggaggc ttggtccagc ctggggggtc cctgagactc      60
   tcctgtgcag tctctggact cgacctcagt agctactaca tgcaatgggt ccgtcaggct     120
   ccagggaagg ggctggagtg ggtcggagtc attggtatca atgataacac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agc                                  333
<210> 164
   <211> 1326
   <212> DNA
   <213> Artificial Sequence
<220>
   <223> Description of Artificial Sequence: Synthetic polynucleotide
<400> 164
   gaggtgcagc ttgtggagtc tgggggaggc ttggtccagc ctggggggtc cctgagactc      60
   tcctgtgcag tctctggact cgacctcagt agctactaca tgcaatgggt ccgtcaggct     120
   ccagggaagg ggctggagtg ggtcggagtc attggtatca atgataacac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacgc gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                              1326
<210> 165
   <211> 39
   <212> DNA
   <213> Oryctolagus cuniculus
<400> 165
   caggccagtc agagtgttta tgataacaac tacctagcc 39
<210> 166
   <211> 21
   <212> DNA
   <213> Oryctolagus cuniculus
<400> 166
   tctacatcca ctctggcatc t 21
<210> 167
   <211> 39
   <212> DNA
   <213> Oryctolagus cuniculus
<400> 167
   ctaggcagtt atgattgtag tagtggtgat tgttttgtt 39
<210> 168
   <211> 15
   <212> DNA
   <213> Oryctolagus cuniculus
<400> 168
   agctactaca tgcaa 15
<210> 169
   <211> 48
   <212> DNA
   <213> Oryctolagus cuniculus
<400> 169
   gtcattggta tcaatgataa cacatactac gcgagctggg cgaaaggc 48
<210> 170
   <211> 9
   <212> DNA
   <213> Oryctolagus cuniculus
<400> 170
   ggggacatc 9
<210> 171
   <211> 339
   <212> DNA
   <213> Artificial Sequence
<220>
   <223> Description of Artificial Sequence: Synthetic polynucleotide
<400> 171
   caagtgctga cccagactcc atcccccgtg tctgcagctg tgggaagcac agtcaccatc 60
   aattgccagg ccagtcagag tgtttatcat aacacctacc tggcctggta tcagcagaaa 120
   ccagggcagc ctcccaaaca actgatctat gatgcatcca ctctggcgtc tggggtccca 180
   tcgcggttca gcggcagtgg atctgggaca cagttcactc tcaccatcag cggcgtgcag 240
   tgtaacgatg ctgccgctta ctactgtctg ggcagttatg attgtactaa tggtgattgt 300
   tttgttttcg gcggagggac cgaggtggtg gtcaaacgt 339
<210> 172
   <211> 660
   <212> DNA
   <213> Artificial Sequence
<220>
   <223> Description of Artificial Sequence: Synthetic polynucleotide
<400> 172
   caagtgctga cccagactcc atcccccgtg tctgcagctg tgggaagcac agtcaccatc 60
   aattgccagg ccagtcagag tgtttatcat aacacctacc tggcctggta tcagcagaaa 120
   ccagggcagc ctcccaaaca actgatctat gatgcatcca ctctggcgtc tggggtccca 180
   tcgcggttca gcggcagtgg atctgggaca cagttcactc tcaccatcag cggcgtgcag 240
   tgtaacgatg ctgccgctta ctactgtctg ggcagttatg attgtactaa tggtgattgt 300
   tttgttttcg gcggagggac cgaggtggtg gtcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 173
   <211> 327
   <212> DNA
   <213> Artificial Sequence
<220>
   <223> Description of Artificial Sequence: Synthetic polynucleotide
<400> 173
   cagtcgctgg aggagtccgg gggtcgcctg gtcacgcctg ggacacccct gacactcacc        60
   tgttccgtct ctggcatcga cctcagtggc tactacatga actgggtccg ccaggctcca       120
   gggaaggggc tggaatggat cggagtcatt ggtattaatg gtgccacata ctacgcgagc       180
   tgggcgaaag gccgattcac catctccaaa acctcgtcga ccacggtgga tctgaaaatg       240
   accagtctga caaccgagga cacggccacc tatttctgtg ccagagggga catctggggc       300
   ccgggcaccc tcgtcaccgt ctcgagc                                           327
<210> 174
   <211> 1320
   <212> DNA
   <213> Artificial Sequence
<220>
   <223> Description of Artificial Sequence: Synthetic polynucleotide
<400> 174
   cagtcgctgg aggagtccgg gggtcgcctg gtcacgcctg ggacacccct gacactcacc        60
   tgttccgtct ctggcatcga cctcagtggc tactacatga actgggtccg ccaggctcca       120
   gggaaggggc tggaatggat cggagtcatt ggtattaatg gtgccacata ctacgcgagc       180
   tgggcgaaag gccgattcac catctccaaa acctcgtcga ccacggtgga tctgaaaatg       240
   accagtctga caaccgagga cacggccacc tatttctgtg ccagagggga catctggggc       300
   ccgggcaccc tcgtcaccgt ctcgagcgcc tccaccaagg gcccatcggt cttccccctg       360
   gcaccctcct ccaagagcac ctctgggggc acagcggccc tgggctgcct ggtcaaggac       420
   tacttccccg aaccggtgac ggtgtcgtgg aactcaggcg ccctgaccag cggcgtgcac       480
   accttcccgg ctgtcctaca gtcctcagga ctctactccc tcagcagcgt ggtgaccgtg       540
   ccctccagca gcttgggcac ccagacctac atctgcaacg tgaatcacaa gcccagcaac 600
   accaaggtgg acaagagagt tgagcccaaa tcttgtgaca aaactcacac atgcccaccg 660
   tgcccagcac ctgaactcct ggggggaccg tcagtcttcc tcttcccccc aaaacccaag 720
   gacaccctca tgatctcccg gacccctgag gtcacatgcg tggtggtgga cgtgagccac 780
   gaagaccctg aggtcaagtt caactggtac gtggacggcg tggaggtgca taatgccaag 840
   acaaagccgc gggaggagca gtacgccagc acgtaccgtg tggtcagcgt cctcaccgtc 900
   ctgcaccagg actggctgaa tggcaaggag tacaagtgca aggtctccaa caaagccctc 960
   ccagccccca tcgagaaaac catctccaaa gccaaagggc agccccgaga accacaggtg 1020
   tacaccctgc ccccatcccg ggaggagatg accaagaacc aggtcagcct gacctgcctg 1080
   gtcaaaggct tctatcccag cgacatcgcc gtggagtggg agagcaatgg gcagccggag 1140
   aacaactaca agaccacgcc tcccgtgctg gactccgacg gctccttctt cctctacagc 1200
   aagctcaccg tggacaagag caggtggcag caggggaacg tcttctcatg ctccgtgatg 1260
   catgaggctc tgcacaacca ctacacgcag aagagcctct ccctgtctcc gggtaaatga 1320
<210> 175
   <211> 39
   <212> DNA
   <213> Oryctolagus cuniculus
<400> 175
   caggccagtc agagtgttta tcataacacc tacctggcc 39
<210> 176
   <211> 21
   <212> DNA
   <213> Oryctolagus cuniculus
<400> 176
   gatgcatcca ctctggcgtc t 21
<210> 177
   <211> 39
   <212> DNA
   <213> Oryctolagus cuniculus
<400> 177
   ctgggcagtt atgattgtac taatggtgat tgttttgtt 39
<210> 178
   <211> 15
   <212> DNA
   <213> Oryctolagus cuniculus
<400> 178
   ggctactaca tgaac 15
<210> 179
   <211> 48
   <212> DNA
   <213> Oryctolagus cuniculus
<400> 179
   gtcattggta ttaatggtgc cacatactac gcgagctggg cgaaaggc 48
<210> 180
   <211> 9
   <212> DNA
   <213> Oryctolagus cuniculus
<400> 180
   ggggacatc 9
<210> 181
   <211> 339
   <212> DNA
   <213> Artificial Sequence
<220>
   <223> Description of Artificial Sequence: Synthetic polynucleotide
<400> 181
   caagtgctga cccagtctcc atcctccctg tctgcatctg taggagacag agtcaccatc 60
   aattgccagg ccagtcagag tgtttatcat aacacctacc tggcctggta tcagcagaaa 120
   ccagggaaag ttcctaagca actgatctat gatgcatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtactaa tggtgattgt 300
   tttgttttcg gcggaggaac caaggtggaa atcaaacgt 339
<210> 182
   <211> 660
   <212> DNA
   <213> Artificial Sequence
<220>
   <223> Description of Artificial Sequence: Synthetic polynucleotide
<400> 182
   caagtgctga cccagtctcc atcctccctg tctgcatctg taggagacag agtcaccatc 60
   aattgccagg ccagtcagag tgtttatcat aacacctacc tggcctggta tcagcagaaa 120
   ccagggaaag ttcctaagca actgatctat gatgcatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtactaa tggtgattgt 300
   tttgttttcg gcggaggaac caaggtggaa atcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 183
   <211> 333
   <212> DNA
   <213> Artificial Sequence
<220>
   <223> Description of Artificial Sequence: Synthetic polynucleotide
<400> 183
   gaggtgcagc ttgtggagtc tgggggaggc ttggtccagc ctggggggtc cctgagactc 60
   tcctgtgcag tctctggaat cgacctcagt ggctactaca tgaactgggt ccgtcaggct 120
   ccagggaagg ggctggagtg ggtcggagtc attggtatta atggtgccac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agc 333
<210> 184
   <211> 1326
   <212> DNA
   <213> Artificial Sequence
<220>
   <223> Description of Artificial Sequence: Synthetic polynucleotide
<400> 184
   gaggtgcagc ttgtggagtc tgggggaggc ttggtccagc ctggggggtc cctgagactc 60
   tcctgtgcag tctctggaat cgacctcagt ggctactaca tgaactgggt ccgtcaggct 120
   ccagggaagg ggctggagtg ggtcggagtc attggtatta atggtgccac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agcgcctcca ccaagggccc atcggtcttc 360
   cccctggcac cctcctccaa gagcacctct gggggcacag cggccctggg ctgcctggtc 420
   aaggactact tccccgaacc ggtgacggtg tcgtggaact caggcgccct gaccagcggc 480
   gtgcacacct tcccggctgt cctacagtcc tcaggactct actccctcag cagcgtggtg 540
   accgtgccct ccagcagctt gggcacccag acctacatct gcaacgtgaa tcacaagccc 600
   agcaacacca aggtggacaa gagagttgag cccaaatctt gtgacaaaac tcacacatgc 660
   ccaccgtgcc cagcacctga actcctgggg ggaccgtcag tcttcctctt ccccccaaaa 720
   cccaaggaca ccctcatgat ctcccggacc cctgaggtca catgcgtggt ggtggacgtg 780
   agccacgaag accctgaggt caagttcaac tggtacgtgg acggcgtgga ggtgcataat 840
   gccaagacaa agccgcggga ggagcagtac gccagcacgt accgtgtggt cagcgtcctc 900
   accgtcctgc accaggactg gctgaatggc aaggagtaca agtgcaaggt ctccaacaaa 960
   gccctcccag cccccatcga gaaaaccatc tccaaagcca aagggcagcc ccgagaacca 1020
   caggtgtaca ccctgccccc atcccgggag gagatgacca agaaccaggt cagcctgacc 1080
   tgcctggtca aaggcttcta tcccagcgac atcgccgtgg agtgggagag caatgggcag 1140
   ccggagaaca actacaagac cacgcctccc gtgctggact ccgacggctc cttcttcctc 1200
   tacagcaagc tcaccgtgga caagagcagg tggcagcagg ggaacgtctt ctcatgctcc 1260
   gtgatgcatg aggctctgca caaccactac acgcagaaga gcctctccct gtctccgggt 1320
   aaatga 1326
<210> 185
   <211> 39
   <212> DNA
   <213> Oryctolagus cuniculus
<400> 185
   caggccagtc agagtgttta tcataacacc tacctggcc 39
<210> 186
   <211> 21
   <212> DNA
   <213> Oryctolagus cuniculus
<400> 186
   gatgcatcca ctctggcatc t 21
<210> 187
   <211> 39
   <212> DNA
   <213> Oryctolagus cuniculus
<400> 187
   ctgggcagtt atgattgtac taatggtgat tgttttgtt 39
<210> 188
   <211> 15
   <212> DNA
   <213> Oryctolagus cuniculus
<400> 188
   ggctactaca tgaac 15
<210> 189
   <211> 48
   <212> DNA
   <213> Oryctolagus cuniculus
<400> 189
   gtcattggta ttaatggtgc cacatactac gcgagctggg cgaaaggc 48
<210> 190
   <211> 9
   <212> DNA
   <213> Oryctolagus cuniculus
<400> 190
   ggggacatc 9
<210> 191
   <211> 339
   <212> DNA
   <213> Artificial Sequence
<220>
   <223> Description of Artificial Sequence: Synthetic polynucleotide
<400> 191
   caagtgctga cccagtctcc atcctccctg tctgcatctg taggagacag agtcaccatc      60
   aattgccagg ccagtcagag tgtttatcat aacacctacc tggcctggta tcagcagaaa     120
   ccagggaaag ttcctaagca actgatctat gatgcatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtactaa tggtgattgt     300
   tttgttttcg gcggaggaac caaggtggaa atcaaacgt                            339
<210> 192
   <211> 660
   <212> DNA
   <213> Artificial Sequence
<220>
   <223> Description of Artificial Sequence: Synthetic polynucleotide
<400> 192
   caagtgctga cccagtctcc atcctccctg tctgcatctg taggagacag agtcaccatc 60
   aattgccagg ccagtcagag tgtttatcat aacacctacc tggcctggta tcagcagaaa 120
   ccagggaaag ttcctaagca actgatctat gatgcatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtactaa tggtgattgt 300
   tttgttttcg gcggaggaac caaggtggaa atcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 193
   <211> 333
   <212> DNA
   <213> Artificial Sequence
<220>
   <223> Description of Artificial Sequence: Synthetic polynucleotide
<400> 193
   gaggtgcagc ttgtggagtc tgggggaggc ttggtccagc ctggggggtc cctgagactc 60
   tcctgtgcag tctctggaat cgacctcagt ggctactaca tgaactgggt ccgtcaggct 120
   ccagggaagg ggctggagtg ggtcggagtc attggtatta atggtgccac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agc 333
<210> 194
   <211> 1326
   <212> DNA
   <213> Artificial Sequence
<220>
   <223> Description of Artificial Sequence: Synthetic polynucleotide
<400> 194
   gaggtgcagc ttgtggagtc tgggggaggc ttggtccagc ctggggggtc cctgagactc      60
   tcctgtgcag tctctggaat cgacctcagt ggctactaca tgaactgggt ccgtcaggct     120
   ccagggaagg ggctggagtg ggtcggagtc attggtatta atggtgccac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacgc gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                              1326
<210> 195
   <211> 39
   <212> DNA
   <213> Oryctolagus cuniculus
<400> 195
   caggccagtc agagtgttta tcataacacc tacctggcc 39
<210> 196
   <211> 21
   <212> DNA
   <213> Oryctolagus cuniculus
<400> 196
   gatgcatcca ctctggcatc t 21
<210> 197
   <211> 39
   <212> DNA
   <213> Oryctolagus cuniculus
<400> 197
   ctgggcagtt atgattgtac taatggtgat tgttttgtt 39
<210> 198
   <211> 15
   <212> DNA
   <213> Oryctolagus cuniculus
<400> 198
   ggctactaca tgaac 15
<210> 199
   <211> 48
   <212> DNA
   <213> Oryctolagus cuniculus
<400> 199
   gtcattggta ttaatggtgc cacatactac gcgagctggg cgaaaggc 48
<210> 200
   <211> 9
   <212> DNA
   <213> Oryctolagus cuniculus
<400> 200
   ggggacatc 9
<210> 201
   <211> 339
   <212> DNA
   <213> Artificial Sequence
<220>
   <223> Description of Artificial Sequence: Synthetic polynucleotide
<400> 201
   caagtgctga cccagactgc atcccccgtg tctgcagctg tgggaagcac agtcaccatc        60
   aattgccagg ccagtcagag tgtttataat tacaactacc ttgcctggta tcagcagaaa       120
   ccagggcagc ctcccaagca actgatctat tctacatcca ctctggcatc tggggtctca       180
   tcgcgattca aaggcagtgg atctgggaca cagttcactc tcaccatcag cgacgtgcag       240
   tgtgacgatg ctgccactta ctactgtcta ggcagttatg actgtagtac tggtgattgt       300
   tttgttttcg gcggagggac cgaggtggtg gtcaaacgt                              339
<210> 202
   <211> 660
   <212> DNA
   <213> Artificial Sequence
<220>
   <223> Description of Artificial Sequence: Synthetic polynucleotide
<400> 202
   caagtgctga cccagactgc atcccccgtg tctgcagctg tgggaagcac agtcaccatc        60
   aattgccagg ccagtcagag tgtttataat tacaactacc ttgcctggta tcagcagaaa       120
   ccagggcagc ctcccaagca actgatctat tctacatcca ctctggcatc tggggtctca       180
   tcgcgattca aaggcagtgg atctgggaca cagttcactc tcaccatcag cgacgtgcag       240
   tgtgacgatg ctgccactta ctactgtcta ggcagttatg actgtagtac tggtgattgt       300
   tttgttttcg gcggagggac cgaggtggtg gtcaaacgta cggtggctgc accatctgtc       360
   ttcatcttcc cgccatctga tgagcagttg aaatctggaa ctgcctctgt tgtgtgcctg       420
   ctgaataact tctatcccag agaggccaaa gtacagtgga aggtggataa cgccctccaa       480
   tcgggtaact cccaggagag tgtcacagag caggacagca aggacagcac ctacagcctc       540
   agcagcaccc tgacgctgag caaagcagac tacgagaaac acaaagtcta cgcctgcgaa       600
   gtcacccatc agggcctgag ctcgcccgtc acaaagagct tcaacagggg agagtgttag       660
<210> 203
   <211> 330
   <212> DNA
   <213> Artificial Sequence
<220>
   <223> Description of Artificial Sequence: Synthetic polynucleotide
<400> 203
   caggagcagc tgaaggagtc cgggggtcgc ctggtcacgc ctgggacatc cctgacactc        60
   acctgcaccg tctctggaat cgacctcagt aaccactaca tgcaatgggt ccgccaggct       120
   ccagggaagg ggctggagtg gatcggagtc gttggtatta atggtcgcac atactacgcg       180
   agctgggcga aaggccgatt caccatctcc agaacctcgt cgaccacggt ggatctgaaa       240
   atgaccaggc tgacaaccga ggacacggcc acctatttct gtgccagagg ggacatctgg       300
   ggcccaggca ccctggtcac cgtctcgagc                                        330
<210> 204
   <211> 1323
   <212> DNA
   <213> Artificial Sequence
<220>
   <223> Description of Artificial Sequence: Synthetic polynucleotide
<400> 204
   caggagcagc tgaaggagtc cgggggtcgc ctggtcacgc ctgggacatc cctgacactc        60
   acctgcaccg tctctggaat cgacctcagt aaccactaca tgcaatgggt ccgccaggct       120
   ccagggaagg ggctggagtg gatcggagtc gttggtatta atggtcgcac atactacgcg       180
   agctgggcga aaggccgatt caccatctcc agaacctcgt cgaccacggt ggatctgaaa       240
   atgaccaggc tgacaaccga ggacacggcc acctatttct gtgccagagg ggacatctgg       300
   ggcccaggca ccctggtcac cgtctcgagc gcctccacca agggcccatc ggtcttcccc       360
   ctggcaccct cctccaagag cacctctggg ggcacagcgg ccctgggctg cctggtcaag       420
   gactacttcc ccgaaccggt gacggtgtcg tggaactcag gcgccctgac cagcggcgtg       480
   cacaccttcc cggctgtcct acagtcctca ggactctact ccctcagcag cgtggtgacc       540
   gtgccctcca gcagcttggg cacccagacc tacatctgca acgtgaatca caagcccagc      600
   aacaccaagg tggacaagag agttgagccc aaatcttgtg acaaaactca cacatgccca      660
   ccgtgcccag cacctgaact cctgggggga ccgtcagtct tcctcttccc cccaaaaccc      720
   aaggacaccc tcatgatctc ccggacccct gaggtcacat gcgtggtggt ggacgtgagc      780
   cacgaagacc ctgaggtcaa gttcaactgg tacgtggacg gcgtggaggt gcataatgcc      840
   aagacaaagc cgcgggagga gcagtacgcc agcacgtacc gtgtggtcag cgtcctcacc      900
   gtcctgcacc aggactggct gaatggcaag gagtacaagt gcaaggtctc caacaaagcc      960
   ctcccagccc ccatcgagaa aaccatctcc aaagccaaag ggcagccccg agaaccacag     1020
   gtgtacaccc tgcccccatc ccgggaggag atgaccaaga accaggtcag cctgacctgc     1080
   ctggtcaaag gcttctatcc cagcgacatc gccgtggagt gggagagcaa tgggcagccg     1140
   gagaacaact acaagaccac gcctcccgtg ctggactccg acggctcctt cttcctctac     1200
   agcaagctca ccgtggacaa gagcaggtgg cagcagggga acgtcttctc atgctccgtg     1260
   atgcatgagg ctctgcacaa ccactacacg cagaagagcc tctccctgtc tccgggtaaa     1320
   tga                                                                   1323
<210> 205
   <211> 39
   <212> DNA
   <213> Oryctolagus cuniculus
<400> 205
   caggccagtc agagtgttta taattacaac taccttgcc 39
<210> 206
   <211> 21
   <212> DNA
   <213> Oryctolagus cuniculus
<400> 206
   tctacatcca ctctggcatc t 21
<210> 207
   <211> 39
   <212> DNA
   <213> Oryctolagus cuniculus
<400> 207
   ctaggcagtt atgactgtag tactggtgat tgttttgtt 39
<210> 208
   <211> 15
   <212> DNA
   <213> Oryctolagus cuniculus
<400> 208
   aaccactaca tgcaa 15
<210> 209
   <211> 48
   <212> DNA
   <213> Oryctolagus cuniculus
<400> 209
   gtcgttggta ttaatggtcg cacatactac gcgagctggg cgaaaggc 48
<210> 210
   <211> 9
   <212> DNA
   <213> Oryctolagus cuniculus
<400> 210
   ggggacatc 9
<210> 211
   <211> 339
   <212> DNA
   <213> Artificial Sequence
<220>
   <223> Description of Artificial Sequence: Synthetic polynucleotide
<400> 211
   caagtgctga cccagtctcc atcctccctg tctgcatctg taggagacag agtcaccatc 60
   aattgccagg ccagtcagag tgtttacaat tacaactacc ttgcctggta tcagcagaaa 120
   ccagggaaag ttcctaagca actgatctat tctacatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtagtac tggtgattgt 300
   tttgttttcg gcggaggaac caaggtggaa atcaaacgt 339
<210> 212
   <211> 660
   <212> DNA
   <213> Artificial Sequence
<220>
   <223> Description of Artificial Sequence: Synthetic polynucleotide
<400> 212
   caagtgctga cccagtctcc atcctccctg tctgcatctg taggagacag agtcaccatc 60
   aattgccagg ccagtcagag tgtttacaat tacaactacc ttgcctggta tcagcagaaa 120
   ccagggaaag ttcctaagca actgatctat tctacatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtagtac tggtgattgt 300
   tttgttttcg gcggaggaac caaggtggaa atcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 213
   <211> 333
   <212> DNA
   <213> Artificial Sequence
<220>
   <223> Description of Artificial Sequence: Synthetic polynucleotide
<400> 213
   gaggtgcagc ttgtggagtc tgggggaggc ttggtccagc ctggggggtc cctgagactc 60
   tcctgtgcag tctctggaat cgacctcagt aaccactaca tgcaatgggt ccgtcaggct 120
   ccagggaagg ggctggagtg ggtcggagtc gttggtatca atggtcgcac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agc 333
<210> 214
   <211> 1326
   <212> DNA
   <213> Artificial Sequence
<220>
   <223> Description of Artificial Sequence: Synthetic polynucleotide
<400> 214
   gaggtgcagc ttgtggagtc tgggggaggc ttggtccagc ctggggggtc cctgagactc 60
   tcctgtgcag tctctggaat cgacctcagt aaccactaca tgcaatgggt ccgtcaggct 120
   ccagggaagg ggctggagtg ggtcggagtc gttggtatca atggtcgcac atactacgcg 180
   agctgggcga aaggccgatt caccatctcc agagacaatt ccaagaccac ggtgtatctt 240
   caaatgaaca gcctgagagc tgaggacact gctgtgtatt tctgtgctag aggggacatc 300
   tggggccaag ggaccctcgt caccgtctcg agcgcctcca ccaagggccc atcggtcttc 360
   cccctggcac cctcctccaa gagcacctct gggggcacag cggccctggg ctgcctggtc 420
   aaggactact tccccgaacc ggtgacggtg tcgtggaact caggcgccct gaccagcggc 480
   gtgcacacct tcccggctgt cctacagtcc tcaggactct actccctcag cagcgtggtg 540
   accgtgccct ccagcagctt gggcacccag acctacatct gcaacgtgaa tcacaagccc 600
   agcaacacca aggtggacaa gagagttgag cccaaatctt gtgacaaaac tcacacatgc 660
   ccaccgtgcc cagcacctga actcctgggg ggaccgtcag tcttcctctt ccccccaaaa 720
   cccaaggaca ccctcatgat ctcccggacc cctgaggtca catgcgtggt ggtggacgtg 780
   agccacgaag accctgaggt caagttcaac tggtacgtgg acggcgtgga ggtgcataat 840
   gccaagacaa agccgcggga ggagcagtac gccagcacgt accgtgtggt cagcgtcctc 900
   accgtcctgc accaggactg gctgaatggc aaggagtaca agtgcaaggt ctccaacaaa 960
   gccctcccag cccccatcga gaaaaccatc tccaaagcca aagggcagcc ccgagaacca 1020
   caggtgtaca ccctgccccc atcccgggag gagatgacca agaaccaggt cagcctgacc 1080
   tgcctggtca aaggcttcta tcccagcgac atcgccgtgg agtgggagag caatgggcag 1140
   ccggagaaca actacaagac cacgcctccc gtgctggact ccgacggctc cttcttcctc 1200
   tacagcaagc tcaccgtgga caagagcagg tggcagcagg ggaacgtctt ctcatgctcc 1260
   gtgatgcatg aggctctgca caaccactac acgcagaaga gcctctccct gtctccgggt 1320
   aaatga 1326
<210> 215
   <211> 39
   <212> DNA
   <213> Oryctolagus cuniculus
<400> 215
   caggccagtc agagtgttta caattacaac taccttgcc 39
<210> 216
   <211> 21
   <212> DNA
   <213> Oryctolagus cuniculus
<400> 216
   tctacatcca ctctggcatc t 21
<210> 217
   <211> 39
   <212> DNA
   <213> Oryctolagus cuniculus
<400> 217
   ctgggcagtt atgattgtag tactggtgat tgttttgtt 39
<210> 218
   <211> 15
   <212> DNA
   <213> Oryctolagus cuniculus
<400> 218
   aaccactaca tgcaa 15
<210> 219
   <211> 48
   <212> DNA
   <213> Oryctolagus cuniculus
<400> 219
   gtcgttggta tcaatggtcg cacatactac gcgagctggg cgaaaggc 48
<210> 220
   <211> 9
   <212> DNA
   <213> Oryctolagus cuniculus
<400> 220
   ggggacatc 9
<210> 221
   <211> 339
   <212> DNA
   <213> Artificial Sequence
<220>
   <223> Description of Artificial Sequence: Synthetic polynucleotide
<400> 221
   caagtgctga cccagactcc atcccccgtg tctgcagctg tgggaagcac agtcaccatc      60
   aattgccagg ccagtcagaa tgtttataat aacaactacc tagcctggta tcagcagaaa     120
   ccagggcagc ctcccaagca actgatctat tctacgtcca ctctggcatc tggggtctca     180
   tcgcgattca gaggcagtgg atctgggaca cagttcactc tcaccatcag cgacgtgcag     240
   tgtgacgatg ctgccactta ctactgtcta ggcagttatg attgtagtcg tggtgattgt     300
   tttgttttcg gcggagggac cgaggtggtg gtcaaacgt                            339
<210> 222
   <211> 660
   <212> DNA
   <213> Artificial Sequence
<220>
   <223> Description of Artificial Sequence: Synthetic polynucleotide
<400> 222
   caagtgctga cccagactcc atcccccgtg tctgcagctg tgggaagcac agtcaccatc      60
   aattgccagg ccagtcagaa tgtttataat aacaactacc tagcctggta tcagcagaaa     120
   ccagggcagc ctcccaagca actgatctat tctacgtcca ctctggcatc tggggtctca     180
   tcgcgattca gaggcagtgg atctgggaca cagttcactc tcaccatcag cgacgtgcag     240
   tgtgacgatg ctgccactta ctactgtcta ggcagttatg attgtagtcg tggtgattgt     300
   tttgttttcg gcggagggac cgaggtggtg gtcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 223
   <211> 327
   <212> DNA
   <213> Artificial Sequence
<220>
   <223> Description of Artificial Sequence: Synthetic polynucleotide
<400> 223
   cagtcgctgg aggagtccgg gggtcgcctg gtcacgcctg ggacacccct gacactcacc      60
   tgcacagtct ctggaatcgg cctcagtagc tactacatgc agtgggtccg ccagtctcca     120
   gggagggggc tggaatggat cggagtcatt ggtagtgatg gtaagacata ctacgcgacc     180
   tgggcgaaag gccgattcac catctccaag acctcgtcga ccacggtgga tctgagaatg     240
   gccagtctga caaccgagga cacggccacc tatttctgta ccagagggga catctggggc     300
   ccggggaccc tcgtcaccgt ctcgagc                                         327
<210> 224
   <211> 1320
   <212> DNA
   <213> Artificial Sequence
<220>
   <223> Description of Artificial Sequence: Synthetic polynucleotide
<400> 224
   cagtcgctgg aggagtccgg gggtcgcctg gtcacgcctg ggacacccct gacactcacc      60
   tgcacagtct ctggaatcgg cctcagtagc tactacatgc agtgggtccg ccagtctcca     120
   gggagggggc tggaatggat cggagtcatt ggtagtgatg gtaagacata ctacgcgacc     180
   tgggcgaaag gccgattcac catctccaag acctcgtcga ccacggtgga tctgagaatg     240
   gccagtctga caaccgagga cacggccacc tatttctgta ccagagggga catctggggc     300
   ccggggaccc tcgtcaccgt ctcgagcgcc tccaccaagg gcccatcggt cttccccctg     360
   gcaccctcct ccaagagcac ctctgggggc acagcggccc tgggctgcct ggtcaaggac     420
   tacttccccg aaccggtgac ggtgtcgtgg aactcaggcg ccctgaccag cggcgtgcac     480
   accttcccgg ctgtcctaca gtcctcagga ctctactccc tcagcagcgt ggtgaccgtg     540
   ccctccagca gcttgggcac ccagacctac atctgcaacg tgaatcacaa gcccagcaac     600
   accaaggtgg acaagagagt tgagcccaaa tcttgtgaca aaactcacac atgcccaccg     660
   tgcccagcac ctgaactcct ggggggaccg tcagtcttcc tcttcccccc aaaacccaag     720
   gacaccctca tgatctcccg gacccctgag gtcacatgcg tggtggtgga cgtgagccac     780
   gaagaccctg aggtcaagtt caactggtac gtggacggcg tggaggtgca taatgccaag     840
   acaaagccgc gggaggagca gtacgccagc acgtaccgtg tggtcagcgt cctcaccgtc     900
   ctgcaccagg actggctgaa tggcaaggag tacaagtgca aggtctccaa caaagccctc     960
   ccagccccca tcgagaaaac catctccaaa gccaaagggc agccccgaga accacaggtg    1020
   tacaccctgc ccccatcccg ggaggagatg accaagaacc aggtcagcct gacctgcctg    1080
   gtcaaaggct tctatcccag cgacatcgcc gtggagtggg agagcaatgg gcagccggag    1140
   aacaactaca agaccacgcc tcccgtgctg gactccgacg gctccttctt cctctacagc    1200
   aagctcaccg tggacaagag caggtggcag caggggaacg tcttctcatg ctccgtgatg    1260
   catgaggctc tgcacaacca ctacacgcag aagagcctct ccctgtctcc gggtaaatga    1320
<210> 225
   <211> 39
   <212> DNA
   <213> Oryctolagus cuniculus
<400> 225
   caggccagtc agaatgttta taataacaac tacctagcc 39
<210> 226
   <211> 21
   <212> DNA
   <213> Oryctolagus cuniculus
<400> 226
   tctacgtcca ctctggcatc t 21
<210> 227
   <211> 39
   <212> DNA
   <213> Oryctolagus cuniculus
<400> 227
   ctaggcagtt atgattgtag tcgtggtgat tgttttgtt 39
<210> 228
   <211> 15
   <212> DNA
   <213> Oryctolagus cuniculus
<400> 228
   agctactaca tgcag 15
<210> 229
   <211> 48
   <212> DNA
   <213> Oryctolagus cuniculus
<400> 229
   gtcattggta gtgatggtaa gacatactac gcgacctggg cgaaaggc 48
<210> 230
   <211> 9
   <212> DNA
   <213> Oryctolagus cuniculus
<400> 230
   ggggacatc 9
<210> 231
   <211> 339
   <212> DNA
   <213> Artificial Sequence
<220>
   <223> Description of Artificial Sequence: Synthetic polynucleotide
<400> 231
   caagtgctga cccagtctcc atcctccctg tctgcatctg taggagacag agtcaccatc      60
   aattgccagg ccagtcagaa tgtttacaat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtagtcg tggtgattgt     300
   tttgttttcg gcggaggaac caaggtggaa atcaaacgt                            339
<210> 232
   <211> 660
   <212> DNA
   <213> Artificial Sequence
<220>
   <223> Description of Artificial Sequence: Synthetic polynucleotide
<400> 232
   caagtgctga cccagtctcc atcctccctg tctgcatctg taggagacag agtcaccatc      60
   aattgccagg ccagtcagaa tgtttacaat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtagtcg tggtgattgt     300
   tttgttttcg gcggaggaac caaggtggaa atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 233
   <211> 333
   <212> DNA
   <213> Artificial Sequence
<220>
   <223> Description of Artificial Sequence: Synthetic polynucleotide
<400> 233
   gaggtgcagc ttgtggagtc tgggggaggc ttggtccagc ctggggggtc cctgagactc 60
   tcctgtgcag tctctggaat cggcctcagt agctactaca tgcaatgggt ccgtcaggct 120
   ccagggaagg ggctggagtg ggtcggagtc attggtagtg atggtaagac atactacgcg 180
   acctgggcga aaggccgatt caccatctcc agagacaatt ccaagaccac ggtgtatctt 240
   caaatgaaca gcctgagagc tgaggacact gctgtgtatt tctgtaccag aggggacatc 300
   tggggccaag ggaccctcgt caccgtctcg agc 333
<210> 234
   <211> 1326
   <212> DNA
   <213> Artificial Sequence
<220>
   <223> Description of Artificial Sequence: Synthetic polynucleotide
<400> 234
   gaggtgcagc ttgtggagtc tgggggaggc ttggtccagc ctggggggtc cctgagactc 60
   tcctgtgcag tctctggaat cggcctcagt agctactaca tgcaatgggt ccgtcaggct 120
   ccagggaagg ggctggagtg ggtcggagtc attggtagtg atggtaagac atactacgcg 180
   acctgggcga aaggccgatt caccatctcc agagacaatt ccaagaccac ggtgtatctt 240
   caaatgaaca gcctgagagc tgaggacact gctgtgtatt tctgtaccag aggggacatc 300
   tggggccaag ggaccctcgt caccgtctcg agcgcctcca ccaagggccc atcggtcttc 360
   cccctggcac cctcctccaa gagcacctct gggggcacag cggccctggg ctgcctggtc 420
   aaggactact tccccgaacc ggtgacggtg tcgtggaact caggcgccct gaccagcggc 480
   gtgcacacct tcccggctgt cctacagtcc tcaggactct actccctcag cagcgtggtg      540
   accgtgccct ccagcagctt gggcacccag acctacatct gcaacgtgaa tcacaagccc      600
   agcaacacca aggtggacaa gagagttgag cccaaatctt gtgacaaaac tcacacatgc      660
   ccaccgtgcc cagcacctga actcctgggg ggaccgtcag tcttcctctt ccccccaaaa      720
   cccaaggaca ccctcatgat ctcccggacc cctgaggtca catgcgtggt ggtggacgtg      780
   agccacgaag accctgaggt caagttcaac tggtacgtgg acggcgtgga ggtgcataat      840
   gccaagacaa agccgcggga ggagcagtac gccagcacgt accgtgtggt cagcgtcctc      900
   accgtcctgc accaggactg gctgaatggc aaggagtaca agtgcaaggt ctccaacaaa      960
   gccctcccag cccccatcga gaaaaccatc tccaaagcca aagggcagcc ccgagaacca     1020
   caggtgtaca ccctgccccc atcccgggag gagatgacca agaaccaggt cagcctgacc     1080
   tgcctggtca aaggcttcta tcccagcgac atcgccgtgg agtgggagag caatgggcag     1140
   ccggagaaca actacaagac cacgcctccc gtgctggact ccgacggctc cttcttcctc     1200
   tacagcaagc tcaccgtgga caagagcagg tggcagcagg ggaacgtctt ctcatgctcc     1260
   gtgatgcatg aggctctgca caaccactac acgcagaaga gcctctccct gtctccgggt     1320
   aaatga                                                                1326
<210> 235
   <211> 39
   <212> DNA
   <213> Oryctolagus cuniculus
<400> 235
   caggccagtc agaatgttta caataacaac tacctagcc 39
<210> 236
   <211> 21
   <212> DNA
   <213> Oryctolagus cuniculus
<400> 236
   tctacatcca ctctggcatc t 21
<210> 237
   <211> 39
   <212> DNA
   <213> Oryctolagus cuniculus
<400> 237
   ctgggcagtt atgattgtag tcgtggtgat tgttttgtt 39
<210> 238
   <211> 15
   <212> DNA
   <213> Oryctolagus cuniculus
<400> 238
   agctactaca tgcaa 15
<210> 239
   <211> 48
   <212> DNA
   <213> Oryctolagus cuniculus
<400> 239
   gtcattggta gtgatggtaa gacatactac gcgacctggg cgaaaggc 48
<210> 240
   <211> 9
   <212> DNA
   <213> Oryctolagus cuniculus
<400> 240
   ggggacatc 9
<210> 241
   <211> 339
   <212> DNA
   <213> Artificial Sequence
<220>
   <223> Description of Artificial Sequence: Synthetic polynucleotide
<400> 241
   caggtgctga cccagactgc atcccccgtg tctccagctg tgggaagcac agtcaccatc 60
   aattgccggg ccagtcagag tgtttattat aacaactacc tagcctggta tcagcagaaa 120
   ccagggcagc ctcccaagca actgatctat tctacatcca ctctggcatc tggggtctca 180
   tcgcggttca aaggcagtgg atctgggaca cagttcactc tcaccatcag cgacgtgcag 240
   tgtgacgatg ctgccactta ctactgtcta ggcagttatg attgtagtaa tggtgattgt 300
   tttgttttcg gcggagggac cgaggtggtg gtcaaacgt 339
<210> 242
   <211> 660
   <212> DNA
   <213> Artificial Sequence
<220>
   <223> Description of Artificial Sequence: Synthetic polynucleotide
<400> 242
   caggtgctga cccagactgc atcccccgtg tctccagctg tgggaagcac agtcaccatc 60
   aattgccggg ccagtcagag tgtttattat aacaactacc tagcctggta tcagcagaaa 120
   ccagggcagc ctcccaagca actgatctat tctacatcca ctctggcatc tggggtctca 180
   tcgcggttca aaggcagtgg atctgggaca cagttcactc tcaccatcag cgacgtgcag 240
   tgtgacgatg ctgccactta ctactgtcta ggcagttatg attgtagtaa tggtgattgt      300
   tttgttttcg gcggagggac cgaggtggtg gtcaaacgta cggtggctgc accatctgtc      360
   ttcatcttcc cgccatctga tgagcagttg aaatctggaa ctgcctctgt tgtgtgcctg      420
   ctgaataact tctatcccag agaggccaaa gtacagtgga aggtggataa cgccctccaa      480
   tcgggtaact cccaggagag tgtcacagag caggacagca aggacagcac ctacagcctc      540
   agcagcaccc tgacgctgag caaagcagac tacgagaaac acaaagtcta cgcctgcgaa      600
   gtcacccatc agggcctgag ctcgcccgtc acaaagagct tcaacagggg agagtgttag      660
<210> 243
   <211> 327
   <212> DNA
   <213> Artificial Sequence
<220>
   <223> Description of Artificial Sequence: Synthetic polynucleotide
<400> 243
   cagtcgctgg aggagtccgg gggtcgcctg gtcacgcctg gaggatccct gacactcacc       60
   tgcacagtct ctggaatcga cgtcactaac tactatatgc aatgggtccg ccaggctcca      120
   gggaaggggc tggaatggat cggagtcatt ggtgtgaatg gtaagagata ctacgcgagc      180
   tgggcgaaag gccgattcac catctccaaa acctcgtcga ccacggtgga tctgaaaatg      240
   accagtctga caaccgagga cacggccacc tatttctgtg ccagaggcga catctggggc      300
   ccggggaccc tcgtcaccgt ctcgagc                                          327
<210> 244
   <211> 1320
   <212> DNA
   <213> Artificial Sequence
<220>
   <223> Description of Artificial Sequence: Synthetic polynucleotide
<400> 244
   cagtcgctgg aggagtccgg gggtcgcctg gtcacgcctg gaggatccct gacactcacc      60
   tgcacagtct ctggaatcga cgtcactaac tactatatgc aatgggtccg ccaggctcca     120
   gggaaggggc tggaatggat cggagtcatt ggtgtgaatg gtaagagata ctacgcgagc     180
   tgggcgaaag gccgattcac catctccaaa acctcgtcga ccacggtgga tctgaaaatg     240
   accagtctga caaccgagga cacggccacc tatttctgtg ccagaggcga catctggggc     300
   ccggggaccc tcgtcaccgt ctcgagcgcc tccaccaagg gcccatcggt cttccccctg     360
   gcaccctcct ccaagagcac ctctgggggc acagcggccc tgggctgcct ggtcaaggac     420
   tacttccccg aaccggtgac ggtgtcgtgg aactcaggcg ccctgaccag cggcgtgcac     480
   accttcccgg ctgtcctaca gtcctcagga ctctactccc tcagcagcgt ggtgaccgtg     540
   ccctccagca gcttgggcac ccagacctac atctgcaacg tgaatcacaa gcccagcaac     600
   accaaggtgg acaagagagt tgagcccaaa tcttgtgaca aaactcacac atgcccaccg     660
   tgcccagcac ctgaactcct ggggggaccg tcagtcttcc tcttcccccc aaaacccaag     720
   gacaccctca tgatctcccg gacccctgag gtcacatgcg tggtggtgga cgtgagccac     780
   gaagaccctg aggtcaagtt caactggtac gtggacggcg tggaggtgca taatgccaag     840
   acaaagccgc gggaggagca gtacgccagc acgtaccgtg tggtcagcgt cctcaccgtc     900
   ctgcaccagg actggctgaa tggcaaggag tacaagtgca aggtctccaa caaagccctc     960
   ccagccccca tcgagaaaac catctccaaa gccaaagggc agccccgaga accacaggtg    1020
   tacaccctgc ccccatcccg ggaggagatg accaagaacc aggtcagcct gacctgcctg    1080
   gtcaaaggct tctatcccag cgacatcgcc gtggagtggg agagcaatgg gcagccggag    1140
   aacaactaca agaccacgcc tcccgtgctg gactccgacg gctccttctt cctctacagc    1200
   aagctcaccg tggacaagag caggtggcag caggggaacg tcttctcatg ctccgtgatg    1260
   catgaggctc tgcacaacca ctacacgcag aagagcctct ccctgtctcc gggtaaatga    1320
<210> 245
   <211> 39
   <212> DNA
   <213> Oryctolagus cuniculus
<400> 245
   cgggccagtc agagtgttta ttataacaac tacctagcc 39
<210> 246
   <211> 21
   <212> DNA
   <213> Oryctolagus cuniculus
<400> 246
   tctacatcca ctctggcatc t 21
<210> 247
   <211> 39
   <212> DNA
   <213> Oryctolagus cuniculus
<400> 247
   ctaggcagtt atgattgtag taatggtgat tgttttgtt 39
<210> 248
   <211> 15
   <212> DNA
   <213> Oryctolagus cuniculus
<400> 248
   aactactata tgcaa 15
<210> 249
   <211> 48
   <212> DNA
   <213> Oryctolagus cuniculus
<400> 249
   gtcattggtg tgaatggtaa gagatactac gcgagctggg cgaaaggc 48
<210> 250
   <211> 9
   <212> DNA
   <213> Oryctolagus cuniculus
<400> 250
   ggcgacatc 9
<210> 251
   <211> 339
   <212> DNA
   <213> Artificial Sequence
<220>
   <223> Description of Artificial Sequence: Synthetic polynucleotide
<400> 251
   caagtgctga cccagtctcc atcctccctg tctgcatctg taggagacag agtcaccatc      60
   aattgccggg ccagtcagag tgtttactat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtagtaa tggtgattgt     300
   tttgttttcg gcggaggaac caaggtggaa atcaaacgt                            339
<210> 252
   <211> 660
   <212> DNA
   <213> Artificial Sequence
<220>
   <223> Description of Artificial Sequence: Synthetic polynucleotide
<400> 252
   caagtgctga cccagtctcc atcctccctg tctgcatctg taggagacag agtcaccatc      60
   aattgccggg ccagtcagag tgtttactat aacaactacc tagcctggta tcagcagaaa     120
   ccagggaaag ttcctaagca actgatctat tctacatcca ctctggcatc tggggtccca     180
   tctcgtttca gtggcagtgg atctgggaca gatttcactc tcaccatcag cagcctgcag     240
   cctgaagatg ttgcaactta ttactgtctg ggcagttatg attgtagtaa tggtgattgt     300
   tttgttttcg gcggaggaac caaggtggaa atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 253
   <211> 333
   <212> DNA
   <213> Artificial Sequence
<220>
   <223> Description of Artificial Sequence: Synthetic polynucleotide
<400> 253
   gaggtgcagc ttgtggagtc tgggggaggc ttggtccagc ctggggggtc cctgagactc      60
   tcctgtgcag tctctggaat cgacgtcact aactactaca tgcaatgggt ccgtcaggct     120
   ccagggaagg ggctggagtg ggtcggagtc attggtgtga atggtaagag atactacgcg     180
   agctgggcga aaggccgatt caccatctcc agagacaatt ccaagaccac ggtgtatctt     240
   caaatgaaca gcctgagagc tgaggacact gctgtgtatt tctgtgccag aggggacatc     300
   tggggccaag ggaccctcgt caccgtctcg agc                                  333
<210> 254
   <211> 1326
   <212> DNA
   <213> Artificial Sequence
<220>
   <223> Description of Artificial Sequence: Synthetic polynucleotide
<400> 254
   gaggtgcagc ttgtggagtc tgggggaggc ttggtccagc ctggggggtc cctgagactc      60
   tcctgtgcag tctctggaat cgacgtcact aactactaca tgcaatgggt ccgtcaggct     120
   ccagggaagg ggctggagtg ggtcggagtc attggtgtga atggtaagag atactacgcg     180
   agctgggcga aaggccgatt caccatctcc agagacaatt ccaagaccac ggtgtatctt     240
   caaatgaaca gcctgagagc tgaggacact gctgtgtatt tctgtgccag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacaa gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                               1326
<210> 255
   <211> 39
   <212> DNA
   <213> Oryctolagus cuniculus
<400> 255
   cgggccagtc agagtgttta ctataacaac tacctagcc 39
<210> 256
   <211> 21
   <212> DNA
   <213> Oryctolagus cuniculus
<400> 256
   tctacatcca ctctggcatc t 21
<210> 257
   <211> 39
   <212> DNA
   <213> Oryctolagus cuniculus
<400> 257
   ctgggcagtt atgattgtag taatggtgat tgttttgtt 39
<210> 258
   <211> 15
   <212> DNA
   <213> Oryctolagus cuniculus
<400> 258
   aactactaca tgcaa 15
<210> 259
   <211> 48
   <212> DNA
   <213> Oryctolagus cuniculus
<400> 259
   gtcattggtg tgaatggtaa gagatactac gcgagctggg cgaaaggc 48
<210> 260
   <211> 9
   <212> DNA
   <213> Oryctolagus cuniculus
<400> 260
   ggggacatc 9
<210> 261
   <211> 339
   <212> DNA
   <213> Artificial Sequence
<220>
   <223> Description of Artificial Sequence: Synthetic polynucleotide
<400> 261
   gccatcgtga tgacccagac tccatcttcc aagtctgtcc ctgtgggaga cacagtcacc      60
   atcaattgcc aggccagtga gagtctttat aataacaacg ccttggcctg gtttcagcag     120
   aaaccagggc agcctcccaa gcgcctgatc tatgatgcat ccaaactggc atctggggtc     180
   ccatcgcggt tcagtggcgg tgggtctggg acacagttca ctctcaccat cagtggcgtg     240
   cagtgtgacg atgctgccac ttactactgt ggaggctaca gaagtgatag tgttgatggt     300
   gttgctttcg ccggagggac cgaggtggtg gtcaaacgt                            339
<210> 262
   <211> 660
   <212> DNA
   <213> Artificial Sequence
<220>
   <223> Description of Artificial Sequence: Synthetic polynucleotide
<400> 262
   gccatcgtga tgacccagac tccatcttcc aagtctgtcc ctgtgggaga cacagtcacc      60
   atcaattgcc aggccagtga gagtctttat aataacaacg ccttggcctg gtttcagcag     120
   aaaccagggc agcctcccaa gcgcctgatc tatgatgcat ccaaactggc atctggggtc     180
   ccatcgcggt tcagtggcgg tgggtctggg acacagttca ctctcaccat cagtggcgtg     240
   cagtgtgacg atgctgccac ttactactgt ggaggctaca gaagtgatag tgttgatggt     300
   gttgctttcg ccggagggac cgaggtggtg gtcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgttag     660
<210> 263
   <211> 333
   <212> DNA
   <213> Artificial Sequence
<220>
   <223> Description of Artificial Sequence: Synthetic polynucleotide
<400> 263
   cagtcggtgg aggagtccgg gggaggcctg gtccagcctg agggatccct gacactcacc      60
   tgcacagcct ctggattcga cttcagtagc aatgcaatgt ggtgggtccg ccaggctcca     120
   gggaaggggc tggagtggat cggatgcatt tacaatggtg atggcagcac atactacgcg     180
   agctgggtga atggccgatt ctccatctcc aaaacctcgt cgaccacggt gactctgcaa     240
   ctgaatagtc tgacagtcgc ggacacggcc acgtattatt gtgcgagaga tcttgacttg     300
   tggggcccgg gcaccctcgt caccgtctcg agc                                   333
<210> 264
   <211> 1326
   <212> DNA
   <213> Artificial Sequence
<220>
   <223> Description of Artificial Sequence: Synthetic polynucleotide
<400> 264
   cagtcggtgg aggagtccgg gggaggcctg gtccagcctg agggatccct gacactcacc      60
   tgcacagcct ctggattcga cttcagtagc aatgcaatgt ggtgggtccg ccaggctcca     120
   gggaaggggc tggagtggat cggatgcatt tacaatggtg atggcagcac atactacgcg     180
   agctgggtga atggccgatt ctccatctcc aaaacctcgt cgaccacggt gactctgcaa     240
   ctgaatagtc tgacagtcgc ggacacggcc acgtattatt gtgcgagaga tcttgacttg     300
   tggggcccgg gcaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacaa gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                               1326
<210> 265
   <211> 39
   <212> DNA
   <213> Oryctolagus cuniculus
<400> 265
   caggccagtg agagtcttta taataacaac gccttggcc 39
<210> 266
   <211> 21
   <212> DNA
   <213> Oryctolagus cuniculus
<400> 266
   gatgcatcca aactggcatc t 21
<210> 267
   <211> 36
   <212> DNA
   <213> Oryctolagus cuniculus
<400> 267
   ggaggctaca gaagtgatag tgttgatggt gttgct 36
<210> 268
   <211> 15
   <212> DNA
   <213> Oryctolagus cuniculus
<400> 268
   agcaatgcaa tgtgg 15
<210> 269
   <211> 51
   <212> DNA
   <213> Oryctolagus cuniculus
<400> 269
   tgcatttaca atggtgatgg cagcacatac tacgcgagct gggtgaatgg c 51
<210> 270
   <211> 12
   <212> DNA
   <213> Oryctolagus cuniculus
<400> 270
   gatcttgact tg 12
<210> 271
   <211> 339
   <212> DNA
   <213> Artificial Sequence
<220>
   <223> Description of Artificial Sequence: Synthetic polynucleotide
<400> 271
   caagtgctga cccagtctcc atcctccctg tctgcatctg taggagacag agtcaccatc 60
   aattgccagg ccagtcagaa tgtttacaat aacaactacc tagcctggta tcagcagaaa 120
   ccagggaaag ttcctaagca actgatctat tctacatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtagtcg tggtgattgt 300
   tttgttttcg gcggaggaac caaggtggaa atcaaacgt 339
<210> 272
   <211> 660
   <212> DNA
   <213> Artificial Sequence
<220>
   <223> Description of Artificial Sequence: Synthetic polynucleotide
<400> 272
   caagtgctga cccagtctcc atcctccctg tctgcatctg taggagacag agtcaccatc 60
   aattgccagg ccagtcagaa tgtttacaat aacaactacc tagcctggta tcagcagaaa 120
   ccagggaaag ttcctaagca actgatctat tctacatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtagtcg tggtgattgt 300
   tttgttttcg gcggaggaac caaggtggaa atcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 273
   <211> 333
   <212> DNA
   <213> Artificial Sequence
<220>
   <223> Description of Artificial Sequence: Synthetic polynucleotide
<400> 273
   gaggtgcagc ttgtggagtc tgggggaggc ttggtccagc ctggggggtc cctgagactc 60
   tcctgtgcag tctctggaat cggcctcagt agctactaca tgcaatgggt ccgtcaggct 120
   ccagggaagg ggctggagtg ggtcggagtc attggtagtg atggtaagac atactacgcg 180
   acctgggcga aaggccgatt caccatctcc agagacaatt ccaagaccac ggtgtatctt 240
   caaatgaaca gcctgagagc tgaggacact gctgtgtatt tctgtaccag aggggacatc 300
   tggggccaag ggaccctcgt caccgtctcg agc 333
<210> 274
   <211> 1326
   <212> DNA
   <213> Artificial Sequence
<220>
   <223> Description of Artificial Sequence: Synthetic polynucleotide
<400> 274
   gaggtgcagc ttgtggagtc tgggggaggc ttggtccagc ctggggggtc cctgagactc 60
   tcctgtgcag tctctggaat cggcctcagt agctactaca tgcaatgggt ccgtcaggct 120
   ccagggaagg ggctggagtg ggtcggagtc attggtagtg atggtaagac atactacgcg 180
   acctgggcga aaggccgatt caccatctcc agagacaatt ccaagaccac ggtgtatctt 240
   caaatgaaca gcctgagagc tgaggacact gctgtgtatt tctgtaccag aggggacatc 300
   tggggccaag ggaccctcgt caccgtctcg agcgcctcca ccaagggccc atcggtcttc 360
   cccctggcac cctcctccaa gagcacctct gggggcacag cggccctggg ctgcctggtc 420
   aaggactact tccccgaacc ggtgacggtg tcgtggaact caggcgccct gaccagcggc 480
   gtgcacacct tcccggctgt cctacagtcc tcaggactct actccctcag cagcgtggtg 540
   accgtgccct ccagcagctt gggcacccag acctacatct gcaacgtgaa tcacaagccc 600
   agcaacacca aggtggacgc gagagttgag cccaaatctt gtgacaaaac tcacacatgc 660
   ccaccgtgcc cagcacctga actcctgggg ggaccgtcag tcttcctctt ccccccaaaa 720
   cccaaggaca ccctcatgat ctcccggacc cctgaggtca catgcgtggt ggtggacgtg 780
   agccacgaag accctgaggt caagttcaac tggtacgtgg acggcgtgga ggtgcataat 840
   gccaagacaa agccgcggga ggagcagtac gccagcacgt accgtgtggt cagcgtcctc 900
   accgtcctgc accaggactg gctgaatggc aaggagtaca agtgcaaggt ctccaacaaa 960
   gccctcccag cccccatcga gaaaaccatc tccaaagcca aagggcagcc ccgagaacca 1020
   caggtgtaca ccctgccccc atcccgggag gagatgacca agaaccaggt cagcctgacc 1080
   tgcctggtca aaggcttcta tcccagcgac atcgccgtgg agtgggagag caatgggcag 1140
   ccggagaaca actacaagac cacgcctccc gtgctggact ccgacggctc cttcttcctc 1200
   tacagcaagc tcaccgtgga caagagcagg tggcagcagg ggaacgtctt ctcatgctcc 1260
   gtgatgcatg aggctctgca caaccactac acgcagaaga gcctctccct gtctccgggt 1320
   aaatga 1326
<210> 275
   <211> 39
   <212> DNA
   <213> Oryctolagus cuniculus
<400> 275
   caggccagtc agaatgttta caataacaac tacctagcc 39
<210> 276
   <211> 21
   <212> DNA
   <213> Oryctolagus cuniculus
<400> 276
   tctacatcca ctctggcatc t 21
<210> 277
   <211> 39
   <212> DNA
   <213> Oryctolagus cuniculus
<400> 277
   ctgggcagtt atgattgtag tcgtggtgat tgttttgtt 39
<210> 278
   <211> 15
   <212> DNA
   <213> Oryctolagus cuniculus
<400> 278
   agctactaca tgcaa 15
<210> 279
   <211> 48
   <212> DNA
   <213> Oryctolagus cuniculus
<400> 279
   gtcattggta gtgatggtaa gacatactac gcgacctggg cgaaaggc 48
<210> 280
   <211> 9
   <212> DNA
   <213> Oryctolagus cuniculus
<400> 280
   ggggacatc 9
<210> 281
   <211> 37
   <212> PRT
   <213> Homo sapiens
<220>
   <223> C-term amidated
<400> 281
   Ala Cys Asp Thr Ala Thr Cys Val Thr His Arg Leu Ala Gly Leu Leu
   1               5                   10                  15
   Ser Arg Ser Gly Gly Val Val Lys Asn Asn Phe Val Pro Thr Asn Val
               20                  25                  30
   Gly Ser Lys Ala Phe
           35
<210> 282
   <211> 37
   <212> PRT
   <213> Homo sapiens
<220>
   <223> C-term amidated
<400> 282
   Ala Cys Asn Thr Ala Thr Cys Val Thr His Arg Leu Ala Gly Leu Leu
   1               5                   10                  15
   Ser Arg Ser Gly Gly Met Val Lys Ser Asn Phe Val Pro Thr Asn Val
               20                  25                  30
   Gly Ser Lys Ala Phe
   35
<210> 283
   <211> 105
   <212> PRT
   <213> Homo sapiens
<400> 283
<210> 284
   <211> 330
   <212> PRT
   <213> Homo sapiens
<400> 284
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
   85 90 95
   Arg Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Ala Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Glu Glu
   225 230 235 240
   Met Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330

## Description

### FIELD OF THE INVENTION

This invention pertains to the use of antibodies against human Calcitonin Gene Related Peptide ("CGRP") and fragments thereof (including Fab fragments) which specifically bind to CGRP and promote glucose uptake and utilization in peripheral tissue and/or inhibit hepatic glucose production. Exemplary embodiments of the subject methods may preserve functional pancreatic beta cells, thereby slowing the progression to overt diabetes. The description also pertains to methods of screening for diseases and disorders associated with insulin resistance (including disorders of glucose, carbohydrate and fat metabolism). Also described herein are methods of preventing or treating diseases and disorders associated with insulin resistance by administering said antibodies or fragments thereof.

This application contains a Biological Sequence Listing which has been submitted in ASCII format via EFS-Web. Said ASCII copy, created on July 1, 2014, is named "4325703013.txt" and is 203,678 bytes in size.

### BACKGROUND OF THE INVENTION

Calcitonin Gene Related Peptide (CGRP) is produced as a multifunctional neuropeptide of 37 amino acids in length. Two forms of CGRP, a CGRP-alpha and a CGRP-beta form exist in humans and both have similar activities. CGRP-alpha and CGRP-beta differ by three amino acids in humans, and are derived from different genes. The CGRP family of peptides also includes amylin, adrenomedullin, and calcitonin, although each has distinct receptors and biological activities. Doods, H., Curr. Op. Invest. Drugs, 2(9):1261-68 (2001). Within the CGRP protein family, amino acid residues at putative receptor binding sites are conserved, although overall homology varies. For example, human CGRP and amylin share 46% amino acid sequence identity overall while human calcitonin and CGRP share 15% amino acid sequence identity. Wimalawansa, S.J., Endocrine Rev. 17(5):533-585 (1996).

The biological effects of CGRP are mediated via the CGRP receptor (CGRP-R), which consists of a seven-transmembrane component, in conjunction with receptor-associated membrane protein (RAMP). CGRP-R further requires the activity of the receptor component protein (RCP), which is essential for an efficient coupling to adenylate cyclase through G proteins and the production of cAMP Doods, H., Curr. Op. Invest. Drugs, 2(9):1261-68 (2001).

CGRP is found throughout the peripheral and central nervous system and influences the cardiovascular, nervous and endocrine systems. When CGRP is released from tissues such as trigeminal nerves, it can result in a sequential activation and release of neuropeptides within the meninges, to mediate neurogenic inflammation that is characterized by vasodilation, vessel leakage, and mast-cell degradation. Durham, P.L., New Eng. J. Med., 350 (11):1073-75 (2004). CGRP is thought to play a prominent role in the development of migraines. It has been shown that elevated levels of CGRP identified in plasma from jugular venous blood during the headache phase of migraines, to the exclusion of other neuropeptides. Arulmozhi, D.K., et al., Vas. Pharma., 43: 176-187 (2005). Additionally, CGRP antagonism has been shown to be effective for treatment of migraine (Olesen et al., N Engl J Med. 2004 Mar 11;350(11):1104-10).

In addition to nervous tissue, CGRP receptors have been identified in cardiovascular tissue, adrenal gland, pituitary gland, kidney, pancreas and bone. Wimalawansa, S.J., Endocrine Rev. 17(5):533-585 (1996). In *in vitro* studies, both CGRP and amylin were found to inhibit insulin secretion using isolated pancreatic tissue, counteract the insulin-stimulated rate of glycogen synthesis in a dose-dependent manner and block the effects of insulin in isolated hepatocytes (Gomez-Foix et al. Biochem. J. 276:607-610, 1991). Additionally, Leighton and Cooper (Nature, 335(6191):632-5, 1988) report that rat CGRP-1 inhibited basal and insulin-stimulated rates of glycogen synthesis in stripped rat soleus muscle *in vitro.*

Glucose homeostasis is maintained by balancing glycogen synthesis with glycogenolysis by the hormone glucagon and glucose utilization and uptake into tissue by the hormone insulin. The presence of glucose normally stimulates insulin production, which functions to increase the transport rate of glucose into skeletal muscle, myocytes, brain and adipocytes. Insulin also normally inhibits lipid degradation in adipocytes. In the earliest stages of pre-diabetes or Type 2 diabetes tissues develop insulin resistance, but pancreatic beta cells compensate by secreting increasing levels of insulin. Eventually as muscle and liver insulin resistance increases, the pancreatic beta cell ability to compensate becomes exhausted and exogenous insulin is required.

The inability to strictly regulate glucose homeostasis as a result of impaired insulin synthesis and glucose utilization can have profound metabolic and detrimental health effects. The most common is development of persistently high blood sugar (hyperglycemia) leading to insulin resistance and a diagnosis of Type II diabetes. In 2011, in the U.S. 25,600,000 people aged 20 and older were diagnosed as having diabetes, of which 95% was Type 2 diabetes. (Centers for Disease Control and Prevention. National Diabetes Fact Sheet, 2011. Atlanta, GA: Centers for Disease Control and Prevention, US Department of Health and Human Services; 2011.) Medical costs for the diabetic are on average as twice as high as the non-diabetic person due to the increased risk for heart attack, stroke, renal complications and neuropathy. Imperatore et al. Am J Epidemiol. 160(6): 531-539 (2004). Without significant changes, the CDC predicts by 2050, 1 in 3 adults in the U.S. could have diabetes. Boyle et al. Popul. Health Metr. 8:29 (2010). Worldwide, in 2011 the total number of people diagnosed with diabetes was estimated at 366 million people, increasing to 552 million by 2030. (International Diabetes Foundation, IDF Diabetes Atlas, Fifth Ed.)

The role of CGRP in glucose metabolism is not clearly defined in the literature. Studies in isolated hepatocytes have demonstrated that CGRP and amylin inhibit insulin-stimulated rate of glycogen synthesis (Gomez-Foix et al. Biochem. J. 276:607-610, 1991). Beaumont et al. Br J Pharmacol. Jul;115(5):713-5 (1995) found CGRP receptors do not mediate the effects of muscle glucose metabolism. Tanaka et al. Exp. Clin Endocrinol Diabetes 121:280-285 (2013) states that in a rat model an anti-CGRP antibody produced a slight extension of first-phase insulin secretion with a small change in insulin secretion, however, the study did not report whether the antibody was cross-reactive against other calcitonin family peptides, potentially confounding the results. Finally, prior U.S. patents have purported to show that CGRP is an amylin agonist and administration of CGRP polypeptide (as opposed to a CGRP antagonist) may treat diabetes (see, e.g., U.S. Pat. Nos. 5,641,744 and 5,175,145).

US 5266561 A (published: 30 November 1993) reports "Treatment of type 2 diabetes". US 5364841 A (published: 25 November 1994) reports "Treatment of obesity and essential hypertension and related disorders". US 2012/294797 A1 (published: 22 November 2012) reports "Anti-CGRP compositions and use thereof'. Derosa et al., reports "Optimizing combination treatment in the management of type 2 diabetes" (Vascular Health and Risk Management, 2007, vol. 3(5): pp. 665-671). Halimi et al., reports "Combination treatment in the management of type 2 diabetes: focus on vildagliptin and metformin as a single tablet" (Vascular Health and Risk Management, 2008, vol. 4(3): pp. 481-492.)

### SUMMARY OF THE INVENTION

The invention is defined by the claims. Specifically the present invention provides an anti-human Calcitonin Gene Related Peptide (CGRP) antibody or antibody fragment for use in a method of ameliorating or reducing the symptoms of, or treating, or preventing or controlling hyperglycemia in a subject in need thereof, wherein said anti-human CGRP antibody or antibody fragment comprises: (i) a variable light (V_{L}) chain comprising complementarity determining region (CDR)1, CDR2 and CDR3 polypeptides having the amino acid sequences of SEQ ID NOS: 55, 56 and 57, respectively; and (ii) a variable heavy (V_{H}) chain comprising CDR1, CDR2 and CDR3 polypeptides having the amino acid sequences of SEQ ID NOS: 58, 59 and 60, respectively.

Also described herein is a composition comprising an anti-human CGRP antibody or antibody fragment for use in a method of increasing peripheral and/or hepatic glucose utilization in a subject in need thereof, the use comprising administering an effective amount of the composition to said subject.

Also described herein is a composition comprising an anti-human CGRP antibody or antibody fragment for use in a method of decreasing insulin resistance in a subject in need thereof, the use comprising administering an effective amount of the composition to said subject.

Also described herein is a composition comprising an anti-human CGRP antibody or antibody fragment for use in a method of treating, preventing or controlling obesity in a subject in need thereof the use comprising administering an effective amount of the composition to said subject.

Also described herein is a composition comprising an anti-human CGRP antibody or antibody fragment for use in a method to achieve sustained normoglycemia in a subject in need thereof the use comprising administering an effective amount of the composition to said subject.

Also described herein is a composition comprising an anti-human CGRP antibody or antibody fragment for use in a method for increasing the ratio of lean tissue to body fat in a subject in need thereof, comprising administering an effective amount of the composition to said subject.

The subject methods may be effective to treat or delay the onset of type II diabetes and/or obesity. For example, the need for administering exogenous insulin may be delayed. The method may be effective to prevent or slow the loss of pancreatic beta cells. For example, without intent to be limited by theory, it is thought that the method may allow pancreatic beta cells of an insulin-resistant human or non-human animal to rest, thereby preventing loss of functional pancreatic beta cells.

Said subject may have been diagnosed with pre-diabetes or may exhibit one or more risk factors for development of type II diabetes.

The subject may be pre-menopausal, perimenopausal, menopausal or postmenopausal.

The subject may exhibit one or more symptoms of pre-diabetes such as fasting blood glucose level of between 100 mg/dL and 125 mg/dl; blood sugar level of between 140 mg/dL and 199 mg/dL two hours after ingesting a 75 gram glucose solution or a glucose solution of 1.75 grams of glucose per kilogram of body weight, to a maximum dose of 75 grams; and/or glycated hemoglobin of between 5.7 percent and 6.4 percent.

The subject may exhibit one or more symptoms of diabetes, such as fasting blood glucose level greater than 125 mg/dl; blood sugar level of at least 200 mg/dL two hours after ingesting a 75 gram glucose solution or a glucose solution of 1.75 grams of glucose per kilogram of body weight, to a maximum dose of 75 grams; and/or glycated hemoglobin of at least 6.5 percent.

The subject may exhibit one or more risk factors for development of type II diabetes, such as a family history of type II diabetes; one or more parents or siblings previously diagnosed with type II diabetes; dyslipidemia; total blood triglyceride levels of at least 200 mg/dL; blood high density lipoprotein level less than 35 mg/dL; obesity; body mass index greater than 25 kg/m²; history of gestational diabetes; previously gave birth to an infant with birth weight greater than 9 lbs.; hypertension; systolic blood pressure of at least 140 mmHg; diastolic blood pressure of at least 90 mmHg; previous measurement of fasting blood glucose of at least 99 mg/dL; vascular disease; Polycystic Ovarian Syndrome; or acanthosis nigricans.

The subject may have been diagnosed with type II diabetes.

The subject may be refractory to treatment with at least one compound selected from the group consisting of: GLP-1, exenatide-1, exendin, exendin analog, exendin agonist, liraglutide, exenatide LAR, a DPP-4 antagonist, a GLP-1 receptor agonist, and another GLP-1 agonist; or such compound may be contraindicated for administration to the subject.

The uses may further comprise administering to said subject an anti-diabetic agent or anti-obesity agent other than an anti-human CGRP antibody or antibody fragment. Said anti-diabetic agent or anti-obesity agent may comprise one or more of amylin, amylin agonist, sulfonylureas, calcitonin, glucagon, PPAR-gamma agonists, GPL-1 receptor agonists, dipeptidyl peptidase IV inhibitor, amylin analogs, biguanides, dopamine D2 receptor agonists, meglitinides, alpha-glucosidase inhibitor, antidyslipidemic bile acid sequestrant, exendin, exendin analog, exendin agonist, gastrin inhibitory peptide (GIP), incretin peptide, insulin, SGLT2 inhibitor, a glucose reabsorption inhibitor, fenofibrate, fibrate, an anti-ghrelin antibody or antibody fragment, an fibroblast growth factor receptor (FGFR)-1(IIIb), FGFR-1(IIIc), antibody or antibody fragment, and/or FGFR-4(IIIc), an anti-CD38 antibody or antibody fragment, an anti-MIC-1 antibody, or MIC-1 binding fragment, metformin or a combination of any of the foregoing.

In an exemplary embodiment, said anti-diabetic agent is metformin.

The method may be effective to cause weight loss.

The administered anti-human CGRP antibody or antibody fragment may not significantly increase insulin secretion in vivo, e.g., may not significantly increase insulin secretion above normal physiological levels in vivo, or may not significantly increase insulin secretion relative to the level of insulin secretion prior to administration of the anti-human CGRP antibody or antibody fragment.

The administered anti-human CGRP antibody or antibody fragment may not result in an increased incidence in pancreatitis or the expression of markers or cytokines associated with pancreatic inflammation.

Said composition may further comprise a pharmaceutically acceptable carrier.

Said anti-human CGRP antibody or antibody fragment may for administration to said subject at a dosage between about 0.1 and 100.0 mg/kg of body weight of recipient subject.

Said anti-human CGRP antibody or antibody fragment may be a human antibody. Said anti-human CGRP antibody or antibody fragment may be non-naturally occurring. Said anti-human CGRP antibody or antibody fragment may be a non-naturally occurring antibody fragment. Said anti-human CGRP antibody or antibody fragment may be a humanized antibody or fragment thereof. Said anti-human CGRP antibody or antibody fragment may be a chimeric antibody.

The anti-human CGRP antibody or antibody fragments described herein may specifically bind to the same linear or conformational epitope(s) and/or may compete for binding to the same or overlapping linear or conformational epitope(s) on an intact CGRP polypeptide or fragment thereof as an anti-human CGRP antibody selected from the group consisting of: (a) Ab1 comprising the V_{L} of SEQ ID NO:2 and the V_{H} of SEQ ID NO:4; (b) Ab2 comprising the V_{L} of SEQ ID NO:12 and the V_{H} of SEQ ID NO:14; (c) Ab3 comprising the V_{L} of SEQ ID NO:22 and the V_{H} of SEQ ID NO:24; (d) Ab4 comprising the V_{L} of SEQ ID NO:32 and the V_{H} of SEQ ID NO:34; (e) Ab5 comprising the V_{L} of SEQ ID NO:42 and the V_{H} of SEQ ID NO:44; (f) Ab6 comprising the V_{L} of SEQ ID NO:52 and the V_{H} of SEQ ID NO:54; (g) Ab7 comprising the V_{L} of SEQ ID NO:62 and the V_{H} of SEQ ID NO:64; (h) Ab8 comprising the V_{L} of SEQ ID NO:52 and the V_{H} of SEQ ID NO: 54; (i) Ab9 comprising the V_{L} of SEQ ID NO: 62 and the V_{H} of SEQ ID NO: 64; (j) Ab10 comprising the V_{L} of SEQ ID NO:72 and the V_{H} of SEQ ID NO:74; (k) Ab11 comprising the V_{L} of SEQ ID NO:82 and the V_{H} of SEQ ID NO:84; (l) Ab12 comprising the V_{L} of SEQ ID NO:92 and the V_{H} of SEQ ID NO:94; (m) Ab13 comprising the V_{L} of SEQ ID NO:102 and the V_{H} of SEQ ID NO:104; and (n) Ab14 comprising the V_{L} of SEQ ID NO:112 and the V_{H} of SEQ ID NO:114.

The anti-human CGRP antibody or antibody fragment for use according to the present invention comprises: (i) a variable light (V_{L}) chain comprising complementarity determining region (CDR)1, CDR2 and CDR3 polypeptides having the amino acid sequences of SEQ ID NOS: 55, 56 and 57, respectively; and (ii) a variable heavy (V_{H}) chain comprising CDR1, CDR2 and CDR3 polypeptides having the amino acid sequences of SEQ ID NOS: 58, 59 and 60, respectively. The anti-human CGRP antibody or antibody fragments described herein may comprise at least one, at least two, at least three, at least four, at least five, or all six CDRs contained in an antibody selected from the group consisting of: (a) Ab1 comprising the V_{L} of SEQ ID NO:2 and the V_{H} of SEQ ID NO:4; (b) Ab2 comprising the V_{L} of SEQ ID NO:12 and the V_{H} of SEQ ID NO:14; (c) Ab3 comprising the V_{L} of SEQ ID NO:22 and the V_{H} of SEQ ID NO:24; (d) Ab4 comprising the V_{L} of SEQ ID NO:32 and the V_{H} of SEQ ID NO:34; (e) Ab5 comprising the V_{L} of SEQ ID NO:42 and the V_{H} of SEQ ID NO:44; (f) Ab6 comprising the V_{L} of SEQ ID NO:52 and the V_{H} of SEQ ID NO:54; (g) Ab7 comprising the V_{L} of SEQ ID NO:62 and the V_{H} of SEQ ID NO:64; (h) Ab8 comprising the V_{L} of SEQ ID NO:52 and the V_{H} of SEQ ID NO:54; (i) Ab9 comprising the V_{L} of SEQ ID NO:62 and the V_{H} of SEQ ID NO:64; (j) Ab10 comprising the V_{L} of SEQ ID NO:72 and the V_{H} of SEQ ID NO:74; (k) Ab11 comprising the V_{L} of SEQ ID NO:82 and the V_{H} of SEQ ID NO:84; (l) Ab12 comprising the V_{L} of SEQ ID NO:92 and the V_{H} of SEQ ID NO:94; (m) Ab13 comprising the V_{L} of SEQ ID NO:102 and the V_{H} of SEQ ID NO:104; and (n) Ab14 comprising the V_{L} of SEQ ID NO:112 and the V_{H} of SEQ ID NO:114.

The anti-human CGRP antibody or antibody fragments described herein may have a polypeptide sequence at least 80%, at least 85%, at least 90%, at least 95%, at least 96%, at least 97%, at least 98%, or at least 99% identical to an antibody selected from the group consisting of: (a) Ab1 comprising the V_{L} of SEQ ID NO:2 and the V_{H} of SEQ ID NO:4; (b) Ab2 comprising the V_{L} of SEQ ID NO:12 and the V_{H} of SEQ ID NO:14; (c) Ab3 comprising the V_{L} of SEQ ID NO:22 and the V_{H} of SEQ ID NO:24; (d) Ab4 comprising the V_{L} of SEQ ID NO:32 and the V_{H} of SEQ ID NO:34; (e) Ab5 comprising the V_{L} of SEQ ID NO:42 and the V_{H} of SEQ ID NO:44; (f) Ab6 comprising the V_{L} of SEQ ID NO:52 and the V_{H} of SEQ ID NO:54; (g) Ab7 comprising the V_{L} of SEQ ID NO:62 and the V_{H} of SEQ ID NO:64; (h) Ab8 comprising the V_{L} of SEQ ID NO:52 and the V_{H} of SEQ ID NO:54; (i) Ab9 comprising the V_{L} of SEQ ID NO: 62 and the V_{H} of SEQ ID NO: 64; (j) Ab10 comprising the V_{L} of SEQ ID NO:72 and the V_{H} of SEQ ID NO:74; (k) Ab11 comprising the V_{L} of SEQ ID NO:82 and the V_{H} of SEQ ID NO:84; (l) Ab12 comprising the V_{L} of SEQ ID NO:92 and the V_{H} of SEQ ID NO:94; (m) Ab13 comprising the V_{L} of SEQ ID NO:102 and the V_{H} of SEQ ID NO:104; and (n) Ab14 comprising the V_{L} of SEQ ID NO:112 and the V_{H} of SEQ ID NO:114.

The anti-human CGRP antibody or antibody fragments described herein may comprise an antibody selected from the group consisting of: (a) Ab1 comprising the V_{L} of SEQ ID NO:2 and the V_{H} of SEQ ID NO:4; (b) Ab2 comprising the V_{L} of SEQ ID NO:12 and the V_{H} of SEQ ID NO:14; (c) Ab3 comprising the V_{L} of SEQ ID NO:22 and the V_{H} of SEQ ID NO:24; (d) Ab4 comprising the V_{L} of SEQ ID NO:32 and the V_{H} of SEQ ID NO:34; (e) Ab5 comprising the V_{L} of SEQ ID NO:42 and the V_{H} of SEQ ID NO:44; (f) Ab6 comprising the V_{L} of SEQ ID NO:52 and the V_{H} of SEQ ID NO:54; (g) Ab7 comprising the V_{L} of SEQ ID NO:62 and the V_{H} of SEQ ID NO:64; (h) Ab8 comprising the V_{L} of SEQ ID NO: 52 and the V_{H} of SEQ ID NO: 54; (i) Ab9 comprising the V_{L} of SEQ ID NO:62 and the V_{H} of SEQ ID NO:64; (j) Ab10 comprising the V_{L} of SEQ ID NO:72 and the V_{H} of SEQ ID NO:74; (k) Ab11 comprising the V_{L} of SEQ ID NO:82 and the V_{H} of SEQ ID NO:84; (l) Ab12 comprising the V_{L} of SEQ ID NO:92 and the V_{H} of SEQ ID NO:94; (m) Ab13 comprising the V_{L} of SEQ ID NO:102 and the V_{H} of SEQ ID NO:104; and (n) Ab14 comprising the V_{L} of SEQ ID NO:112 and the V_{H} of SEQ ID NO:114.

The anti-human CGRP antibody or antibody fragment may comprise a human, chimeric or humanized antibody. The anti-human CGRP antibody or antibody fragment may comprise a Fab, F(ab')₂, scFv, IgNar, or MetMab or another monovalent antibody fragment.

The disclosure provides a composition suitable for use in a method as described herein, e.g., as recited in the preceding paragraphs, which may comprise an effective amount of an anti-human CGRP antibody or antibody fragment and an anti-diabetic or anti-obesity agent other than an anti-human CGRP antibody or antibody fragment. The anti-human CGRP antibody or antibody fragment may one described herein, e.g., which may specifically bind to the same linear or conformational epitope(s), may compete for binding to the same or overlapping linear or conformational epitope(s) on an intact CGRP polypeptide or fragment thereof as, may have a polypeptide sequence at least 80%, at least 85%, at least 90%, at least 95%, at least 96%, at least 97%, at least 98%, or at least 99% identical to, or may comprises, an anti-human CGRP antibody selected from the group consisting of (a) Ab1 comprising the V_{L} of SEQ ID NO:2 and the V_{H} of SEQ ID NO:4; (b) Ab2 comprising the V_{L} of SEQ ID NO:12 and the V_{H} of SEQ ID NO:14; (c) Ab3 comprising the V_{L} of SEQ ID NO:22 and the V_{H} of SEQ ID NO:24; (d) Ab4 comprising the V_{L} of SEQ ID NO:32 and the V_{H} of SEQ ID NO:34; (e) Ab5 comprising the V_{L} of SEQ ID NO:42 and the V_{H} of SEQ ID NO:44; (f) Ab6 comprising the V_{L} of SEQ ID NO:52 and the V_{H} of SEQ ID NO:54; (g) Ab7 comprising the V_{L} of SEQ ID NO:62 and the V_{H} of SEQ ID NO:64; (h) Ab8 comprising the V_{L} of SEQ ID NO:52 and the V_{H} of SEQ ID NO:54; (i) Ab9 comprising the V_{L} of SEQ ID NO:62 and the V_{H} of SEQ ID NO:64; (j) Ab10 comprising the V_{L} of SEQ ID NO:72 and the V_{H} of SEQ ID NO:74; (k) Ab11 comprising the V_{L} of SEQ ID NO:82 and the V_{H} of SEQ ID NO:84; (l) Ab12 comprising the V_{L} of SEQ ID NO:92 and the V_{H} of SEQ ID NO:94; (m) Ab13 comprising the V_{L} of SEQ ID NO:102 and the V_{H} of SEQ ID NO:104; and (n) Ab14 comprising the V_{L} of SEQ ID NO:112 and the V_{H} of SEQ ID NO:114.

Said anti-diabetic or anti-obesity agent comprises one or more of amylin, amylin agonist, sulfonylureas, calcitonin, glucagon, PPAR-gamma agonists, GPL-1 receptor agonists, dipeptidyl peptidase IV inhibitor, amylin analogs, biguanides, dopamine D2 receptor agonists, meglitinides, alpha-glucosidase inhibitor, antidyslipidemic bile acid sequestrant, exendin, exendin analog, exendin agonist, gastrin inhibitory peptide (GIP), incretin peptide, insulin, SGLT2 inhibitor, a glucose reabsorption inhibitor, fenofibrate, fibrate, metformin, an anti-ghrelin antibody or antibody fragment, an fibroblast growth factor receptor (FGFR)-1(IIIb), FGFR-1(IIIc), antibody or antibody fragment, and/or FGFR-4(IIIc), an anti-CD38 antibody or antibody fragment, an anti-MIC-1 antibody or MIC-1 binding fragment, or a combination of any of the foregoing. For example, said other anti-diabetic or anti-obesity agent may comprise metformin.

### BRIEF DESCRIPTION OF THE SEVERAL VIEWS OF THE DRAWINGS

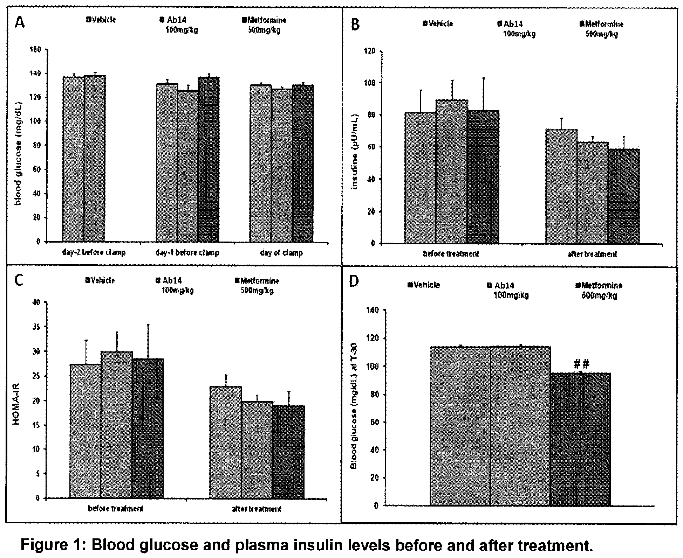

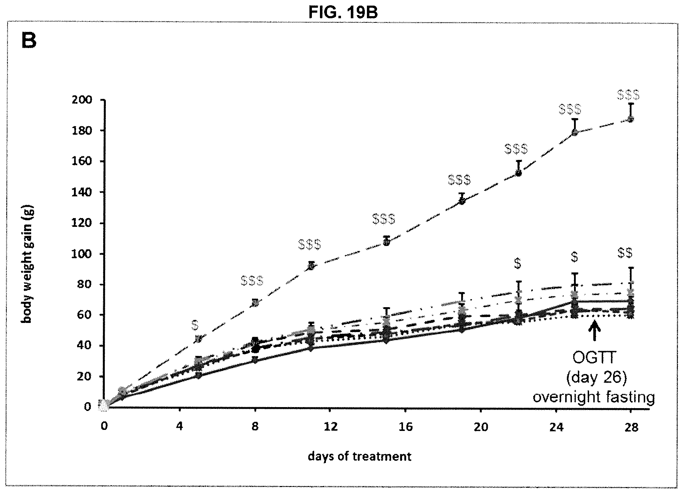

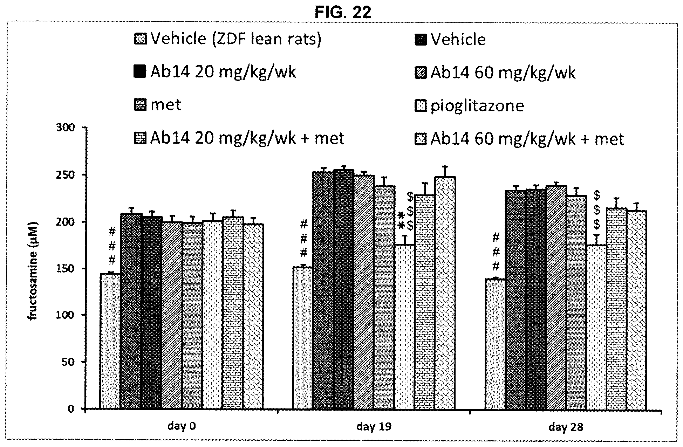

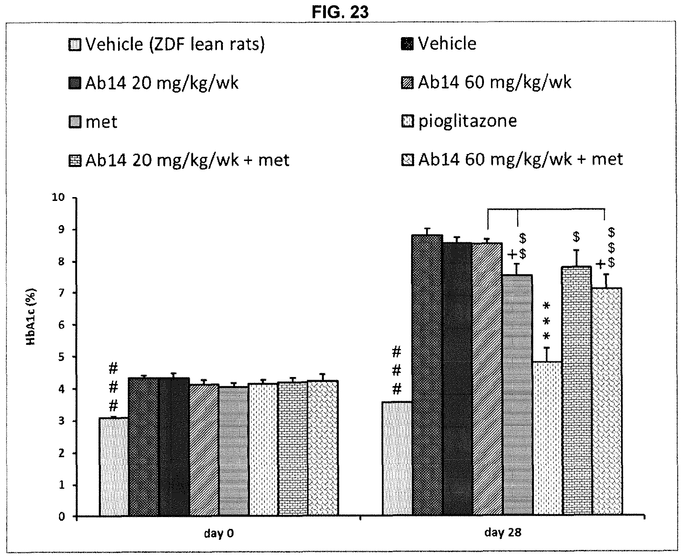

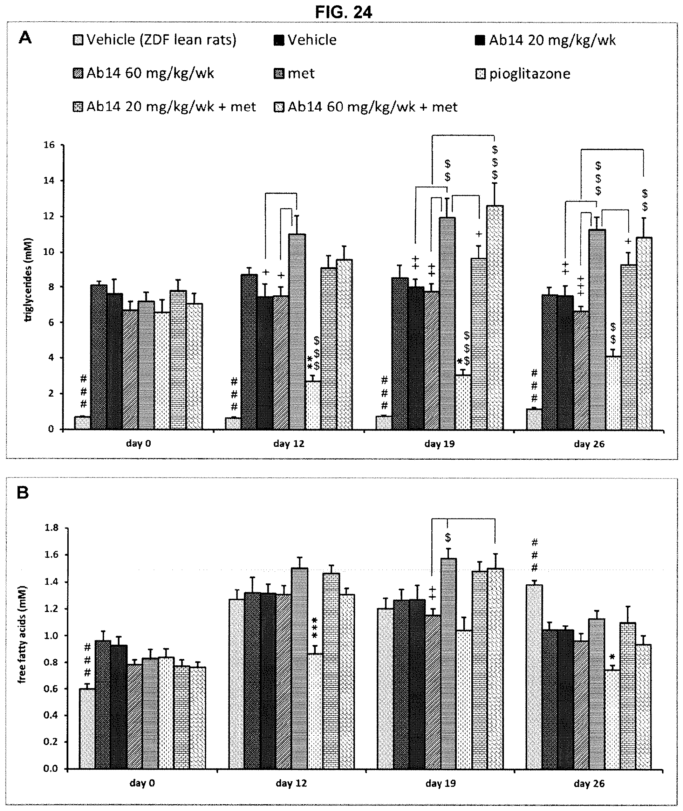

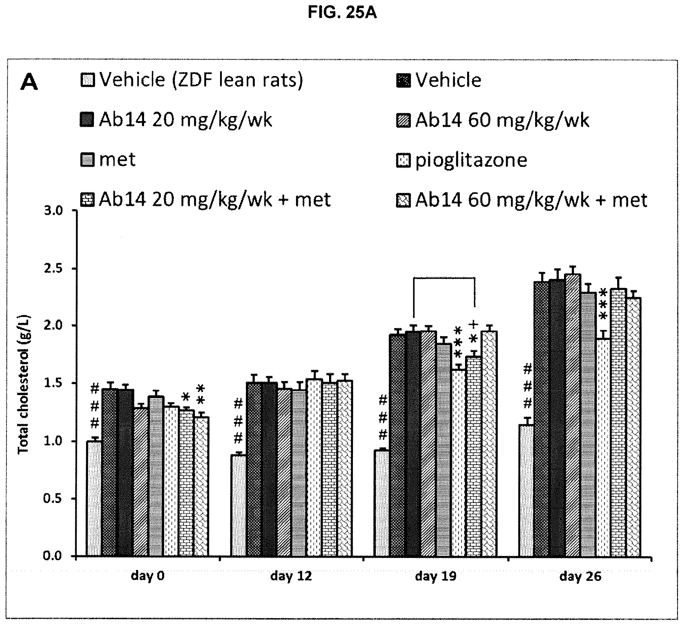

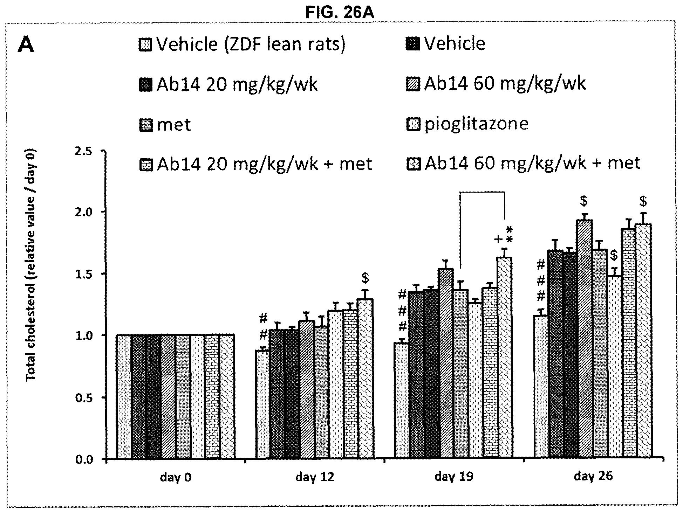

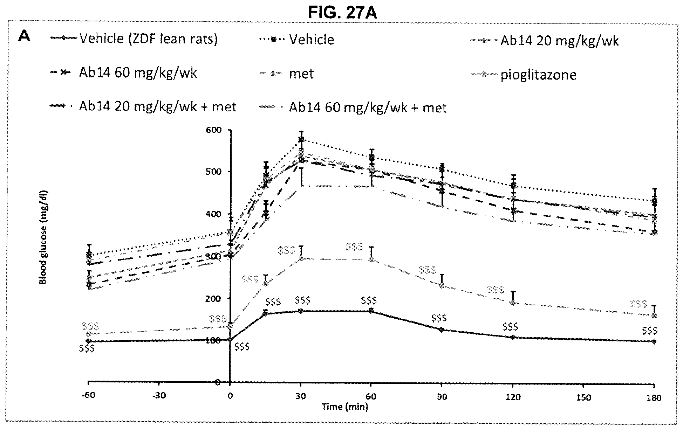

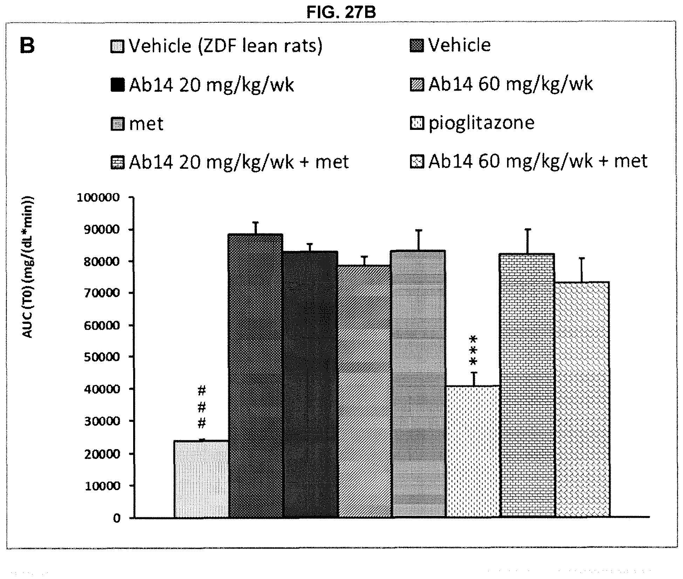

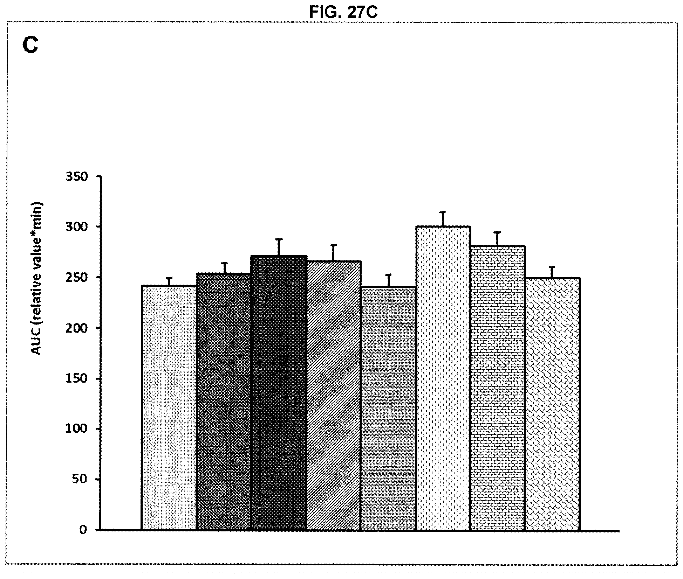

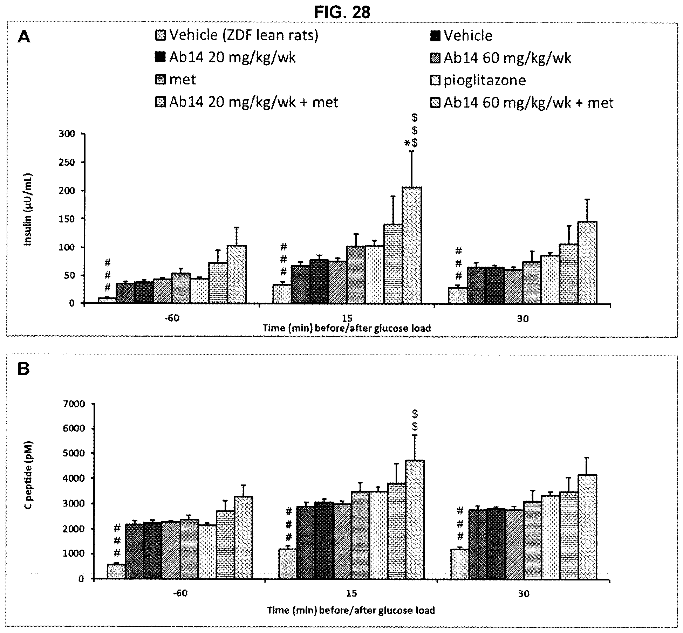

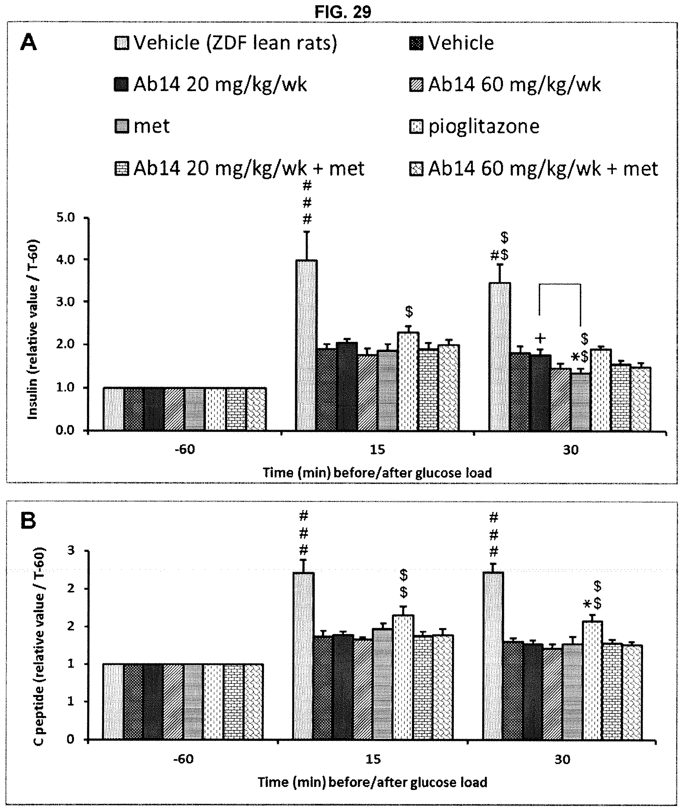

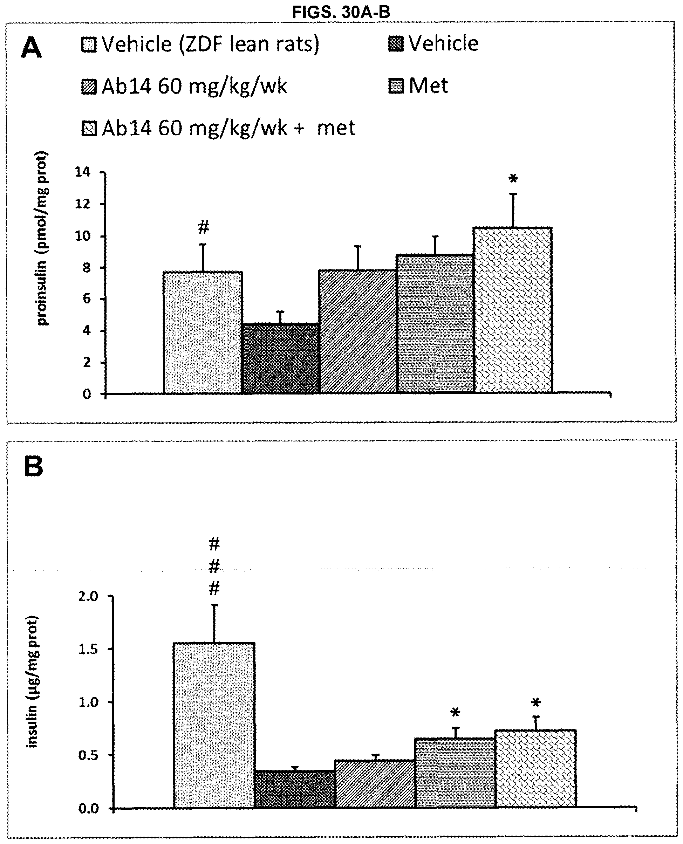

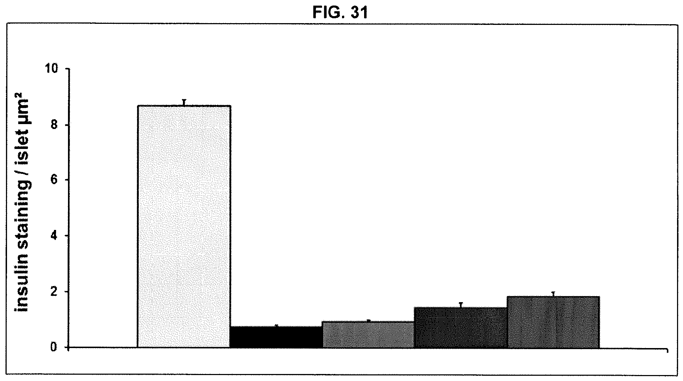

**FIG. 1A-D**. Blood glucose and plasma insulin levels before and after treatment. Results are expressed as the mean ± SEM. ## p<0.01 vs vehicle with an ANOVA one way + Dunnett's post test. A: Blood glucose was measured in fed condition before treatments, 18h after treatment with vehicle, Ab14 and metformin and 42h after treatment with vehicle and Ab14. B: Plasma insulin was measured in fed condition before treatments, 18h after treatment with metformin and 42h after treatment with vehicle and Ab14. C: HOMA-IR (insulin resistance index = glucose (mM) X insulin (µU/mL) / 22.5) was calculated before treatment, 18h after treatment with metformin and 42h after treatment with vehicle and Ab14 D: Blood glucose was measured in fasted condition just before the clamp (24h after treatment with metformin and 48h after treatment with vehicle and Ab14). Legend: Leftmost bar in each group, vehicle; middle bar in each group, Ab14 treatment; rightmost bar in each group, metformin treatment.

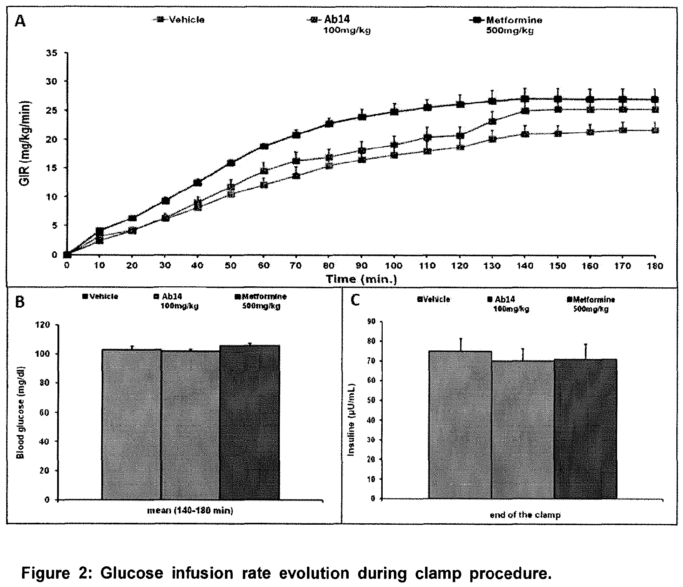

**FIG. 2A-C.** Glucose infusion rate evolution during clamp procedure (A), blood glucose mean during steady state (B) and plasma insulin levels at the end of the clamp (C). Results are expressed as the mean ± SEM. A: ^{∗}p<0.05, ^{∗∗}p<0.01, ^{∗∗∗}p<0.001 vs vehicle with an ANOVA two way with Bonferroni's post test. Legend for FIG. 2A: upper line, metformin treatment; middle line, Ab14 treatment; lower line, vehicle treatment (at 180 min time point). Legend for FIG. 2B-2C: Leftmost bar in each group, vehicle; middle bar in each group, Ab14 treatment; rightmost bar in each group, metformin treatment.

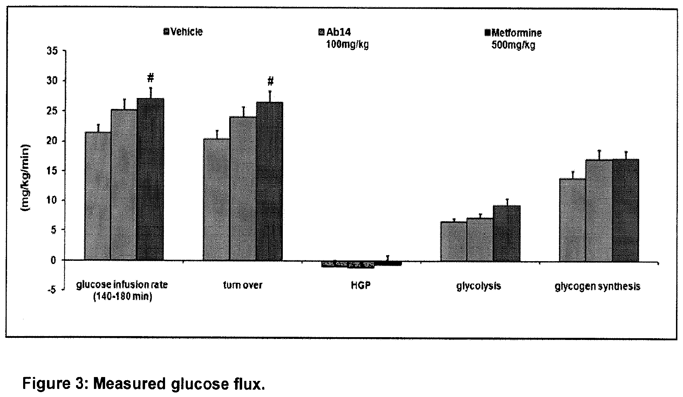

**FIG. 3****.** Measured glucose flux. Results are expressed as the mean ± SEM. # p<0.05 vs vehicle with an ANOVA one way with Dunnett's post test. Clamps were performed under 6 hours fasting conditions. 0.3U/Kg/h insulin and ³H-glucose were perfused for 180 minutes. Glucose infusion rate, whole body turn over, hepatic glucose production (HGP), glycolysis and glycogen synthesis means were calculated between 140 and 180 minutes corresponding to the steady state. Legend: Leftmost bar in each group, vehicle; middle bar in each group, Ab14 treatment; rightmost bar in each group, metformin treatment.

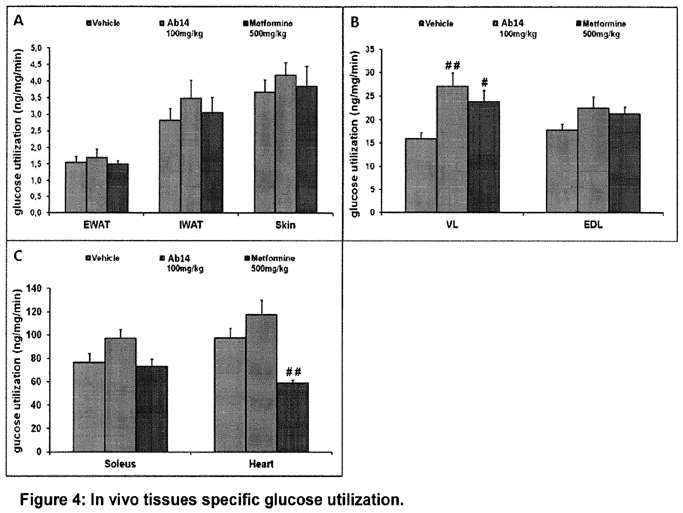

**FIG. 4A-C.** In vivo tissues specific glucose utilization. Results are expressed as the mean ± SEM. # p<0.05, ## p<0.01 vs vehicle with an ANOVA one way with Dunnett's post test. A: glucose utilization in epididymal white adipose tissue (EWAT), inguinal white adipose tissue (IWAT), and skin (as negative control). B: glucose utilization in mixed vastus lateralis muscle (VL) and glycolytic extensor digitorum longus muscle (EDL). C: glucose utilization in oxidative soleus muscle and heart apex. Legend: Leftmost bar in each group, vehicle; middle bar in each group, Ab14 treatment; rightmost bar in each group, metformin treatment.

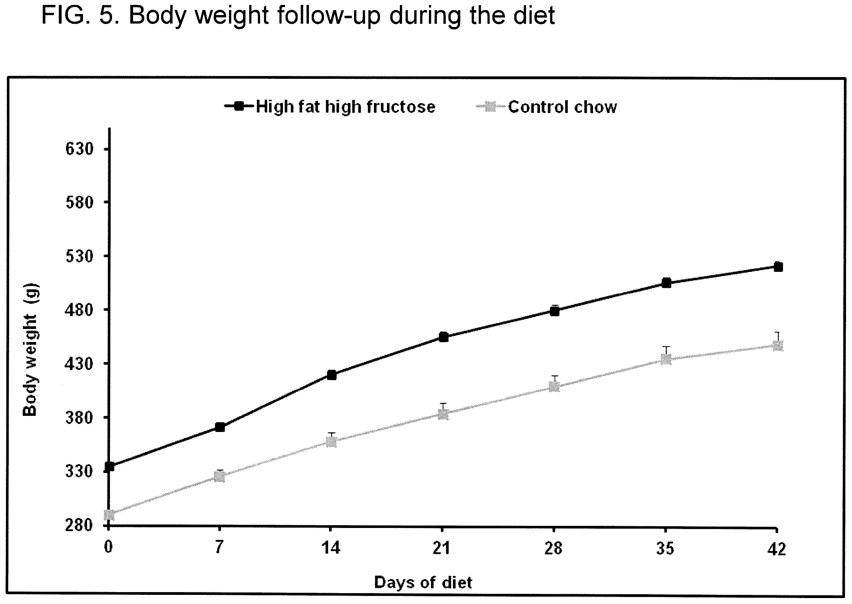

**FIG. 5****.** Average body weight over time for animals fed a high-fat fructose diet or control animals fed normal chow. Legend: Upper line, high fat high fructose diet; lower line, control chow.

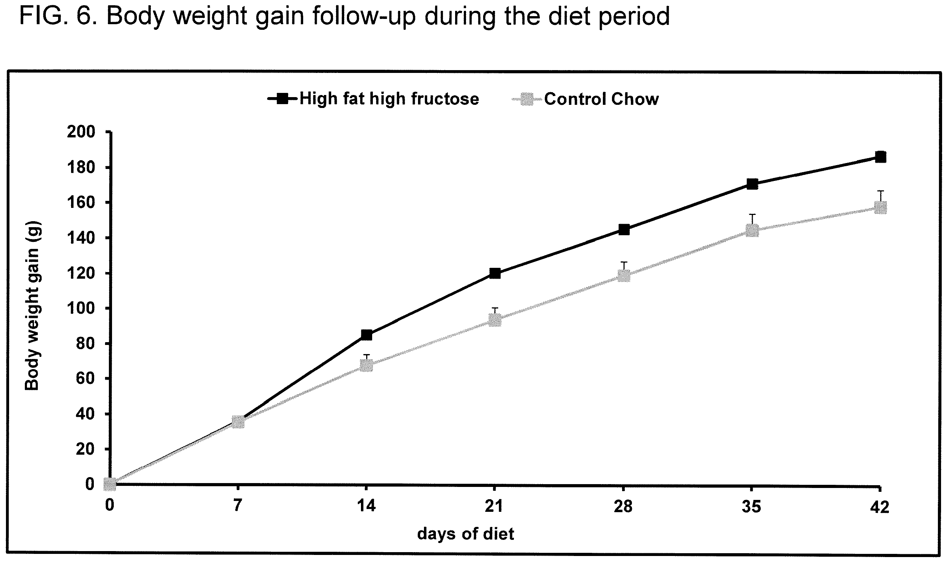

**FIG. 6****.** Body weight gain over time for the animal groups shown in FIG. 5. Upper line: high fat high fructose diet; lower line: control chow. Legend: Upper line, high fat high fructose diet; lower line, control chow.

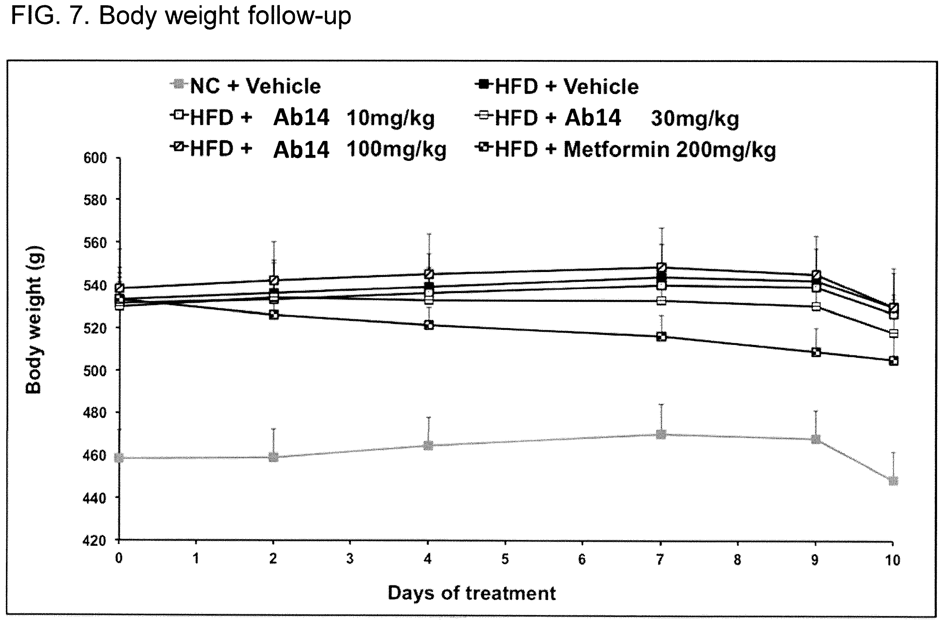

**FIG. 7****.** Body weight gain over time for high-fat diet fed animals after treatment with Ab14 (10, 30, or 100 mg/kg) or metformin, as well as vehicle-treated animals and control animals fed normal chow. Treatment was administered on day 0. Lines on graph in order from lowest to highest at day 7 are: normal chow (NC) plus vehicle; high fat diet (HFD) plus metformin; HFD plus Ab14 30 mg/kg; HFD plus Ab14 10 mg/kg; HFD plus vehicle; HFD plus Ab14 100 mg/kg.

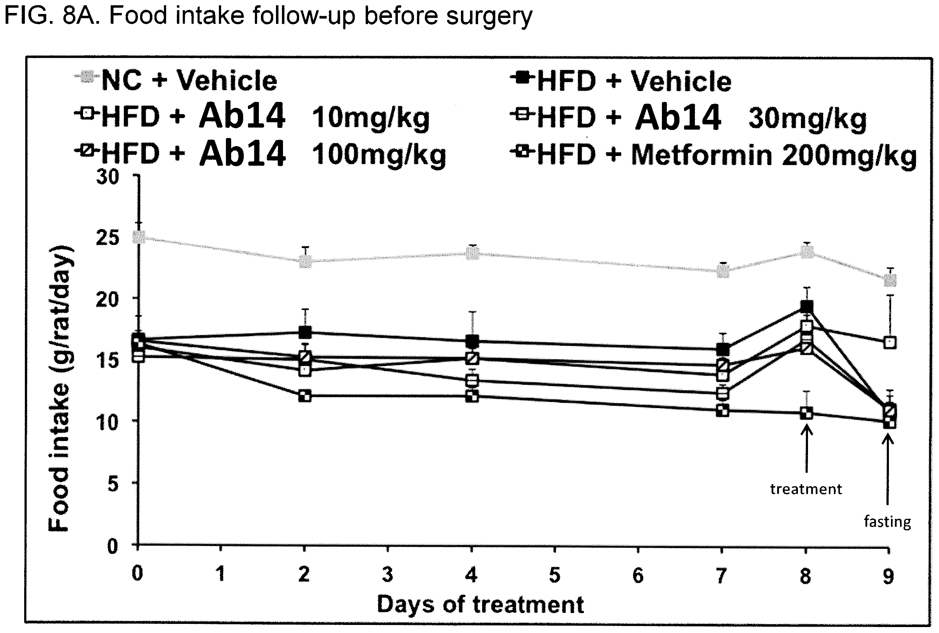

**FIG. 8A****.** Food intake for the animals shown in FIG. 7. Lines on graph in order from lowest to highest at day 7 are: high fat diet (HFD) plus metformin; HFD plus Ab14 30 mg/kg; HFD plus Ab14 10 mg/kg; HFD plus Ab14 100 mg/kg; HFD plus vehicle; normal chow (NC) plus vehicle.

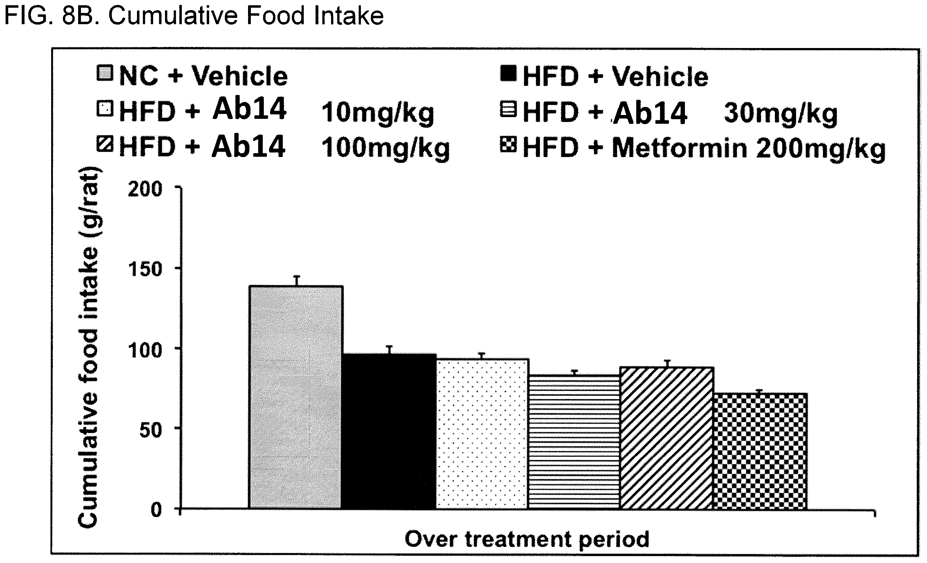

**FIG 8B****.** Cumulative food intake for the animals shown in FIG. 7. Legend: order of bars from left to right is: normal chow (NC) plus vehicle; high fat diet (HFD) plus vehicle; HFD plus Ab14 10 mg/kg; HFD plus Ab14 30 mg/kg; HFD plus Ab14 100 mg/kg; HFD plus metformin.

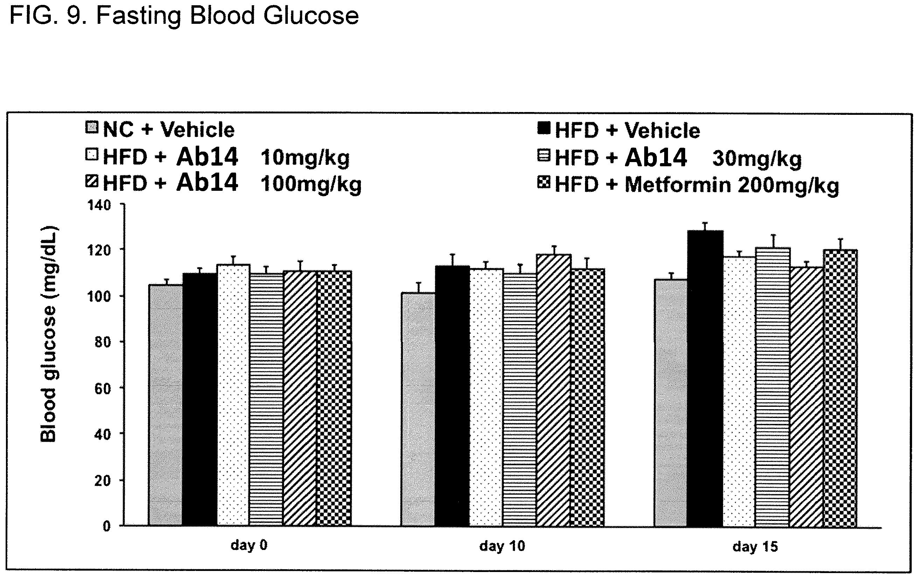

**FIG. 9****.** Fasting blood glucose for high-fat diet fed animals after treatment with Ab14 (10, 30, or 100 mg/kg) or metformin, as well as vehicle-treated animals and control animals fed normal chow. Treatment was administered on day 0. Legend: order of bars from left to right is as in FIG. 8B.

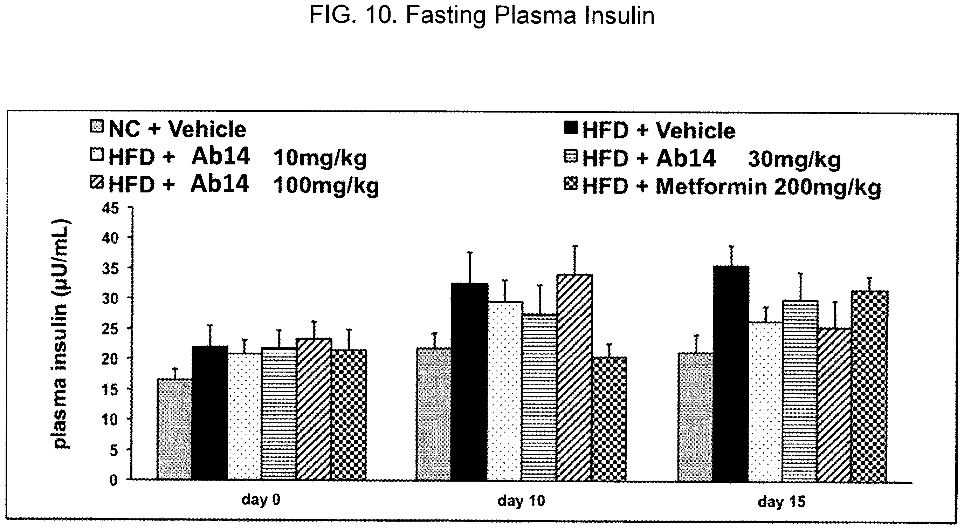

**FIG. 10****.** Fasting plasma insulin for high-fat diet fed animals after treatment with Ab14 (10, 30, or 100 mg/kg) or metformin, as well as vehicle-treated animals and control animals fed normal chow. Treatment was administered on day 0. Legend: order of bars from left to right in each group is as in FIG. 8B.

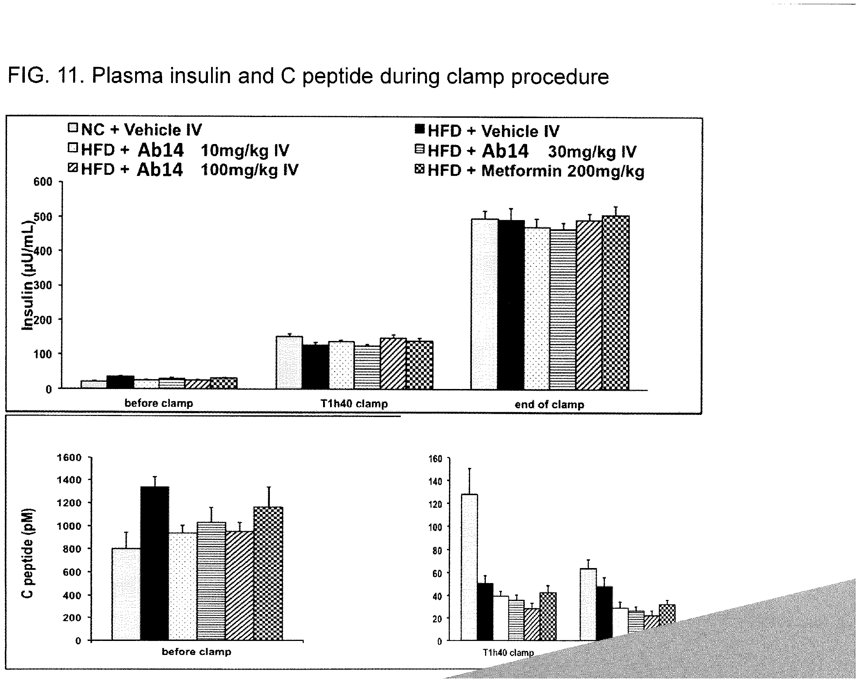

**FIG. 11****.** Plasma insulin (upper panel) and C peptide (lower left and right panels) before and during glucose clamp performed after 15 days of treatment with Ab14 or metformin. Animals were fed a high fat diet for 6 weeks prior to treatment. Legend: order of bars from left to right is as in FIG. 8B.

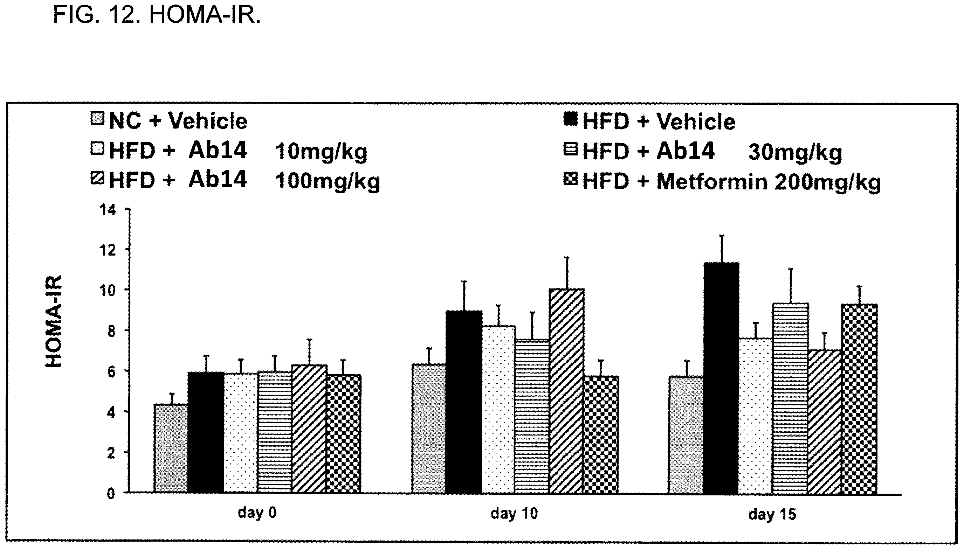

**FIG. 12****.** HOMA-IR for high-fat diet fed animals after treatment with Ab14 (10, 30, or 100 mg/kg) or metformin, as well as vehicle-treated animals and control animals fed normal chow. Treatment was administered on day 0. Legend: order of bars from left to right is as in FIG. 8B.

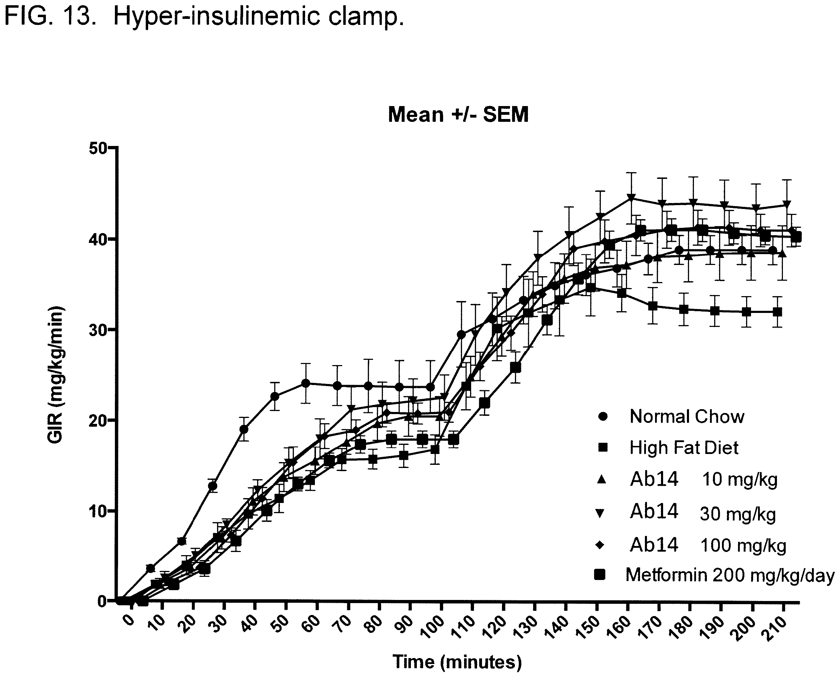

**FIG. 13****.** Glucose infusion rate for glucose clamp performed after 15 days of treatment with Ab14 or metformin, as well as vehicle-treated animals and control animals fed normal chow. Animals were fed a high fat diet for 6 weeks prior to treatment. Glucose clamp was performed at two different insulin infusion rates (5mU/kg/min, steady state achieved at approx. 70-100 min, and 15mU/kg/min, steady state achieved at approx. 170-210 min.) Legend: circle markers, normal chow; medium square markers, high fat diet (HFD) plus vehicle; upward-pointing triangles, HFD plus Ab14 10 mg/kg; downward-pointing triangles, HFD plus Ab14 30 mg/kg; diamonds, HFD plus Ab14 100 mg/kg; large squares, HFD plus metformin. Error bars shown are the mean plus or minus SEM.

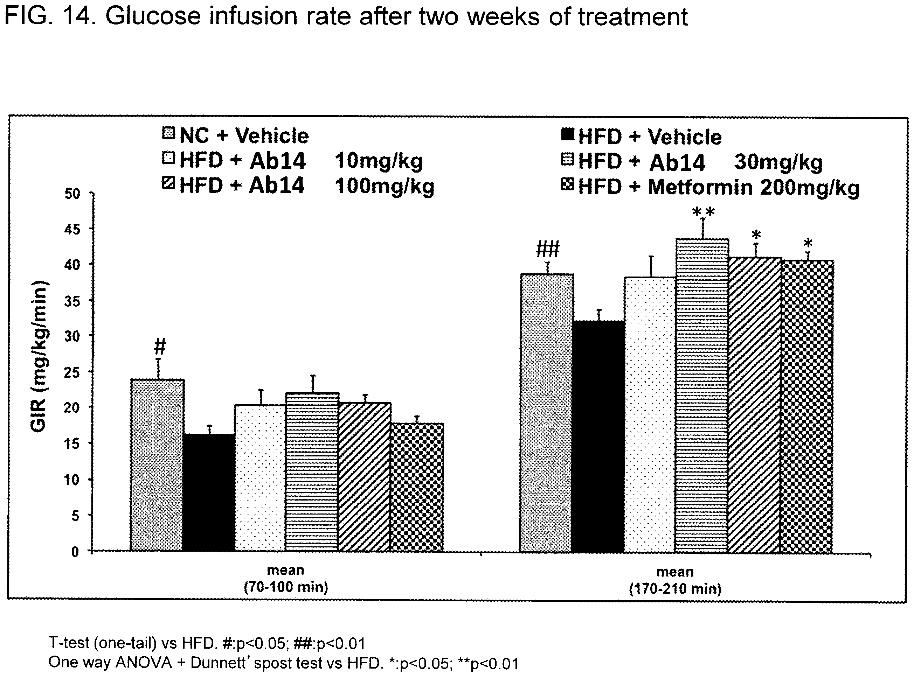

**FIG. 14****.** Mean glucose infusion rate during steady state for the glucose clamp experiments shown in FIG. 13. Glucose infusion rates are shown for the low and high insulin infusion rates (5mU/kg/min, steady state achieved at approx. 70-100 min, and 15mU/kg/min, steady state achieved at approx. 170-210 min.) Order of the bars in each group is as in FIG. 8B.

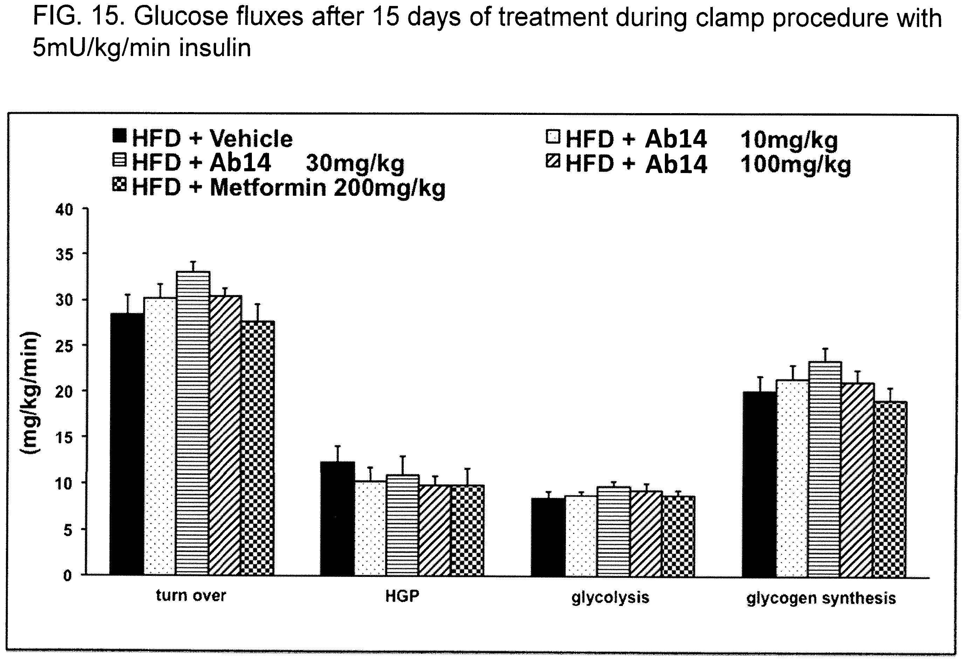

**FIG. 15****.** Mean glucose fluxes during the glucose clamp experiments shown in FIG. 13. Results are shown for the lower (5mU/kg/min) insulin infusion rate, steady state achieved at approx. 70-100 min. Legend: order of bars from left to right in each group is: high fat diet (HFD) plus vehicle; HFD plus Ab14 10 mg/kg; HFD plus Ab14 30 mg/kg; HFD plus Ab14 100 mg/kg; HFD plus metformin.

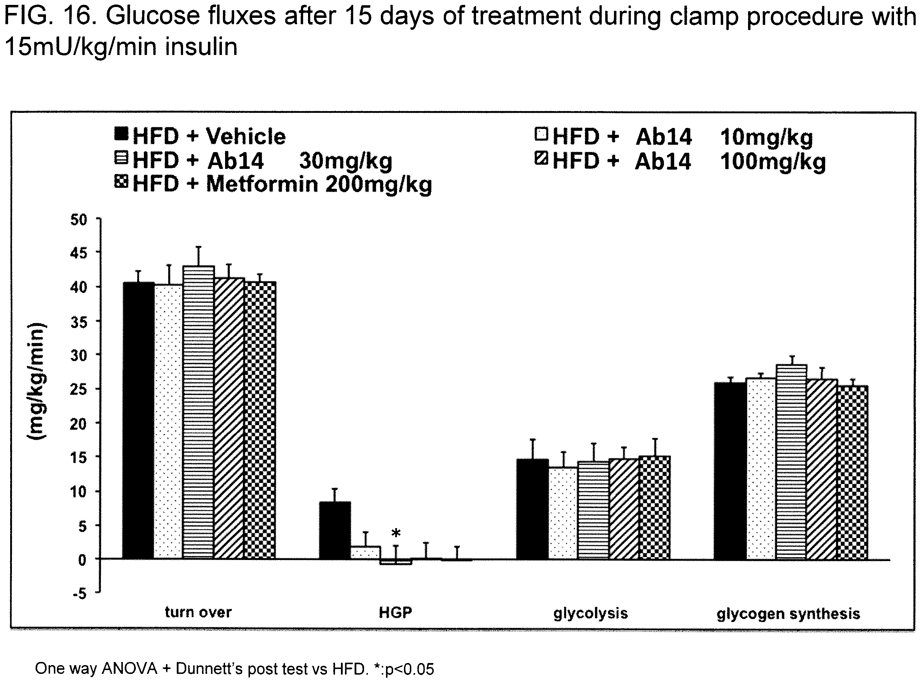

**FIG. 16****.** Mean glucose fluxes during the glucose clamp experiments shown in FIG. 13. Results are shown for the higher (15mU/kg/min) insulin infusion rate, steady state achieved at approx. 170-210 min. Legend: order of bars in each group is as in FIG. 15.

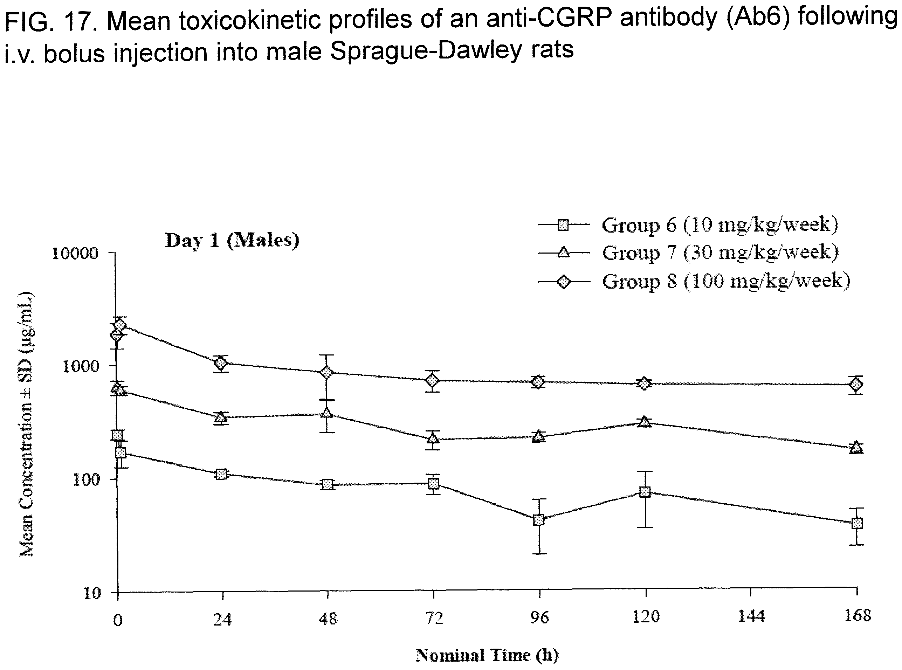

**FIG. 17****.** Mean toxicokinetic profiles of an anti-CGRP antibody (specifically, Ab6) following i.v. bolus injection into male Sprague-Dawley rats. Plasma concentration over time is shown for 168 hours (7 days), supporting weekly dosing as performed in the Examples below. Legend: square markers, Ab14 10 mg/kg/week; upward-pointing triangle markers, Ab14 30 mg/kg/week; diamond markers, Ab14 100 mg/kg/week.

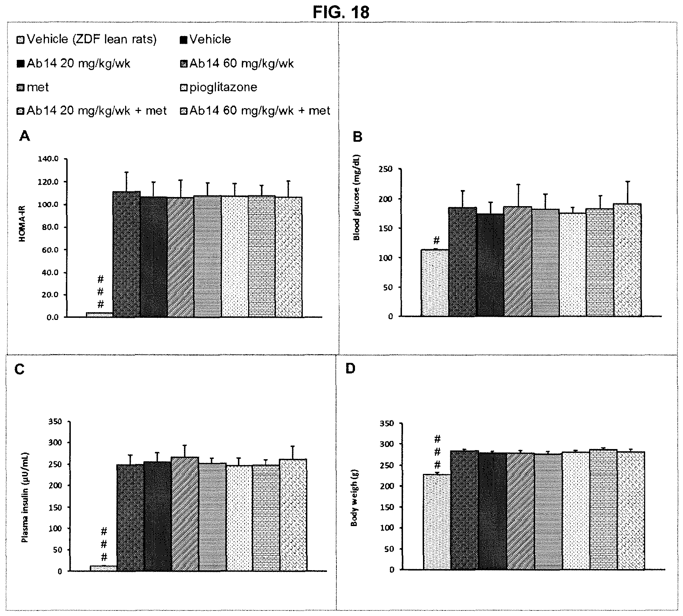

**FIG. 18A-D.** 6 hours fasting HOMA-IR (A), blood glucose (B), plasma insulin (C) and body weight (D) in 8-week old ZDF rats. Results are expressed as mean ± SEM.
   # p<0.05; ### p<0.001 vs vehicle ZDF (Mann Whitney). The order of the bars (from left to right) in FIGS. 18A-D is: (1) vehicle 1 and vehicle 2 treated ZDF lean rats; (2) vehicle 1 and vehicle 2 treated ZDF rats; (3) Ab14 20 mg/kg/week and vehicle 2 treated ZDF rats; (4) Ab14 60 mg/kg/week and vehicle 2 treated ZDF rats; (5) vehicle 1 and metformin ( "met") 200 mg/kg/day treated ZDF rats; (6) vehicle 1 and pioglitazone 10 mg/kg/day treated ZDF rats; (7) Ab14 20 mg/kg/week and metformin 200 mg/kg/day treated ZDF rats; and (8) Ab14 60 mg/kg/week and metformin 200 mg/kg/day treated ZDF rats.

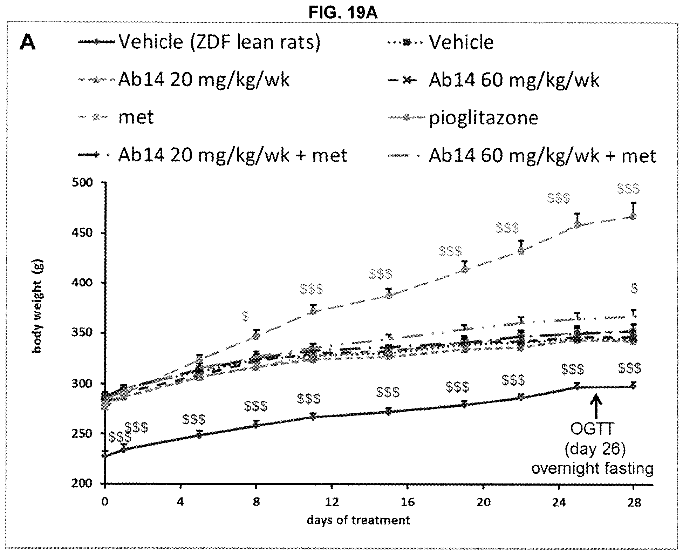

**FIG. 19A-B.** Body weight (A) and body weight gain (B) follow-up. Results are expressed as mean±SEM.
   $ p<0.05 (FIG. 19A: pioglitazone treated rats at day 8; Ab14 60mg/kg/wk + metformin treated rats at day 28; FIG. 19B: Ab14 60mg/kg/wk + metformin treated rats at days 22 and 25); $$ p<0.01 (FIG. 19B: Ab14 60mg/kg/wk + metformin treated rats at day 28); $$$ p<0.001 vs vehicle ZDF (FIG. 19A: pioglitazone treated rats at all time points between days 11-28; vehicle treated ZDF lean rats at all time points) (2-way ANOVA + Bonferroni's post test).
**FIG. 20A-B.** Food intake follow-up (A) and cumulative food consumption (B). Results are expressed as mean±SEM. The order of the bars in FIG. 20B is the same as in FIGS. 18A-D.
   $ p<0.05 (FIG. 20A: pioglitazone treated rats at 20 and 22 days); $$ p<0.01 (FIG. 20A: pioglitazone treated rats at 15 days); $$$ p<0.001 vs vehicle ZDF (FIG. 20a: vehicle treated ZDF lean rats at all time points) (2-way ANOVA + Bonferroni's post test)
   ## p<0.01 vs vehicle ZDF (Mann Whitney)
**FIG. 21A-D.** Blood glucose (A), plasma insulin (B), HOMA-IR (C) and C peptide (D) in 6-hours (day 0) or overnight (days 12, 19, 26) fasting conditions. Results are expressed as mean±SEM. The order of the bars in each group in FIGS. 21A-D is the same as in FIGS. 18A-D.
   # p<0.05; ### p<0.001 vs vehicle ZDF (Mann Whitney)
   ^{∗} p<0.05; ^{∗∗} p<0.01; ^{∗∗∗} p<0.001 vs vehicle ZDF (Kruskal-Wallis + Dunn's post test)
   ++ p<0.01; vs metformin group and AB14 20mg/kg + metformin group (1-way ANOVA + Newman-Keuls post test)
   $ p<0.05; $$ p<0.01 vs vehicle ZDF (2-way ANOVA + Bonferroni's post test)
**FIG. 22****.** Fructosamine levels. Results are expressed as mean±SEM. The order of the bars in each group in FIG. 22 is the same as in FIGS. 18A-D.
   ### p<0.001 vs vehicle ZDF (Mann Whitney)
   ^{∗∗} p<0.01 vs vehicle ZDF (Kruskal-Wallis + Dunn's post test)
   $$$ p<0.001 vs vehicle ZDF (2-way ANOVA + Bonferroni's post test)
**FIG. 23****.** HbAlc levels. Results are expressed as mean±SEM. The order of the bars in each group in FIG. 23 is the same as in FIGS. 18A-D.
   ### p<0.001 vs vehicle ZDF (Mann Whitney)
   ^{∗∗∗} p<0.001 vs vehicle ZDF (Kruskal-Wallis + Dunn's post test)
   + p<0.05; vs AB14 60mg/kg group (1-way ANOVA + Newman-Keuls post test)
   $ p<0.05; $$ p<0.01; $$$ p<0.001 vs vehicle ZDF (2-way ANOVA + Bonferroni's post test)
**FIG. 24A-B.** Plasma triglycerides (A) and free fatty acids (B) levels in 6-hours (day 0) or overnight (days 12, 19, and 26) fasting conditions. Results are expressed as mean±SEM. The order of the bars in each group in FIGS. 24A-D is the same as in FIGS. 18A-D.
   ### p<0.001 vs vehicle ZDF (Mann Whitney)
   ^{∗} p<0.05; ^{∗∗} p<0.01; ^{∗∗∗} p<0.001 vs vehicle ZDF (Kruskal-Wallis + Dunn's post test)
   + p<0.05; ++ p<0.01; +++ p<0.001 vs metformin group or Ab14 60mg/kg + metformin group (1-way ANOVA + Newman-Keuls post test)
   $ p<0.05; $$ p<0.01; $$$ p<0.001 vs vehicle ZDF (2-way ANOVA + Bonferroni's post test)
**FIG. 25A-C.** Plasma Total cholesterol (A), HDL-cholesterol (B) and non HDL-cholesterol (C) levels. Results are expressed as mean±SEM. The order of the bars in each group in FIGS. 25A-C is the same as in FIGS. 18A-D.
   # p<0.05; ## p<0.01; ### p<0.001 vs vehicle ZDF (Mann Whitney)
   ^{∗} p<0.05; ^{∗∗} p<0.01; ^{∗∗∗} p<0.001 vs vehicle ZDF (1-way ANOVA + Dunnett's post test)
   + p<0.05; ++ p<0.01 vs metformin group or Ab14 + metformin groups (1-way ANOVA + Newman-Keuls post test)
   $$ p<0.01 vs vehicle ZDF (2-way ANOVA + Bonferroni's post test)
**FIG. 26A-C.** Plasma Total cholesterol (A), HDL-cholesterol (B) and non HDL-cholesterol (C) levels relative to the day 0. Results are expressed as mean±SEM. The order of the bars in each group in FIGS. 26A-C is the same as in FIGS. 18A-D.
   # p<0.05; ## p<0.01; ### p<0.001 vs vehicle ZDF (unpaired t-test)
   ^{∗} p<0.05; ^{∗∗} p<0.01 vs vehicle ZDF (1-way ANOVA + Dunnett's post test)
   + p<0.05; vs metformin group (1-way ANOVA + Newman-Keuls post test)
   $ p<0.05; $$$ p<0.001 vs vehicle ZDF (2-way ANOVA + Bonferroni's post test)
**FIG. 27A-C.** Oral glucose tolerance test on day 26 in overnight fasting conditions (A), area under the curve (AUC) calculated from the blood glucose measured on T0 (B) and calculated from relative value vs T0 (C). Results are expressed as mean±SEM. The order of the bars in FIGS. 27B-C is the same as in FIGS. 18A-D.
   $$$ p<0.001 vs vehicle ZDF (2-way ANOVA + Bonferroni's post test) (FIG 27A: all time points shown for vehicle-treated ZDF lean rats and pioglitazone-treated rats).
   ### p<0.001 vs vehicle ZDF (Mann Whitney)
   ^{∗∗∗} p<0.001 vs vehicle ZDF (Kruskal-Wallis + Dunn's post test)
**FIG. 28A-B.** Plasma insulin (A) and C peptide (B) levels during oral glucose tolerance test on day 26. Results are expressed as mean±SEM. The order of the bars in each group in FIGS. 28A-B is the same as in FIGS. 18A-D.
   ### p<0.001 vs vehicle ZDF (Mann Whitney)
   ^{∗} p<0.05 vs vehicle ZDF (Kruskal-Wallis + Dunn's post test)
   $$ p<0.01; $$$ p<0.001 vs vehicle ZDF (2-way ANOVA + Bonferroni's post test)
**FIG. 29A-B.** Relative expression from T-60 of plasma insulin (A) and C peptide (B) levels during oral glucose tolerance test on day 26. Results are expressed as mean±SEM. The order of the bars in each group in FIGS. 29A-B is the same as in FIGS. 18A-D.
   # p<0.05; ### p<0.001 vs vehicle ZDF (Mann Whitney)
   ^{∗} p<0.05 vs vehicle ZDF (1-way ANOVA + Dunnett's post test)
   + p<0.05; vs metformin group (1-way ANOVA + Newman-Keuls post test)
   $ p<0.05; $$ p<0.01 vs vehicle ZDF (2-way ANOVA + Bonferroni's post test)
**FIG. 30A-C.** Pancreas content: proinsulin (A), insulin (B) and proinsulin/insulin ratio (C). Results are expressed as mean ± SEM. The order of bars (from left to right) in FIGS. 30A-C is: vehicle 1 and vehicle 2 treated ZDF lean rats; vehicle 1 and vehicle 2 treated ZDF rats; Ab14 60 mg/kg/week and vehicle 2 treated ZDF rats; vehicle 1 and metformin 200 mg/kg/day treated ZDF rats; and Ab14 60 mg/kg/week and metformin 200 mg/kg/day treated ZDF rats.
   # p<0.05; ### p<0.001 vs vehicle ZDF (Mann Whitney)
   ^{∗} p<0.05 vs vehicle ZDF (1-way ANOVA + Newman-Keuls post test)
**FIG. 31****.** Pancreas immunohistochemical analysis: insulin labelling quantification. The order of bars (from left to right) in FIG. 31 is: vehicle 1 and vehicle 2 treated ZDF lean rats; vehicle 1 and vehicle 2 treated ZDF rats; Ab14 60 mg/kg/week and vehicle 2 treated ZDF rats; vehicle 1 and metformin 200 mg/kg/day treated ZDF rats; and Ab14 60 mg/kg/week and metformin 200 mg/kg/day treated ZDF rats.

### DETAILED DESCRIPTION

The present invention is defined by the claims.

The present inventors discovered that anti-CGRP antibodies produced significantly increased glucose utilization in peripheral muscle when compared to metformin, without any apparent increase in the glucose utilization rate in white adipose tissue. Moreover, the anti-CGRP antibodies described herein increased glucose utilization in heart, whereas metformin produced a decrease in the glucose utilization rate in the heart. Additionally, anti-CGRP antibodies described herein inhibited hepatic glucose production, similarly to the effect obtained from administration of metformin.

The anti-CGRP antibody, Ab14, which is a potent functional antagonist, was evaluated in preclinical animal models of normal and altered glucose metabolism to determine its effects on insulin sensitivity and glycemic control in normal rats (Example 1), in hyperinsulinemic but normoglycemic diet-induced obese (DIO) rats that had been fed a high fat/high fructose diet for six weeks to induce the metabolic syndrome (Example 2), and in Zucker diabetic fatty (ZDF) rats that were progressing from a prediabetic (hyperinsulinemic, normoglycemic) state to an overtly diabetic (hypoinsulinemic, hyperglycemic) state (Example 3).

In Example 1, a hyperinsulinemic-euglycemic clamp study was performed with Ab14 to determine its effects on whole body insulin sensitivity as well as on the insulin sensitivity of specific tissues in normal rats that are normoglycemic, normoinsulinemic, and have normal whole body and tissue-specific insulin sensitivities. Ab14 was given intravenously as a single 100 mg/kg administration to normal rats 48 hrs prior to a hyperinsulinemic-euglycemic clamp procedure. Results from the evaluation of plasma glucose and insulin levels measured just prior to the clamp procedure indicated that Ab14 reduced plasma insulin levels relative to vehicle-treated controls without altering plasma glucose levels. The resulting reductions in HOMA-IR indicated improvements in whole body insulin sensitivity.

The hyperinsulinemic-euglycemic clamp procedure confirmed this improvement in whole body insulin sensitivity by CGRP antagonism, where at steady state, both the glucose infusion rate and whole body glucose turnover (utilization) rate were elevated relative to vehicle-treated controls. Increased glucose infusion rate and whole body glucose turnover with a constant insulin infusion was indicative of increased whole body insulin sensitivity.

Consistent with the increase in glucose infusion rate and whole body glucose turnover, hepatic glucose utilization for glycolysis and glycogen synthesis were increased by Ab14 relative to vehicle-treated controls and hepatic glucose production was reduced. These observations are indicative of increased hepatic insulin sensitivity by CGRP antagonism resulting in an increased hepatic utilization of the greater supply of internalized glucose for both energy generation and for storage while at the same time inhibiting de novo hepatic glucose production.

CGRP antagonism also increased glucose utilization in glycolytic as well as oxidative skeletal muscle (vastus lateralis, indicative of mixed glycolytic plus oxidative; extensor digitorum longus, indicative of glycolytic, and soleus, indicative of oxidative). The greatest increases in glucose utilization occurred in the mixed metabolic vastus lateralis. These observations are indicative of increased skeletal muscle insulin sensitivity caused by CGRP antagonism. CGRP antagonism also increased cardiac glucose utilization. In contrast, glucose utilization rates in visceral or subcutaneous fat depots were not affected, suggesting that CGRP antagonism did not substantially increase insulin sensitivity in white adipose tissue.

As mentioned above, the animals used in this study were normoglycemic rats with normal whole body and tissue-specific insulin sensitivities, rendering improvements in insulin sensitivity in these animals more difficult to demonstrate. Therefore, although improvements in some of the individual endpoints evaluated in this study did not reach statistical significance, the observation that they trended in the same direction as those that did suggests that increased study power would allow additional measured parameters to reach statistical significance. Furthermore, since in general there is a high degree of translation of the results of hyperinsulinemic-euglycemic clamp studies performed in experimental animals to hyperinsulinemic-euglycemic clamp studies performed in the clinical setting, these observations suggest the potential for CGRP antagonism to improve whole body and tissue-specific insulin sensitivity in humans.

In Example 2, the effects of CGRP antagonism by chronic administration of Ab14 on hepatic and peripheral insulin sensitivity in insulin-resistant animals were evaluated in rats made hyperinsulinemic and insulin-resistant but not hyperglycemic by prolonged feeding of a high fat/high fructose diet. Rats were fed a diet containing 69% fat and 14% fructose for seven weeks prior to initiation of compound administration to induce the metabolic syndrome. At the end of the seven week diet treatment period, rats continued to receive the high fat/high fructose diet and in addition were given Ab14 intravenously at doses of 0 mg/kg (vehicle), 10mg/kg, 30 mg/kg, or 100 mg/kg once a week for 2 weeks.

When compared to the high fat diet fed vehicle-treated control group, CGRP antagonism had no effect on food consumption or body weight, indicating that effects of CGRP antagonism on the additional parameters evaluated below were not a result of caloric restriction or weight loss.

At the end of the treatment period, all doses of Ab14 reduced HOMA-IR relative to vehicle-treated controls, indicative of improvements in whole body insulin sensitivity. This reduction in HOMA-IR was primarily due to a reduction in plasma insulin levels, which occurred at all doses of Ab14. The reduction in plasma insulin was a result of diminished insulin production rather than increased insulin degradation, since plasma C-peptide, a bi-product of pancreatic insulin synthesis, was reduced in parallel to the reduction in plasma insulin. Plasma glucose levels were only slightly reduced by the lower two doses of Ab14 but were substantially reduced relative to vehicle-treated controls by the 100 mg/kg dose of Ab14.

Immediately after the final day of treatment, a two-step hyperinsulinemic-euglycemic clamp procedure was performed using first, infusion of a physiological amount of insulin and second, a supraphysiological insulin concentration. All three doses of Ab14 increased the steady state glucose infusion rate relative to vehicle-treated controls after both physiological and supraphysiological insulin infusion concentrations. This is consistent with an improvement in whole body insulin sensitivity. All three doses of Ab14 also increased whole body glucose turnover (utilization) rates, increased hepatic glucose utilization for glycolysis and glycogen synthesis, and inhibited hepatic glucose production relative to vehicle-treated controls after infusion of physiological insulin concentrations, consistent with improvements in both whole body and hepatic insulin sensitivity. Hepatic glucose production was also completely prevented after infusion of supraphysiological insulin concentrations.

The similarities between the acute effects of CGRP antagonism in normal rats (Example 1) and its chronic effects in normoglycemic, hyperinsulinemic, insulin-resistant rats (Example 2) indicate the ability of CGRP antagonism to function chronically to treat established insulin resistance. In addition, as mentioned above, since in general there is a high degree of translation of the results of hyperinsulinemic-euglycemic clamp studies performed in experimental animals to hyperinsulinemic-euglycemic clamp studies performed in the clinical setting, these observations suggest the potential for CGRP antagonism to improve whole body and tissue-specific insulin sensitivity in insulin-resistant humans with pre-diabetes or with the metabolic syndrome. Finally, the ability of CGRP antagonism to reduce plasma glucose levels in these normoglycemic animals, albeit only at the highest dose evaluated, suggests the potential for CGRP antagonism to also reduce plasma glucose levels in hyperglycemic patients.

In Example 3, the effects of chronic administration of Ab14 on glucose control was evaluated in ZDF rats that were progressing from a prediabetic (hyperinsulinemic, normoglycemic) state to an overtly diabetic (hypoinsulinemic, hyperglycemic) state. These animals develop prediabetes, characterized by marked hyperinsulinemia to compensate for their developing insulin resistance, but with little to no hyperglycemia, by seven weeks of age. This rapidly progresses to overt diabetes, characterized by hypoinsulinemia, as a result of pancreatic beta cell failure, and marked hyperglycemia by 10-12 weeks of age.

At 8 weeks of age, ZDF rats were screened according to their HOMA-IR and treated with Ab14 at 20 or 60 mg/kg once weekly for 28 days. In addition to evaluating the actions of the CGRP antagonist Ab14 on glycemic control in this animal model, the effects of CGRP antagonism in combination with the marketed drug metformin (200 mg/kg/day) were also evaluated. Metformin alone produced a partial prevention of the rise in fasting blood glucose, a partial prevention of the reduction in plasma insulin and C-peptide levels, a complete prevention of the reduction in pancreatic proinsulin levels, a partial prevention in the reduction in pancreatic insulin levels, and a reduction in pancreatic islet vacuolation, hyperplasia, and fibrosis that were of magnitudes similar to those described above for the high dose of Ab14. However, the combination of Ab14 and metformin produced a substantially greater prevention of the rise in fasting blood glucose, the reduction in plasma insulin and C-peptide levels, the reductions in pancreatic proinsulin and insulin levels, and the reduction in pancreatic islet fibrosis than either compound alone. This suggests that the effects of metformin may be enhanced by a CGRP antagonist such as Ab14.

Additionally, the combination of Ab14 and metformin resulted in a substantial reduction in HbAlc levels (a marker of hemoglobin glycation) and to a lesser extent a reduction in fructosamine levels (a marker of plasma albumin glycation) relative to vehicle-treated controls after 28 days of treatment. This is consistent with the greater reduction of plasma glucose levels produced by the combination of Ab14 plus metformin than with either agent alone. These results suggest that the combination of a CGRP antagonist and metformin can favorably affect hyperglycemia-mediated diabetic complications.

Similarly, the combination of the high dose of Ab14 with metformin showed an improvement in glucose excursion and glucose AUC relative to vehicle-treated animals after administration of the glucose bolus during an oral glucose tolerance test (oGTT) performed on day 26 of the study. Moreover, because by day 26 of the study the beta cell destruction in these animals had progressed past the point of their ability to increase insulin secretion in response to a glucose challenge, it is expected that an even more substantial improvement in glucose excursion and glucose AUC with the combination of Ab14 plus metformin could have been observed had the oGTT been performed two weeks earlier or at another time point prior to complete beta cell destruction.

The ZDF rat used in Example 3 is a very severe model of diabetes progression that advances rapidly from an insulin-resistant prediabetic state to overt diabetes with complete beta-cell destruction occurring over a time-course of only a few weeks. This limits the opportunity to evaluate modulations of disease progression, rendering compound-related improvements in disease progression and beta-cell protection difficult to demonstrate in these animals. Therefore, any demonstration of a modest delay in disease progression by the CGRP antagonist Ab14 as outlined above suggests the potential to also affect disease progression in the clinic. In addition, the ability to improve the overall treatment efficacy through combination of CGRP antagonism with metformin also supports improved efficacy of combination therapy in the clinic.

The results of the examples presented in this application indicate that CGRP antagonism has the ability to improve whole body insulin sensitivity, hepatic insulin sensitivity, and skeletal muscle insulin sensitivity. These improvements can be observed acutely or chronically in normal animals that are normoinsulinemic and normoglycemic and have normal insulin sensitivity, as well as in insulin-resistant animals that are hyperinsulinemic but not yet hyperglycemic. These results indicate that CGRP antagonism should decrease the insulin resistance that presents in patients with the metabolic syndrome, prediabetes, or other prediabetic conditions and further that CGRP antagonism may be capable of slowing the progression of these diseases to overt diabetes.

In addition, the ability of the CGRP antagonist Ab14 to reduce the hyperinsulinemia present in insulin-resistant animals by reducing pancreatic insulin secretion suggests that Ab14 may have a pancreatic beta-cell sparing effect by allowing the pancreas of an insulin-resistant animal to rest. This may further delay the progression of the metabolic syndrome, prediabetes, and other prediabetic conditions to overt diabetes in the clinic.

Furthermore, the ability of Ab14 to reduce plasma glucose levels in insulin-resistant, hyperinsulinemic but normoglycemic rats and to slow the progression from prediabetes to overt diabetes in ZDF rats and to maintain a reduction of plasma glucose levels in overtly diabetic animals that have little to no residual ability to increase insulin production indicates that CGRP antagonism may have the ability to affect disease progression not only in the prediabetic states outlined above but also in overt diabetes.

Thus, taken together, the results of these studies clearly indicate that a CGRP antagonist such as Ab14 may favorably affect insulin resistance and abnormal glucose control in a clinical setting both in patients with prediabetic conditions and also in patients with developing or overt diabetes.

Finally, the ability of the CGRP antagonist Ab14 to enhance the actions of metformin in the ZDF rat suggests that an Ab14-metformin combination therapy has the potential to be of a superior clinical efficacy relative to either Ab14 or metformin alone for treating patients with prediabetic conditions, patients with developing diabetes, and patients with overt diabetes.

### Definitions

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention, which will be limited only by the appended claims. As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

*Calcitonin Gene Related Peptide* (*CGRP*): As used herein, CGRP encompasses not only the following *Homo sapiens* CGRP-alpha and *Homo sapiens* CGRP-beta amino acid sequences available from American Peptides (Sunnyvale CA) and Bachem (Torrance, CA):

CGRP-alpha: ACDTATCVTHRLAGLLSRSGGVVKNNFVPTNVGSKAF-NH₂ (SEQ ID NO: 281), wherein the N-terminal phenylalanine is amidated. Except where indicated otherwise, in general references to "CGRP" typically refer to CGRP-alpha. CGRP-alpha is referred to interchangeably as αCGRP or α-CGRP.

CGRP-beta: ACNTATCVTHRLAGLLSRSGGMVKSNFVPTNVGSKAF-NH₂ (SEQ ID NO: 282), wherein the N-terminal phenylalanine is amidated; but also any membrane-bound forms of these CGRP amino acid sequences, as well as mutants (muteins), splice variants, isoforms, orthologs, homologues and variants of this sequence. CGRP-beta is referred to interchangeably as βCGRP or β-CGRP.

*Normoglycemia*: In the present disclosure, the terms normoglycemia or euglycemia refer to the state of having a normal blood glucose concentration. An exemplary normal blood glucose concentration in humans is between 70 mg/dl and 99 mg/dl in fasting adults, and between 70 mg/dl and 140 mg/dl in postprandial adults. Sustained normoglycemia refers to maintenance of normoglycemia for an extensive period of time, e.g., at least one day, at least two days, at least one week, at least two weeks, at least one month, or longer.

*Mating competent yeast species:* In the present specification this is intended to broadly encompass any diploid or tetraploid yeast which can be grown in culture. Such species of yeast may exist in a haploid, diploid, or other polyploid form. The cells of a given ploidy may, under appropriate conditions, proliferate for an indefinite number of generations in that form. Diploid cells can also sporulate to form haploid cells. Sequential mating can result in tetraploid strains through further mating or fusion of diploid strains. The present specification contemplates the use of haploid yeast, as well as diploid or other polyploid yeast cells produced, for example, by mating or spheroplast fusion.

In one example of the specification, the mating competent yeast is a member of the *Saccharomycetaceae* family, which includes the genera *Arxiozyma*; *Ascobotryozyma*; *Citeromyces*; *Debaryomyces*; *Dekkera; Eremothecium; Issatchenkia*; *Kazachstania*; *Kluyveromyces; Kodamaea*; *Lodderomyces; Pachysolen; Pichia; Saccharomyces*; *Saturnispora*; *Tetrapisispora; Torulaspora; Williopsis;* and *Zygosaccharomyces.* Other types of yeast potentially useful in the specification include *Yarrowia; Rhodosporidium*; *Candida; Hansenula; Filobasidium*; *Sporidiobolus*; *Bullera*; *Leucosporidium* and *Filobasidiella.*

In an example of the specification, the mating competent yeast is a member of the genus *Pichia.* In a further example of the specification, the mating competent yeast of the genus *Pichia* is one of the following species: *Pichia pastoris, Pichia methanolica,* and *Hansenula polymorpha* (*Pichia angusta*). In an exemplified example of the specification, the mating competent yeast of the genus *Pichia* is the species *Pichia pastoris.*

*Haploid Yeast Cell:* A cell having a single copy of each gene of its normal genomic (chromosomal) complement.

*Polyploid Yeast Cell:* A cell having more than one copy of its normal genomic (chromosomal) complement.

*Diploid Yeast Cell:* A cell having two copies (alleles) of essentially every gene of its normal genomic complement, typically formed by the process of fusion (mating) of two haploid cells.

*Tetraploid Yeast Cell:* A cell having four copies (alleles) of essentially every gene of its normal genomic complement, typically formed by the process of fusion (mating) of two haploid cells. Tetraploids may carry two, three, four or more different expression cassettes. Such tetraploids might be obtained in *S*. *cerevisiae* by selective mating homozygotic heterothallic a/a and alpha/alpha diploids and in *Pichia* by sequential mating of haploids to obtain auxotrophic diploids. For example, a [met his] haploid can be mated with [ade his] haploid to obtain diploid [his]; and a [met arg] haploid can be mated with [ade arg] haploid to obtain diploid [arg]; then the diploid [his] x diploid [arg] to obtain a tetraploid prototroph. It will be understood by those of skill in the art that reference to the benefits and uses of diploid cells may also apply to tetraploid cells.

*Yeast Mating:* The process by which two haploid yeast cells naturally fuse to form one diploid yeast cell.

*Meiosis:* The process by which a diploid yeast cell undergoes reductive division to form four haploid spore products. Each spore may then germinate and form a haploid vegetatively growing cell line.

*Selectable Marker:* A selectable marker is a gene or gene fragment that confers a growth phenotype (physical growth characteristic) on a cell receiving that gene as, for example through a transformation event. The selectable marker allows that cell to survive and grow in a selective growth medium under conditions in which cells that do not receive that selectable marker gene cannot grow. Selectable marker genes generally fall into several types, including positive selectable marker genes such as a gene that confers on a cell resistance to an antibiotic or other drug, temperature when two temperature sensitive ("ts") mutants are crossed or a ts mutant is transformed; negative selectable marker genes such as a biosynthetic gene that confers on a cell the ability to grow in a medium without a specific nutrient needed by all cells that do not have that biosynthetic gene, or a mutagenized biosynthetic gene that confers on a cell inability to grow by cells that do not have the wild type gene; and the like. Suitable markers include but are not limited to: ZEO; G418; LYS3; MET1; MET3a; ADE1; ADE3; URA3; and the like.

*Expression Vector:* These DNA vectors contain elements that facilitate manipulation for the expression of a foreign protein within the target host cell. Conveniently, manipulation of sequences and production of DNA for transformation is first performed in a bacterial host, *e.g. E. coli,* and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, (b) complement auxotrophic deficiencies, or (c) supply critical nutrients not available from complex media. Exemplary vectors and methods for transformation of yeast are described, for example, in Burke, D., Dawson, D., & Stearns, T. (2000). "Methods in Yeast Genetics: A Cold Spring Harbor Laboratory Course Manual." Plainview, N.Y.: Cold Spring Harbor Laboratory Press.

Expression vectors for use in the methods of the specification will further include yeast specific sequences, including a selectable auxotrophic or drug marker for identifying transformed yeast strains. A drug marker may further be used to amplify copy number of the vector in a yeast host cell.

The polypeptide coding sequence of interest is operably linked to transcriptional and translational regulatory sequences that provide for expression of the polypeptide in yeast cells. These vector components may include, but are not limited to, one or more of the following: an enhancer element, a promoter, and a transcription termination sequence. Sequences for the secretion of the polypeptide may also be included, e.g. a signal sequence, and the like. A yeast origin of replication is optional, as expression vectors are often integrated into the yeast genome. In one example of the specification, the polypeptide of interest is operably linked, or fused, to sequences providing for optimized secretion of the polypeptide from yeast diploid cells.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites or alternatively via a PCR/recombination method familiar to those skilled in the art (Gateway^{®} Technology; Invitrogen, Carlsbad California). If such sites do not exist, the synthetic oligonucleotide adapters or linkers are used in accordance with conventional practice.

Promoters are untranslated sequences located upstream (5') to the start codon of a structural gene (generally within about 100 to 1000 bp) that control the transcription and translation of particular nucleic acid sequences to which they are operably linked. Such promoters fall into several classes: inducible, constitutive, and repressible promoters (that increase levels of transcription in response to absence of a repressor). Inducible promoters may initiate increased levels of transcription from DNA under their control in response to some change in culture conditions, *e.g*., the presence or absence of a nutrient or a change in temperature.

The yeast promoter fragment may also serve as the site for homologous recombination and integration of the expression vector into the same site in the yeast genome; alternatively a selectable marker is used as the site for homologous recombination. *Pichia* transformation is described in Cregg et al. Mol. Cell. Biol. 5:3376-3385, 1985.

Examples of suitable promoters from *Pichia* include the AOX1 and promoter (Cregg et al. Mol. Cell. Biol. 9:1316-1323, (1989)); ICL1 promoter (Menendez et al. Yeast 20(13):1097-108, (2003)); glyceraldehyde-3-phosphate dehydrogenase promoter (GAP) (Waterham et al. Gene 186(1):37-44 (1997)); and FLD1 promoter (Shen et al. Gene 216(1):93-102 (1998)). The *GAP* promoter is a strong constitutive promoter and the AOX and FLD1 promoters are inducible.

Other yeast promoters include ADH1, alcohol dehydrogenase II, GAL4, PHO3, PHO5, Pyk, and chimeric promoters derived therefrom. Additionally, non-yeast promoters may be used in the specification such as mammalian, insect, plant, reptile, amphibian, viral, and avian promoters. Most typically the promoter will comprise a mammalian promoter (potentially endogenous to the expressed genes) or will comprise a yeast or viral promoter that provides for efficient transcription in yeast systems.

The polypeptides of interest may be recombinantly produced not only directly, but also as a fusion polypeptide with a heterologous polypeptide, *e.g*. a signal sequence or other polypeptide having a specific cleavage site at the N-terminus of the mature protein or polypeptide. In general, the signal sequence may be a component of the vector, or it may be a part of the polypeptide coding sequence that is inserted into the vector. The heterologous signal sequence selected preferably is one that is recognized and processed through one of the standard pathways available within the host cell. The *S*. *cerevisiae* alpha factor pre-pro signal has proven effective in the secretion of a variety of recombinant proteins from *P. pastoris.* Other yeast signal sequences include the alpha mating factor signal sequence, the invertase signal sequence, and signal sequences derived from other secreted yeast polypeptides. Additionally, these signal peptide sequences may be engineered to provide for enhanced secretion in diploid yeast expression systems. Other secretion signals of interest also include mammalian signal sequences, which may be heterologous to the protein being secreted, or may be a native sequence for the protein being secreted. Signal sequences include pre-peptide sequences, and in some instances may include propeptide sequences. Many such signal sequences are known in the art, including the signal sequences found on immunoglobulin chains, *e.g*., K28 preprotoxin sequence, PHA-E, FACE, human MCP-1, human serum albumin signal sequences, human Ig heavy chain, human Ig light chain, and the like. For example, see Hashimoto et. al. Protein Eng 11(2) 75 (1998); and Kobayashi et. al. Therapeutic Apheresis 2(4) 257 (1998).

Transcription may be increased by inserting a transcriptional activator sequence into the vector. These activators are cis-acting elements of DNA, usually about from 10 to 300 bp, which act on a promoter to increase its transcription. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter.

Expression vectors used in eukaryotic host cells may also contain sequences necessary for the termination of transcription and for stabilizing the mRNA. Such sequences are commonly available from 3' to the translation termination codon, in untranslated regions of eukaryotic or viral DNAs or cDNAs. These regions contain nucleotide segments transcribed as polyadenylated fragments in the untranslated portion of the mRNA.

Construction of suitable vectors containing one or more of the above-listed components employs standard ligation techniques or PCR/recombination methods. Isolated plasmids or DNA fragments are cleaved, tailored, and re-ligated in the form desired to generate the plasmids required or via recombination methods. For analysis to confirm correct sequences in plasmids constructed, the ligation mixtures are used to transform host cells, and successful transformants selected by antibiotic resistance (e.g. ampicillin or Zeocin) where appropriate. Plasmids from the transformants are prepared, analyzed by restriction endonuclease digestion and/or sequenced.

As an alternative to restriction and ligation of fragments, recombination methods based on att sites and recombination enzymes may be used to insert DNA sequences into a vector. Such methods are described, for example, by Landy Ann. Rev. Biochem. 58:913-949 (1989); and are known to those of skill in the art. Such methods utilize intermolecular DNA recombination that is mediated by a mixture of lambda and *E. coli* -encoded recombination proteins. Recombination occurs between specific attachment (*att*) sites on the interacting DNA molecules. For a description of att sites see Weisberg and Landy (1983) "Site-Specific Recombination in Phage Lambda, in Lambda II", Weisberg, ed. (Cold Spring Harbor, NY:Cold Spring Harbor Press), pp. 211-250. The DNA segments flanking the recombination sites are switched, such that after recombination, the *att* sites are hybrid sequences comprised of sequences donated by each parental vector. The recombination can occur between DNAs of any topology.

*Att* sites may be introduced into a sequence of interest by ligating the sequence of interest into an appropriate vector; generating a PCR product containing *att* B sites through the use of specific primers; generating a cDNA library cloned into an appropriate vector containing att sites; and the like.

*Folding,* as used herein, refers to the three-dimensional structure of polypeptides and proteins, where interactions between amino acid residues act to stabilize the structure. While non-covalent interactions are important in determining structure, usually the proteins of interest will have intra- and/or intermolecular covalent disulfide bonds formed by two cysteine residues. For naturally occurring proteins and polypeptides or derivatives and variants thereof, the proper folding is typically the arrangement that results in optimal biological activity, and can conveniently be monitored by assays for activity, *e.g*. ligand binding, enzymatic activity, *etc.*

In some instances, for example where the desired product is of synthetic origin, assays based on biological activity will be less meaningful. The proper folding of such molecules may be determined on the basis of physical properties, energetic considerations, modeling studies, and the like.

The expression host may be further modified by the introduction of sequences encoding one or more enzymes that enhance folding and disulfide bond formation, *i*.*e*. foldases, chaperonins, *etc.* Such sequences may be constitutively or inducibly expressed in the yeast host cell, using vectors, markers, *etc.* as known in the art. Preferably the sequences, including transcriptional regulatory elements sufficient for the desired pattern of expression, are stably integrated in the yeast genome through a targeted methodology.

For example, the eukaryotic PDI is not only an efficient catalyst of protein cysteine oxidation and disulfide bond isomerization, but also exhibits chaperone activity. Co-expression of PDI can facilitate the production of active proteins having multiple disulfide bonds. Also of interest is the expression of BIP (immunoglobulin heavy chain binding protein); cyclophilin; and the like. In one example of the specification, each of the haploid parental strains expresses a distinct folding enzyme, *e.g.* one strain may express BIP, and the other strain may express PDI or combinations thereof.

The terms *"desired protein"* or *"desired antibody"* are used interchangeably and refer generally to a parent antibody specific to a target, i.e., CGRP or a chimeric or humanized antibody or a binding portion thereof derived therefrom as described herein. The term "antibody" is intended to include any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." A source for producing antibodies useful as starting material according to the specification is rabbits. Numerous antibody-coding sequences have been described; and others may be raised by methods well-known in the art. Examples thereof include chimeric antibodies, human antibodies and other non-human mammalian antibodies, humanized antibodies, single chain antibodies (such as scFvs), camelbodies, nanobodies, IgNAR (single-chain antibodies derived from sharks), small-modular immunopharmaceuticals (SMIPs), and antibody fragments such as Fab, F(ab')₂ and the like. See, Streltsov VA, et al., Structure of a shark IgNAR antibody variable domain and modeling of an early-developmental isotype, Protein Sci. Nov;14(11):2901-9 (2005), Epub 2005 Sep 30; Greenberg AS, et al., A new antigen receptor gene family that undergoes rearrangement and extensive somatic diversification in sharks, Nature, Mar 9;374(6518):168-73 (1995); Nuttall SD, et al., Isolation of the new antigen receptor from wobbegong sharks, and use as a scaffold for the display of protein loop libraries, Mol Immunol. Aug;38(4):313-26 (2001); Hamers-Casterman C, et al., Naturally occurring antibodies devoid of light chains, Nature. 1993 Jun 3;363(6428):446-8; Gill DS, et al., Biopharmaceutical drug discovery using novel protein scaffolds, Curr Opin Biotechnol. Dec;17(6):653-8 (2006), Epub 2006 Oct 19.

For example, antibodies or antigen binding fragments may be produced by genetic engineering. In this technique, as with other methods, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones, which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid ("aa") substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (*e.g.,* a functional domain, catalytic amino acid residues, *etc*)*.* Variants also include fragments of the polypeptides disclosed herein, particularly biologically active fragments and/or fragments corresponding to functional domains. Techniques for in vitro mutagenesis of cloned genes are known. Also included in the subject specification are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (V_{L} and V_{H}), obtained from antibody producing cells of one species with the constant light and heavy chain regions from another. Typically chimeric antibodies utilize rodent or rabbit variable regions and human constant regions, in order to produce an antibody with predominantly human domains. The production of such chimeric antibodies is well known in the art, and may be achieved by standard means (as described, e.g., in U. S. Patent No. 5,624,659). It is further contemplated that the human constant regions of chimeric antibodies of the specification may be selected from IgG1, IgG2, IgG3, or IgG4 constant regions.

Humanized antibodies are engineered to contain even more human-like immunoglobulin domains, and incorporate only the complementarity-determining regions of the animal-derived antibody. This is accomplished by examination of the sequence of the hyper-variable loops of the variable regions of the monoclonal antibody to fit them to the structure of the human antibody chains. Although facially complex, the process is straightforward in practice. See, e.g., U.S. Patent No. 6,187,287.

In addition to entire immunoglobulins (or their recombinant counterparts), immunoglobulin fragments comprising the epitope binding site (e.g., Fab, F(ab')₂, or other fragments) may be synthesized. "Fragment," or minimal immunoglobulins may be designed utilizing recombinant immunoglobulin techniques. For instance, "Fv" immunoglobulins for use in the present specification may be produced by synthesizing a fused variable light chain region and a variable heavy chain region. Combinations of antibodies are also of interest, *e.g*. diabodies, which comprise two distinct Fv specificities. In another example of the specification, SMIPs (small molecule immunopharmaceuticals), camelbodies, nanobodies, and IgNAR are encompassed by immunoglobulin fragments.

Immunoglobulins and fragments thereof may be modified post-translationally, *e.g.* to add effector moieties such as chemical linkers, detectable moieties, such as fluorescent dyes, enzymes, toxins, substrates, bioluminescent materials, radioactive materials, chemiluminescent moieties and the like, or specific binding moieties, such as streptavidin, avidin, or biotin, and the like may be utilized in the methods and compositions of the present specification. Examples of additional effector molecules are provided *infra.*

A polynucleotide sequence "corresponds" to a polypeptide sequence if translation of the polynucleotide sequence in accordance with the genetic code yields the polypeptide sequence (i.e., the polynucleotide sequence "encodes" the polypeptide sequence), one polynucleotide sequence "corresponds" to another polynucleotide sequence if the two sequences encode the same polypeptide sequence.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally occurring mutational events do not give rise to a heterologous region of DNA as defined herein.

A "coding sequence" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide. A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism or host cell. Typical vectors include recombinant viruses (for polynucleotides) and liposomes (for polypeptides). A "DNA vector" is a replicon, such as plasmid, phage or cosmid, to which another polynucleotide segment may be attached so as to bring about the replication of the attached segment. An "expression vector" is a DNA vector which contains regulatory sequences which will direct polypeptide synthesis by an appropriate host cell. This usually means a promoter to bind RNA polymerase and initiate transcription of mRNA, as well as ribosome binding sites and initiation signals to direct translation of the mRNA into a polypeptide(s). Incorporation of a polynucleotide sequence into an expression vector at the proper site and in correct reading frame, followed by transformation of an appropriate host cell by the vector, enables the production of a polypeptide encoded by said polynucleotide sequence.

"Amplification" of polynucleotide sequences is the *in vitro* production of multiple copies of a particular nucleic acid sequence. The amplified sequence is usually in the form of DNA. A variety of techniques for carrying out such amplification are described in a review article by Van Brunt (Bio/Technol., 8(4):291-294 (1990)). Polymerase chain reaction or PCR is a prototype of nucleic acid amplification, and use of PCR herein should be considered exemplary of other suitable amplification techniques.

The general structure of antibodies in vertebrates now is well understood (Edelman, G. M., Ann. N.Y. Acad. Sci., 190: 5 (1971)). Antibodies consist of two identical light polypeptide chains of molecular weight approximately 23,000 Daltons (the "light chain"), and two identical heavy chains of molecular weight 53,000-70,000 (the "heavy chain"). The four chains are joined by disulfide bonds in a "Y" configuration wherein the light chains bracket the heavy chains starting at the mouth of the "Y" configuration. The "branch" portion of the "Y" configuration is designated the Fab region; the stem portion of the "Y" configuration is designated the Fc region. The amino acid sequence orientation runs from the N-terminal end at the top of the "Y" configuration to the C-terminal end at the bottom of each chain. The N-terminal end possesses the variable region having specificity for the antigen that elicited it, and is approximately 100 amino acids in length, there being slight variations between light and heavy chain and from antibody to antibody.

The variable region is linked in each chain to a constant region that extends the remaining length of the chain and that within a particular class of antibody does not vary with the specificity of the antibody (i.e., the antigen eliciting it). There are five known major classes of constant regions that determine the class of the immunoglobulin molecule (IgG, IgM, IgA, IgD, and IgE corresponding to γ, µ, α, δ, and ε (gamma, mu, alpha, delta, or epsilon) heavy chain constant regions). The constant region or class determines subsequent effector function of the antibody, including activation of complement (Kabat, E. A., Structural Concepts in Immunology and Immunochemistry, 2nd Ed., p. 413-436, Holt, Rinehart, Winston (1976)), and other cellular responses (Andrews, D. W., et al., Clinical Immunobiology, pp 1-18, W. B. Sanders (1980); Kohl, S., et al., Immunology, 48: 187 (1983)); while the variable region determines the antigen with which it will react. Light chains are classified as either κ (kappa) or λ (lambda). Each heavy chain class can be prepared with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

The expression "variable region" or "VR" refers to the domains within each pair of light and heavy chains in an antibody that are involved directly in binding the antibody to the antigen. Each heavy chain has at one end a variable domain (V_{H}) followed by a number of constant domains. Each light chain has a variable domain (V_{L}) at one end and a constant domain at its other end; the constant domain of the light chain is aligned with the first constant domain of the heavy chain, and the light chain variable domain is aligned with the variable domain of the heavy chain.

The expressions "complementarity determining region," "hypervariable region," or "CDR" refer to one or more of the hyper-variable or complementarity determining regions (CDRs) found in the variable regions of light or heavy chains of an antibody (*See* Kabat, E. A. et al., Sequences of Proteins of Immunological Interest, National Institutes of Health, Bethesda, Md., (1987)). These expressions include the hypervariable regions as defined by Kabat et al. ("Sequences of Proteins of Immunological Interest," Kabat E., et al., US Dept. of Health and Human Services, 1983) or the hypervariable loops in 3-dimensional structures of antibodies (Chothia and Lesk, J Mol. Biol. 196 901-917 (1987)). The CDRs in each chain are held in close proximity by framework regions and, with the CDRs from the other chain, contribute to the formation of the antigen binding site. Within the CDRs there are select amino acids that have been described as the selectivity determining regions (SDRs) which represent the critical contact residues used by the CDR in the antibody-antigen interaction (Kashmiri, S., Methods, 36:25-34 (2005)).

The expressions "framework region" or "FR" refer to one or more of the framework regions within the variable regions of the light and heavy chains of an antibody (*See* Kabat, E. A. et al., Sequences of Proteins of Immunological Interest, National Institutes of Health, Bethesda, Md., (1987)). These expressions include those amino acid sequence regions interposed between the CDRs within the variable regions of the light and heavy chains of an antibody.

### Anti-CGRP Antibodies and Binding Fragments Thereof Having Binding Activity for CGRP

Exemplary examples of the present methods comprise administering anti-CGRP antibodies and fragments thereof to subject. Exemplary anti-CGRP antibodies and fragments are described in U.S. patent publication no. 2012/0294797, and additional exemplary anti-CGRP antibodies as described in the paragraphs that follow.

### Antibody Ab1

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 5; SEQ ID NO: 6; and SEQ ID NO: 7 which correspond to the complementarity-determining regions (CDRs, or hypervariable regions) of the variable light chain sequence of SEQ ID NO: 1 or the light chain sequence of SEQ ID NO: 2, and/or one or more of the polypeptide sequences of SEQ ID NO: 8; SEQ ID NO: 9; and SEQ ID NO: 10 which correspond to the complementarity-determining regions (CDRs, or hypervariable regions) of the variable heavy chain sequence of SEQ ID NO: 3 or the heavy chain sequence of SEQ ID NO: 4, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 1 or SEQ ID NO: 2. In another example of the specification, antibody fragments of the specification comprise, or alternatively consist of, the polypeptide sequence of SEQ ID NO: 3 or SEQ ID NO: 4.

In a further example of the specification, fragments of the antibody having binding specificity to CGRP comprise, or alternatively consist of, one or more of the polypeptide sequences of SEQ ID NO: 5; SEQ ID NO: 6; and SEQ ID NO: 7 which correspond to the complementarity-determining regions (CDRs, or hypervariable regions) of the variable light chain sequence of SEQ ID NO: 1 or the light chain sequence of SEQ ID NO: 2.

In a further example of the specification, fragments of the antibody having binding specificity to CGRP comprise, or alternatively consist of, one or more of the polypeptide sequences of SEQ ID NO: 8; SEQ ID NO: 9; and SEQ ID NO: 10 which correspond to the complementarity-determining regions (CDRs, or hypervariable regions) of the variable heavy chain sequence of SEQ ID NO: 3 or the heavy chain sequence of SEQ ID NO: 4.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 1; the variable heavy chain region of SEQ ID NO: 3; the complementarity-determining regions (SEQ ID NO: 5; SEQ ID NO: 6; and SEQ ID NO: 7) of the variable light chain region of SEQ ID NO: 1; and the complementarity-determining regions (SEQ ID NO: 8; SEQ ID NO: 9; and SEQ ID NO: 10) of the variable heavy chain region of SEQ ID NO: 3.

In a particularly preferred example of the specification, the chimeric anti- CGRP antibody is Ab1, comprising, or alternatively consisting of, SEQ ID NO: 2 and SEQ ID NO: 4, and having at least one of the biological activities set forth herein.

In a further preferred example of the specification, antibody fragments comprise, or alternatively consist of, Fab (fragment antigen binding) fragments having binding specificity for CGRP. With respect to antibody Ab1, the Fab fragment includes the variable light chain sequence of SEQ ID NO: 1 and the variable heavy chain sequence of SEQ ID NO: 3. This example of the specification further contemplates additions, deletions, and variants of SEQ ID NO: 1 and/or SEQ ID NO: 3 in said Fab while retaining binding specificity for CGRP.

In one example of the specification described herein (infra), Fab fragments may be produced by enzymatic digestion (e.g., papain) of Ab1. In another example of the specification, anti-CGRP antibodies such as Ab1 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab2

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 15; SEQ ID NO: 16; and SEQ ID NO: 17 which correspond to the complementarity-determining regions (CDRs, or hypervariable regions) of the variable light chain sequence of SEQ ID NO: 11 or the light chain sequence of SEQ ID NO: 12, and/or one or more of the polypeptide sequences of SEQ ID NO: 18; SEQ ID NO: 19; and SEQ ID NO: 20 which correspond to the complementarity-determining regions (CDRs, or hypervariable regions) of the variable heavy chain sequence of SEQ ID NO: 13 or the heavy chain sequence of SEQ ID NO: 14, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 11 or SEQ ID NO: 12. In another example of the specification, antibody fragments of the specification comprise, or alternatively consist of, the polypeptide sequence of SEQ ID NO: 13 or SEQ ID NO: 14.

In a further example of the specification, fragments of the antibody having binding specificity to CGRP comprise, or alternatively consist of, one or more of the polypeptide sequences of SEQ ID NO: 15; SEQ ID NO: 16; and SEQ ID NO: 17 which correspond to the complementarity-determining regions (CDRs, or hypervariable regions) of the variable light chain sequence of SEQ ID NO: 11 or the light chain sequence of SEQ ID NO: 12.

In a further example of the specification, fragments of the antibody having binding specificity to CGRP comprise, or alternatively consist of, one or more of the polypeptide sequences of SEQ ID NO: 18; SEQ ID NO: 19; and SEQ ID NO: 20 which correspond to the complementarity-determining regions (CDRs, or hypervariable regions) of the variable heavy chain sequence of SEQ ID NO: 13 or the heavy chain sequence of SEQ ID NO: 14.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 11; the variable heavy chain region of SEQ ID NO: 13; the complementarity-determining regions (SEQ ID NO: 15; SEQ ID NO: 16; and SEQ ID NO: 17) of the variable light chain region of SEQ ID NO: 11; and the complementarity-determining regions (SEQ ID NO: 18; SEQ ID NO: 19; and SEQ ID NO: 20) of the variable heavy chain region of SEQ ID NO: 13.

In an example of the specification, the humanized anti- CGRP antibody is Ab2, comprising, or alternatively consisting of, SEQ ID NO: 12 and SEQ ID NO: 14, and having at least one of the biological activities set forth herein.

In a further example of the specification, antibody fragments comprise, or alternatively consist of, Fab (fragment antigen binding) fragments having binding specificity for CGRP. With respect to antibody Ab2, the Fab fragment includes the variable light chain sequence of SEQ ID NO: 11 and the variable heavy chain sequence of SEQ ID NO: 13. This example of the specification further contemplates additions, deletions, and variants of SEQ ID NO: 11 and/or SEQ ID NO: 13 in said Fab while retaining binding specificity for CGRP.

In one example of the specification described herein (infra), Fab fragments may be produced by enzymatic digestion (e.g., papain) of Ab2. In another example of the specification, anti-CGRP antibodies such as Ab2 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab3

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 25; SEQ ID NO: 26; and SEQ ID NO: 27 which correspond to the complementarity-determining regions (CDRs, or hypervariable regions) of the variable light chain sequence of SEQ ID NO: 21 or the light chain sequence of SEQ ID NO: 22, and/or one or more of the polypeptide sequences of SEQ ID NO: 28; SEQ ID NO: 29; and SEQ ID NO: 30 which correspond to the complementarity-determining regions (CDRs, or hypervariable regions) of the variable heavy chain sequence of SEQ ID NO: 23 or the heavy chain sequence of SEQ ID NO: 24, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 21 or SEQ ID NO: 22. In another example of the specification, antibody fragments of the specification comprise, or alternatively consist of, the polypeptide sequence of SEQ ID NO: 23 or SEQ ID NO: 24.

In a further example of the specification, fragments of the antibody having binding specificity to CGRP comprise, or alternatively consist of, one or more of the polypeptide sequences of SEQ ID NO: 25; SEQ ID NO: 26; and SEQ ID NO: 27 which correspond to the complementarity-determining regions (CDRs, or hypervariable regions) of the variable light chain sequence of SEQ ID NO: 21 or the light chain sequence of SEQ ID NO: 22.

In a further example of the specification, fragments of the antibody having binding specificity to CGRP comprise, or alternatively consist of, one or more of the polypeptide sequences of SEQ ID NO: 28; SEQ ID NO: 29; and SEQ ID NO: 30 which correspond to the complementarity-determining regions (CDRs, or hypervariable regions) of the variable heavy chain sequence of SEQ ID NO: 23 or the heavy chain sequence of SEQ ID NO: 24.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 21; the variable heavy chain region of SEQ ID NO: 23; the complementarity-determining regions (SEQ ID NO: 25; SEQ ID NO: 26; and SEQ ID NO: 27) of the variable light chain region of SEQ ID NO: 21; and the complementarity-determining regions (SEQ ID NO: 28; SEQ ID NO: 29; and SEQ ID NO: 30) of the variable heavy chain region of SEQ ID NO: 23.

In an example of the specification, the chimeric anti-CGRP antibody is Ab3, comprising, or alternatively consisting of, SEQ ID NO: 22 and SEQ ID NO: 24, and having at least one of the biological activities set forth herein.

In a further example of the specification, antibody fragments comprise, or alternatively consist of, Fab (fragment antigen binding) fragments having binding specificity for CGRP. With respect to antibody Ab3, the Fab fragment includes the variable light chain sequence of SEQ ID NO: 21 and the variable heavy chain sequence of SEQ ID NO: 23. This example of the specification further contemplates additions, deletions, and variants of SEQ ID NO: 21 and/or SEQ ID NO: 23 in said Fab while retaining binding specificity for CGRP.

In one example of the specification described herein (infra), Fab fragments may be produced by enzymatic digestion (e.g., papain) of Ab3. In another example of the specification, anti-CGRP antibodies such as Ab3 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab4

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:
QSLEESGGRLVTPGTPLTLTCSVSGIDLSGYYMNWVRQAPGKGLEWIGVIGINGA TYYASWAKGRFTISKTSSTTVDLKMTSLTTEDTATYFCARGDIWGPGTLVTVSS (SEQ ID NO: 33).

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 35; SEQ ID NO: 36; and SEQ ID NO: 37 which correspond to the complementarity-determining regions (CDRs, or hypervariable regions) of the variable light chain sequence of SEQ ID NO: 31 or the light chain sequence of SEQ ID NO: 32, and/or one or more of the polypeptide sequences of SEQ ID NO: 38; SEQ ID NO: 39; and SEQ ID NO: 40 which correspond to the complementarity-determining regions (CDRs, or hypervariable regions) of the variable heavy chain sequence of SEQ ID NO: 33 or the heavy chain sequence of SEQ ID NO: 34, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 31 or SEQ ID NO: 32. In another example of the specification, antibody fragments of the specification comprise, or alternatively consist of, the polypeptide sequence of SEQ ID NO: 33 or SEQ ID NO: 34.

In a further example of the specification, fragments of the antibody having binding specificity to CGRP comprise, or alternatively consist of, one or more of the polypeptide sequences of SEQ ID NO: 35; SEQ ID NO: 36; and SEQ ID NO: 37 which correspond to the complementarity-determining regions (CDRs, or hypervariable regions) of the variable light chain sequence of SEQ ID NO: 31 or the light chain sequence of SEQ ID NO: 32.

In a further example of the specification, fragments of the antibody having binding specificity to CGRP comprise, or alternatively consist of, one or more of the polypeptide sequences of SEQ ID NO: 38; SEQ ID NO: 39; and SEQ ID NO: 40 which correspond to the complementarity-determining regions (CDRs, or hypervariable regions) of the variable heavy chain sequence of SEQ ID NO: 33 or the heavy chain sequence of SEQ ID NO: 34.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 31; the variable heavy chain region of SEQ ID NO: 33; the complementarity-determining regions (SEQ ID NO: 35; SEQ ID NO: 36; and SEQ ID NO: 37) of the variable light chain region of SEQ ID NO: 31; and the complementarity-determining regions (SEQ ID NO: 38; SEQ ID NO: 39; and SEQ ID NO: 40) of the variable heavy chain region of SEQ ID NO: 33.

In an example of the specification, the humanized anti-CGRP antibody is Ab4, comprising, or alternatively consisting of, SEQ ID NO: 32 and SEQ ID NO: 34, and having at least one of the biological activities set forth herein.

In a further example of the specification, antibody fragments comprise, or alternatively consist of, Fab (fragment antigen binding) fragments having binding specificity for CGRP. With respect to antibody Ab4, the Fab fragment includes the variable light chain sequence of SEQ ID NO: 31 and the variable heavy chain sequence of SEQ ID NO: 33. This example of the specification further contemplates additions, deletions, and variants of SEQ ID NO: 31 and/or SEQ ID NO: 33 in said Fab while retaining binding specificity for CGRP.

In one example of the specification described herein (infra), Fab fragments may be produced by enzymatic digestion (e.g., papain) of Ab4. In another example of the specification, anti-CGRP antibodies such as Ab4 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab5

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 45; SEQ ID NO: 46; and SEQ ID NO: 47 which correspond to the complementarity-determining regions (CDRs, or hypervariable regions) of the variable light chain sequence of SEQ ID NO: 41 or the light chain sequence of SEQ ID NO: 42, and/or one or more of the polypeptide sequences of SEQ ID NO: 48; SEQ ID NO: 49; and SEQ ID NO: 50 which correspond to the complementarity-determining regions (CDRs, or hypervariable regions) of the variable heavy chain sequence of SEQ ID NO: 43 or the heavy chain sequence of SEQ ID NO: 44, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 41 or SEQ ID NO: 42. In another example of the specification, antibody fragments of the specification comprise, or alternatively consist of, the polypeptide sequence of SEQ ID NO: 43 or SEQ ID NO: 44.

In a further example of the specification, fragments of the antibody having binding specificity to CGRP comprise, or alternatively consist of, one or more of the polypeptide sequences of SEQ ID NO: 45; SEQ ID NO: 46; and SEQ ID NO: 47 which correspond to the complementarity-determining regions (CDRs, or hypervariable regions) of the variable light chain sequence of SEQ ID NO: 41 or the light chain sequence of SEQ ID NO: 42.

In a further example of the specification, fragments of the antibody having binding specificity to CGRP comprise, or alternatively consist of, one or more of the polypeptide sequences of SEQ ID NO: 48; SEQ ID NO: 49; and SEQ ID NO: 50 which correspond to the complementarity-determining regions (CDRs, or hypervariable regions) of the variable heavy chain sequence of SEQ ID NO: 43 or the heavy chain sequence of SEQ ID NO: 44.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 41; the variable heavy chain region of SEQ ID NO: 43; the complementarity-determining regions (SEQ ID NO: 45; SEQ ID NO: 46; and SEQ ID NO: 47) of the variable light chain region of SEQ ID NO: 41; and the complementarity-determining regions (SEQ ID NO: 48; SEQ ID NO: 49; and SEQ ID NO: 50) of the variable heavy chain region of SEQ ID NO: 43.

In an example of the specification, the chimeric anti-CGRP antibody is Ab5, comprising, or alternatively consisting of, SEQ ID NO: 42 and SEQ ID NO: 44, and having at least one of the biological activities set forth herein.

In a further example of the specification, antibody fragments comprise, or alternatively consist of, Fab (fragment antigen binding) fragments having binding specificity for CGRP. With respect to antibody Ab5, the Fab fragment includes the variable light chain sequence of SEQ ID NO: 41 and the variable heavy chain sequence of SEQ ID NO: 43. This example of the specification further contemplates additions, deletions, and variants of SEQ ID NO: 41 and/or SEQ ID NO: 43 in said Fab while retaining binding specificity for CGRP.

In one example of the specification described herein (infra), Fab fragments may be produced by enzymatic digestion (e.g., papain) of Ab5. In another example of the specification, anti-CGRP antibodies such as Ab5 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab6

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 52, and/or one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 54, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 51 or SEQ ID NO: 52. In another example of the specification, antibody fragments of the specification comprise, or alternatively consist of, the polypeptide sequence of SEQ ID NO: 53 or SEQ ID NO: 54.

In a further example of the specification, fragments of the antibody having binding specificity to CGRP comprise, or alternatively consist of, one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 52.

In a further example of the specification, fragments of the antibody having binding specificity to CGRP comprise, or alternatively consist of, one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 54.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 51; the variable heavy chain region of SEQ ID NO: 53; the complementarity-determining regions (SEQ ID NO: 55; SEQ ID NO: 56; and SEQ ID NO: 57) of the variable light chain region of SEQ ID NO: 51; and the complementarity-determining regions (SEQ ID NO: 58; SEQ ID NO: 59; and SEQ ID NO: 60) of the variable heavy chain region of SEQ ID NO: 53.

In an example of the specification, the humanized anti- CGRP antibody is Ab6, comprising, or alternatively consisting of, SEQ ID NO: 52 and SEQ ID NO: 54, and having at least one of the biological activities set forth herein.

In a further example of the specification, antibody fragments comprise, or alternatively consist of, Fab (fragment antigen binding) fragments having binding specificity for CGRP. With respect to antibody Ab6, the Fab fragment includes the variable light chain sequence of SEQ ID NO: 51 and the variable heavy chain sequence of SEQ ID NO: 53. This example of the specification further contemplates additions, deletions, and variants of SEQ ID NO: 51 and/or SEQ ID NO: 53 in said Fab while retaining binding specificity for CGRP.

In one example of the specification described herein (infra), Fab fragments may be produced by enzymatic digestion (e.g., papain) of Ab6. In another example of the specification, anti-CGRP antibodies such as Ab6 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab7

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 65; SEQ ID NO: 66; and SEQ ID NO: 67 which correspond to the complementarity-determining regions (CDRs, or hypervariable regions) of the variable light chain sequence of SEQ ID NO: 61 or the light chain sequence of SEQ ID NO: 62, and/or one or more of the polypeptide sequences of SEQ ID NO: 68; SEQ ID NO: 69; and SEQ ID NO: 70 which correspond to the complementarity-determining regions (CDRs, or hypervariable regions) of the variable heavy chain sequence of SEQ ID NO: 63 or the heavy chain sequence of SEQ ID NO: 64, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 61 or SEQ ID NO: 62. In another example of the specification, antibody fragments of the specification comprise, or alternatively consist of, the polypeptide sequence of SEQ ID NO: 63 or SEQ ID NO: 64.

In a further example of the specification, fragments of the antibody having binding specificity to CGRP comprise, or alternatively consist of, one or more of the polypeptide sequences of SEQ ID NO: 65; SEQ ID NO: 66; and SEQ ID NO: 67 which correspond to the complementarity-determining regions (CDRs, or hypervariable regions) of the variable light chain sequence of SEQ ID NO: 61 or the light chain sequence of SEQ ID NO: 62.

In a further example of the specification, fragments of the antibody having binding specificity to CGRP comprise, or alternatively consist of, one or more of the polypeptide sequences of SEQ ID NO: 68; SEQ ID NO: 69; and SEQ ID NO: 70 which correspond to the complementarity-determining regions (CDRs, or hypervariable regions) of the variable heavy chain sequence of SEQ ID NO: 63 or the heavy chain sequence of SEQ ID NO: 64.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 61; the variable heavy chain region of SEQ ID NO: 63; the complementarity-determining regions (SEQ ID NO: 65; SEQ ID NO: 66; and SEQ ID NO: 67) of the variable light chain region of SEQ ID NO: 61; and the complementarity-determining regions (SEQ ID NO: 68; SEQ ID NO: 69; and SEQ ID NO: 70) of the variable heavy chain region of SEQ ID NO: 63.

In an example of the specification, the chimeric anti-CGRP antibody is Ab7, comprising, or alternatively consisting of, SEQ ID NO: 62 and SEQ ID NO: 64, and having at least one of the biological activities set forth herein.

In a further example of the specification, antibody fragments comprise, or alternatively consist of, Fab (fragment antigen binding) fragments having binding specificity for CGRP. With respect to antibody Ab7, the Fab fragment includes the variable light chain sequence of SEQ ID NO: 61 and the variable heavy chain sequence of SEQ ID NO: 63. This example of the specification further contemplates additions, deletions, and variants of SEQ ID NO: 61 and/or SEQ ID NO: 63 in said Fab while retaining binding specificity for CGRP.

In one example of the specification described herein (infra), Fab fragments may be produced by enzymatic digestion (e.g., papain) of Ab7. In another example of the specification, anti-CGRP antibodies such as Ab7 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab8

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 75; SEQ ID NO: 76; and SEQ ID NO: 77 which correspond to the complementarity-determining regions (CDRs, or hypervariable regions) of the variable light chain sequence of SEQ ID NO: 71 or the light chain sequence of SEQ ID NO: 72, and/or one or more of the polypeptide sequences of SEQ ID NO: 78; SEQ ID NO: 79; and SEQ ID NO: 80 which correspond to the complementarity-determining regions (CDRs, or hypervariable regions) of the variable heavy chain sequence of SEQ ID NO: 73 or the heavy chain sequence of SEQ ID NO: 74, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 71 or SEQ ID NO: 72. In another example of the specification, antibody fragments of the specification comprise, or alternatively consist of, the polypeptide sequence of SEQ ID NO: 73 or SEQ ID NO: 74.

In a further example of the specification, fragments of the antibody having binding specificity to CGRP comprise, or alternatively consist of, one or more of the polypeptide sequences of SEQ ID NO: 75; SEQ ID NO: 76; and SEQ ID NO: 77 which correspond to the complementarity-determining regions (CDRs, or hypervariable regions) of the variable light chain sequence of SEQ ID NO: 71 or the light chain sequence of SEQ ID NO: 72.

In a further example of the specification, fragments of the antibody having binding specificity to CGRP comprise, or alternatively consist of, one or more of the polypeptide sequences of SEQ ID NO: 78; SEQ ID NO: 79; and SEQ ID NO: 80 which correspond to the complementarity-determining regions (CDRs, or hypervariable regions) of the variable heavy chain sequence of SEQ ID NO: 73 or the heavy chain sequence of SEQ ID NO: 74.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 71; the variable heavy chain region of SEQ ID NO: 73; the complementarity-determining regions (SEQ ID NO: 75; SEQ ID NO: 76; and SEQ ID NO: 77) of the variable light chain region of SEQ ID NO: 71; and the complementarity-determining regions (SEQ ID NO: 78; SEQ ID NO: 79; and SEQ ID NO: 80) of the variable heavy chain region of SEQ ID NO: 73.

In an example of the specification, the humanized anti-CGRP antibody is Ab8, comprising, or alternatively consisting of, SEQ ID NO: 72 and SEQ ID NO: 74, and having at least one of the biological activities set forth herein.

In a further example of the specification, antibody fragments comprise, or alternatively consist of, Fab (fragment antigen binding) fragments having binding specificity for CGRP. With respect to antibody Ab8, the Fab fragment includes the variable light chain sequence of SEQ ID NO: 71 and the variable heavy chain sequence of SEQ ID NO: 73. This example of the specification further contemplates additions, deletions, and variants of SEQ ID NO: 71 and/or SEQ ID NO: 73 in said Fab while retaining binding specificity for CGRP.

In one example of the specification described herein (infra), Fab fragments may be produced by enzymatic digestion (e.g., papain) of Ab8. In another example of the specification, anti-CGRP antibodies such as Ab8 or Fab fragments thereof may be produced via expression in mammalian cells such as CHO, NSO or HEK 293 cells, fungal, insect, or microbial systems such as yeast cells (for example diploid yeast such as diploid *Pichia)* and other yeas strains. Suitable *Pichia* species include, but are not limited to, *Pichia pastoris.*

### Antibody Ab9

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 85; SEQ ID NO: 86; and SEQ ID NO: 87 which correspond to the complementarity-determining regions (CDRs, or hypervariable regions) of the variable light chain sequence of SEQ ID NO: 81 or the light chain sequence of SEQ ID NO: 82, and/or one or more of the polypeptide sequences of SEQ ID NO: 88; SEQ ID NO: 89; and SEQ ID NO: 90 which correspond to the complementarity-determining regions (CDRs, or hypervariable regions) of the variable heavy chain sequence of SEQ ID NO: 83 or the heavy chain sequence of SEQ ID NO: 84, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 81 or SEQ ID NO: 82. In another example of the specification, antibody fragments of the specification comprise, or alternatively consist of, the polypeptide sequence of SEQ ID NO: 83 or SEQ ID NO: 84.

In a further example of the specification, fragments of the antibody having binding specificity to CGRP comprise, or alternatively consist of, one or more of the polypeptide sequences of SEQ ID NO: 85; SEQ ID NO: 86; and SEQ ID NO: 87 which correspond to the complementarity-determining regions (CDRs, or hypervariable regions) of the variable light chain sequence of SEQ ID NO: 81 or the light chain sequence of SEQ ID NO: 82.

In a further example of the specification, fragments of the antibody having binding specificity to CGRP comprise, or alternatively consist of, one or more of the polypeptide sequences of SEQ ID NO: 88; SEQ ID NO: 89; and SEQ ID NO: 90 which correspond to the complementarity-determining regions (CDRs, or hypervariable regions) of the variable heavy chain sequence of SEQ ID NO: 83 or the heavy chain sequence of SEQ ID NO: 84.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 81; the variable heavy chain region of SEQ ID NO: 83; the complementarity-determining regions (SEQ ID NO: 85; SEQ ID NO: 86; and SEQ ID NO: 87) of the variable light chain region of SEQ ID NO: 81; and the complementarity-determining regions (SEQ ID NO: 88; SEQ ID NO: 89; and SEQ ID NO: 90) of the variable heavy chain region of SEQ ID NO: 83.

In an example of the specification, the chimeric anti-CGRP antibody is Ab9, comprising, or alternatively consisting of, SEQ ID NO: 82 and SEQ ID NO: 84, and having at least one of the biological activities set forth herein.

In a further example of the specification, antibody fragments comprise, or alternatively consist of, Fab (fragment antigen binding) fragments having binding specificity for CGRP. With respect to antibody Ab9, the Fab fragment includes the variable light chain sequence of SEQ ID NO: 81 and the variable heavy chain sequence of SEQ ID NO: 83. This example of the specification further contemplates additions, deletions, and variants of SEQ ID NO: 81 and/or SEQ ID NO: 83 in said Fab while retaining binding specificity for CGRP.

In one example of the specification described herein (infra), Fab fragments may be produced by enzymatic digestion (e.g., papain) of Ab9. In another example of the specification, anti-CGRP antibodies such as Ab9 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab10

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 95; SEQ ID NO: 96; and SEQ ID NO: 97 which correspond to the complementarity-determining regions (CDRs, or hypervariable regions) of the variable light chain sequence of SEQ ID NO: 91 or the light chain sequence of SEQ ID NO: 92, and/or one or more of the polypeptide sequences of SEQ ID NO: 98; SEQ ID NO: 99; and SEQ ID NO: 100 which correspond to the complementarity-determining regions (CDRs, or hypervariable regions) of the variable heavy chain sequence of SEQ ID NO: 93 or the heavy chain sequence of SEQ ID NO: 94, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 91 or SEQ ID NO: 92. In another example of the specification, antibody fragments of the specification comprise, or alternatively consist of, the polypeptide sequence of SEQ ID NO: 93 or SEQ ID NO: 94.

In a further example of the specification, fragments of the antibody having binding specificity to CGRP comprise, or alternatively consist of, one or more of the polypeptide sequences of SEQ ID NO: 95; SEQ ID NO: 96; and SEQ ID NO: 97 which correspond to the complementarity-determining regions (CDRs, or hypervariable regions) of the variable light chain sequence of SEQ ID NO: 91 or the light chain sequence of SEQ ID NO: 92.

In a further example of the specification, fragments of the antibody having binding specificity to CGRP comprise, or alternatively consist of, one or more of the polypeptide sequences of SEQ ID NO: 98; SEQ ID NO: 99; and SEQ ID NO: 100 which correspond to the complementarity-determining regions (CDRs, or hypervariable regions) of the variable heavy chain sequence of SEQ ID NO: 93 or the heavy chain sequence of SEQ ID NO: 94.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 91; the variable heavy chain region of SEQ ID NO: 93; the complementarity-determining regions (SEQ ID NO: 95; SEQ ID NO: 96; and SEQ ID NO: 97) of the variable light chain region of SEQ ID NO: 91; and the complementarity-determining regions (SEQ ID NO: 98; SEQ ID NO: 99; and SEQ ID NO: 100) of the variable heavy chain region of SEQ ID NO: 93.

In an example of the specification, the humanized anti-CGRP antibody is Ab10, comprising, or alternatively consisting of, SEQ ID NO: 92 and SEQ ID NO: 94, and having at least one of the biological activities set forth herein.

In a further example of the specification, antibody fragments comprise, or alternatively consist of, Fab (fragment antigen binding) fragments having binding specificity for CGRP. With respect to antibody Ab10, the Fab fragment includes the variable light chain sequence of SEQ ID NO: 91 and the variable heavy chain sequence of SEQ ID NO: 93. This example of the specification further contemplates additions, deletions, and variants of SEQ ID NO: 91 and/or SEQ ID NO: 93 in said Fab while retaining binding specificity for CGRP.

In one example of the specification described herein (infra), Fab fragments may be produced by enzymatic digestion (e.g., papain) of Ab10. In another example of the specification, anti-CGRP antibodies such as Ab10 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab11

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 105; SEQ ID NO: 106; and SEQ ID NO: 107 which correspond to the complementarity-determining regions (CDRs, or hypervariable regions) of the variable light chain sequence of SEQ ID NO: 101 or the light chain sequence of SEQ ID NO: 102, and/or one or more of the polypeptide sequences of SEQ ID NO: 108; SEQ ID NO: 109; and SEQ ID NO: 110 which correspond to the complementarity-determining regions (CDRs, or hypervariable regions) of the variable heavy chain sequence of SEQ ID NO: 103 or the heavy chain sequence of SEQ ID NO: 104, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 101 or SEQ ID NO: 102. In another example of the specification, antibody fragments of the specification comprise, or alternatively consist of, the polypeptide sequence of SEQ ID NO: 103 or SEQ ID NO: 104.

In a further example of the specification, fragments of the antibody having binding specificity to CGRP comprise, or alternatively consist of, one or more of the polypeptide sequences of SEQ ID NO: 105; SEQ ID NO: 106; and SEQ ID NO: 107 which correspond to the complementarity-determining regions (CDRs, or hypervariable regions) of the variable light chain sequence of SEQ ID NO: 101 or the light chain sequence of SEQ ID NO: 102.

In a further example of the specification, fragments of the antibody having binding specificity to CGRP comprise, or alternatively consist of, one or more of the polypeptide sequences of SEQ ID NO: 108; SEQ ID NO: 109; and SEQ ID NO: 110 which correspond to the complementarity-determining regions (CDRs, or hypervariable regions) of the variable heavy chain sequence of SEQ ID NO: 103 or the heavy chain sequence of SEQ ID NO: 104.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 101; the variable heavy chain region of SEQ ID NO: 103; the complementarity-determining regions (SEQ ID NO: 105; SEQ ID NO: 106; and SEQ ID NO: 107) of the variable light chain region of SEQ ID NO: 101; and the complementarity-determining regions (SEQ ID NO: 108; SEQ ID NO: 109; and SEQ ID NO: 110) of the variable heavy chain region of SEQ ID NO: 103.

In an example of the specification, the chimeric anti-CGRP antibody is Ab11, comprising, or alternatively consisting of, SEQ ID NO: 102 and SEQ ID NO: 104, and having at least one of the biological activities set forth herein.

In a further example of the specification, antibody fragments comprise, or alternatively consist of, Fab (fragment antigen binding) fragments having binding specificity for CGRP. With respect to antibody Ab11, the Fab fragment includes the variable light chain sequence of SEQ ID NO: 101 and the variable heavy chain sequence of SEQ ID NO: 103. This example of the specification further contemplates additions, deletions, and variants of SEQ ID NO: 101 and/or SEQ ID NO: 103 in said Fab while retaining binding specificity for CGRP.

In one example of the specification described herein (infra), Fab fragments may be produced by enzymatic digestion (e.g., papain) of Ab11. In another example of the specification, anti-CGRP antibodies such as Ab11 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab12

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 115; SEQ ID NO: 116; and SEQ ID NO: 117 which correspond to the complementarity-determining regions (CDRs, or hypervariable regions) of the variable light chain sequence of SEQ ID NO: 111 or the light chain sequence of SEQ ID NO: 112, and/or one or more of the polypeptide sequences of SEQ ID NO: 118; SEQ ID NO: 119; and SEQ ID NO: 120 which correspond to the complementarity-determining regions (CDRs, or hypervariable regions) of the variable heavy chain sequence of SEQ ID NO: 113 or the heavy chain sequence of SEQ ID NO: 114, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 111 or SEQ ID NO: 112. In another example of the specification, antibody fragments of the specification comprise, or alternatively consist of, the polypeptide sequence of SEQ ID NO: 113 or SEQ ID NO: 114.

In a further example of the specification, fragments of the antibody having binding specificity to CGRP comprise, or alternatively consist of, one or more of the polypeptide sequences of SEQ ID NO: 115; SEQ ID NO: 116; and SEQ ID NO: 117 which correspond to the complementarity-determining regions (CDRs, or hypervariable regions) of the variable light chain sequence of SEQ ID NO: 111 or the light chain sequence of SEQ ID NO: 112.

In a further example of the specification, fragments of the antibody having binding specificity to CGRP comprise, or alternatively consist of, one or more of the polypeptide sequences of SEQ ID NO: 118; SEQ ID NO: 119; and SEQ ID NO: 120 which correspond to the complementarity-determining regions (CDRs, or hypervariable regions) of the variable heavy chain sequence of SEQ ID NO: 113 or the heavy chain sequence of SEQ ID NO: 114.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 111; the variable heavy chain region of SEQ ID NO: 113; the complementarity-determining regions (SEQ ID NO: 115; SEQ ID NO: 116; and SEQ ID NO: 117) of the variable light chain region of SEQ ID NO: 111; and the complementarity-determining regions (SEQ ID NO: 118; SEQ ID NO: 119; and SEQ ID NO: 120) of the variable heavy chain region of SEQ ID NO: 113.

In an example of the specification, the humanized anti-CGRP antibody is Ab12, comprising, or alternatively consisting of, SEQ ID NO: 112 and SEQ ID NO: 114, and having at least one of the biological activities set forth herein.

In a further example of the specification, antibody fragments comprise, or alternatively consist of, Fab (fragment antigen binding) fragments having binding specificity for CGRP. With respect to antibody Ab12, the Fab fragment includes the variable light chain sequence of SEQ ID NO: 111 and the variable heavy chain sequence of SEQ ID NO: 113. This example of the specification further contemplates additions, deletions, and variants of SEQ ID NO: 111 and/or SEQ ID NO: 113 in said Fab while retaining binding specificity for CGRP.

In one example of the specification described herein (infra), Fab fragments may be produced by enzymatic digestion (e.g., papain) of Ab12. In another example of the specification, anti-CGRP antibodies such as Ab12 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab13

In one example, the specification includes chimeric antibodies having binding specificity to CGRP and possessing a variable light chain sequence comprising the sequence set forth below:

The specification also includes chimeric antibodies having binding specificity to CGRP and possessing a light chain sequence comprising the sequence set forth below:

The specification further includes chimeric antibodies having binding specificity to CGRP and possessing a variable heavy chain sequence comprising the sequence set forth below:

The specification also includes chimeric antibodies having binding specificity to CGRP and possessing a heavy chain sequence comprising the sequence set forth below:

The specification further contemplates antibodies comprising one or more of the polypeptide sequences of SEQ ID NO: 125; SEQ ID NO: 126; and SEQ ID NO: 127 which correspond to the complementarity-determining regions (CDRs, or hypervariable regions) of the variable light chain sequence of SEQ ID NO: 121 or the light chain sequence of SEQ ID NO: 122, and/or one or more of the polypeptide sequences of SEQ ID NO: 128; SEQ ID NO: 129; and SEQ ID NO: 130 which correspond to the complementarity-determining regions (CDRs, or hypervariable regions) of the variable heavy chain sequence of SEQ ID NO: 123 or the heavy chain sequence of SEQ ID NO: 124, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 121 or SEQ ID NO: 122. In another example of the specification, antibody fragments of the specification comprise, or alternatively consist of, the polypeptide sequence of SEQ ID NO: 123 or SEQ ID NO: 124.

In a further example of the specification, fragments of the antibody having binding specificity to CGRP comprise, or alternatively consist of, one or more of the polypeptide sequences of SEQ ID NO: 125; SEQ ID NO: 126; and SEQ ID NO: 127 which correspond to the complementarity-determining regions (CDRs, or hypervariable regions) of the variable light chain sequence of SEQ ID NO: 121 or the light chain sequence of SEQ ID NO: 122.

In a further example of the specification, fragments of the antibody having binding specificity to CGRP comprise, or alternatively consist of, one or more of the polypeptide sequences of SEQ ID NO: 128; SEQ ID NO: 129; and SEQ ID NO: 130 which correspond to the complementarity-determining regions (CDRs, or hypervariable regions) of the variable heavy chain sequence of SEQ ID NO: 123 or the heavy chain sequence of SEQ ID NO: 124.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 121; the variable heavy chain region of SEQ ID NO: 123; the complementarity-determining regions (SEQ ID NO: 125; SEQ ID NO: 126; and SEQ ID NO: 127) of the variable light chain region of SEQ ID NO: 121; and the complementarity-determining regions (SEQ ID NO: 128; SEQ ID NO: 129; and SEQ ID NO: 130) of the variable heavy chain region of SEQ ID NO: 123.

In an example of the specification, the chimeric anti-CGRP antibody is Ab13, comprising, or alternatively consisting of, SEQ ID NO: 122 and SEQ ID NO: 124, and having at least one of the biological activities set forth herein.

In a further example of the specification, antibody fragments comprise, or alternatively consist of, Fab (fragment antigen binding) fragments having binding specificity for CGRP. With respect to antibody Ab13, the Fab fragment includes the variable light chain sequence of SEQ ID NO: 121 and the variable heavy chain sequence of SEQ ID NO: 123. This example of the specification further contemplates additions, deletions, and variants of SEQ ID NO: 121 and/or SEQ ID NO: 123 in said Fab while retaining binding specificity for CGRP.

In one example of the specification described herein (infra), Fab fragments may be produced by enzymatic digestion (e.g., papain) of Ab13. In another example of the specification, anti-CGRP antibodies such as Ab13 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

### Antibody Ab14

In one example, the specification includes humanized antibodies having binding specificity to CGRP and possessing a variable light chain sequence comprising the sequence set forth below:

The specification also includes humanized antibodies having binding specificity to CGRP and possessing a light chain sequence comprising the sequence set forth below:

The specification further includes humanized antibodies having binding specificity to CGRP and possessing a variable heavy chain sequence comprising the sequence set forth below:

The specification also includes humanized antibodies having binding specificity to CGRP and possessing a heavy chain sequence comprising the sequence set forth below:

The specification further contemplates antibodies comprising one or more of the polypeptide sequences of SEQ ID NO: 135; SEQ ID NO: 136; and SEQ ID NO: 137 which correspond to the complementarity-determining regions (CDRs, or hypervariable regions) of the variable light chain sequence of SEQ ID NO: 131 or the light chain sequence of SEQ ID NO: 132, and/or one or more of the polypeptide sequences of SEQ ID NO: 138; SEQ ID NO: 139; and SEQ ID NO: 140 which correspond to the complementarity-determining regions (CDRs, or hypervariable regions) of the variable heavy chain sequence of SEQ ID NO: 133 or the heavy chain sequence of SEQ ID NO: 134, or combinations of these polypeptide sequences. In another example of the specification, the antibodies of the specification or fragments thereof comprise, or alternatively consist of, combinations of one or more of the CDRs, the variable heavy and variable light chain sequences, and the heavy and light chain sequences set forth above, including all of them.

The specification also contemplates fragments of the antibody having binding specificity to CGRP. In one example of the specification, antibody fragments of the specification comprise, or alternatively consist of, the polypeptide sequence of SEQ ID NO: 131 or SEQ ID NO: 132. In another example of the specification, antibody fragments of the specification comprise, or alternatively consist of, the polypeptide sequence of SEQ ID NO: 133 or SEQ ID NO: 134.

In a further example of the specification, fragments of the antibody having binding specificity to CGRP comprise, or alternatively consist of, one or more of the polypeptide sequences of SEQ ID NO: 135; SEQ ID NO: 136; and SEQ ID NO: 137 which correspond to the complementarity-determining regions (CDRs, or hypervariable regions) of the variable light chain sequence of SEQ ID NO: 131 or the light chain sequence of SEQ ID NO: 132.

In a further example of the specification, fragments of the antibody having binding specificity to CGRP comprise, or alternatively consist of, one or more of the polypeptide sequences of SEQ ID NO: 138; SEQ ID NO: 139; and SEQ ID NO: 140 which correspond to the complementarity-determining regions (CDRs, or hypervariable regions) of the variable heavy chain sequence of SEQ ID NO: 133 or the heavy chain sequence of SEQ ID NO: 134.

The specification also contemplates antibody fragments which include one or more of the antibody fragments described herein. In one example of the specification, fragments of the antibodies having binding specificity to CGRP comprise, or alternatively consist of, one, two, three or more, including all of the following antibody fragments: the variable light chain region of SEQ ID NO: 131; the variable heavy chain region of SEQ ID NO: 133; the complementarity-determining regions (SEQ ID NO: 135; SEQ ID NO: 136; and SEQ ID NO: 137) of the variable light chain region of SEQ ID NO: 131; and the complementarity-determining regions (SEQ ID NO: 138; SEQ ID NO: 139; and SEQ ID NO: 140) of the variable heavy chain region of SEQ ID NO: 133.

In an example of the specification, the humanized anti-CGRP antibody is Ab14, comprising, or alternatively consisting of, SEQ ID NO: 132 and SEQ ID NO: 134, and having at least one of the biological activities set forth herein.

In a further example of the specification, antibody fragments comprise, or alternatively consist of, Fab (fragment antigen binding) fragments having binding specificity for CGRP. With respect to antibody Ab14, the Fab fragment includes the variable light chain sequence of SEQ ID NO: 131 and the variable heavy chain sequence of SEQ ID NO: 133. This example of the specification further contemplates additions, deletions, and variants of SEQ ID NO: 131 and/or SEQ ID NO: 133 in said Fab while retaining binding specificity for CGRP.

In one example of the specification described herein (infra), Fab fragments may be produced by enzymatic digestion (e.g., papain) of Ab14. In another example of the specification, anti-CGRP antibodies such as Ab14 or Fab fragments thereof may be produced via expression in mammalian cells such as CHO, NSO or HEK 293 cells, fungal, insect, or microbial systems such as yeast cells (for example diploid yeast such as diploid *Pichia)* and other yeast strains. Suitable *Pichia* species include, but are not limited to, *Pichia pastoris.*

In another example, antibody fragments may be present in one or more of the following non-limiting forms: Fab, Fab', F(ab')₂, Fv and single chain Fv antibody forms. In a preferred example, the anti-CGRP antibodies described herein further comprises the kappa constant light chain sequence comprising the sequence set forth below:

In another preferred example, the anti-CGRP antibodies described herein further comprises the gamma-1 constant heavy chain polypeptide sequence comprising the sequence set forth below:

In another example, the specification contemplates an isolated anti-CGRP antibody comprising a V_{H} polypeptide sequence selected from: SEQ ID NO: 3, 13, 23, 33, 43, 53, 63, 73, 83, 93, 103, 113, 123, or 133, or a variant thereof; and further comprising a V_{L} polypeptide sequence selected from: SEQ ID NO: 1, 11, 21, 31, 41, 51, 61, 71, 81, 91, 101, 111, 121, or 131, or a variant thereof, wherein one or more of the framework residues (FR residues) in said V_{H or} V_{L} polypeptide has been substituted with another amino acid residue resulting in an anti-CGRP antibody that specifically binds CGRP. The specification contemplates humanized and chimeric forms of these antibodies. The chimeric antibodies may include an Fc derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19 constant regions.

In one example of the specification, the antibodies or V_{H} or V_{L} polypeptides originate or are selected from one or more rabbit B cell populations prior to initiation of the humanization process referenced herein.

In another example of the specification, the anti-CGRP antibodies and fragments thereof do not have binding specificity for CGRP-R. In a further example of the specification, the anti-CGRP antibodies and fragments thereof inhibit the association of CGRP with CGRP-R. In another example of the specification, the anti-CGRP antibodies and fragments thereof inhibit the association of CGRP with CGRP-R and/or additional proteins and/or multimers thereof, and/or antagonize the biological effects thereof.

As stated above, antibodies and fragments thereof may be modified post-translationally to add effector moieties such as chemical linkers, detectable moieties such as for example fluorescent dyes, enzymes, substrates, bioluminescent materials, radioactive materials, and chemiluminescent moieties, or functional moieties such as for example streptavidin, avidin, biotin, a cytotoxin, a cytotoxic agent, and radioactive materials.

Antibodies or fragments thereof may also be chemically modified to provide additional advantages such as increased solubility, stability and circulating time (in vivo half-life) of the polypeptide, or decreased immunogenicity (See U.S. Pat. No. 4,179,337). The chemical moieties for derivatization may be selected from water soluble polymers such as polyethylene glycol, ethylene glycol/propylene glycol copolymers, carboxymethylcellulose, dextran, polyvinyl alcohol and the like. The antibodies and fragments thereof may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa. Branched polyethylene glycols are described, for example, in U.S. Pat. No. 5,643,575; Morpurgo et al., Appl. Biochem. Biotechnol. 56:59-72 (1996); Vorobjev et al., Nucleosides Nucleotides 18:2745-2750 (1999); and Caliceti et al., Bioconjug. Chem. 10:638-646 (1999).

There are a number of attachment methods available to those skilled in the art, See e.g., EP 0 401 384 (coupling PEG to G-CSF), See also Malik et al., Exp. Hematol. 20:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

As suggested above, polyethylene glycol may be attached to proteins via linkage to any of a number of amino acid residues. For example, polyethylene glycol can be linked to polypeptides via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) or to more than one type of amino acid residue (e.g., lysine, histidine, aspartic acid, glutamic acid, cysteine and combinations thereof).

Alternatively, antibodies or fragments thereof may have increased in vivo half-lives via fusion with albumin (including but not limited to recombinant human serum albumin or fragments or variants thereof (See, e.g., U.S. Pat. No. 5,876,969, issued Mar. 2, 1999, EP Patent 0 413 622, and U.S. Pat. No. 5,766,883, issued Jun. 16, 1998)) or other circulating blood proteins such as transferrin or ferritin. In a preferred example, polypeptides and/or antibodies of the present specification (including fragments or variants thereof) are fused with the mature form of human serum albumin (i.e., amino acids 1-585 of human serum albumin as shown in FIGS. 1 and 2 of EP Patent 0 322 094). Polynucleotides encoding fusion proteins of the specification are also encompassed by the specification.

Regarding detectable moieties, further exemplary enzymes include, but are not limited to, horseradish peroxidase, acetylcholinesterase, alkaline phosphatase, beta-galactosidase and luciferase. Further exemplary fluorescent materials include, but are not limited to, rhodamine, fluorescein, fluorescein isothiocyanate, umbelliferone, dichlorotriazinylamine, phycoerythrin and dansyl chloride. Further exemplary chemiluminescent moieties include, but are not limited to, luminol. Further exemplary bioluminescent materials include, but are not limited to, luciferin and aequorin. Further exemplary radioactive materials include, but are not limited to, Iodine 125 (¹²⁵I), Carbon 14 (¹⁴C), Sulfur 35 (³⁵S), Tritium (³H) and Phosphorus 32 (³²P).

Regarding functional moieties, exemplary cytotoxic agents include, but are not limited to, methotrexate, aminopterin, 6-mercaptopurine, 6-thioguanine, cytarabine, 5-fluorouracil dacarbazine; alkylating agents such as mechlorethamine, thiotepa chlorambucil, melphalan, carmustine (BSNU), mitomycin C, lomustine (CCNU), 1-methylnitrosourea, cyclophosphamide, mechlorethamine, busulfan, dibromomannitol, streptozotocin, mitomycin C, cis-dichlorodiammineplatinum (II) (DDP), cisplatin, carboplatin (Paraplatin); anthracyclines include daunorubicin (formerly daunomycin), doxorubicin (Adriamycin), detorubicin, carminomycin, idarubicin, epirubicin, mitoxantrone and bisantrene; antibiotics include dactinomycin (actinomycin D), bleomycin, calicheamicin, mithramycin, and anthramycin (AMC); and antimitotic agents such as the vinca alkaloids, vincristine and vinblastine. Other cytotoxic agents include paclitaxel (Taxol), ricin, pseudomonas exotoxin, gemcitabine, cytochalasin B, gramicidin D, ethidium bromide, emetine, etoposide, teniposide, colchicine, dihydroxy anthracin dione, 1-dehydrotestosterone, glucocorticoids, procaine, tetracaine, lidocaine, propranolol, puromycin, procarbazine, hydroxyurea, asparaginase, corticosteroids, mitotane (O,P'-(DDD)), interferons, and mixtures of these cytotoxic agents.

Further cytotoxic agents include, but are not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine and bleomycin. Toxic enzymes from plants and bacteria such as ricin, diphtheria toxin and Pseudomonas toxin may be conjugated to the humanized or chimeric antibodies, or binding fragments thereof, to generate cell-type-specific-killing reagents (Youle, et al., Proc. Nat'l Acad. Sci. USA 77:5483 (1980); Gilliland, et al., Proc. Nat'l Acad. Sci. USA 77:4539 (1980); Krolick, et al., Proc. Nat'l Acad. Sci. USA 77:5419 (1980)).

Other cytotoxic agents include cytotoxic ribonucleases as described by Goldenberg in U.S. Pat. No. 6,653,104. Examples of the specification also relate to radioimmunoconjugates where a radionuclide that emits alpha or beta particles is stably coupled to the antibody, or binding fragments thereof, with or without the use of a complex-forming agent. Such radionuclides include beta-emitters such as Phosphorus-32 (³²P), Scandium-47 (⁴⁷Sc), Copper-67 (⁶⁷Cu), Gallium-67 (⁶⁷Ga), Yttrium-88 (⁸⁸Y), Yttrium-90 (⁹⁰Y), Iodine-125 (¹²⁵I), Iodine-131 (¹³¹I), Samarium-153 (¹⁵³Sm), Lutetium-177 (¹⁷⁷Lu), Rhenium-186 (¹⁸⁶Re) or Rhenium-188 (¹⁸⁸Re), and alpha-emitters such as Astatine-211 (²¹¹At), Lead-212 (²¹²Pb), Bismuth-212 (²¹²Bi) or -213 (²¹³Bi) or Actinium-225 (²²⁵Ac).

Methods are known in the art for conjugating an antibody or binding fragment thereof to a detectable moiety and the like, such as for example those methods described by Hunter et al, Nature 144:945 (1962); David et al, Biochemistry 13:1014 (1974); Pain et al, J. Immunol. Meth. 40:219 (1981); and Nygren, J., Histochem. and Cytochem. 30:407 (1982).

Examples described herein further include variants and equivalents that are substantially homologous to the antibodies, antibody fragments, diabodies, SMIPs, camelbodies, nanobodies, IgNAR, polypeptides, variable regions and CDRs set forth herein. These may contain, e.g., conservative substitution mutations, (i.e., the substitution of one or more amino acids by similar amino acids). For example, conservative substitution refers to the substitution of an amino acid with another within the same general class, e.g., one acidic amino acid with another acidic amino acid, one basic amino acid with another basic amino acid, or one neutral amino acid by another neutral amino acid. What is intended by a conservative amino acid substitution is well known in the art.

In another example, the specification contemplates polypeptide sequences having at least 90% or greater sequence homology to any one or more of the polypeptide sequences of antibody fragments, variable regions and CDRs set forth herein. More preferably, the specification contemplates polypeptide sequences having at least 95% or greater sequence homology, even more preferably at least 98% or greater sequence homology, and still more preferably at least 99% or greater sequence homology to any one or more of the polypeptide sequences of antibody fragments, variable regions and CDRs set forth herein. Methods for determining homology between nucleic acid and amino acid sequences are well known to those of ordinary skill in the art.

In another example, the specification further contemplates the above-recited polypeptide homologs of the antibody fragments, variable regions and CDRs set forth herein further having anti-CGRP activity. Non-limiting examples of anti-CGRP activity are set forth herein.

In another example, the specification further contemplates the generation and use of anti-idiotypic antibodies that bind any of the foregoing sequences. In an exemplary example, such an anti-idiotypic antibody could be administered to a subject who has received an anti-CGRP antibody to modulate, reduce, or neutralize, the effect of the anti-CGRP antibody. Such anti-idiotypic antibodies could also be useful for treatment of an autoimmune disease characterized by the presence of anti-CGRP antibodies. A further exemplary use of such anti-idiotypic antibodies is for detection of the anti-CGRP antibodies of the present specification, for example to monitor the levels of the anti-CGRP antibodies present in a subject's blood or other bodily fluids.

The present specification also contemplates anti-CGRP antibodies comprising any of the polypeptide or polynucleotide sequences described herein substituted for any of the other polynucleotide sequences described herein. For example, without limitation thereto, the present specification contemplates antibodies comprising the combination of any of the variable light chain and variable heavy chain sequences described herein, and further contemplates antibodies resulting from substitution of any of the CDR sequences described herein for any of the other CDR sequences described herein.

### Additional Examples of the Specification

In another example, the specification contemplates one or more anti-human CGRP antibodies or antibody fragments thereof which specifically bind to the same linear or conformational epitope(s) and/or competes for binding to the same linear or conformational epitope(s) on an intact human CGRP polypeptide or fragment thereof as an anti-human CGRP antibody selected from Ab1, Ab2, Ab3, Ab4, Ab5, Ab6, Ab7, Ab8, Ab9, Ab10, Ab11, Ab12, Ab13, or Ab14. Said one or more anti-human CGRP antibodies or antibody fragments thereof may be non-naturally occurring, such as humanized or chimeric antibodies, non-naturally occurring antibody fragments, antibodies incorporating a tag or label, etc. In a preferred example, the anti-human CGRP antibody or fragment thereof specifically binds to the same linear or conformational epitope(s) and/or competes for binding to the same linear or conformational epitope(s) on an intact human CGRP polypeptide or a fragment thereof as Ab3, Ab6, Ab13, or Ab14.

A preferred example of the specification is directed to chimeric or humanized antibodies and fragments thereof (including Fab fragments) having binding specificity for CGRP and inhibiting biological activities mediated by the binding of CGRP to the CGRP receptor. In an example of the specification, the chimeric or humanized anti-CGRP antibodies are selected from Ab3, Ab6, Ab13, or Ab14.

In another example of the specification, the anti-human CGRP antibody is an antibody which specifically binds to the same linear or conformational epitopes on an intact CGRP polypeptide or fragment thereof that is (are) specifically bound by Ab3, Ab6, Ab13, or Ab14 as ascertained by epitopic mapping using overlapping linear peptide fragments which span the full length of the native human CGRP polypeptide.

The specification is also directed to an anti-CGRP antibody that binds with the same CGRP epitope and/or competes with an anti-CGRP antibody for binding to CGRP as an antibody or antibody fragment disclosed herein, including but not limited to an anti-CGRP antibody selected from Ab1, Ab2, Ab3, Ab4, Ab5, Ab6, Ab7, Ab8, Ab9, Ab10, Ab11, Ab12, Ab13, or Ab14.

In another example, the specification is also directed to an isolated anti-CGRP antibody or antibody fragment comprising one or more of the CDRs contained in the V_{H} polypeptide sequences selected from: 3, 13, 23, 33, 43, 53, 63, 73, 83, 93, 103, 113, 123, or 133, or a variant thereof, and/or one or more of the CDRs contained in the V_{L} polypeptide sequences selected from: 1, 11, 21, 31, 41, 51, 61, 71, 81, 91, 101, 111, 121, or 131, or a variant thereof.

The specification further contemplates that the one or more anti-human CGRP antibodies discussed above are aglycosylated or if glycosylated contain only mannose residues; that contain an Fc region that has been modified to alter effector function, half-life, proteolysis, and/or glycosylation; are human, humanized, single chain or chimeric; and are a humanized antibody derived from a rabbit (parent) anti-human CGRP antibody.

The specification further contemplates one or more anti-human CGRP antibodies wherein the framework regions (FRs) in the variable light region and the variable heavy regions of said antibody respectively are human FRs which are unmodified or which have been modified by the substitution of one or more human FR residues in the variable light or heavy chain region with the corresponding FR residues of the parent rabbit antibody, and wherein said human FRs have been derived from human variable heavy and light chain antibody sequences which have been selected from a library of human germline antibody sequences based on their high level of homology to the corresponding rabbit variable heavy or light chain regions relative to other human germline antibody sequences contained in the library.

In one example of the specification, the anti-human CGRP antibody or fragment specifically binds to CGRP expressing human cells and/or to circulating soluble CGRP molecules *in vivo,* including CGRP expressed on or by human cells in a patient with a disease associated with cells that express CGRP.

The specification further contemplates anti-human CGRP antibodies or fragments directly or indirectly attached to a detectable label or therapeutic agent.

The specification also contemplates one or more nucleic acid sequences which result in the expression of an anti-human CGRP antibody or antibody fragment as set forth above, including those comprising, or alternatively consisting of, yeast or human preferred codons. The specification also contemplates vectors (including plasmids or recombinant viral vectors) comprising said nucleic acid sequence(s). The specification also contemplates host cells or recombinant host cells expressing at least one of the antibodies set forth above, including a mammalian, yeast, bacterial, and insect cells. In a preferred example, the host cell is a yeast cell. In a further preferred example, the yeast cell is a diploidal yeast cell. In an exemplified example, the yeast cell is a Pichia yeast.

The specification also contemplates a method of treatment comprising administering to a patient with a disease or condition associated with CGRP expressing cells a therapeutically effective amount of at least one anti-human CGRP antibody or fragment described herein. The specification also contemplates that the treatment method may involve the administration of two or more anti-CGRP antibodies or fragments thereof and disclosed herein. If more than one antibody is administered to the patient, the multiple antibodies may be administered simultaneously or concurrently, or may be staggered in their administration.

The anti-CGRP activity of the anti-CGRP antibodies of the present specification, and fragments thereof having binding specificity to CGRP, may also be described by their strength of binding or their affinity for CGRP. In one example of the specification, the anti-CGRP antibodies of the present specification, and fragments thereof having binding specificity to CGRP, bind to CGRP with a dissociation constant (K_{D}) of less than or equal to 5×10⁻⁷ M, 10⁻⁷ M, 5×10⁻⁸ M, 10⁻⁸ M, 5×10⁻⁹ M, 10⁻⁹ M, 5×10⁻¹⁰ M, 10⁻¹⁰ M, 5×10⁻¹¹ M, 10⁻¹¹ M, 5×10⁻¹² M, 10⁻¹² M, 5×10⁻¹³ M, or 10⁻¹³ M. Preferably, the anti-CGRP antibodies and fragments thereof bind CGRP with a dissociation constant of less than or equal to 10⁻¹¹ M, 5×10⁻¹² M, or 10⁻¹² M. In another example of the specification, the anti-CGRP antibodies of the present specification, and fragments thereof having binding specificity to CGRP, bind to a linear or conformational CGRP epitope.

In another example of the specification, the anti-CGRP activity of the anti-CGRP antibodies of the present specification, and fragments thereof having binding specificity to CGRP, bind to CGRP with an off-rate of less than or equal to 10⁻⁴ S⁻¹, 5×10⁻⁵ S⁻¹, 10⁻⁵ S⁻¹, 5×10⁻⁶ S⁻¹, 10⁻⁶ S⁻¹, 5×10⁻⁷ S⁻¹, or 10⁻⁷S⁻¹.

In a further example of the specification, the anti-CGRP activity of the anti-CGRP antibodies of the present specification, and fragments thereof having binding specificity to CGRP, exhibit anti-CGRP activity by preventing, ameliorating or reducing the symptoms of, or alternatively treating, diseases and disorders associated with CGRP. Non-limiting examples of diseases and disorders associated with CGRP are set forth herein.

### Polynucleotides Encoding Anti-CGRP Antibody Polypeptides

In exemplary examples, the anti-CGRP antibodies may be encoded by polynucleotide sequences set forth in the biological sequence listing contained herein, or other encoding polynucleotides as may be readily identified by one of ordinary skill in the art. Examples thereof include the polynucleotide of SEQ ID NO: 141 (encoding the polypeptide of SEQ ID NO: 1), the polynucleotide of SEQ ID NO: 142 (encoding the polypeptide of SEQ ID NO: 2), the polynucleotide of SEQ ID NO: 143 (encoding the polypeptide of SEQ ID NO: 3), the polynucleotide of SEQ ID NO: 144 (encoding the polypeptide of SEQ ID NO: 4), the polynucleotide of SEQ ID NO: 151 (encoding the polypeptide of SEQ ID NO: 11), the polynucleotide of SEQ ID NO: 152 (encoding the polypeptide of SEQ ID NO: 12), the polynucleotide of SEQ ID NO: 153 (encoding the polypeptide of SEQ ID NO: 13), the polynucleotide of SEQ ID NO: 154 (encoding the polypeptide of SEQ ID NO: 14), the polynucleotide of SEQ ID NO: 161 (encoding the polypeptide of SEQ ID NO: 21), the polynucleotide of SEQ ID NO: 162 (encoding the polypeptide of SEQ ID NO: 22), the polynucleotide of SEQ ID NO: 163 (encoding the polypeptide of SEQ ID NO: 23), the polynucleotide of SEQ ID NO: 164 (encoding the polypeptide of SEQ ID NO: 24), the polynucleotide of SEQ ID NO: 171 (encoding the polypeptide of SEQ ID NO: 31), the polynucleotide of SEQ ID NO: 172 (encoding the polypeptide of SEQ ID NO: 32), the polynucleotide of SEQ ID NO: 173 (encoding the polypeptide of SEQ ID NO: 33), the polynucleotide of SEQ ID NO: 174 (encoding the polypeptide of SEQ ID NO: 34), the polynucleotide of SEQ ID NO: 181 (encoding the polypeptide of SEQ ID NO: 41), the polynucleotide of SEQ ID NO: 182 (encoding the polypeptide of SEQ ID NO: 42), the polynucleotide of SEQ ID NO: 183 (encoding the polypeptide of SEQ ID NO: 43), the polynucleotide of SEQ ID NO: 184 (encoding the polypeptide of SEQ ID NO: 44), the polynucleotide of SEQ ID NO: 191 (encoding the polypeptide of SEQ ID NO: 51), the polynucleotide of SEQ ID NO: 192 (encoding the polypeptide of SEQ ID NO: 52), the polynucleotide of SEQ ID NO: 193 (encoding the polypeptide of SEQ ID NO: 53), the polynucleotide of SEQ ID NO: 194 (encoding the polypeptide of SEQ ID NO: 54), the polynucleotide of SEQ ID NO: 201 (encoding the polypeptide of SEQ ID NO: 61), the polynucleotide of SEQ ID NO: 202 (encoding the polypeptide of SEQ ID NO: 62), the polynucleotide of SEQ ID NO: 203 (encoding the polypeptide of SEQ ID NO: 63), the polynucleotide of SEQ ID NO: 204 (encoding the polypeptide of SEQ ID NO: 64), the polynucleotide of SEQ ID NO: 211 (encoding the polypeptide of SEQ ID NO: 71), the polynucleotide of SEQ ID NO: 212 (encoding the polypeptide of SEQ ID NO: 72), the polynucleotide of SEQ ID NO: 213 (encoding the polypeptide of SEQ ID NO: 73), the polynucleotide of SEQ ID NO: 214 (encoding the polypeptide of SEQ ID NO: 74), the polynucleotide of SEQ ID NO: 221 (encoding the polypeptide of SEQ ID NO: 81), the polynucleotide of SEQ ID NO: 222 (encoding the polypeptide of SEQ ID NO: 82), the polynucleotide of SEQ ID NO: 223 (encoding the polypeptide of SEQ ID NO: 83), the polynucleotide of SEQ ID NO: 224 (encoding the polypeptide of SEQ ID NO: 84), the polynucleotide of SEQ ID NO: 231 (encoding the polypeptide of SEQ ID NO: 91), the polynucleotide of SEQ ID NO: 232 (encoding the polypeptide of SEQ ID NO: 92), the polynucleotide of SEQ ID NO: 233 (encoding the polypeptide of SEQ ID NO: 93), the polynucleotide of SEQ ID NO: 234 (encoding the polypeptide of SEQ ID NO: 94), the polynucleotide of SEQ ID NO: 241 (encoding the polypeptide of SEQ ID NO: 101), the polynucleotide of SEQ ID NO: 242 (encoding the polypeptide of SEQ ID NO: 102), the polynucleotide of SEQ ID NO: 243 (encoding the polypeptide of SEQ ID NO: 103), the polynucleotide of SEQ ID NO: 244 (encoding the polypeptide of SEQ ID NO: 104), the polynucleotide of SEQ ID NO: 251 (encoding the polypeptide of SEQ ID NO: 111), the polynucleotide of SEQ ID NO: 252 (encoding the polypeptide of SEQ ID NO: 112), the polynucleotide of SEQ ID NO: 253 (encoding the polypeptide of SEQ ID NO: 113), the polynucleotide of SEQ ID NO: 254 (encoding the polypeptide of SEQ ID NO: 114), the polynucleotide of SEQ ID NO: 261 (encoding the polypeptide of SEQ ID NO: 121), the polynucleotide of SEQ ID NO: 262 (encoding the polypeptide of SEQ ID NO: 122), the polynucleotide of SEQ ID NO: 263 (encoding the polypeptide of SEQ ID NO: 123), the polynucleotide of SEQ ID NO: 264 (encoding the polypeptide of SEQ ID NO: 124), the polynucleotide of SEQ ID NO: 271 (encoding the polypeptide of SEQ ID NO: 131), the polynucleotide of SEQ ID NO: 272 (encoding the polypeptide of SEQ ID NO: 132), the polynucleotide of SEQ ID NO: 273 (encoding the polypeptide of SEQ ID NO: 133), or the polynucleotide of SEQ ID NO: 274 (encoding the polypeptide of SEQ ID NO: 134).

### B-cell Screening and Isolation

In one example, the present specification contemplates the preparation and isolation of a clonal population of antigen-specific B cells that may be used for isolating at least one CGRP antigen-specific cell, which can be used to produce a monoclonal antibody against CGRP, which is specific to a desired CGRP antigen, or a nucleic acid sequence corresponding to such an antibody. Methods of preparing and isolating said clonal population of antigen-specific B cells are taught, for example, in U.S. patent publication no. US 2007/0269868 to Carvalho-Jensen et al. Methods of preparing and isolating said clonal population of antigen-specific B cells are also taught herein in the examples. Methods of "enriching" a cell population by size or density are known in the art. *See,* e.g., U.S. Patent 5,627,052. These steps can be used in addition to enriching the cell population by antigen-specificity.

### Methods of Humanizing Antibodies

In another example, the present specification contemplates methods for humanizing antibody heavy and light chains. Methods for humanizing antibody heavy and light chains which may be applied to anti-CGRP antibodies are taught, for example, in U.S. patent application publication no. US 2009/0022659 to Olson et al.*,* and in U.S. patent no. 7,935,340 to Garcia-Martinez et al.

### Screening Assays

The specification also includes screening assays designed to assist in the identification of diseases and disorders associated with CGRP in patients exhibiting symptoms of a CGRP associated disease or disorder. For example, the present specification includes assays that detect insulin insensitivity (resistance) or glucose utilization in a subject. Said subject may optionally be in a fasted state or post-prandial state.

In one example of the specification, the anti-CGRP antibodies of the specification, or CGRP binding fragments thereof, are used to detect the presence of CGRP in a biological sample obtained from a patient exhibiting symptoms of a disease or disorder associated with CGRP. The presence of CGRP, or elevated levels thereof when compared to pre-disease levels of CGRP in a comparable biological sample, may be beneficial in diagnosing a disease or disorder associated with CGRP.

Another example of the specification provides a diagnostic or screening assay to assist in diagnosis of diseases or disorders associated with CGRP in patients exhibiting symptoms of a CGRP associated disease or disorder identified herein, comprising assaying the level of CGRP expression in a biological sample from said patient using a post-translationally modified anti-CGRP antibody or binding fragment thereof. The anti-CGRP antibody or binding fragment thereof may be post-translationally modified to include a detectable moiety such as set forth previously in the disclosure.

The CGRP level in the biological sample may be determined using a modified anti-CGRP antibody or binding fragment thereof as set forth herein, and comparing the level of CGRP in the biological sample against a standard level of CGRP (e.g., the level in normal biological samples). The skilled clinician would understand that some variability may exist between normal biological samples, and would take that into consideration when evaluating results. In one example of the specification, the anti-CGRP antibodies of the specification may be used to correlate CGRP expression levels with a particular stage of impaired glucose metabolism. For example, correlating levels of circulating CGRP with glucose and/or insulin levels will allow for establishing the level of insulin insensitivity, or hyperglycemia. Insulin sensitivity may additionally be measured in a subject using methods known in the art, for example as described in Muniyappa et al. (Am J Physiol Endocrinol Metab 294:E15-E26, 2008). In brief, insulin sensitivity may be measured using a variety of methods including hyperinsulinemic euglycemic glucose clamp, the insulin suppression test, QUICKI, HOMA, 1/insulin, or the Matusda index. One skilled in the art would be able to measure CGRP in numerous subjects in order to establish ranges of CGRP expression that correspond to clinically defined stages of diabetic development or pre-diabetes.

The above-recited assay may also be useful in monitoring a disease or disorder, where the level of CGRP obtained in a biological sample from a patient believed to have a CGRP associated disease or disorder is compared with the level of CGRP in prior biological samples from the same patient, in order to ascertain whether the CGRP level in said patient has changed with, for example, a treatment regimen. One skilled in the art would understand that by measuring CGRP in the patient at different intervals, the progression of the impairment to an individual's ability metabolize glucose can be determined.

The specification is also directed to a method of *in vivo* imaging which detects the presence of cells expressing CGRP comprising administering a diagnostically effective amount of a diagnostic composition. Said detection can be useful as part of a planning regimen for the design of an effective treatment protocol for diabetes or patients at risk for developing diabetes.

In one example, the methods of the specification include one or more compositions used for treating impaired glucose metabolism, such as insulin resistance, impaired insulin secretion or hyperglycemia in combination with the anti-CGRP antibodies disclosed herein. Of particular interest are, for example, one or more of sulfonylureas, PPAR-gamma agonists, GPL-1 receptor agonists, dipeptidyl peptidase IV inhibitor, amylin analogs, biguanides, dopamine D2 receptor agonists, meglitinides, alpha-glucosidase inhibitor, antidyslipidemic bile acid sequestrant, insulin, cytokine therapy, gene therapy, and antibody therapy, as well as an anti-CGRP antibody or fragment thereof. Examples of biguanides include: Metformin such as Glucophage and Glucophage XR (Bristol Myers Squibb/Merck Serono), Fortamet (Watson), Glumetza (Biovail/Depomed/Santarus), and generics. Examples of sulfonylureas include Glimepiride such as Amaryl (Sanofi) and generics; Glipizide such as Glucotrol and Glucotrol XL (Pfizer) and generics; Glyburide/glibenclamide such as Diabeta (Sanofi), Micronase/Glynase (Pfizer) and generics; Metformin + glyburide such as Glucovance (Bristol Myers Squibb), Suguan M (Sanofi-Aventis), GlicoRest, GlucoNorm (Abiogen), Bi-Euglucon (Roche) and generics; Metformin + glipizide such as Metaglip (Bristol Myers Squibb), and generics. Examples of PPAR-gamma agonists include: Rosiglitazone such as Avandia (GlaxoSmithKline); Pioglitazone such as Actos (Takeda) and generics; Rosiglitazone + metformin such as Avandamet (GlaxoSmithKline); Pioglitazone + metformin such as Actoplus Met XR (Takeda); Pioglitazone + glimepiride such as Avandaryl/Avaglim (GlaxoSmithKline); Pioglitazone + glimepiride such as Duetact/Tandemact/Sonias (Takeda). Examples of GLP-1 receptor agonists include: Exenatide such as Byetta (Bristol Myers Squibb/AstraZeneca); Liraglutide such as Victoza (Novo Nordisk); Exenatide LAR such as Bydureon (Bristol Myers Squibb/AstraZeneca). Examples of Dipeptidyl peptidase IV (DPP-IV or DPP4) inhibitors include: Sitagliptin such as Januvia, Merck; Vildagliptin such as Galvus (Novartis); Saxagliptin such as Onglyza (Bristol Myers Squibb/AstraZeneca); Alogliptin such as Nesina (Takeda/Furiex); Linagliptin such as Trazenta (Boehringer Ingelheim/Eli Lilly); Teneligliptin such as Tenelia (Mitsubishi Tanabe/Daiichi Sankyo); Sitagliptin + metformin such as Janumet (Merck) and Janumet XR (Merck); Sitagliptin + simvastatin such as JuviSync (Merck); Vildagliptin + metformin such as Eurcreas (Novartis); Saxagliptin + metformin such as Kombiglyze/Kombiglyze XR (AstraZeneca/Bristol Myers Squibb); Alogliptin + pioglitazone such as Liovel (Takeda/Furiex); Linagliptin + metformin such as Jentadueto (Boehringer Ingelheim/Eli Lilly). Examples of Meglitinides include: Repaglinide such as GlucoNorm/Prandin/NovoNorm (Daiichi Sankyo/Fournier Pharma/Novo Nordisk); Nateglinide such as Starlix (Novartis), Fastic (Daiichi Sankyo), Starsis (Astellas) and generics; Mitiglinide such as Glufast (Kissei/Takeda). Examples of Alpha-glucosidase inhibitors include: Acarbose such as Precose/Glucobay (Bayer) and generics; Miglitol such as Glyset (Pfizer), Diastabol (Sanofi), Seibule (Sanwa Kagaku) and generics; voglibose such as Basen (Takeda) and generics. Example of a bile acid sequestrants include: Colesevelam such as Cholestagel (Sanofi), Welchol (Daiichi Sankyo). Example of a Dopamine D2 receptor agonist includes Bromocriptine such as Cycloset (Santarus). Example of an amylin analogue includes Pramlintide such as Symlin (Bristol Myers Squibb/AstraZeneca). Examples of fast-acting insulins include: insulin lispro such as Humalog (Eli Lilly); Insulin aspart such as NovoLog (Novo Nordisk), NovoRapid (Novo Nordisk), Insulin glulisine such as Apidra (Sanofi). Examples of regular human insulins include: Humulin/Umuline Rapide (Eli Lilly), Novolin R (Novo Nordisk), Actrapid (Sanofi). Examples of intermediate-acting insulins include: Humulin N (Eli Lilly), Novolin N (Novo Nordisk). Examples of long-lasting insulins include: Insulin glargine such as Lantus (Sanofi) and insulin detemir such as Levemir (Novo Nordisk).

The present specification further provides for a kit for detecting binding of an anti-CGRP antibody of the specification to CGRP. In particular, the kit may be used to detect the presence of a CGRP specifically reactive with an anti-CGRP antibody of the specification or an immunoreactive fragment thereof. The kit may also include an antibody bound to a substrate, a secondary antibody reactive with the antigen and a reagent for detecting a reaction of the secondary antibody with the antigen. Such a kit may be an ELISA kit and can comprise the substrate, primary and secondary antibodies when appropriate, and any other necessary reagents such as detectable moieties, enzyme substrates, and color reagents, for example as described herein. The diagnostic kit may also be in the form of an immunoblot kit. The diagnostic kit may also be in the form of a chemiluminescent kit (Meso Scale Discovery, Gaithersburg, MD). The diagnostic kit may also be a lanthanide-based detection kit (PerkinElmer, San Jose, CA).

A skilled clinician would understand that a biological sample includes, but is not limited to, sera, plasma, urine, saliva, mucous, pleural fluid, synovial fluid and spinal fluid.

### Methods of Ameliorating or Reducing Symptoms of, or Treating, or Preventing, Diseases and Disorders Associated with, CGRP

In another example of the specification, anti-CGRP antibodies described herein, or fragments thereof, are useful for ameliorating or reducing the symptoms of, or treating, or preventing, diseases and disorders associated with CGRP. Anti-CGRP antibodies described herein, or fragments thereof, as well as combinations, can also be administered in a therapeutically effective amount to patients in need of treatment of diseases and disorders associated with CGRP in the form of a pharmaceutical composition as described in greater detail below.

In another example of the specification, anti-CGRP antibodies described herein, or fragments thereof, are useful for ameliorating or reducing the symptoms of, or treating, or preventing, impaired glucose tolerance, insulin resistance (insensitivity), impaired insulin secretion, lipotoxicity, hyperglycemia, pancreatic beta cell failure as a result of diabetes, pre-diabetes, Type 1 diabetes, Type 2 diabetes, or gestational diabetes.

In exemplary examples, the anti-CGRP antibodies described herein, or fragments thereof, may be administered to an individual at risk of developing diabetes, e.g., an individual diagnosed with pre-diabetes. Without intent to be limited by theory, it is believed that by restoring insulin sensitivity, the subject anti-CGRP antibodies may be able to delay or prevent the progression to diabetes.

In additional exemplary examples, the anti-CGRP antibodies described herein, or fragments thereof, may be administered to a patient that does not achieve normoglycemia with administration of another treatment, e.g., treatment with metformin, pioglitazone, a sulfonylurea, a glinide, an oral thiazolidinedione (TZD) such as pioglitazone, a glucagon-like peptide 1 (GLP-1) agonist such as exenatide, a DPP4 inhibitor such as sitagliptin, vildagliptin, saxagliptin, alogliptin, linagliptin, or teneligliptin, or a combination therapy such as metformin and pioglitazone, metformin and a sulfonylurea, metformin and a glinide, metformin and a TZD, metformin and pioglitazone, metformin and a GLP-1 agonist, metformin and exenatide, sitagliptin and metformin, sitagliptin and simvastatin, vildagliptin and metformin, saxagliptin and metformin, alogliptin and pioglitazone, or linagliptin and metformin.

In an additional exemplary example, anti-CGRP antibodies described herein, or fragments thereof, are administered for prevention or treatment of obesity, e.g., to individuals having a body mass index of at least 25. Without intent to be limited by theory, it is believed that the subject anti-CGRP antibodies may increase peripheral and/or hepatic glucose utilization, thereby increasing metabolic rate and contributing to weight loss. Said anti-CGRP antibodies may be administered in combination with another anti-obesity agent such as orlistat, rimonabant, sibutramine, a peptide YY (PYY, a 36 amino acid peptide that reduces appetite), a PYY analog, a CB-1 antagonist, rimonabant, a leptin, a leptin analog, or a phentermine.

### Administration

In one example of the specification, the anti-CGRP antibodies described herein, or CGRP binding fragments thereof, as well as combinations of said antibodies or antibody fragments, are administered to a subject at a concentration of between about 0.1 and 100.0 mg/kg of body weight of recipient subject. In an example of the specification, the anti-CGRP antibodies described herein, or CGRP binding fragments thereof, as well as combinations of said antibodies or antibody fragments, are administered to a subject at a concentration of about 0.4 mg/kg of body weight of recipient subject. In another example of the specification, the anti-CGRP antibodies described herein, or CGRP binding fragments thereof, as well as combinations of said antibodies or antibody fragments, are administered to a recipient subject with a frequency of once every twenty-six weeks or less, such as once every sixteen weeks or less, once every eight weeks or less, once every four weeks or less, once every two weeks or less, once every week or less, or once daily or less.

Fab fragments may be administered every two weeks or less, every week or less, once daily or less, multiple times per day, and/or every few hours. In one example of the specification, a patient receives Fab fragments of 0.1 mg/kg to 40 mg/kg per day given in divided doses of 1 to 6 times a day, or in a sustained release form, effective to obtain desired results.

It is to be understood that the concentration of the antibody or Fab administered to a given patient may be greater or lower than the exemplary administration concentrations set forth above in the two preceding paragraphs.

A person of skill in the art would be able to determine an effective dosage and frequency of administration through routine experimentation, for example guided by the disclosure herein and the teachings in Goodman, L. S., Gilman, A., Brunton, L. L., Lazo, J. S., & Parker, K. L. (2006). Goodman & Gilman's the pharmacological basis of therapeutics. New York: McGraw-Hill; Howland, R. D., Mycek, M. J., Harvey, R. A., Champe, P. C., & Mycek, M. J. (2006). Pharmacology. Lippincott's illustrated reviews. Philadelphia: Lippincott Williams & Wilkins; and Golan, D. E. (2008). Principles of pharmacology: the pathophysiologic basis of drug therapy. Philadelphia, Pa., [etc.]: Lippincott Williams & Wilkins.

In another example of the specification, the anti-CGRP antibodies described herein, or CGRP binding fragments thereof, as well as combinations of said antibodies or antibody fragments, are administered to a subject in a pharmaceutical formulation.

A "pharmaceutical composition" refers to a chemical or biological composition suitable for administration to a mammal. Such compositions may be specifically formulated for administration *via* one or more of a number of routes, including but not limited to buccal, epicutaneous, epidural, inhalation, intraarterial, intracardial, intracerebroventricular, intradermal, intramuscular, intranasal, intraocular, intraperitoneal, intraspinal, intrathecal, intravenous, oral, parenteral, rectally *via* an enema or suppository, subcutaneous, subdermal, sublingual, transdermal, and transmucosal. In addition, administration can occur by means of injection, powder, liquid, gel, drops, or other means of administration.

In one example of the specification, the anti-CGRP antibodies described herein, or CGRP binding fragments thereof, as well as combinations of said antibodies or antibody fragments, may be optionally administered in combination with one or more active agents. Such active agents include analgesic, anti-histamine, antipyretic, anti-inflammatory, antibiotic, antiviral, and anti-cytokine agents. Active agents include agonists, antagonists, and modulators of TNF-α, IL-2, IL-4, IL-6, IL-10, IL-12, IL-13, IL-18, IFN-α, IFN-γ, BAFF, CXCL13, IP-10, VEGF, EPO, EGF, HRG, Hepatocyte Growth Factor (HGF), Hepcidin, including antibodies reactive against any of the foregoing, and antibodies reactive against any of their receptors. Active agents also include but are not limited to 2-Arylpropionic acids, Aceclofenac, Acemetacin, Acetylsalicylic acid (Aspirin), Alclofenac, Alminoprofen, Amoxiprin, Ampyrone, Arylalkanoic acids, Azapropazone, Benorylate/Benorilate, Benoxaprofen, Bromfenac, Carprofen, Celecoxib, Choline magnesium salicylate, Clofezone, COX-2 inhibitors, Dexibuprofen, Dexketoprofen, Diclofenac, Diflunisal, Droxicam, Ethenzamide, Etodolac, Etoricoxib, Faislamine, fenamic acids, Fenbufen, Fenoprofen, Flufenamic acid, Flunoxaprofen, Flurbiprofen, Ibuprofen, Ibuproxam, Indometacin, Indoprofen, Kebuzone, Ketoprofen, Ketorolac, Lornoxicam, Loxoprofen, Lumiracoxib, Magnesium salicylate, Meclofenamic acid, Mefenamic acid, Meloxicam, Metamizole, Methyl salicylate, Mofebutazone, Nabumetone, Naproxen, N-Arylanthranilic acids, Nerve Growth Factor (NGF), Oxametacin, Oxaprozin, Oxicams, Oxyphenbutazone, Parecoxib, Phenazone, Phenylbutazone, Phenylbutazone, Piroxicam, Pirprofen, profens, Proglumetacin, Pyrazolidine derivatives, Rofecoxib, Salicyl salicylate, Salicylamide, Salicylates, Substance P, Sulfinpyrazone, Sulindac, Suprofen, Tenoxicam, Tiaprofenic acid, Tolfenamic acid, Tolmetin, and Valdecoxib.

An anti-histamine can be any compound that opposes the action of histamine or its release from cells (e.g., mast cells). Anti-histamines include but are not limited to acrivastine, astemizole, azatadine, azelastine, betatastine, brompheniramine, buclizine, cetirizine, cetirizine analogues, chlorpheniramine, clemastine, CS 560, cyproheptadine, desloratadine, dexchlorpheniramine, ebastine, epinastine, fexofenadine, HSR 609, hydroxyzine, levocabastine, loratidine, methscopolamine, mizolastine, norastemizole, phenindamine, promethazine, pyrilamine, terfenadine, and tranilast.

Antibiotics include but are not limited to Amikacin, Aminoglycosides, Amoxicillin, Ampicillin, Ansamycins, Arsphenamine, Azithromycin, Azlocillin, Aztreonam, Bacitracin, Carbacephem, Carbapenems, Carbenicillin, Cefaclor, Cefadroxil, Cefalexin, Cefalothin, Cefalotin, Cefamandole, Cefazolin, Cefdinir, Cefditoren, Cefepime, Cefixime, Cefoperazone, Cefotaxime, Cefoxitin, Cefpodoxime, Cefprozil, Ceftazidime, Ceftibuten, Ceftizoxime, Ceftobiprole, Ceftriaxone, Cefuroxime, Cephalosporins, Chloramphenicol, Cilastatin, Ciprofloxacin, Clarithromycin, Clindamycin, Cloxacillin, Colistin, Co-trimoxazole, Dalfopristin, Demeclocycline, Dicloxacillin, Dirithromycin, Doripenem, Doxycycline, Enoxacin, Ertapenem, Erythromycin, Ethambutol, Flucloxacillin, Fosfomycin, Furazolidone, Fusidic acid, Gatifloxacin, Geldanamycin, Gentamicin, Glycopeptides, Herbimycin, Imipenem, Isoniazid, Kanamycin, Levofloxacin, Lincomycin, Linezolid, Lomefloxacin, Loracarbef, Macrolides, Mafenide, Meropenem, Meticillin, Metronidazole, Mezlocillin, Minocycline, Monobactams, Moxifloxacin, Mupirocin, Nafcillin, Neomycin, Netilmicin, Nitrofurantoin, Norfloxacin, Ofloxacin, Oxacillin, Oxytetracycline, Paromomycin, Penicillin, Penicillins, Piperacillin, Platensimycin, Polymyxin B, Polypeptides, Prontosil, Pyrazinamide, Quinolones, Quinupristin, Rifampicin, Rifampin, Roxithromycin, Spectinomycin, Streptomycin, Sulfacetamide, Sulfamethizole, Sulfanilimide, Sulfasalazine, Sulfisoxazole, Sulfonamides, Teicoplanin, Telithromycin, Tetracycline, Tetracyclines, Ticarcillin, Tinidazole, Tobramycin, Trimethoprim, Trimethoprim-Sulfamethoxazole, Troleandomycin, Trovafloxacin, and Vancomycin.

Active agents also include Aldosterone, Beclometasone, Betamethasone, Corticosteroids, Cortisol, Cortisone acetate, Deoxycorticosterone acetate, Dexamethasone, Fludrocortisone acetate, Glucocorticoids, Hydrocortisone, Methylprednisolone, Prednisolone, Prednisone, Steroids, and Triamcinolone. Any suitable combination of these active agents is also contemplated.

A "pharmaceutical excipient" or a "pharmaceutically acceptable excipient" is a carrier, usually a liquid, in which an active therapeutic agent is formulated. In one example of the specification, the active therapeutic agent is a humanized antibody described herein, or one or more fragments thereof. The excipient generally does not provide any pharmacological activity to the formulation, though it may provide chemical and/or biological stability, and release characteristics. Exemplary formulations can be found, for example, in Remington's Pharmaceutical Sciences, 19th Ed., Grennaro, A., Ed., 1995.

As used herein "pharmaceutically acceptable carrier" or "excipient" includes any and all solvents, dispersion media, coatings, antibacterial and antifungal agents, isotonic and absorption delaying agents that are physiologically compatible. In one example, the carrier is suitable for parenteral administration. Alternatively, the carrier can be suitable for intravenous, intraperitoneal, intramuscular, or sublingual administration. Pharmaceutically acceptable carriers include sterile aqueous solutions or dispersions and sterile powders for the extemporaneous preparation of sterile injectable solutions or dispersions. The use of such media and agents for pharmaceutically active substances is well known in the art. Except insofar as any conventional media or agent is incompatible with the active compound, use thereof in the pharmaceutical compositions of the specification is contemplated. Supplementary active compounds can also be incorporated into the compositions.

Pharmaceutical compositions typically must be sterile and stable under the conditions of manufacture and storage. The specification contemplates that the pharmaceutical composition is present in lyophilized form. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol), and suitable mixtures thereof. The specification further contemplates the inclusion of a stabilizer in the pharmaceutical composition. The proper fluidity can be maintained, for example, by the maintenance of the required particle size in the case of dispersion and by the use of surfactants.

In many cases, it will be preferable to include isotonic agents, for example, sugars, polyalcohols such as mannitol, sorbitol, or sodium chloride in the composition. By including an agent such as, monostearate salts and gelatin, the absorption of the injectable compositions can be prolonged. Moreover, the alkaline polypeptide can be formulated in a time-release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are known to those skilled in the art.

For each of the recited examples, the compounds can be administered by a variety of dosage forms. Any biologically-acceptable dosage form known to persons of ordinary skill in the art, and combinations thereof, are contemplated. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal), infusions, and combinations thereof.

The above description of various illustrated examples of the specification is not intended to be exhaustive or to limit the specification to the precise form disclosed. While specific examples of, and examples for, the specification are described herein for illustrative purposes, various equivalent modifications are possible within the scope of the specification, as those skilled in the relevant art will recognize. The teachings provided herein of the specification can be applied to other purposes, other than the examples described above.

These and other changes can be made to the specification in light of the above detailed description. In general, in the following claims, the terms used should not be construed to limit the specification to the specific examples disclosed in the specification and the claims. Accordingly, the specification is not limited by the disclosure, but instead the scope of the specification is to be determined entirely by the following claims.

The specification may be practiced in ways other than those particularly described in the foregoing description and examples. Numerous modifications and variations of the specification are possible in light of the above teachings and, therefore, are within the scope of the appended claims.

Certain teachings related to methods for obtaining a clonal population of antigen-specific B cells were disclosed in U.S. Provisional patent application no. 60/801,412, filed May 19, 2006.

Certain teachings related to humanization of rabbit-derived monoclonal antibodies and preferred sequence modifications to maintain antigen binding affinity were disclosed in International Application No. PCT/US2008/064421, corresponding to International Publication No. WO/2008/144757, entitled "Novel Rabbit Antibody Humanization Methods and Humanized Rabbit Antibodies", filed May 21, 2008.

Certain teachings related to producing antibodies or fragments thereof using mating competent yeast and corresponding methods were disclosed in U.S. Patent application no. 11/429,053, filed May 8, 2006, (U.S. Patent Application Publication No. US2006/0270045).

Certain CGRP antibody polynucleotides and polypeptides are disclosed in the sequence listing accompanying this patent application filing.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g. amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

### EXAMPLES

### Example 1 - Reference Example

Normal rats were treated with Ab14 100mg/kg (via intravenous route, single administration 48h before clamps procedure) and the effects were compared to metformin 500mg/kg (via oral route, 2 administrations 24h and 4h before clamp procedure). The antibody used in this example consisted of the light and heavy polypeptide chains of SEQ ID NOs 132 and 134.

Blood glucose was measured in fed conditions before treatment, 18h after treatment with metformin and Ab14 and 42h after treatment with Ab14. The 2 compounds did not affect blood glucose in these conditions. Blood glucose was measured in fasted condition, just before clamp and only metformin had a significant decreasing effect (17%). Plasma insulin, measured in fed condition before treatment and 18h after treatment with metformin and 42h after treatment with Ab14, was slightly decreased by the 2 compounds, as well as the insulin resistance index HOMA-IR (not significant).

Plasma samples were obtained from Ab14 treated animals just prior to the clamp procedure, 48-hours post treatment, Ab14 concentration was determined. The results of this analysis confirmed systemic exposures ranging from 642 to 797 µg/mL Ab14 in rats undergoing the clamp procedure.

To assess the effect on whole body insulin sensitivity, a clamp procedure was performed using 0.3U/kg/h insulin and ³H-glucose. Rats were fasted for 6 hours before 180 minutes of perfusion. Steady state was reached after 140 minutes of infusion and the means of glucose infusion rate (GIR), whole body glucose turn over (GTO), hepatic glucose production (HGP), glycolysis, and glycogen synthesis were calculated from 140 min. to 180 min. A bolus of 14-C-2-deoxyglucose was administered 1 hour before the end of the clamp to measure tissue specific glucose utilization. As expected, metformin significantly increased GIR (27%) and GTO (30%), by increasing glycolysis and glycogen synthesis. Metformin increased glucose utilization in the mixed vastus lateralis muscle (VL, 49% p<0.05), in the glycolytic extensor digitorum longus muscle (EDL, 19% NS) and decreased glucose utilization in the heart (-39%, p<0.01), presumably due to the stimulation of myocardial fatty acid oxidation1. Ab14 tended to increase GIR and GTO (NS) and had a stronger effect than metformin on glucose utilization in VL (70%, p<0.01), in EDL (26%, NS), and in the oxidative soleus muscle (27%, NS). It also tended to increase glucose utilization in the heart (21%, NS). Similar to metformin, Ab14 did not affect glucose utilization rate in white adipose tissues (deep and subcutaneous).

In conclusion, Ab14 had a good trend to improve whole body glucose utilization in normal rats after acute treatment with a significant effect upon the glucose utilization rate in muscles (VL).

### Methods

Male Sprague Dawley rats were housed in housing cages (1500 cm² × 21 cm) throughout the experimental phase. Animals' cages litters were changed once a week. They were housed in groups of 3-4 animals during acclimation period and then individual housing after surgery until clamp procedure. Inverted 12hours light cycle (at 08:00 am lights off), 22 ± 2 °C and 55 ± 10 % relative humidity. Standard diet (RM1 (E) 801492, SDS) and tap water were provided ad libitum.

After 2 weeks of acclimation period, rats were anesthetized (isoflurane) and a catheter was implemented in the femoral vein. A recovering period was followed for 5-6 days before the clamp procedure.

Blood glucose (BG) was measured between 07:30 and 08:00 (just before the light off) am from the tip of the tail with glucometer and a blood collection (on EDTA) was performed just after to measure plasma insulin. The table below describes the conditions:

| | **2 days before clamp (blood volume)** | **1 day before clamp (blood volume)** | **The day of the clamp (blood volume)** |
|---|---|---|---|
| **Group 1** | BG + insulin (~40µL) | BG (∼1µL) | BG + insulin (~40µL) |
| **Group 2** | BG + insulin (~40µL) | BG (∼1µL) | BG + insulin + exposure (∼200µL) |
| **Group 3** | - | BG + insulin (~40µL) | BG + insulin (∼40µL) |

Plasma samples were kept at -80°C until insulin measurement (using ELISA method).

A sample of blood (~200 µL) was obtained just prior to the clamp procedure for each Group 2 animal, processed to plasma (~60 µL), and maintained at -80°C for subsequent determination of Ab14 concentrations utilizing a Meso Scale Discovery (MSD) ELISA platform.

Vehicle and Ab14 were administered by i.v. route 48h before the T0 of clamp procedure (at 02:00 pm two days before the clamp).

Metformin was administered by p.o. route 24h and 4h before the T0 of clamp procedure (at 02:00 pm the day before the clamp and at 10:00 am the day of the clamp).

The rats were fasted 6 hours before the start of the clamp (at ~8h, just after the blood collection).

The hyperinsulinemic-euglycemic clamp was performed using ³H-glucose as a tracer and 0.3U/kg/h insulin infusion from 02:00 pm (T0) to 05:00 pm (T+3h). A glucose solution was infused in parallel and the infusion rate was adjusted to reach the steady state (~100 +/-10 mg/dL). Blood glucose was measured from the tip of the tail using glucometers every 10 minutes. Blood was collected (10µL) from the tip of the tail during the last hour (steady state) and the following parameters were assessed: Glucose infusion rate; Whole body glucose utilization rate; Hepatic glucose production rate; Whole body glycogen and glycolytic rates.

To determine the individual tissue glucose utilization rate, a bolus injection of 100 µCi per rat of deoxy-D-glucose 2-¹⁴C (¹⁴C-2-DOG) through the femoral vein was performed 60 min before the end of the D-[3-³H]-glucose infusion. Plasma ¹⁴C-2-DOG disappearance and glucose concentration were determined in 10µL drops of blood sampled from the tip of the tail vein at 0, 5, 10, 15, 20, 25, 30, 45, and 60 minutes after the injection. At the end of the experiment, vastus lateralis (VL), extensor digitorum longus (EDL) and soleus muscles, epididymal and inguinal white adipose tissues, heart apex, and skin (as negative control) were dissected, flash frozen and kept at -80°C. A piece of each tissue was dissolved in 1M NaOH and then neutralized with 1M HCl. D-2-¹⁴C deoxyglucose 6-phosphate. D-2-¹⁴C deoxyglucose was differentially precipitated by the use of a zinc hydroxide (0.3M) solution or a perchloric acid solution (6%). Both radioactivity contents were measured to evaluate the glucose uptake expressed as ng/mg/min.

Plasma insulin level was measured at the end of the clamp.

Statistical analyses were performed using GraphPad prism software. Histograms were analyzed using an ANOVA one way with a Dunnett's post test and curves were analyzed using an ANOVA two ways with a Bonferroni's post test. A difference was considered significant when p value was <0.05. NS: not significant.

### Results and discussion

**Blood glucose and insulin measurement.** In fed conditions, blood glucose level was not affected 42h after treatment with Ab 14 100mg/kg and 18h after treatment with metformin 500mg/kg compared to the vehicle group (FIG. 1A). At the same time, Ab14 and metformin decreased plasma insulin level by 11% and 18% respectively (non significant, FIG. IB). Then the index of insulin resistance HOMA-IR was decreased in a similar manner compared to the vehicle group (FIG. 1C). On the other hand, in 6 hours fasting condition, metformin significantly decreased blood glucose by 17% 30h after treatment (FIG. ID).

**Whole body Glucose fluxes**. Hyperinsulinemic euglycemic clamps were performed in 6 hours fasting conditions 48h after the single administration of Ab14 100mg/kg, and 4h after the last administration of metformin 500mg/kg.

Metformin significantly increased GIR evolution from 60 minutes after the start of the infusion compared to vehicle group. Ab14 had a trend to increase the GIR evolution mostly after 130 minutes infusion (FIG. 2A).

The plateau of glucose infusion rate was reached from 140 min in all groups. The blood glucose level was similar in all groups during this steady state (FIG. 2B). Plasma insulin level at the end of the clamp was also similar in all groups (FIG. 2C). The plasma insulin level reached at the end of the clamp was almost the same as that measured in fed conditions, i.e., the dose of insulin used to obtain hyperinsulinemia was physiological.

Glucoses fluxes were then calculated from 140 minutes to 180 minutes of infusion (FIG. 3). Ab14 and metformin increased the glucose infusion rate by 18% (NS) and 27% (p<0.05) respectively, as well as the glucose turn over by 18% (NS) and 30% (p<0.05) respectively. The hepatic glucose production was totally inhibited by this supraphysiological dose of insulin in the 3 groups. Ab14 did not affect glycolysis rate whereas metformin increased it by 43% (NS). Ab14 increased glycogen synthesis rate by 23% similarly to metformin (NS).

Individual tissue glucose utilization rates were also determined using a bolus injection of 100 µCi per rat of deoxy-D-glucose 2-¹⁴C (¹⁴C-2-DOG) through the femoral vein introduced 60 min before the end of the D-[3-³H]-glucose infusion. Both radioactivity contents were measured to evaluate the glucose uptake expressed as ng/mg/min. As shown in FIG. 4A-C, metformin increased glucose utilization in the mixed vastus lateralis muscle (VL, 49% p<0.05), in the glycolytic extensor digitorum longus muscle (EDL, 19% NS) and decreased glucose utilization in the heart (-39%, p<0.01), which is a known effect of metformin thought to be due to the stimulation of myocardial fatty acid oxidation. Ab14 tended to increase glucose infusion rate and whole body glucose turn over (NS) and had a stronger effect than metformin on glucose utilization in the VL (70%, p<0.01), in EDL (26%, NS), and in the oxidative soleus muscle (27%, NS). It also tended to increase glucose utilization in the heart (21%, NS). Similar to metformin, Ab14 did not affect glucose utilization rate in white adipose tissues (deep and subcutaneous).

**Ab14 Plasma Concentration Analysis.** The Ab14 plasma concentrations for Group 2 animals undergoing the clamp procedure ranged from 642 to 797 µg/mL and supported up to 48-hours of systemic exposure.

**Conclusion.** Acute treatment with Ab14 slightly decreased plasma insulin and tended to increase whole body glucose utilization by increasing glycogen synthesis and muscle glucose utilization.

### Example 2 - Reference Example

This example assesses the ability of Ab14 to improve insulin sensitivity in a rat model of insulin resistance. In this model, rats were fed with a high fat (69%) and high fructose (14%) diet (HFD) for 6 weeks to induce glucose intolerance, with the plasma insulin level becoming significantly increased and glycaemia becoming slightly increased compared to control animals fed normal chow. The antibody used in this example consisted of the light and heavy polypeptide chains of SEQ ID NOs 132 and 134.

Six-week HFD fed rats were treated for 2 weeks with Ab14, and a 2-step hyperinsulinemic euglycemic clamp was performed to assess insulin sensitivity. A physiological dose of insulin was used during the first step and a pharmacological dose of insulin was used during the second step to assess the effects of peripheral and hepatic insulin sensitivity, respectively, under these two conditions.

### Summary

After 6 weeks of HFD rats were randomized to treatment groups according to their glucose intolerance (AUC calculation during an oral glucose tolerance test (OGTT)) and their HOMA-IR (insulin resistance index). HFD rats were treated for 15 days (i.v., two administrations one week apart) with Ab14 at 10, 30 and 100 mg/kg/week, or daily with metformin 200mg/kg/day in drinking water.

Body weight and food consumption were measured 3 times per week until 10 days of treatment. HOMA-IR was measured on day 10 and 15. A 2-step clamp (5mU/kg/min and then 15mU/kg/min insulin) was performed on day 15 or 16. Glucose Turnover (GTO) was assessed using ³H-glucose tracer infusion during the clamp procedure.

At the end of the study, when compared to control rats fed normal chow, HFD rats were observed to exhibit significant increases in body weight, fasting blood glucose, plasma insulin, and C peptide, as well as a significant decrease in glucose infusion rate (GIR) during the 2-step hyperinsulinemic euglycemic clamp.

When compared to the HFD plus vehicle control group, Ab14 treatment had no effects upon body weight or food consumption while the metformin group was significantly decreased in both parameters.

Ab14 at 100 mg/kg significantly decreased HOMA-IR by 38% after 15 days of treatment (by decreasing fasting blood glucose as well as plasma insulin). Metformin had a non-significant (ns) decreasing effect on HOMA-IR on day 15. C-peptide was also significantly decreased by Ab14 treatment (by 30% with 10 mg/kg and 29% with 100 mg/kg).

Increased GIR (ns) was observed in Ab14 treated groups when compared to the HFD vehicle control group during the first step of the clamp, while metformin had an increasing effect upon GIR that was comparatively less in magnitude. Ab14 at 30 or 100 mg/kg and metformin treatment significantly increased GIR during the second step of the clamp (by 36, 28, and 27% respectively).

Ab14 at 30 mg/kg tended to increase GTO when compared to the HFD vehicle control group during the first step (by 17%, ns) of the clamp procedure. Ab14 at 30 mg/kg had a slight increasing effect (ns) upon glycolysis and glycogen synthesis during both clamp steps.

Hepatic glucose production (HGP) was slightly but not significantly decreased by Ab14 or metformin treatment during the first clamp step (between 11-20 %). During the second step, HGP was non-significantly decreased by Ab14 at 10 mg/kg (by 78%) when compared to the HFD vehicle control group, and HGP was completely inhibited by Ab14 at 30 or 100 mg/kg and by metformin.

In conclusion, improved insulin resistance (mainly hepatic) was observed following intravenous administration with Ab14 in the HFD rat model.

### Methods

82 Sprague Dawley rats (8 weeks old at start of study, average weight about 250 grams) were housed in housing cages (904 cm² × 23 cm) throughout the experimental phase. Animals' cages litters were changed 3 times per week. They were housed in groups of 2-3 animals during acclimation, HFD and treatment period. Then rats were individually housing after surgery until the clamp procedure. The rats were housed with an inverted 12 hour light cycle (at 08:00 am lights off), with temperature maintained at 22 ± 2 °C and 55 ± 10 % relative humidity. At least 5 days of acclimation period was provided before commencement of HFD feeding. During the acclimation phase, standard diet (RM1 (E) 801492, SDS) and tap water were provided ad libitum.

After the acclimation phase, 10 rats were fed with normal chow (NC) whereas 72 rats were fed with HFD (RD1, SAFE) throughout the experiment.

The high fat diet composition was as follows (Kcal%): Protein: 17.3%; Carbohydrate (fructose): 14%; Fat (lard) ): 68.7%; cholesterol 1.65%, cholic acid 0.65%.

After 6 weeks of HFD feeding, the rats were fasted for 6 hours, and a glucose tolerance test was performed. The rats presenting the lowest AUC (~17%) were excluded from the study. The remaining rats were then randomly allocated to the different groups according to their AUC (glucose tolerance index) and HOMA-IR (insulin resistance index).

Ab14 (10, 30, 100mg/kg) and the vehicle were weekly administered via i.v. route (via the caudal vein, under isoflurane anaesthesia), in the morning, on day 1 and day 8 of treatment.

Metformin (200mg/kg/day) was administered in drinking water for ~2 weeks until the clamp procedure. Rats treated with metformin were treated with vehicle on day 1 and day 8 (via the caudal vein).

The test groups were as follows:
Group 1: NC + vehicle i.v. (n=10)
Group 2: HFD + vehicle i.v. (n=10)
Group 3: HFD + Ab14 10mg/kg i.v. (n=10)
Group 4: HFD + Ab14 30mg/kg i.v. (n=10)
Group 5: HFD + Ab14 100mg/kg i.v. (n=10)
Group 6: HFD + metformin 200mg/kg in drinking water + vehicle i.v. (n=10)

During the last week of HFD feeding, before the screening, water consumption was measured 3 times in the week to evaluate the metformin dilution in tap water.

After 6 weeks of normal chow and HFD, the 82 rats were fasted from 08:00am to 02:00pm (6 hours). A glucose bolus was administered (2.5g/kg) at 02:00pm (t0). Blood glucose was measured (using glucometer, in a blood drop collected from the tail tip) on t-30, 0, 15, 30, 60, 90, 120, 150 min. Blood was collected from the tip tail (40µL on EDTA) on t-30 to measure plasma insulin (ELISA method).

Area under the curve (AUC) was calculated. The 12 HFD fed rats presenting the highest AUC were considered as the less glucose intolerant and were excluded from the study. The 60 remaining rats were randomly allocated to the 6 groups, according to homogeneous AUC, HOMA-IR, and body weight.

Body weight was measured once a week during the first six week of HFD. Body weight was measured 3 times per week during the first 10 days of treatment. Food consumption was measured over 48h or 72h, just before treatment and 3 times per week during treatment until surgery procedure (day 11).

Before the start of the treatment (the day of OGTT) and on day 10 of treatment, all rats were fasted from 08:00am. At 01:30pm, blood was collected from the from the tip tail (40µL on EDTA). Blood glucose (using glucometers) and plasma insulin (ELISA method) were measured.

On day 11, rats were anesthetized (isoflurane) and a catheter was implanted in the femoral vein. A recovery period was followed for 4 days before the clamp procedure.

The morning of the clamp, rats were fasted 6 hours (from 8:00am to 02:00pm). A sample of blood (~160 µL, on EDTA) was collected from the tip of the tail, just prior to the clamp procedure (at ~01:00pm) from each rat in group 3, 4, 5 and 6, processed to plasma (~60 µL), and maintained at -80°C until assessment of Ab14 concentrations. Although the control and metformin groups were not analyzed for antibody concentration, a similar amount of blood was collected (and discarded) from the rats of groups 1, 2 and 7.

On day 15 or 16, the 2-step hyperinsulinemic-euglycemic clamp was performed using ³H- glucose as a tracer (except in the normal chow group), and 5mU/kg/min insulin infusion from 02:00 pm (T0) to 04:00 pm (T+2h), followed by 15mU/kg/min insulin infusion from 04:00 pm to 05:30 pm (t+3.5h). A glucose solution was infused in parallel and the infusion rate was adjusted to reach the steady state (100 +/-10 mg/dL). Blood glucose was measured from the tip of the tail using glucometers every 10 minutes. Blood was collected (10µL) regularly from the tip of the tail during the steady states of each step.

The following parameters were assessed: Glucose infusion rate (in all groups); Whole body glucose utilization rate (except in the normal chow group); Hepatic glucose production rate (except in the normal chow group); Whole body glycogen and glycolytic rates (except in the normal chow group).

Moreover, except in the normal chow group, 1 hour before the end of the clamp experiment, a bolus injection of ¹⁴C-2DOG was performed and samples of the following tissues were collected at the end of the clamp and retained for further evaluation:

Vastus lateralis (VL) muscle; Extensor digitorum longus (EDL) muscle; Soleus muscle heart apex; epididymal white adipose tissue inguinal white adipose tissue skin (negative control).

Plasma insulin and C peptide levels were measured just before the infusion starts (~T-30min.), at the end of the steady state of step 1 (T2h) and step 2 (T3.5h). For that, blood collection was performed from the tip of the tail (~100µL, on EDTA).

Statistical analyses were performed using GraphPad prism software. Curves were analyzed using ANOVA two ways with a Bonferroni's post-test. Histograms were analyzed using a t-test to compare the HFD plus vehicle control group and the normal chow plus vehicle control group. Histograms were analyzed using an ANOVA one way with a Dunnett's post-test to compare Ab14 and metformin groups against the HFD plus vehicle control group. A difference was considered significant when the p-value was <0.05. NS: not significant.

### Results

**Animal model and screening.** 8-week old rats were fed with a fructose enriched high fat diet (HFD, 69%fat and 16% fructose) for 7 weeks before the start of the treatment. Body weight was 522±5g in the HFD group vs 448±13g in control group fed normal chow. There was then a 16% increase of body weight (p<0.001 with a t-test on day 42 under HFD (FIG. 5). After 7 weeks, body weight was increased by 187±3g in HFD population vs 158±9g in the control group fed normal chow (p<0.001 with a t-test on day 42, FIG. 6).

During the 7th week of HFD, an oral glucose tolerance test was performed to assess glucose intolerance in the HFD population. The blood glucose level remained higher until 150 min after the glucose administration in the HFD population (not shown). The AUC calculated relatively to the T0 was significantly higher (9%) in HFD rats compared to control chow-fed rats (not shown).

The HOMA-IR (insulin resistance index) was calculated on t-30 of OGTT. The rats presenting the higher AUC and the higher HOMA-IR, were randomly allocated to the 6 groups. AUC was higher (~9%, not shown) in HFD groups compared to the control chow-fed group, as well as the HOMA-IR (34%, not shown) and the body weight (~17%, p<0.001, FIG. 7)

**Body weight and food intake follow-up.** Body weight was followed for 10 days of treatment. Ab14 treatment had no effects upon body weight. Body weight of control chow-fed rats remained significantly lower than HFD vehicle rats (FIG. 7). Ab14 (30mg/kg) slightly decreased (ns) and Metformin 200 mg/kg significantly decreased body weight gain from the second day of treatment onward.

Food consumption was lower in HFD vehicle than in control chow-fed group as expected. Ab14 treatment had no effect on the follow-up food intake (FIG. 8A) or on cumulative food intake (FIG. 8Bnot shown). Metformin significantly decreased cumulative food consumption by 25%. The fasting of animals prior to surgical procedures disrupted the food consumption measurements between day 9 and 10.

**Biochemical parameters.** Fasting blood glucose increased in the HFD vehicle group when compared to the control chow-fed group by 5% (ns), 11% (ns), and 20% (p<0.001) on days 0, 10 and 15 respectively. Treatment with Ab14 or metformin had no effect on day 10. Treatment with Ab14 at 100 mg/kg had a significant decreasing effect on fasting blood glucose on day 15 when compared to the HFD vehicle group (FIG. 9).

Fasting plasma insulin was increased by 33% (ns), 49% (ns) and 67% (p<0.01) in HFD vehicle group when compared to the control chow-fed group on days 0, 10 and 15 respectively. Ab14 treatment had no effect on day 10 whereas metformin decreased plasma insulin by 37% (ns). Ab14 treatment at 10, 30 or 100 mg/kg decreased plasma insulin by 26, 16, or 18% (ns), respectively, after 15 days of treatment, and metformin decreased plasma insulin by 11% (ns, FIG. 10).

As expected, the plasma C-peptide level profile on day 15 was similar to the plasma insulin level, but the effects were more marked and less variable. The C-peptide was significantly increased by 67% in HFD vehicle group as compared to the control chow-fed group. Ab14 treatment at 10, 30, or 100 mg/kg decreased C-peptide level by 30 % (p<0.05), 23 % (ns), and 29% (p<0.05), respectively, and metformin decreased C-peptide by 13% (ns) (FIG. 11, lower left panel).

HOMA-IR, an index of insulin resistance, was increased in the HFD vehicle group as compared to the control chow-fed group by 36% on day 0 (ns), by 42 % on day 10 (ns) and by 98% on day 15 (p<0.01). Compared to HFD vehicle, Ab14 had no effect after 10 days of treatment whereas metformin had a decreasing effect (ns) by 36%. After 15 days of treatment, Ab14 at 10, 30 or 100 mg/kg decreased HOMA-IR by 33% (ns), 17% (ns) and 38% (p<0.05), respectively, and metformin tended to decrease HOMA-IR by 18% (ns, FIG. 12).

**Hyper-insulinemic clamp.** FIG. 13 shows the glucose infusion rate (GIR) over time during the 2-step hyper-insulinemic clamp. During the first step (5mU/kg/min insulin), hepatic glucose production (HGP) was incompletely inhibited and the glucose infusion rates (GIR) were lower than during the second step (15mU/kg/min insulin) when hepatic glucose production was inhibited.

GIR for the control chow-fed group was higher than the HFD vehicle group during both of the clamp steps, and confirmed that HFD rats had an insulin-resistant phenotype after 8-9 weeks of diet. Metformin had no effect on GIR during the first clamp step, while the GIR plateau was slightly higher (ns) in Ab14 treated groups. All treated groups were observed with GIR plateaus higher than the HFD vehicle group during the second clamp step, with significant differences observed for metformin and Ab14 at 30 or 100 mg/kg (FIG. 13). Statistical significance was evaluated using a two-way ANOVA with Bonferroni's post test versus HFD. During the first clamp step, the GIR was significantly different only for the normal chow control, vehicle treated rats at the 50 and 60 minute time points (p<0.01 and p<0.05 respectively). During the second clamp step, the GIR was significantly different for the HFD rats treated with 30 mg/kg Ab14 at the 160-210 minute time points (p<0.05 at 160 minutes and p<0.01 for the 170-210 minute time points), for the HFD rats treated with 100 mg/kg Ab14 at the 170-210 minute time points (p<0.01 at 190 minutes and p<0.05 for the 170-180 and 200-210 minute time points), and for the HFD rats treated with metformin at the 170-210 minute time points (p<0.01 at 180 and 190 minutes, and p<0.05 at the 170 and 200-210 minute time points).

The GIR means were calculated for each plateau (FIG. 14). GIR was significantly decreased in HFD vehicle group compared to control chow-fed group by 32% (p<0.05) and 17% (p<0.01) during the first and the second steps, respectively. Ab14 at 10, 30, or 100 mg/kg increased GIR (ns) during the first step (by 26, 37, and 29% respectively), and metformin had also an increasing (ns) effect by 11%. During the second step, all treatments increased GIR as compared to the HFD vehicle group: Ab14 10, 30, or 100mg/kg by 19% (ns), 36% (p<0.01), and 28% (p<0.05), respectively, and metformin by 27% (p<0.05).

Blood glucose means during the two clamp steps corresponded to a euglycemic state as expected. Although there was a significant difference between control chow-fed and the HFD vehicle group during the first clamp step, the glycaemia remained in a normal range, and the biological state was the same in both groups (FIG. 14).

Plasma insulin was measured during the clamp procedure. As expected the insulin level was similar between all groups at the end of the two clamp steps. During the first clamp step the insulin concentration was approximately 140µU/mL, and was a physiological level expected during fed conditions. The insulin concentration after the second clamp step was approximately 490µU/mL, which was a pharmacological level (FIG. 11, upper panel).

C-peptide was also measured during the clamp procedure (FIG. 11, lower right panel). During euglycemic conditions the insulin secretion by beta cells was inhibited, and the plasma C-peptide levels were therefore low and not interpretable.

³H-glucose was infused with insulin during the clamp procedure in all HFD groups (not in the control chow-fed group). The whole body glucose fluxes were then calculated. During the first clamp step, the glucose turn over (GTO) was similar in all groups, excluding that Ab14 at 30 mg/kg tended to increase GTO as compared to the HFD vehicle group (17%, ns). Glycolysis and glycogen synthesis tended to increase, 15 and 16%, respectively (ns, FIG. 15), following treatment with Ab14 at 30 mg/kg.

During the second clamp step, GTO, glycolysis, and glycogen synthesis were similar in all treated groups, with a slight increase of glycogen synthesis observed in the Ab14 30mg/kg treated group when compared to HFD vehicle group (by 10%, ns). Ab14 at 30 mg/kg (p<0.05) and 100mg/kg (ns) completely inhibited HGP, as did metformin (ns), and Ab14 treatment at10mg/kg decreased HGP by 78% (ns, FIG. 16).

**Conclusion.** Treatment with Ab14 decreased HOMA-IR by decreasing fasting blood glucose as well as plasma insulin levels in the HFD rat model. Furthermore, liver insulin sensitivity was markedly improved by Ab14, whereas an effect on whole body peripheral insulin sensitivity was not clearly observed. As expected, metformin treatment also improved liver insulin sensitivity.

### Example 3 - Reference Example

This example assesses the effect of Ab14 on glucose metabolism and on glycemic control in a model a rat model of diabetes, the Zucker diabetic fatty (ZDF) rat. The effects of chronic administration of Ab14 on glucose control was evaluated in ZDF rats that were progressing from a prediabetic (hyperinsulinemic, normoglycemic) state to an overtly diabetic (hypoinsulinemic, hyperglycemic) state. These animals develop prediabetes, characterized by marked hyperinsulinemia to compensate for their developing insulin resistance, but with little to no hyperglycemia, by seven weeks of age. This rapidly progresses to overt diabetes, characterized by hypoinsulinemia, as a result of pancreatic beta cell failure, and marked hyperglycemia by 10-12 weeks of age. The antibody used in this example consisted of the light and heavy polypeptide chains of SEQ ID NOs 132 and 134.

### Methods

81 ZDF fa/fa rats (Charles River Laboratories, France) and 10 lean ZDF ?/+ rats (controls) were housed in ventilated and enriched housing cages in groups of 1-2 animals on a normal 12hours light cycle (at 08:00 pm lights off), 22 ± 2 °C and 50 ± 10 % relative humidity. The rats were 7 weeks of age at delivery and were acclimated for one week prior to study commencement. The rats were fed the standard diet for ZDF rats (Purina 5008, Charles River) and tap water were provided *ad libitum.* All animals were monitored at least once daily for any signs of ill health, adverse reactions to treatment, or morbidity throughout the study.

Eight week old male ZDF fa/fa rats were hyperinsulinemic and mildly diabetic. Due to the variability of the blood glucose and insulin levels at this state, the ZDF rats were screened and selected according to their HOMA-IR.

For the groups treated with AB14, the antibody was administered once weekly via the caudal vein (i.v., 5mL/kg) on days 1, 8, 15, and 22 at two different doses 20 mg/kg/week (groups 3 and 7) or 60 mg/kg/week (groups 4 and 8). All other groups were treated once weekly with vehicle 1 (i.v., 5mL/kg). The intravenous treatments were performed in the morning on day 1, 8, 15, and 22 while under isoflurane anaesthesia. The volume of administration was individually adapted according to the most recent body weight.

Metformin (Met) and pioglitazone (PIO) were administered once daily for 28 days, via per os route (p.o., 5mL/kg) between 8:00 and 10:00 am, except that on the day of OGTT or after intravenous treatments, some per os treatments were completed after 10:00am. Group 5, 7, and 8 were treated with 200mg/kg/day metformin, and group 6 was treated with 10mg/kg/day pioglitazone. All other groups were treated daily with vehicle 2 (p.o., 5mL/kg) for 28 days. The most recent body weight was used to calculate the average volume of administration in each group.

The test groups are summarized in the following Table. Met: metformin.

| **Groups** | **n** | **Treatment** | **dose** | **route** | **frequency** |
|---|---|---|---|---|---|
| Group 1 | 10 | Vehicle | - | i.v. | Day 1, 8, 15, 22 |
| (Lean Zucker rats) | | Vehicle | - | p.o. | Daily |
| Group 2 (ZDF rats) | 10 | Vehicle | - | i.v. | Day 1, 8, 15, 22 |
| | | Vehicle | - | p.o. | Daily |
| Group 3 (ZDF rats) | 10 | AB14 | 20 mg/kg/week | i.v. | Day 1, 8, 15, 22 |
| | | Vehicle | - | p.o. | Daily |
| Group 4 (ZDF rats) | 10 | AB14 | 60 mg/kg/week - | i.v. | Day 1, 8, 15, 22 |
| | | Vehicle | | p.o. | Daily |
| Group 5 (ZDF rats) | 10 | Vehicle | - | i.v. | Day 1, 8, 15, 22 |
| | | Met | 200mg/kg/day | p.o. | Daily |
| Group 6 (ZDF rats) | 10 | Vehicle | - | i.v. | Day 1, 8, 15, 22 |
| | | PIO | 10mg/kg/day | p.o. | Daily |
| Group 7 (ZDF rats) | 10 | AB14 | 20 mg/kg/week | i.v. | Day 1, 8, 15, 22 |
| | | Met | 200mg/kg/day | p.o. | Daily |
| Group 8 (ZDF rats) | 10 | AB14 | 60 mg/kg/week | i.v. | Day 1, 8, 15, 22 |
| | | Met | 200 mg/kg/day | p.o. | Daily |

After 1 week of acclimation period, all rats were weighted and fasted for 6 hours (from ∼8:00 am to ∼2:00 pm). At ∼2:00 pm, blood was collected (∼150µL, EDTA) from the tip of the tail. Blood glucose (glucometer) and plasma insulin (ELISA) were measured, and the HOMA-IR (insulin resistance index) was calculated. The 11 ZDF fa/fa rats presenting extreme HOMA-IR values were excluded from the study but were kept housed for 28 days for plasma collection at the end of the study. Then the 70 remaining rats were randomly allocated to 7 treatment groups according to their HOMA-IR and body weight. The lean rats were all kept in group 1 and treated with vehicle only.

Body weight was measured twice a week during the 4 weeks of treatment.

Food consumption was measured just before the screening procedure and then twice weekly over 24h during the first three weeks of treatment. Food consumption was measured once weekly during the week of OGTT (week 4).

A fasting (6 hours on day 0 from 8:00 am to 02:00 pm, and overnight on day 12, 19 and 26 from ~6:00 pm to ∼8:00 am) was performed before each blood collection. Blood was collected at - 2:00 pm, from the tip of the tail, on day 0 (before screening, 150µL, potassium EDTA), and at ∼8:00 am prior to dosing on days 12 (110µL, potassium EDTA), 19 (150µL, potassium EDTA) and 26, (110µL, potassium EDTA).

Fasting blood glucose (glucometers) was measured on days 0, 12, 19, and 26. Fasting plasma insulin, peptide-C (ELISA method), free fatty acids, triglycerides, total cholesterol (colorimetric method), and HDL-cholesterol (phosphotungstate precipitation, colorimetric method) were measured on day 0, and prior to dosing on days 12, 19, and day 26. Non HDL-cholesterol was calculated as total cholesterol - HDL-cholesterol. Fructosamine was measured on days 0, 19 and 28. HbAlc (DCA 2000) was measured on days 0 and 28.

The oral glucose tolerance test (OGTT) was performed as follows. On day 25, rats were fasted at ∼6:00 pm and an oral glucose tolerance test was performed the day after (on day 26). At ∼8:00 am (T-60) a blood collection (110µL, EDTA) was performed for biochemical parameters measurements. One hour after (∼09:00 am), an oral glucose bolus (1.5g/kg) was administered (T0). Blood glucose was measured (glucometer or colorimetric method in case of high glycaemia) on T-60, T0, T15, T30, T60, T90, T120, and T180 minutes. Area under the curve (AUC) was calculated based on the blood glucose values measured at T0. Plasma insulin and C-peptide were measured (ELISA method) on T-60, T15 (~40µL of blood, EDTA), and T30 minutes (~40µL of blood, EDTA).

After 2 hours of food restriction (from 8:00 am to 10:00 am) 80 rats from the study were anesthetized on day 28 with isoflurane. Blood was collected (~3000 µL from abdominal vein, on K2-EDTA) for the determination of AB14 plasma concentrations. Plasma (3 aliquots of ~200µL) was kept at -80°C until testing. The pancreas tissue was then excised. Rats were euthanized by incision of the abdominal vein and aorta.

Each pancreas was divided into 2 parts (longitudinal cut). One piece was fixed in 10% formalin solution for histopathological processing. The other piece of pancreas was flash frozen and kept at -80°C for determination of insulin and proinsulin levels.

The 11 ZDF fa/fa rats excluded from the study were sacrificed after 28 housing days. They were anesthetized with isoflurane. Blood was collected from abdominal vein (maximum volume on potassium EDTA). Plasma samples (2 aliquots of 1 mL each) were frozen for further testing. Rats were euthanized by incision of the abdominal vein and aorta.

Each pancreas sample was homogenized in an acid buffer, and insulin and proinsulin content were measured using ELISA kits in the following groups:
Group 1: Lean rats + vehicles (n=10)
Group 2: ZDF rats + vehicles (n=10)
Group 4: ZDF rats + AB14 60mg/kg/week (n=10)
Group 5: ZDF rats + metformin 200mg/kg/day (n=10)
Group 8: ZDF rats + AB14 60mg/kg/week + metformin 200mg/kg/day (n=10)

Each pancreas sample was fixed in 4% formalin during 24-48 hours maximum; the volume of formalin was 5-10 times higher than the sample volume to assure appropriate fixation. After 48h, the samples were placed in 70% ethanol. Samples were then included in paraffin for histological process in the following groups:
Group 1: Lean rats + vehicles (n=10)
Group 2: ZDF rats + vehicles (n=10)
Group 4: ZDF rats + AB14 60mg/kg/week (n=10)
Group 5: ZDF rats + metformin 200mg/kg/day (n=10)
Group 8: ZDF rats + AB14 60mg/kg/week + metformin 200mg/kg/day (n=10)

After delineating the islets of Langerhans, surface and intensity of the insulin labelling were quantified by image analysis of the labeled (brown) and non-labeled (blue) areas.

The means of vehicle ZDF rats and lean rats were compared using a student test when the Fisher test did not show significant differences in variances. If not, the non-parametric Mann Whitney test was used.

The means of the treated ZDF rats were compared to the vehicle ZDF rats using a 1-way ANOVA + Dunnett's post-test. If the Bartlett's test showed significant differences in variances, the non-parametric Kruskall Wallis + Dunn's post-test was used.

The means of AB14 20mg/kg alone was compared to metformin 200mg/kg alone or in combination with AB14 20mg/kg using a 1-way ANOVA + Newman-Keuls post-test.

The means of AB14 60mg/kg alone was compared to metformin 200mg/kg alone or in combination with AB14 60mg/kg using a 1-way ANOVA + Newman-Keuls post-test.

The curves were analyzed using a 2-way ANOVA + Bonferroni's post-test.

Rats were excluded from analysis if they were an outlier in all or almost all parameters. This resulted in exclusion of four rats, each from a different group.

### Results

As expected in 8-week old ZDF rats, HOMA-IR was strongly increased as compared with lean rats (∼111 vs 3.5, FIG. 18A). ZDF rats were mildly hyperglycemic (∼180 vs 113 mg/dL, FIG. 18B) and hyperinsulinemic (∼250 vs 12.6 µU/mL, FIG. 18C). Body weight was slightly increased in ZDF rats (FIG. 18D).

Compared with lean rats, the body weights of ZDF rats remained higher over the entire treatment period (FIG. 19A), while body weight gain was similar between lean and ZDF rats (FIG. 19B).

Pioglitazone significantly increased body weight compared to the ZDF vehicle rats, from 8 days of treatment and body weight gain was 3-fold higher at the end of the treatment (FIG. 19A and B). All other drug treatments had no significant effect on body weight compared to vehicle ZDF rats. AB14 60mg/kg + metformin 200mg/kg combination significantly increased body weight gain from 22 days of treatment (FIG. 19B).

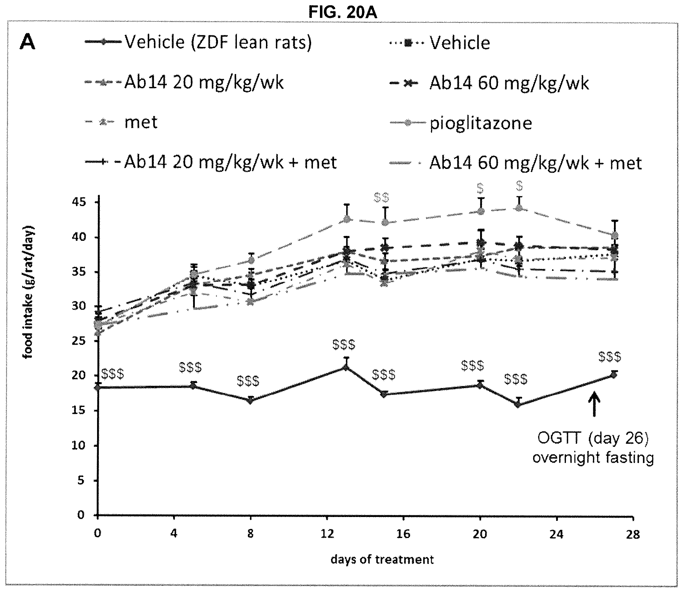

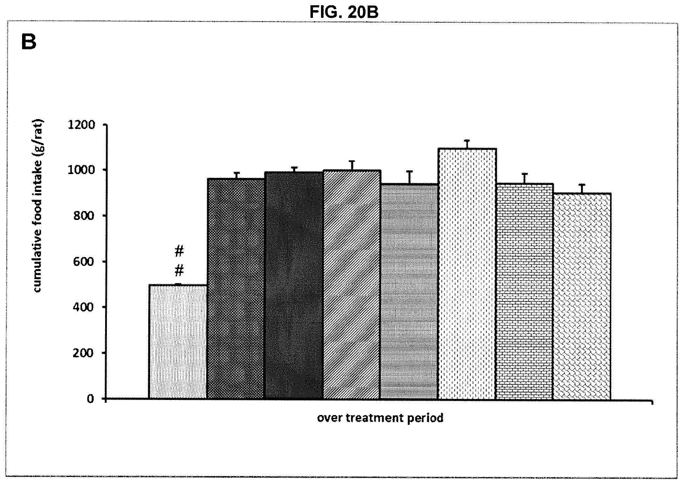

Food intake was ~2-fold increased in vehicle ZDF rats compared to the lean rats (significant on day 13). Rats treated with pioglitazone showed a trend to higher food consumption as compared with ZDF vehicle rats (significant on day 15, 20 and 22, FIG. 20A). Cumulative food intake was increased by 94% in the vehicle ZDF group as compared with the lean group (p<0.01), and by 14% in pioglitazone group as compared with the vehicle ZDF group (NS, FIG. 20B). Other treatments had no effect on food intake as compared with vehicle-treated ZDF rats.

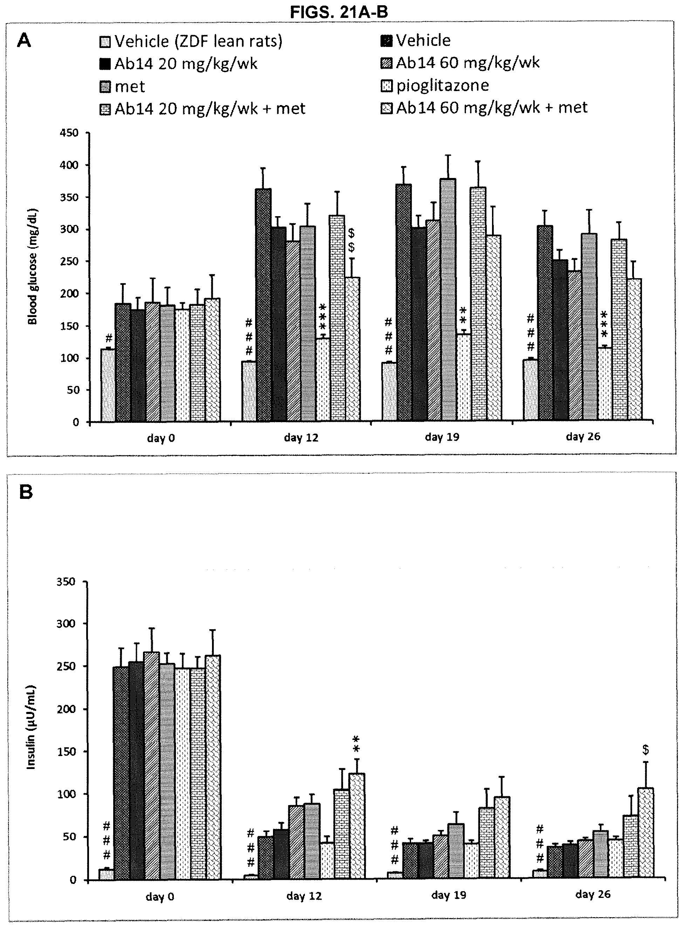

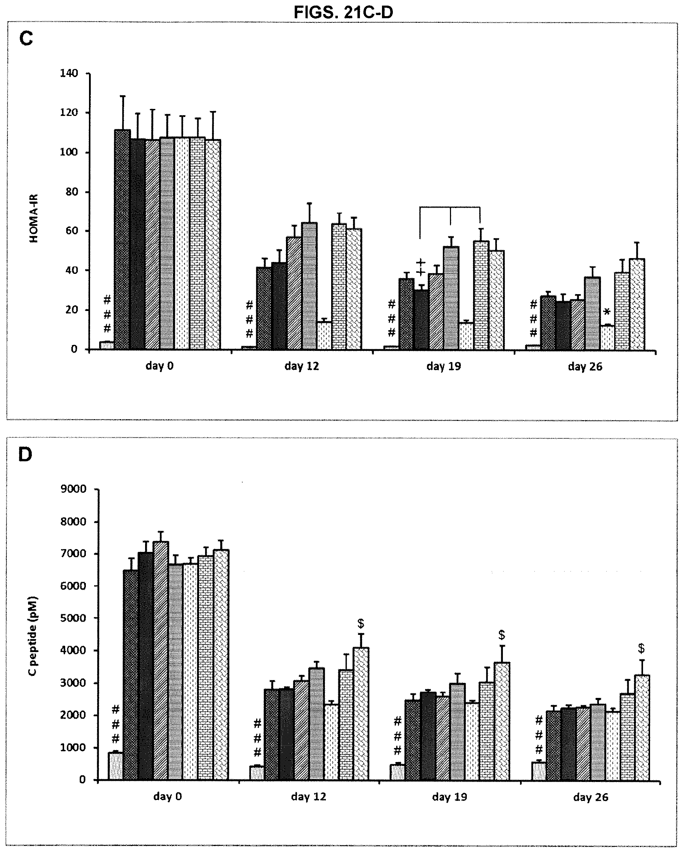

Fasting blood glucose remained in a normal range during the 26 days of treatment in lean rats, either after 6 hours or one night of fasting (FIG. 21A). This was correlated with normal insulin levels (FIG. 21B). In vehicle ZDF rats, overnight fasting blood glucose reached 362 ± 32 mg/dL on day 12 (at about 10 weeks of age) and remained significantly higher than lean rats until the end of the treatment (FIG. 21A). This was correlated with decreasing plasma insulin levels (49.2 ± 6.7, 41.2 ± 4.8, and 36.6 ± 2.7 µU/mL - p<0.001 vs. lean rats, FIG. 21B) and with decreasing plasma C-peptide levels (2813 ± 249, 2472 ± 195, 2156 ± 165 pM - <p<0.001 vs lean rats (FIG. 21D) measured on days 12, 19, and 26. The evolution of the HOMA-IR in lean and vehicle ZDF rats reflected the change in blood glucose and plasma insulin levels (FIG. 21C).

Pioglitazone significantly decreased overnight fasting blood glucose levels to a normal level from 12 days of treatment (p<0.001, FIG. 21A). At both doses AB14 decreased by about 15% the blood glucose after 12, 19 or 26 days of treatment (n.s, FIG. 21A). Compared to AB14, metformin 200mg/kg had similar effect on day 12, but this effect was not observed at day 19 and 26. Compared with ZDF rats treated with vehicle, AB14 20 mg/kg + metformin combination slightly reduced blood glucose on day 12 (12%, ns), and showed no effect on day 19 and day 26 (FIG. 21A). In contrast, the AB14 60 mg/kg combination with metformin significantly reduced blood glucose levels day 12 (38%, p<0.01 vs vehicle treated ZDF rats). Although not statistically significant, the blood reduction was still observed on day 19 and 26 (22% and 27% respectively, FIG. 21A).

Pioglitazone seemed to have no protective effect on insulin secretion as it showed no effect on plasma insulin and C-peptide levels on days 12, 19 and 26 compared to vehicle ZDF rats (FIGs. 21B and D). Hence the reduction in blood glucose levels was related to the insulin sensitizing effect of pioglitazone, which reduced HOMA-IR by 67%, 62% and 54%, on days 12, 19 and 26 respectively, as compared with vehicle ZDF rats (FIG. 21C).

Compared with vehicle-treated ZDF rats, AB14 20mg/kg did not change plasma insulin and C-peptide levels, as well as HOMA-IR on days 12, 19 and 26 (FIG. 21B-D). Meanwhile, AB14 60mg/kg increased plasma insulin levels on days 12, 19 and 26 by 74%, 21% and 19%, respectively (ns vs. vehicle ZDF rats, FIG. 21B).

AB14 60mg/kg increased plasma C-peptide levels on day 12 by 10% (ns), and had no effect on days 19 and 26 (FIG. 21D).

Compared with vehicle-treated ZDF rats, metformin increased plasma insulin levels on days 12, 19 and 26 by 79%, 55% and 48%, respectively (ns, FIG. 21B). Metformin increased plasma C-peptide levels on days 12, 19 and 26 by 23%, 21%, and 9%, (NS vs ZDF rats treated with vehicle, FIG. 21D).

Compared with vehicle-treated ZDF rats, AB14 20mg/kg + metformin combination increased plasma insulin levels on days 12, 19 and 26 by 2-fold (NS, FIG. 21B). AB14 20mg/kg + metformin combination increased plasma C-peptide levels on days 12, 19 and 26 by 21%, 23% and 25%, respectively (NS, FIG. 21D).

Compared with vehicle-treated ZDF rats, AB14 60mg/kg + metformin combination significantly increased plasma insulin levels on days 12, 19 and 26 by a factor 2.5, 2.3 and 2.7 respectively. AB14 60mg/kg + metformin combination significantly increased plasma C-peptide levels from day 12 by 45% (day 12), 48% (day 19), and 52% (day 26) (p<0.05 vs vehicle-treated ZDF rats, FIG. 21D).

In this model where insulin secretion was reduced over time, the increase in HOMA-IR was reflecting an improvement of insulin secretion. Thus an increase of HOMA-IR was observed in metformin alone or in combination with the AB14 treated groups, as compared with vehicle ZDF groups, and this increase was maintained on days 12, 19 and 26 (FIG. 21C).

Compared with lean rats, fructosamine was significantly higher (66%) in 8-week old vehicle-treated ZDF rats (208 ± 6 vs 144 ± 2 µM, p<0.001). Fructosamine levels remained in a similar range in lean rats during the treatment period, but increased in vehicle ZDF rats after 19 (253 ± 5 µM, p<0.001) and 28 (234 ± 6 µM, p<0.001) days of treatment (FIG. 22). As expected, pioglitazone significantly reduced fructosamine levels from day 19 (30% on day 19 and 25% on day 28, p<0.001). AB14 20 and 60mg/kg had no effect on fructosamine levels. Compared with vehicle-treated rats, metformin showed a trend towards lower fructosamine levels only on day 19 (6%, ns). Compared with vehicle-treated ZDF rats, AB14 20mg/kg + metformin combination showed a non significant trend towards lower fructosamine levels on days 19 and 28 by 10% and 8%, respectively. As well the AB14 60mg/kg + metformin combination showed a non significant trend towards lower fructosamine levels (9%) on day 28 (FIG. 22).

Compared with lean rats, HbAlc was higher in 8-week old ZDF rats (4.3 ± 0.1% vs3.1 ± 0.04%), although these values were in a normal range.

In 12-week old ZDF rats, HbAlc reached a pathological value of 8.8 ± 0.2% on day 28, (p<0.001 ZDF vs lean rats, FIG. 23). Compared with vehicle-treated ZDF rats, AB14 20 and 60mg/kg had no effect on HbA1c levels after 28 days of treatment. After 28 days of treatment, pioglitazone and metformin significantly decreased HbAlc by 44 and 15%, respectively (FIG. 23). The combination of metformin with AB14 20mg/kg and with 60mg/kg significantly reduced HbAlc by 11 and 19% respectively (FIG. 23).

Compared with lean rats, plasma triglycerides levels were strongly increased in 8-week old ZDF rats (~8 mM vs ~0.7 mM, FIG. 24A).

Compared with vehicle, pioglitazone strongly decreased plasma triglycerides levels from the 12th day of treatment. AB14 20 mg/kg slightly decreased plasma triglycerides levels on days 12 and 19 (by 15% and 7% respectively, ns), and had no effect on day 26. AB14 60mg/kg slightly decreased plasma triglycerides levels on days 12, 19 and 26 by 14%, 9% and 12%, respectively (ns). Metformin increased plasma triglycerides levels on days 12, 19 and 26 by 26%, 40%, and 49%, respectively (significant from day 19). The metformin + AB14 20 mg/kg combination showed a trend towards higher plasma triglycerides on days 19 and 26 (by 13% and 23%, respectively, ns) as compared with the vehicle ZDF group. Metformin + AB14 60 mg/kg combination showed a trend towards higher plasma triglycerides on days 12, 19 and 26 (by 9%, 48% and 43% respectively, significant from day 19, FIG. 24A).

After 6 hours of fasting, plasma free fatty acids levels were higher in 8-week old ZDF rats than in lean rats (~0.85mM vs. ~0.59mM). After an overnight fasting (maximal lipolytic conditions), free fatty acids levels were similar (∼1.3mM) at 10 and 11 weeks. At 12 weeks of treatment, the lipolytic capacity of ZDF rats was decreased, as shown by lower free fatty acids levels, as compared with lean rats (1.05 ± 0.06 vs 1.38 ± 0.03mM, FIG. 24B). Compared to vehicle ZDF rats, rats treated with pioglitazone showed lower plasma free fatty acids levels by 35% on day 12 (p<0.001), 17% on day 19 (ns) and 30% on day 26 (p<0.05). AB14 20 and 60mg/kg had no effect. Metformin increased free fatty acids levels by 14%, 25% and 8% on days 12, 19 and 26 but not significantly when compared to vehicle ZDF rats. The effect of metformin alone or in combination with AB14 20mg/kg was similar. On the other hand, when combined with AB14 60mg/kg, metformin showed an increasing effect only on day 19 when compared with the vehicle ZDF group (by 20%, NS, FIG. 24B).

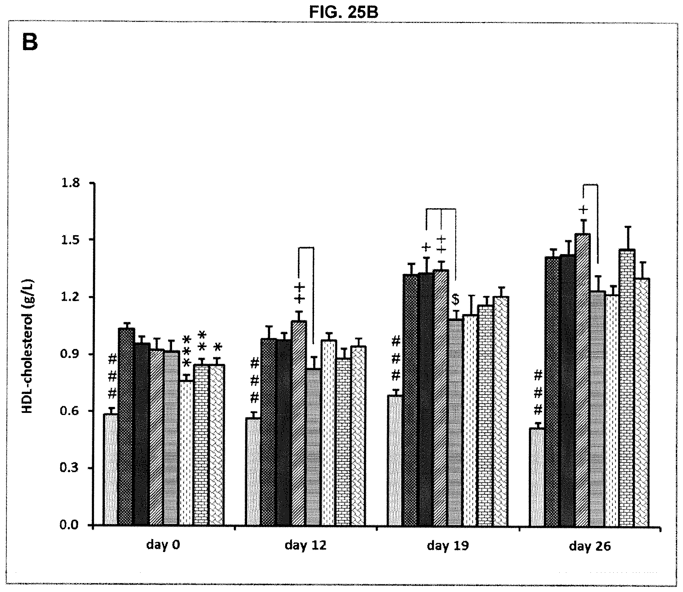

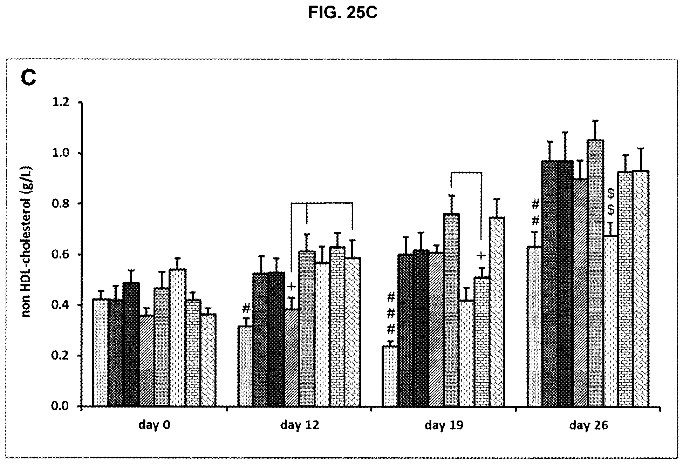

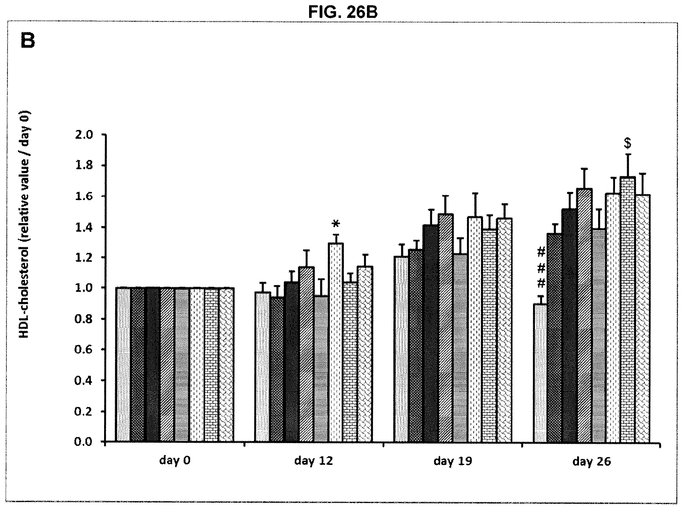

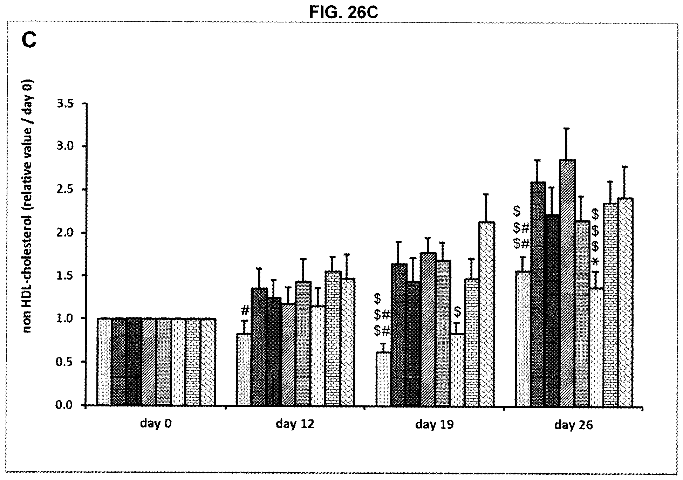

Compared with lean rats, plasma total cholesterol and HDL-cholesterol levels were higher in 8-week old ZDF rats and gradually increased over the 4 following weeks (FIG. 25A-B, FIG. 26A-B). Plasma non-HDL cholesterol levels were similar in lean and ZDF rats at 8 weeks of age, but increased over time in ZDF rats vs lean rats from 10 weeks (FIG. 25C and FIG. 26C). As there was significant difference in total cholesterol and HDL-cholesterol between the ZDF groups at day 0 (FIG. 25), the results were expressed in relative values from day 0 (FIG. 26). As shown in FIG. 26A, pioglitazone tended to prevent the increase in plasma total cholesterol levels overtime. AB14 20 mg/kg and metformin had no effect. AB14 60 mg/kg increased total cholesterol by 8%, 14%, and 15% on days 12, 19, and 26 respectively compared to the vehicle ZDF group. When combined with metformin, AB14 20 mg/kg increased total cholesterol by 15% and 10% on days 12 and 26 respectively, whereas AB14 60 mg/kg increased total cholesterol by 24%, 21% and 13% on days 12, 19 and 26 respectively compared to the vehicle ZDF group. Compared with the vehicle, pioglitazone increased plasma HDL-cholesterol by 38%, 17% and 19% on days 12, 19 and 26 respectively. Metformin alone had no effect. AB14 20 mg/kg and 60 mg/kg, alone or in combination with metformin increased plasma HDL-cholesterol levels by 11 to 22% after 12, 19 and 26 days of treatment. Plasma non HDL-cholesterol levels (FIG. 26C) were similar in all ZDF groups after 12 days of treatment. Compared with vehicle, AB14 20mg/kg, AB14 60 mg/kg, metformin, alone or in combination with AB14 had no effect on non HDL-cholesterol levels. Only pioglitazone significantly decreased plasma non HDL-cholesterol levels by 49% and 47% on day 19 and day 26, respectively.

An oral glucose tolerance test was performed after 26 days of treatment. As compared with vehicle-treated lean rats, blood glucose levels were expectedly higher in vehicle-treated ZDF rats before and after glucose load (FIG. 27A). Compared with vehicle-treated ZDF rats, only pioglitazone-treated ZDF rats showed significantly reduced blood glucose levels at all time points. Compared with vehicle, AB14 60 mg/kg and metformin combination tended to reduced blood glucose levels at t-60 minutes (FIG. 27A), while other drug treatments showed no significant effect. Compared with lean rats, blood glucose area under the curve (AUC) was significantly increased by 3.7-fold in vehicle-treated ZDF rats. Compared with vehicle-treated ZDF rats, rats treated with pioglitazone showed a significant 54% reduction in blood glucose AUC. AB14 20mg/kg, AB14 60 mg/kg and metformin alone or in combination with 20 mg/kg or 60 mg/kg AB14 showed a non significant reduction on AUC (7%, 11%, 6%, 7% and 17%, respectively). The AB14 60 mg/kg + metformin combination was slightly more effective in reducing AUC when compared with AB14 or metformin alone (FIG. 27B).

Plasma insulin and C-peptide levels were measured at 15 and 30 minutes after the glucose load. The concentration versus time profiles were similar for both insulin and C-peptide. Insulin and C-peptide levels were similar between the vehicle, AB14 20 mg/kg and AB14 60 mg/kg treated groups, slightly increased in the metformin and pioglitazone treated groups, and more increased in a dose-dependent manner in groups treated with AB14 20 mg/kg and AB14 60 mg/kg combined with metformin (FIG. 28A-B). The capacity of insulin or C-peptide secretion in response to the glucose charge was evaluated by expressing the results in relative values calculated from T-60 minutes. As expected, vehicle ZDF rats had significantly lost their capacity to secrete insulin and C-peptide in response to the glucose charge as compared with lean rats (FIG. 29A-B). When compared with vehicle-treated ZDF rats, rats treated with pioglitazone had increased insulin secretion by 20% (p<0.05) and 5% at time T15 and T30 respectively. All other treatments had no effect on insulin secretion at time T15. Metformin decreased insulin secretion at time T30 by 26% (p<0.01). AB14 20 mg/kg alone had no effect at time T30, and showed a trend to decrease insulin secretion (by 14%, ns) in combination with metformin. AB14 60 mg/kg alone or combined with metformin showed a trend to decrease insulin secretion by 19% and 18%, respectively (FIG. 29A). As compared to lean rats, C-peptide secretion in response to glucose load (FIG. 29B) was significantly reduced in vehicle-treated ZDF rats by ~40% at time T15 and T30. When compared to vehicle-treated ZDF rats, only pioglitazone significantly increased C-peptide secretion at time T15 and T30 by 21% and 22% respectively.

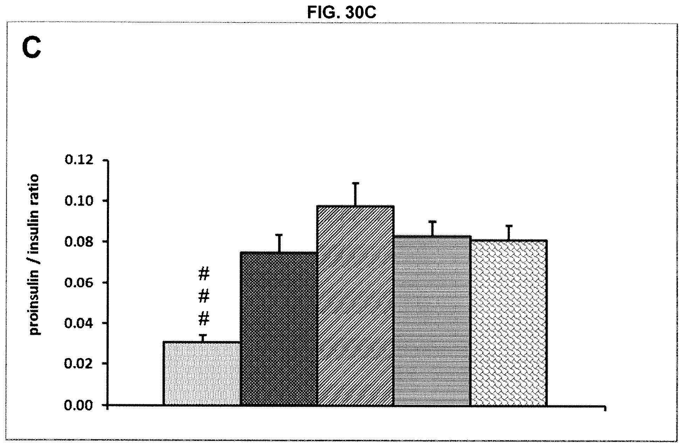

As expected, pancreas proinsulin (FIG. 30A) and insulin (FIG. 30B) levels were significantly lower in 12-week old ZDF rats when compared with lean rats. AB14 60 mg/kg and metformin completely prevented reductions in proinsulin and AB14 60 mg/kg combined with metformin significantly increased proinsulin levels (p<0.05 vs. vehicle) (FIG. 30A). AB14 60 mg/kg slightly increased pancreatic insulin levels, and metformin or AB14 60 mg/kg combined with metformin significantly increased insulin levels (p<0.05 vs. vehicle) (FIG. 30B). Compared with lean rats proinsulin/insulin ratio was significantly increased in ZDF rats, while no change was observed with drug treatments (FIG. 30C).

No evidence for toxicity was reported in this study focusing on microscopic changes in pancreas of lean or ZDF rats treated with either vehicle, AB14, metformin or AB14/metformin combination.

A higher incidence and severity of focal to multifocal large / giant islet(s) - corresponding to islet hyperplasia - and islet fibrosis were noted in ZDF rats given vehicle control (Table 1 and Table 2).

**Table 1. Incidence of Histophathological Observations. All treatment groups were ZDF rats except where indicated otherwise. Met.: metformin 200 mg/kg/day.**

| Observation | Severity | Vehicle only (ZDF Lean rats) | Vehicle only | Ab14 60 mg/kg/wk. | Met. | Ab14 60 mg/kg/wk. + Met. |
|---|---|---|---|---|---|---|
| Islet Fibrosis | 0 | 6 | - | - | - | - |
| | 1 | 2 | - | 4 | 3 | 7 |
| | 2 | 2 | 2 | 3 | 7 | 1 |
| | 3 | - | 7 | 2 | - | 1 |
| Brown pigment-laden | 0 | 4 | - | - | - | - |
| | 1 | 4 | 6 | 7 | 7 | 8 |
| | 2 | 2 | 3 | 2 | 3 | 1 |
| | 3 | - | - | - | - | - |
| Mononuclear cell infiltration | 0 | 4 | - | - | - | - |
| | 1 | 5 | 7 | 9 | 10 | 8 |
| | 2 | 1 | 2 | - | - | 1 |
| | 3 | - | - | - | - | - |
| Islet Cell Vacuolation | 0 | 10 | - | - | 1 | 1 |
| | 1 | - | 1 | 6 | 4 | 7 |
| | 2 | - | 8 | 3 | 5 | 1 |
| | 3 | - | - | - | - | - |
| Islet cell single cell necrosis | 0 | 9 | 3 | 5 | 7 | 3 |
| | 1 | 1 | 6 | 4 | 3 | 6 |
| | 2 | - | - | - | - | - |
| | 3 | - | - | - | - | - |
| Islet cell anisocytosis / anisokaryosis | 0 | 10 | 1 | 5 | 2 | 1 |
| | 1 | - | 8 | 4 | 8 | 8 |
| | 2 | - | - | - | - | - |
| | 3 | - | - | - | - | - |
| Islet granulocytic infiltration | 0 | 8 | 6 | 5 | 6 | 7 |
| | 1 | 2 | 3 | 4 | 4 | 2 |
| | 2 | - | - | - | - | - |
| | 3 | - | - | - | - | - |
| Islet hemorrhage | 0 | 8 | 7 | 7 | 8 | 7 |
| | 1 | 2 | 2 | 2 | 2 | 2 |
| | 2 | - | - | - | - | - |
| | 3 | - | - | - | - | - |
| Islet cell mitosis | 0 | 9 | 6 | 7 | 5 | 8 |
| | 1 | 1 | 3 | 2 | 5 | 1 |
| | 2 | - | - | - | - | - |
| | 3 | - | - | - | - | - |
| Interstitial hemorrhage | 0 | 2 | 2 | 3 | 4 | 2 |
| | 1 | 7 | 5 | 4 | 6 | 5 |
| | 2 | 1 | 2 | 2 | - | 2 |
| | 3 | - | - | - | - | - |
| Interstitial fibroplasia/ fibrosis | 0 | 5 | 5 | 8 | 9 | 8 |
| | 1 | 5 | 3 | 1 | 1 | 1 |
| | 2 | - | 1 | - | - | - |
| | 3 | - | - | - | - | - |
| Arteries, medial / intimal hypertrophy | 0 | 10 | 6 | 6 | 9 | 7 |
| | 1 | - | 2 | 3 | 1 | 2 |
| | 2 | - | 1 | - | - | - |
| | 3 | - | - | - | - | - |
| Pancreatic ductule hyperplasia | 0 | 9 | 3 | 9 | 5 | 8 |
| | 1 | 1 | 6 | - | 5 | 1 |
| | 2 | - | - | - | - | - |
| | 3 | - | - | - | - | - |
| Exocrine cell vacuolation | 0 | 8 | 8 | 8 | 10 | 7 |
| | 1 | 2 | 1 | 1 | - | 2 |
| | 2 | - | - | - | - | - |
| | 3 | - | - | - | - | - |
| Eosinophilic granulocyte infiltration | 0 | 9 | 6 | 8 | 10 | 5 |
| | 1 | 1 | 3 | 1 | - | 3 |
| | 2 | - | - | - | - | 1 |
| | 3 | - | - | - | - | - |
| Large/Giant Islets | 0 | 5 | 1 | - | 1 | - |
| | 1 | 5 | 2 | 4 | 1 | 1 |
| | 2 | - | 5 | 3 | 3 | 5 |
| | 3 | - | 1 | 2 | 5 | 3 |

**Table 2. Histopathological analysis: group mean scores of elementary findings. All treatment groups were ZDF rats except where indicated otherwise. Met.: metformin 200 mg/kg/day.**

| **Organ** | **Findings** | **Vehicle only (ZDF Lean rats)** | **Vehicle only** | **Ab14 60 mg/kg/wk.** | **Met.** | **Ab14 60 mg/kg/wk. + Met.** |
|---|---|---|---|---|---|---|
| **Fat tissue** | Fat tissue necrosis | 0 | 0 | 0 | 0 | 0 |
| **Lymph node** | Brown pigment-laden macrophages | 0 | 1 | 0 | 0 | 1 |
| | Eosinophilic granulocyte infiltration | 1 | 1 | 0.5 | 0.5 | 0 |
| | Erythrophagocytosi s - sinusoidal | 1 | 1.5 | 1 | 0 | 1 |
| | Pink protein rich sinusoidal lymph | 0 | 0 | 0 | 0 | 0 |
| | Sinusoidal hemorrhage | 1 | 0.5 | 0 | 0 | 1 |
| | Sinusoidal mast cells | 0 | 0 | 0 | 0 | 0 |
| **Pancreas** | Arteries, medial / intimal hypertrophy | 0 | 0 | 0 | 0 | 0 |
| | Brown pigment-laden macrophages | 1 | 1 | 1 | 1 | 1 |
| | Eosinophilic granulocyte infiltration | 0 | 0 | 0 | 0 | 0.5 |
| | Exocrine cell vacuolation | 0 | 0 | 0 | 0 | 0 |
| | Interstitial fibroplasia / fibrosis | 0.5 | 0 | 0 | 0 | 0 |
| | Interstitial hemorrhage | 1 | 1 | 1 | 1 | 1 |
| | Islet cell anisocytosis / anisokaryosis | 0 | 1 | 0.5 | 1 | 1 |
| | Islet cell mitosis | 0 | 0 | 0 | 0.5 | 0 |
| | Islet cell single cell necrosis | 0 | 1 | 0.5 | 0 | 1 |
| | Islet cell vacuolation | 0 | 2 | 1 | 1.5 | 1 |
| | Islet fibrosis | 0 | 3 | 2 | 2 | 1 |
| | Islet granulocytic infiltration | 0 | 0 | 0 | 0 | 0 |
| | Islet hemorrhage | 0 | 0 | 0 | 0 | 0 |
| | Large / giant islet(s) | 0.5 | 2 | 1.5 | 2.5 | 2 |
| | Mononuclear cell infiltration | 1 | 1 | 1 | 1 | 1 |
| | Pancreatic ductule hyperplasia | 0 | 1 | 0 | 0.5 | 0 |

Compared with lean rats, vehicle treated ZDF rats had slight islet cell vacuolation and increased incidence and severity of islet fibrosis. There was a consistent trend towards a decrease in vacuolization and islet fibrosis severity with all drug treatments, AB14 60 mg/kg, metformin, or combined metformin with AB14. These effects were more pronounced when the AB14 and metformin were combined.

As expected, pancreas insulin measured by immunohistochemistry showed a reduction in insulin labelling in ZDF rats. As observed from the insulin content measurement (FIG. 30B), drug treatments slightly prevented this insulin labeling reduction with a better effect when AB14 and metformin were combined (FIG. 31).

### Discussion

Eight week old ZDF rats that were markedly insulin-resistant and severely hyperinsulinemic but only mildly hyperglycemic were given Ab14 intravenously at doses of 0 mg/kg (vehicle), 20mg/kg, or 60 mg/kg once a week for 4 weeks. During this time-period, the vehicle-treated controls progressed to overt diabetes and were severely hypoinsulinemic and markedly hyperglycemic by day 12 of the study, consistent with complete pancreatic beta cell failure by 10 weeks of age. This was confirmed at the end of the study by direct measurement of the pancreatic insulin and proinsulin levels, both of which were substantially reduced, and through immunohistochemical assessment of pancreatic insulin labeling, which was also dramatically lowered. In addition, histological analysis conducted at the end of the study also demonstrated an increased incidence and severity of islet vacuolation, islet hyperplasia (large/giant islet(s)), and islet fibrosis in these animals, consistent with diabetic pancreatic islet pathology.

By contrast, the rise in fasting blood glucose in the vehicle-treated controls on day 12 of the study was partially prevented by both doses of Ab14, and this partial prevention was also observed on days 19 and 26 of the study. In addition, the high dose of Ab14 also partially prevented the reduction in plasma insulin and C-peptide levels observed in the vehicle-treated controls on day 12 of the study. This partial prevention was also observed but to a lesser extent on days 19 and 26 of the study, indicative of a modest delay in disease progression (pancreatic beta cell failure) and suggestive of partial pancreatic beta cell protection by the compound. Indeed, the high dose of Ab14 also completely prevented the reduction in pancreatic proinsulin levels observed in the vehicle-treated controls when pancreas tissue was obtained at the end of the study (day 28) and also partially prevented the reduction in pancreatic insulin levels when measured either directly or through immunohistochemical analyses. Furthermore, Ab14 consistently decreased the islet vacuolation, islet fibrosis, and islet hyperplasia noted in the vehicle-treated animals upon histological evaluation at the end of the study, further indicating a favorable impact on diabetic pancreatic islet pathology.

As demonstrated in Example 2, when compared to the vehicle-treated control group, Ab14 had no effect on food consumption or body weight, indicating that effects of Ab14 on the parameters evaluated above were not a result of caloric restriction or weight loss.

## Claims

1. An anti-human Calcitonin Gene Related Peptide (CGRP) antibody or antigen-binding fragment thereof for use in ameliorating or reducing the symptoms of, or treating, or preventing or controlling hyperglycemia in a subject in need thereof, wherein said anti-human CGRP antibody or antigen-binding fragment thereof comprises: (i) a variable light (V_{L}) chain comprising complementarity determining region (CDR)1, CDR2 and CDR3 polypeptides having the amino acid sequences of SEQ ID NOS: 55, 56 and 57, respectively; and (ii) a variable heavy (V_{H}) chain comprising CDR1, CDR2 and CDR3 polypeptides having the amino acid sequences of SEQ ID NOS: 58, 59 and 60, respectively.

2. The anti-human CGRP antibody or antigen-binding fragment thereof for use according to Claim 1, wherein the use is to achieve sustained normoglycemia in the subject.

3. The anti-human CGRP antibody or antigen-binding fragment thereof for use according to Claim 1 or Claim 2, which is for use in combination with an anti-diabetic agent or anti-obesity agent other than an anti-human CGRP antibody or antigen-binding fragment thereof, wherein optionally (i) said anti-diabetic agent or anti-obesity agent comprises one or more of amylin, amylin agonist, sulfonylureas, calcitonin, glucagon, PPAR-gamma agonists, GPL-1 receptor agonists, dipeptidyl peptidase IV inhibitor, amylin analogs, biguanides, dopamine D2 receptor agonists, meglitinides, alpha-glucosidase inhibitor, antidyslipidemic bile acid sequestrant, exendin, exendin analog, exendin agonist, gastrin inhibitory peptide (GIP), incretin peptide, insulin, SGLT2 inhibitor, a glucose reabsorption inhibitor, fenofibrate, fibrate, an anti-ghrelin antibody or antigen-binding fragment thereof, a fibroblast growth factor receptor (FGFR)-1(IIIb), FGFR-1(IIIc), and/or FGFR-4(IIIc) antibody or antigen-binding fragment thereof, an anti-CD38 antibody or antigen-binding fragment thereof, an anti-MIC-1 antibody or antigen-binding fragment thereof, metformin or a combination of any of the foregoing; or (ii) said anti-diabetic agent is metformin.

4. The anti-human CGRP antibody or antigen-binding fragment thereof for use according to any one of the foregoing Claims, further comprising a pharmaceutically acceptable carrier.

5. The anti-human CGRP antibody or antigen-binding fragment thereof for use according to any one of the foregoing Claims, wherein said anti-human CGRP antibody or antigen-binding fragment thereof is to be administered to said subject at a dosage between about 0.1 and 100.0 mg/kg of body weight of recipient subject.

6. The anti-human CGRP antibody or antigen-binding fragment thereof for use according to any one of Claims 1-5, wherein said anti-human CGRP antibody or antigen-binding fragment thereof is a human antibody; is non-naturally occurring; is a non-naturally occurring antibody fragment; is a humanized antibody; or is a chimeric antibody.

7. The anti-human CGRP antibody or antigen-binding fragment thereof for use according to any one of Claims 1-5, wherein said anti-human CGRP antibody or antigen-binding fragment thereof (i) has a polypeptide sequence at least 80%, at least 85%, at least 90%, at least 95%, at least 96%, at least 97%, at least 98%, or at least 99% identical to, or (ii) comprises, an antibody comprising the V_{L} of SEQ ID NO:52 and the V_{H} of SEQ ID NO: 54.

8. The anti-human CGRP antibody or antigen-binding fragment thereof for use according to any of the foregoing Claims, wherein the anti-human CGRP antibody or antigen-binding fragments thereof comprises (i) a human, chimeric or humanized antibody; (ii) a Fab, F(ab')₂, scFv, or IgNar or another monovalent antibody fragment.

9. The anti-human CGRP antibody for use according to any one of the foregoing Claims, wherein said anti-human CGRP antibody comprises an Fc region that has been modified to alter effector function, half-life, proteolysis, and/or glycosylation.

10. The anti-human CGRP antibody or antigen-binding fragment thereof for use according to any one of the foregoing Claims, wherein said anti-human CGRP antibody or antigen-binding fragment thereof is aglycosylated or if glycosylated only comprises mannose residues.

11. The anti-human CGRP antibody or antigen-binding fragment thereof for use according to any one of the foregoing Claims, wherein the anti-human CGRP antibody or antigen-binding fragment thereof comprises the V_{L} of SEQ ID NO: 52 and the V_{H} of SEQ ID NO: 54.

## Patentansprüche

1. Anti-Human - Calcitonin Gene Related Peptide (CGRP) - Antikörper oder Antigen-bindendes Fragment davon zur Verwendung beim Verbessern oder Verringern der Symptome oder Verhüten oder Beherrschen von Hyperglykämie in einer daran bedürftigen Person, wobei der Anti-Human-CGRP-Antikörper oder das Antigen-bindende Fragment davon aufweist: (i) eine variable leichte (V_{L}) Kette, die komplementaritätsbestimmende Region (CDR) 1-, CDR2- und CDR3-Polypeptide mit den Aminosäuresequenzen von SEQ ID NOS: 55, 56 bzw. 57 aufweist; und (ii) eine variable schwere (V_{H}) Kette, die CDR1-, CDR2- und CDR3-Polypeptide mit den Aminosäuresequenzen von SEQ ID NOS: 58, 59 bzw. 60 aufweist.

2. Anti-Human-CGRP-Antikörper oder Antigen-bindendes Fragment davon zur Verwendung nach Anspruch 1, wobei die Verwendung eine anhaltende Normoglykämie in der Person erreichen soll.

3. Anti-Human-CGRP-Antikörper oder Antigen-bindendes Fragment davon zur Verwendung nach Anspruch 1 oder Anspruch 2, zur Verwendung in Kombination mit einem Anti-Diabetes-Mittel oder einem Anti-Obesitas-Mittel anders als ein Anti-Human-CGRP-Antikörper oder Antigen-bindenden Fragment davon ist, wobei optional (i) das Anti-Diabetes-Mittel oder Anti-Obesitas-Mittel ein oder mehr aufweist von Amylin, Amylin-Agonisten, Sulfonylharnstoffen, Calcitonin, Glucagon, PPAR-Gamma-Agonisten, GPL-1-Rezeptor-Agonisten, Dipeptidyl-Peptidase-IV-Inhibitor, Amylin-Analoga, Biguaniden, Dopamin-D2-Rezeptor-Agonisten, Meglitiniden, Alpha-Glucosidase-Inhibitor, antidyslipidämischem Gallensäure-Sequestriermittel, Exendin, Exendin-Analogon, Exendin-Agonist, Gastrin-Hemm-Peptid (GIP), Inkretin-Peptid, Insulin, SGLT-Inhibitor, einem Glukose-Resorptionsinhibitor, Fenofibrat, Fibrat, einem Anti-Ghrelin-Antikörper oder Antigen-bindenden Fragment davon, einem Fibroblasten-Wachstumsfaktor-Rezeptor (FGFR) -1 (IIIb), FGFR-1 (IIIc) und/oder FGFR-4 (IIIc) - Antikörper oder Antigen-bindenden Fragment davon, einem Anti-CD38-Antikörper oder einem Antigen-bindenden Fragment davon, einem Anti-MIC-1-Antikörper oder einem Antigen-bindenden Fragment davon, Metformin oder einer Kombination von irgendwelchen der Vorstehenden; oder (ii) das Anti-Diabetes-Mittel Metformin ist.

4. Anti-Human-CGRP-Antikörper oder Antigen-bindendes Fragment davon zur Verwendung nach einem der vorhergehenden Ansprüche, ferner aufweisend einen pharmazeutisch annehmbaren Träger.

5. Anti-Human-CGRP-Antikörper oder Antigen-bindendes Fragment davon zur Verwendung nach einem der vorhergehenden Ansprüche, wobei der Anti-Human-CGRP-Antikörper oder das Antigen-bindende Fragment davon an die Person in einer Dosierung zwischen etwa 0,1 und 100,0 mg/kg des Körpergewichts der Empfängerperson verabreicht werden soll.

6. Anti-Human-CGRP-Antikörper oder Antigen-bindendes Fragment davon zur Verwendung nach einem der Ansprüche 1-5, wobei der Anti-Human-CGRP-Antikörper oder das Antigen-bindende Fragment davon ein humaner Antikörper ist; nicht natürlich vorkommt; ein nicht-natürlich vorkommendes Antikörperfragment ist; ein humanisierter Antikörper ist; oder ein chimärer Antikörper ist.

7. Anti-Human-CGRP-Antikörper oder Antigen-bindendes Fragment davon zur Verwendung nach einem der Ansprüche 1-5, wobei der Anti-Human-CGRP-Antikörper oder das Antigen-bindende Fragment davon (i) eine Polypeptidsequenz von mindestens 80%, mindestens 85%, mindestens 90%, mindestens 95%, mindestens 96%, mindestens 97%, mindestens 98% oder mindestens 99% identisch zu einem Antikörper mit V_{L} von SEQ ID NO: 52 und dem V_{H} von SEQ ID NO: 54 hat oder (ii) einen solchen Antikörper aufweist.

8. Anti-Human-CGRP-Antikörper oder Antigen-bindendes Fragment davon zur Verwendung nach einem der vorhergehenden Ansprüche, wobei der Anti-Human-CGRP-Antikörper oder das Antigen-bindende Fragmente davon (i) einen humanen, chimären oder humanisierten Antikörper; (ii) ein Fab, F(ab')₂, scFv oder IgNar oder ein anderes monovalentes Antikörperfragment aufweist.

9. Anti-Human-CGRP-Antikörper zur Verwendung nach einem der vorhergehenden Ansprüche, wobei der Anti-Human-CGRP-Antikörper eine Fc-Region aufweist, die modifiziert wurde, um Erfolgsfunktion, Halbwertszeit, Proteolyse und/oder Glykosylierung zu verändern.

10. Anti-Human-CGRP-Antikörper oder Antigen-bindendes Fragment davon zur Verwendung nach einem der vorhergehenden Ansprüche, wobei der Anti-Human-CGRP-Antikörper oder das Antigen-bindende Fragment davon aglycosyliert ist oder, falls glykosyliert, nur Mannosereste aufweist.

11. Anti-Human-CGRP-Antikörper oder Antigen-bindendes Fragment davon zur Verwendung nach einem der vorhergehenden Ansprüche, wobei der Anti-Human-CGRP-Antikörper oder das Antigen-bindende Fragment davon die V_{L} von SEQ ID NO: 52 und die V_{H} von SEQ ID NO: 54 aufweist.

## Revendications

1. Anticorps anti-CGRP (peptide relié au gène calcitonine) humain ou fragment de liaison à l'antigène de celui-ci destiné à être utilisé pour améliorer ou réduire les symptômes de l'hyperglycémie ou pour traiter ou empêcher ou contrôler l'hyperglycémie chez un sujet qui en a besoin, dans lequel ledit anticorps anti-CGRP humain ou fragment de liaison à l'antigène de celui-ci comprend : (i) une chaîne légère variable (V_{L}) comprenant des polypeptides de région déterminant la complémentarité (CDR)1, CDR2 et CDR3 ayant les séquences d'acides aminés SEQ ID NOS : 55, 56 et 57, respectivement ; et (ii) une chaîne lourde variable (V_{H}) comprenant des polypeptides CDR1, CDR2 et CDR3 ayant les séquences d'acides aminés SEQ ID NOS : 58, 59 et 60, respectivement.

2. Anticorps anti-CGRP humain ou fragment de liaison à l'antigène de celui-ci destiné à une utilisation selon la revendication 1, dans lequel l'utilisation a pour but d'atteindre une normoglycémie soutenue chez le sujet.

3. Anticorps anti-CGRP humain ou fragment de liaison à l'antigène de celui-ci destiné à une utilisation selon la revendication 1 ou 2, lequel est destiné à être utilisé en combinaison avec un agent antidiabétique ou agent anti-obésité autre qu'un anticorps anti-CGRP humain ou fragment de liaison à l'antigène de celui-ci, dans lequel éventuellement (i) ledit agent antidiabétique ou agent anti-obésité comprend l'un ou plusieurs parmi l'amyline, l'agoniste de l'amyline, les sulfonylurées, la calcitonine, le glucagon, les agonistes PPAR-gamma, les agonistes du récepteur GPL-1, l'inhibiteur de la dipeptidyl peptidase-IV, les analogues d'amyline, les biguanides, les agonistes des récepteurs dopaminergiques D2, les méglitinides, l'inhibiteur de l'alpha-glucosidase, l'antidyslipidémique chélateur des acides biliaires, l'exendine, l'analogue de l'exendine, l'agoniste de l'exendine, le peptide inhibiteur de la gastrine (GIP), le peptide incrétine, l'insuline, l'inhibiteur du SGLT2, un inhibiteur de la réabsorption du glucose, le fénofibrate, le fibrate, un anticorps antighréline ou fragment de liaison à l'antigène de celui-ci, un anticorps du récepteur du facteur de croissance des fibroblastes (FGFR)1-IIIb, FGFR1-IIIc et / ou FGFR4-IIIc ou fragment de liaison à l'antigène de celui-ci, un anticorps anti-CD38 ou fragment de liaison à l'antigène de celui-ci, un anticorps MIC-1 ou fragment de liaison à l'antigène de celui-ci, la metformine ou une combinaison de l'un quelconque de ces derniers ; ou (ii) ledit agent antidiabétique est la metformine.

4. Anticorps anti-CGRP humain ou fragment de liaison à l'antigène de celui-ci destiné à une utilisation selon l'une quelconque des revendications précédentes, comprenant en outre un support acceptable sur le plan pharmaceutique.

5. Anticorps anti-CGRP humain ou fragment de liaison à l'antigène de celui-ci destiné à une utilisation selon l'une quelconque des revendications précédentes, dans lequel ledit anticorps anti-CGRP humain ou fragment de liaison à l'antigène de celui-ci doit être administré audit sujet à une dose comprise entre environ 0,1 et 100,0 mg/kg de poids corporel du sujet receveur.

6. Anticorps anti-CGRP humain ou fragment de liaison à l'antigène de celui-ci destiné à une utilisation selon l'une quelconque des revendications 1 à 5, dans lequel ledit anticorps anti-CGRP humain ou fragment de liaison à l'antigène est un anticorps humain, n'est pas d'origine naturelle, est un fragment d'anticorps d'origine non naturelle, est un anticorps humanisé, ou un anticorps chimérique.

7. Anticorps anti-CGRP humain ou fragment de liaison à l'antigène de celui-ci destiné à une utilisation selon l'une quelconque des revendications 1 à 5, dans lequel ledit anticorps anti-CGRP humain ou fragment de liaison à l'antigène de celui-ci (i) a une séquence polypeptidique d'au moins 80 %, d'au moins 85 %, d'au moins 90 %, d'au moins 95 %, d'au moins 96 %, d'au moins 97 %, d'au moins 98 %, ou d'au moins 99 % identique à, ou (ii) comprend, un anticorps comprenant le V_{L} de SEQ ID NO : 52 et le V_{H} de SEQ ID NO : 54.

8. Anticorps anti-CGRP humain ou fragment de liaison à l'antigène de celui-ci destiné à une utilisation selon l'une quelconque des revendications précédentes, dans lequel l'anticorps anti-CGRP humain ou fragment de liaison à l'antigène de celui-ci comprend : (i) un anticorps humain, chimérique ou humanisé ; (ii) un fragment Fab, F(ab')₂, scFv, ou IgNar, ou un autre fragment d'anticorps monovalent.

9. Anticorps anti-CGRP humain ou fragment de liaison à l'antigène de celui-ci destiné à une utilisation selon l'une quelconque des revendications précédentes, dans lequel ledit anticorps anti-CGRP humain comprend une région F_{c} qui a été modifiée pour changer la fonction effectrice, la demi-vie, la protéolyse, et / ou la glycosylation.

10. Anticorps anti-CGRP humain ou fragment de liaison à l'antigène de celui-ci destiné à une utilisation selon l'une quelconque des revendications précédentes, dans lequel l'anticorps anti-CGRP humain ou fragment de liaison à l'antigène de celui-ci est aglycosylé ou, s'il est glycosylé, ne comprend que des résidus de mannose.

11. Anticorps anti-CGRP humain ou fragment de liaison à l'antigène de celui-ci destiné à une utilisation selon l'une quelconque des revendications précédentes, dans lequel l'anticorps anti-CGRP humain ou fragment de liaison à l'antigène de celui-ci comprend le V_{L} de SEQ ID NO : 52 et le V_{H} de SEQ ID NO : 54.
